# EUROPEAN PATENT APPLICATION

(11) **EP 1 460 085 A1**
(43) Date of publication of application: **22.09.2004**
(21) Application number: 03006341.6
(22) Date of filing: 20.03.2003
(51) Int. Cl.: C07K 14/36, C12N 15/31

(54) **Genes and proteins for the biosynthesis of the glycopeptide antibiotic teicoplanin**

(71) Applicant: Vicuron Pharmaceuticals, Inc., King of Prussia, PA 19406 (US)
(72) Inventor: Donadio, Stefano, 21046 Malnate (VA) (IT); Sosio, Margherita, 20020 Solaro (MI) (IT); Bianchi, Alessandra, 20025 Legnano (MI) (IT)
(74) Representative: Sgarbi, Renato

(57) **Abstract**

The present invention relates to the field of antibiotics, and more specifically to the isolation of nucleic acid molecules that code for the biosynthetic pathway of the glycopeptide antibiotic teicoplanin. Disclosed are the functions of the gene products involved in teicoplanin production. The present invention provides novel biosynthetic genes that code for teicoplanin production, the encoded polypeptides, the recombinant vectors comprising the nucleic acid sequences that encode said polypeptides, the host cells transformed,with said vectors and methods for producing glycopeptide antibiotics using said transformed host cells, including methods for producing teicoplanin, a precursor thereof, a derivative thereof or a modified glycopeptide different from teicoplanin or a precursor thereof.

SEQUENCE LISTING

<110> Versicor Inc.

<120> Genes and proteins for the biosynthesis of the glycopeptide antibiotic
teicoplanin

<130> BI0009

<160> 50

<170> PatentIn version 3.1

<210> 1

<211> 73882

<212> DNA

<213> Actinoplanes teichomyceticus

<220>

<221> misc_feature

<222> (652)..(1995)

<223> ORF1; positive strandedness

<220>

<221> misc_feature

<222> (2017)..(3234)

<223> ORF2; positive strandedness

<220>

<221> misc_feature

<222> (3227)..(4264)

<223> ORF3; positive strandedness

<220>

<221> misc_feature

<222> (4264)..(4872)

<223> ORF4; positive strandedness

<220>

<221> misc_feature

<222> (5634)..(6173)

<223> ORF5; negative strandedness

<220>

<221> misc_feature

<222> (6286)..(7377)

<223> ORF6; negative strandedness

<220>

<221> misc_feature

<222> (7385)..(8065)

<223> ORF7; negative strandedness

<220>

<221> misc_feature

<222> (8924)..(10105)

<223> ORF8; positive strandedness

<220>

<221> misc_feature

<222> (10597)..(16827)

<223> ORF9; positive strandedness

<220>

<221> misc_feature

<222> (16824)..(20009)

<223> ORF10; positive strandedness

<220>

<221> misc_feature

<222> (20053)..(32256)

<223> ORF11; positive strandedness

<220>

<221> misc_feature

<222> (32276)..(37873)

<223> ORF12; positive strandedness

<220>

<221> misc_feature

<222> (37886)..(38095)

<223> ORF13; positive strandedness

<220>

<221> misc_feature

<222> (38401)..(39222)

<223> ORF14; positive strandedness

<220>

<221> misc_feature

<222> (39268)..(41049)

<223> ORF15; positive strandedness

<220>

<221> misc_feature
<222> (41343)..(43289)
<223> ORF16; positive strandedness

<220>
<221> misc_feature
<222> (43373)..(43585)
<223> ORF17; positive strandedness

<220>
<221> misc_feature
<222> (43696)..(44871)
<223> ORF18; positive strandedness

<220>
<221> misc_feature
<222> (44894)..(46048)
<223> ORF19; positive strandedness

<220>
<221> misc_feature
<222> (46038)..(47234)
<223> ORF20; positive strandedness

<220>
<221> misc_feature
<222> (47484)..(49004)
<223> ORF21; positive strandedness

<220>
<221> misc_feature

<222>  (49166)..(50344)
<223>  ORF22; positive strandedness

<220>
<221>  misc_feature
<222>  (50461)..(51687)
<223>  ORF23; positive strandedness

<220>
<221>  misc_feature
<222>  (51833)..(52804)
<223>  ORF24; positive strandedness

<220>
<221>  misc_feature
<222>  (52948)..(54540)
<223>  ORF25; positive strandedness

<220>
<221>  misc_feature
<222>  (54757)..(56553)
<223>  ORF26; positive strandedness

<220>
<221>  misc_feature
<222>  (56924)..(57979)
<223>  ORF27; positive strandedness

<220>
<221>  misc_feature
<222>  (58746)..(59735)

<223>  ORF28; positive strandedness

<220>
<221>  misc_feature
<222>  (60477)..(62855)
<223>  ORF29; positive strandedness

<220>
<221>  misc_feature
<222>  (63036)..(64157)
<223>  ORF30; positive strandedness

<220>
<221>  misc_feature
<222>  (64154)..(64819)
<223>  ORF31; positive strandedness

<220>
<221>  misc_feature
<222>  (64816)..(66117)
<223>  ORF32; positive strandedness

<220>
<221>  misc_feature
<222>  (66146)..(66952)
<223>  ORF33; positive strandedness

<220>
<221>  misc_feature
<222>  (66991)..(68352)
<223>  ORF34; positive strandedness

<220>

<221>  misc_feature

<222>  (68349)..(68945)

<223>  ORF35; positive strandedness


<220>

<221>  misc_feature

<222>  (69083)..(70180)

<223>  ORF36; positive strandedness


<220>

<221>  misc_feature

<222>  (70266)..(71321)

<223>  ORF37; positive strandedness


<220>

<221>  misc_feature

<222>  (71318)..(72412)

<223>  ORF38; positive strandedness


<220>

<221>  misc_feature

<222>  (72548)..(73300)

<223>  ORF39; positive strandedness


<400>  1
   ccgtggcccg atcccgggcg cggtcgaggt ggccgccgac ccgggcggcg cggtcttcct      60
   cgaccggcgc atccggcatg cccgctcgaa caactactca cagatcgccg cggaagtgca     120
   cccaccggtg ggtgtacacc gcgacaccgc gcccgcggac ctgatggtct cgtgccggtg     180
   cagcggcggc tcctggtcct cccggacggc gccggcgacc acgcctgggg ccactgcgcg     240
   agcgaggctc cgccgcgcgg cctgccgagg gagcacgatc tgctcggccc ggcccaccgg 300
   ccgctgaagc cctaaccggc tgacccagcc tcggcgttgc tgccttcagg taacgttcct 360
   gctggtgctg accgacactc gtcgtcggct gggcaggcgc gcgccgccac ccgcgcccgc 420
   gaccggcgct gtccctcgat ctctcggacc cggcgtcccc ggtgccgtcc acatatcgtc 480
   ggcatatgca aaaattgata cgtggtggca acatcacact tctttgactg agggcgtgag 540
   acacagcgga cccgtccccg tcacccttca gctcctcagt gtcctgccgc tcacccttag 600
   cccggccggg tcgctgtcgc cgcgtttcgg cggtccggcc gccgttcggg catgatcccg 660
   ctcagcctcg aggcgatcgc cgctgttgtc ggggggacgg tcgagggaga ccacacggtg 720
   acggtcaccg cgccggccgt gctcgacggt cggcaggccg agccgggcgg cctcttcgtc 780
   gccttcgccg gcgagcacgt cgacggccac gactacgccg gccaggccgg ccaggcgggc 840
   gcggtcgccg tgctcggctc gcggcccacg gcgcttccca ccgtcgtcgt cgaagacgcc 900
   cggaccgcgc tgcaggccct cgccgccgac gtcgtggcgc ggctgcgtga acggttgacg 960
   ctcgtcgggg tgaccggctc ccagggcaag acgagcacca aggacctgct ggccgccgtg 1020
   ctgtcgagcg ccgcgccgac gatcgccacc aagggcaacc tcaacaacga gctcggcgtg 1080
   cccctcacca tgctgcgcgc cggtgcggcc acccggttcc tcgtcgtcga gatgggcgcc 1140
   cgccgcatcg gtgacctcgc caagctcacc gacctggttc cgctcgacgt cgccgtcgtc 1200
   ctcaacgtcg gcgaggccca cctcggcgtg ttcggatcgc gtgcggccat cgccgagggc 1260
   aagggggagc tggtacgagg gctggtgccc gggggtaccg cgatcctcaa cgcggacgac 1320
   cccagggtgg tcgcgatgcg ctcgctcacc gatggcccgg tgttgacgtt cgggcacgcg 1380
   gaagacgcgg atgtgcgggt gcgcgacctg accctggacc ggctcggccg accgtccttc 1440
   acgctgcgca ccgcggacgc cacggcccgg gtcaagttgc cgatcgtggg cgcccaccag 1500
   gcactcaacg ccgcagccgc ggtggcggcg gcgctttccg ccggcctgac gctcgacgcg 1560
   gccgcggcgc cactggccac cgcctcactg acgaggtggc gcttggagct acgtgacatc 1620
   gccggcggcg tgacactgct caacgactcc tacaacgcca gccccgggtc gacgcgagcc 1680
   gcgctggacg ccctggccgc gatcgagggt ggccgccgcg tcgccgtcct cggccagatg 1740
   ctggagctgg gggacgagag cgaggccgag caccgcgccg tcggggagta cgccgccgcc 1800
   cgggccgacg tggtggtcac cgtcggcgag gcggttcgtc cgcttgccgt cggcgcgggg 1860
   gagcgggcgg tggcagtggc ggacaacggc gcggccgtcg attggcttcg tggccacctc 1920
   gcccccggcg atgtggtgct cgtcaaggca tcccgtgggg cgcgcctcga tgaagtcgcc 1980
   gccgcgctgt cctagccgtg gccgccgcgg cgattcatgt ggcgccgggg cagcgcggac 2040
   gccatccgtc ccgtaacgtg gatttccact acgcgtggtg cgggcttcct tgacaatcag 2100
   ttgacatatc gcgccgcggg cccctccgac gtatcgtcgg catatccaaa accgcatacg 2160
   tgctcgcaac accaccttcc cttgactggg cgcatgggtt acagcgaacc agtacgagca 2220
   gcggcccccc gcacccgatc gctggcctcg tcgtcctccc cggtcgttcc ggcaacgggg 2280
   atcacgatct atggatgcgg cccggatgag gccgccctgt tccgtgagct ggcgcctcgc 2340
   ttcggcgtac tgccgatcat cacggaagcg gcggtatccg aggccaatgt gaatctggca 2400
   tccggaaatc gatgcatcag cgtcgggcat aagactcaga tcacgaatgg cacgcttctc 2460
   gcgctgagcc gatccggtgt gaaatacatc tctacgcgga gcataggcta caaccacatc 2520
   gatgtggcgt atgcgcagag cgtgggtatc tccgtcggca acgtcgccta ctcgccggac 2580
   agcgtggccg attacacgct gatgctgatg ctgatggccg tacgccatat gaaagccatg 2640
   atccgccgcg cggacgttca cgattatcga ttgaacgacg tgcgcgggaa ggaactgcgc 2700
   gatctgaccg tcggggtggt cgggacgggg cgtatcggtt cggccgtcat ggaccggctg 2760
   tggggtttcg gctgccgtgt gctcgcctac gacaatcgac gttcggattc cgctcattat 2820
   gttccgctcg atgatttggt gcggcagagc gacatcgtca cacttcacac cccgctcacc 2880
   gcggccacgc accatcttct caaccaggac cgtatcgcgc agatgaggaa cggtgcgttc 2940
   gtcatcaaca ccgggcgcgg ttcgcttatc gacaccgagg ccctcgtcgc agcgctggaa 3000
   agcgacaaac tgggtggcgc ggcgctcgat gtcctggaag gggaggaggg ggtattctat 3060
   gccgactgcc gtaacaagct cattgagagc aaggcgctgt tacggctaca ggaaatgccc 3120
   aatgtgatca tcagcccgca catggcctac tacacggacc atgcgctcag cgacactgtc 3180
   gagaacagtc tcgtcaactg catgaacttc gagagggaac aacagcatgg gtaagttgaa 3240
   gatcggcatc atcttcggcg gcgtctccga agagcacccc gtctcgatca agtccgcgca 3300
   ggaggtcgcc aagagcctgg acgccgagaa gtacgagccg tactggatcg gcatcacgaa 3360
   ggacggcgcc tggcggctgt gcgacggccc gggcgcggac tgggagatca gcagcgcccg 3420
   gccggccatg ctgtcgccgg accgcaacgt ccacgggctg ctggtccagg aggacggccg 3480
   gtaccgggag atccgcctgg acgtcgtgct gccggttctg cacggcaagc tgggcgagga 3540
   cggcgcgatg cagggcctgt tcgagctgtc cggcatcccc tacgtcggct gtgacgtgca 3600
   gagctccgcg gtctgcatgg acaagtcgct gacctacgcc gtggtcagca gcgcgggcat 3660
   cgcgacgccg cggttctggg tcgtcacgcc ggacgaggcg ccggaccccg cgcagctcac 3720
   ctacccggtc ttcgtgaagc cggcccgctc gggctcctcc ttcggcgtca ccaaggtgtc 3780
   gaccgagcag gagctgaagg ccgcgctgga gaccgcgcgg gagttcgact cgaaggtgct 3840
   gatcgaagag gccgtggtcg gcagcgagat cggttgcgcc atcatgggca acgacccgga 3900
   cctggtcgcc ggcgaggtgg accgggtcgc cctgtcccac ggcttcttcc ggatccacca 3960
   ggagagcgag ccggagcgcg gttccgagaa ctcgaccttc atctgtcccg ccgacatccc 4020
   ggccgaggcg cgctcgctgg tccaggagac ggcgaaggcc atctaccgtg ctctgggatg 4080
   ccgcgggatc gcccgggtgg acatgttcct caaggaggac ggaacagttg tcctcaacga 4140
   ggtcaacacc ctgcccggcc tgacctcgta cagccgttac ccgcgcatga tggcggctgc 4200
   cgggattccg ctctccgcgg tgatcgacca gatcgtgtcg ctggccctgc cgggggacat 4260
   ctgatgaaag acgacttcgc gttcgtggac gagctcgtgc caggcatacg ctgggatgcc 4320
   aagtacgcca cctgggacaa tttcaccggc aagccggtgg acggttacgt tgcgaatcgg 4380
   atcgtcggaa cgaaggcgtt gtgcgctgcc ctggcgcgcg cccaggaaga ggccgagacg 4440
   cttggcttcg gtctgcttct ctgggatggg tatcggccgc aacgcgccgt ggactgcttc 4500
   atgcgctggg cggagcagcc ggaggacggc cgcaagaagc tgcggcacta cccgaacatc 4560
   ggcaggcccg agatgttcga gaaggggtat gtcgccacca agtccggtca cagccgcggc 4620
   agcaccgtcg acttgacgct ctacaacctt gccaccggcg aactggcgtc gatgggcggc 4680
   gaccacgacc tgatggatcc gatctcccac cacggggcca gcggaatcac gccgatcgaa 4740
   gcgaagaacc gtgaatacct ctgctcgatc atggagggct gcggattcgc caggtacgac 4800
   tacgagtggt ggcactacac cctgaagaac gaaccctatc ccgacacata ttttgatttt 4860
   ccgatcgtgt agttgatgcc cgggcaggaa ggggagtcgc gccggagatc cgtcttcgtc 4920
   cggcgtccgc ggtgtgggga aagaccatcg tgacccgggg cgtgacgctc ctttccgccg 4980
   gcggcggctg ctgccggatc ttctcccgtc ccggccgctg ccgtgcacac cgacccggga 5040
   cctgctgcga acgcccggcc ggcgtgactc ggtgcgggcg ggccggggtc ctcggccgcg 5100
   gtccgcggca gccgggcgat tcctggccgg tgtcgctgtt gtgacgctgg tcatgagggt 5160
   gggcgggatc tgacggtgcc cggcaccggc ggcttgtccc tggcagcggc gcgctcgccg 5220
   gaggtgaccg gctcgacacc gcgccggtca ccggccgggt ggcagagccc gtgcgtgtgc 5280
   ggcggctacc tgaccaggag ggcaggcaac cgctgaggct caccagggga gggaccggct 5340
   cggcgatccg gatgtgtcgg acgatgctcg tgctcggccc ggccggcggg aaccctgcct 5400
   cacccggtga ggcctgcccg aactcgctgc cgctctccgc gcacacgggc atcaagaccc 5460
   ggggcctgga cggcctggaa caacgacgcg aacgcgcccg gatgcgctcc gaactccaac 5520
   accgatgggg tcagaccccg gcacgggcgg cggcccgacc aacaccgtcg aggtttccgg 5580
   tcagagcacc agacgtcagg gtgtccgctg cgcgccggcg cggagggctg cgcctacttt 5640
   cgcacgcgga agcgcagcat cgtgacggcg ccggaccggg tgttgctgag aggttccagg 5700
   tcgatcctgt cgaggcttgg gggcgagaag cggacaccgt cgccgagcag caccggtacg 5760
   acatacacca ggatctcgtc gacgagcccg cgccgcaggc actgggcggc cacgtcagca 5820
   ccgaggattt ccaagttctt gccgcccgcg gcgctgcgtg ccgtggcaac ggcttcctcg 5880
   atgtcacagc tgaggaacgt gacggcaggg tccggctcgt ggggaggctc gtgcgtgagg 5940
   acgaactgtg ctcccccgtc gtagggggtg ccctggccgg ccatacgctt ggcgacctcg 6000
   tacgtgcctc ggccgatgag catggcgccc gtggacgcca taacctccgg gaacatgtcc 6060
   ccagtcaggt attcgaagat ccagtccatc gcgtggccgg gaccggcgat gaacccgtcc 6120
   agcgacatcg ccctgttcac gaccactttg ccggcgccgc tcacatcgct catgcgtctt 6180
   ctccaaggat ttgggagccg agggccggat gacagtgcgg ttgctgggga acggatgccg 6240
   cccggcccgg gactcggaac ccgaccggga gccgcggtgg gcggttcagt ttctcggagg 6300
   cgttgtcgcg gctggcagct gcaccgtgac gtgcaggccg cctccagcgc gcggggcggt 6360
   ggtgagtgtt ccgtcgtgcg cgtgggcgat gctcttgacg atcgccaggc cgaggccgac 6420
   acctgcatga tcgtggtgta tgcgctcggt gccgcgctgg aacggttcgg tgagtgtcga 6480
   gaccagctgt gcggagagct gctcgccggt gttttccacg gtgagggtca cactcttggg 6540
   atggacgctg gtcgtgaccc acacgatgcg ctcttccgcc acgttatgca cgatcgcgtt 6600
   gtgcaagagg ttgatcgtca tttgtaacag cagggcacgt gagccgacgg tcggggtgat 6660
   gtcgccggag gcttcgatgg tgacgccgtg cttctccgcc agcggtagga gcgtctcgat 6720
   ggcttcttcg acgttcagcg acaggtcgac atactcgcgc ttgaacgatc gccggtcggc 6780
   gcggctcagc agaagcagtg cctcggtgag gtcgattgcc cgggtgttga cggtgtgaag 6840
   gcgaccgatg agctcgtggg tgcttcgtgc cgggtcggtg cgggcgacgt cgagaagcgt 6900
   tttcgtgatc gccagcggcg tacgcagctc gtgagaggcg ttggctgcga atctctggtg 6960
   ctcggcgatg tgtgcttcga gtcgcgtcag catgatgtcg aaggcgtcgg cgagctcgcg 7020
   gaactcatcc ttgcggccat ccaactcgat gcggtgcgag agcgaaccgt tcgcggccat 7080
   gcgggtggcc tctgtgatgc gggtcagagg agcgagcatc cgaccggcca gaagccaccc 7140
   gccgagcagg ccgaacacca gtaggaacag caggactccg gccgtcgccg gggcgaaggc 7200
   gtgcaagacg atggtcttga tgggcacgag accacgactg ctcatcgcgg tgtccggtag 7260
   gccgcgcagg aggtacaccc acacggtggc gagcaacgag gcgccggcga gcatgaggaa 7320
   gccggcgtag ctcaacgtga gtttgaggcg cacgctcaag ccgggggctc ggtccacggt 7380
   ctcttcacgc acgcccgctg cctggttgcg tggttatgcg atacccgacg ccgggcacgg 7440
   tggcgatgat ctggggctcg ccgagccgtt tgcgcagggc cgaaacggtg atgcggacgg 7500
   cgttggtgaa cgggtccgcg ttctcgtccc aagcccgttc cagcagttcc tcggcgctga 7560
   cgacgccgcc ttcggcggct acgaggacgg cgagcacggc gaactgcttc cgggtcagcg 7620
   cgacgtagcg gtcgtcgcgg tagacctcgc ggcggaacgg gtccagtcgc aggcccgcga 7680
   tctcccgtac aggtggcctg ctgtaggcgc gcctgcggtc gagtgccctg agcctgagca 7740
   cgagctcccg cagttcgaac ggcttggtga ggtagtcgtc ggcgccgagc tcgaacccgg 7800
   atgctttgtc gtcgagccga tcggcagcgg tgagcatgag gatgggcata ccgctccggg 7860
   aggcgacgat gcgttcggcg atctgatcac ccgaggtgcc ggggatgtcg cggtcgagga 7920
   tgacgatgtc gtaggcgttc acgctcagca tttccagagc ggtgtcaccg tcaccggcga 7980
   tgtctgccgc gatcgcttcc aaacggaggc catcccggat agcctccgcc atgtagggtt 8040
   cgtcctcgac gatcaatacg cgcatgcgct gatgttacga gccggcgcgt attgtcggca 8100
   taccgaaaat cgcatgcctt gtaacgagtc acgctgctgt gactgaggac gtgggcgaca 8160
   ctggaccagc acgggcgacc agtctaccgc atccgggccc cgcccccgcc gcgggccccg 8220
   ccggtccgcc ggccgtcatc atccgatcca ccggggcccc gagctgtccg ggtgccgccg 8280
   tcaccccggc gcgggcgccc gccgatggcg tcgcccacgc ttcctccgcc cgaacgtgct 8340
   gcggcacacc ccgatgccgg tggggcgccg ggtgagacag cggcgggccg ggatcgccgc 8400
   cacgccgacc cgtgagcagg gctccgggac gatgcggcca cggcgctggt caccacggcc 8460
   cgccgtcggc tcgtccaacc gcccgatcga gcggctggac atcaccgcac accgcgatct 8520
   caccggcgca tgtatttccg aggtggctcg gcgccgcctg gcctgcgtcc gcgaggcgac 8580
   catcccctcg ccgcggccac cgaccgtcag cggacggcca tgacgagcat tcgcctgctg 8640
   gttccccggc ggcgccggcg ataccgatca accgcgtcca ccgtcgaagg tggcggcgtt 8700
   ctgtacgcct tgcctgattg tgggtaagaa tggcggcggc cgctgaagcc cgcgacgcga 8760
   cgccgtgttt gacgaccggt cgggcgatgg aggatgccga cttcccgcgt atttcagatg 8820
   cgaattcctc gccgcagtac ccgcttcgtt caaaagttcg gcggagccgt gtcggccgat 8880
   acctagcagg gagctgaaag gctccgtgga aaaggatgtg aaaatgcgcg tgctgttttc 8940
   gtcctacggg acacgcggag atgtcgagcc cctggtggca ttggccgtgc gactgcggga 9000
   actcggtgcg gaggtccgga tgtgcgcgtc gccggactac gtggagcggc tggccgaggt 9060
   cgaagtgccg atggtgccga tcggccagcc ggtgcgcgcg ggggcccgca agggtgcagc 9120
   gcctccgggg gcgcccgagg ccgtggccga ggtgatcgcc gagcagttcg acagggtccc 9180
   gccgttcgcc gaagggtgtg acgcggtggt ggccagcggt ctggtgtcca ccgccgtcgc 9240
   cgtgcggtcg gtcgccgaga agctgggcat ccaccactac gcctacgccg tgctctcgcc 9300
   gtcctttctg cgcgcgcccg gcgtgaccga cagccgggac ctgcgggccc gggccaacca 9360
   gggcgcctat cgacagttcg gtggcccgct caacagccat cgggccgcgg tcggcctgcc 9420
   gccggtggag aaggtcatcg actacgcctt caccgagcgc ccctggttgg cggcggaccc 9480
   ggtcctggac ccgctgcggc cgacggaccc ggacgtggtg cagaccggcg cgtggatcct 9540
   gcccgaccag cggccgttgt cggcggagtt ggaggggttc ttgagggctg gctcgcctcc 9600
   ggtgtacgtg ggtttcggca gtgggccggc gcctgccgag gccgcgcggg tggccatcga 9660
   ggcggttcgg gctcagggcc gccgggtggt gctgtccagt ggttgggccg ggttgggccg 9720
   gatcgacgag ggggatgact gtctggtggt gggcgaggtg aaccatcagg tgctgttcgg 9780
   ccgggtggcc gccgtcgtcc accacggcgg ggcgggcacg acgaccgcgg tgacccgggc 9840
   gggcgctccg caggtcgtgg tgccccagaa ggcggatcag ccctactatg ccggtcgggt 9900
   ggctgacctg ggtgtcggcg tggcgcatga cggtccgacc ccgaccgtcg agtccctgtc 9960
   cgcggcgctg gccacggcgt tgaccccggg gatccgggcg cgagcggcgg cggtggccgg 10020
   cacgatccgc accgacggga cgacggtggc cgcgaaactg ctcctcgagg cgatcagccg 10080
   gcagagatcg tcggttcccg cgtgaatcgc gccgggcccg ccgagccggc gtgtcgcttg 10140
   ccgggcagat ctcgccagga ccggtcaccg cataggcgct gacggatgcg tccgtccaag 10200
   tgtcgacaac gttcgcttgg acgtcctcgt ggacatcggc tctgttcagc atgattggga 10260
   ccgccgacct catgtgtcgg gtgagagggg atgccccgtg gtcgtgatgc cgtccgtccg 10320
   gagggtgaca agggtcggcg tcgaccccga ttccgggtcg ttcagcatcc atcccgggac 10380
   gtcacgcgga gtgtttttct ttctccgccg gtacatcgga aaggtggcgc tgtttccttt 10440
   tgtcgccttc ccgatattgc tgatcgcggt cggagctgaa ttggtgccga aacaggtcat 10500
   cgacggcggc gtccgcagga ccggtcgcgg cggtgacggg accgctccgg gtccggaccg 10560
   ccggccggtg cgccgggtgg ctcttgaagg gggacgatga attccgcagc gcaggccaca 10620
   tcgacggtgc cggagctgct cgcccggcag gtgacccggg cccccgatgc ggtggccgtg 10680
   gtggaccggg accgggttct gacgtaccgg gaactcgatg agctcgcggg ccggttgtcc 10740
   ggacgtctga tcggccgggg cgtccgccgc ggggaccgcg tggcggtcct gctggaccgt 10800
   tcggcggacc tggtggtgac gctgctcgcg atctggaagg ccggggcggc gtatgtgccg 10860
   gtcgatgccg gctatcccgc gccgcgtgtg gcgttcatgg tggcggactc gggagcctcc 10920
   cgcatggtgt gctcggccgc gacgcgtgac ggcgtaccgg aggggatcga ggcgatcgtc 10980
   gtcacggatg aggaggcgtt cgaggcctcg gcggccgggg cgcgaccggg agatctggcg 11040
   tacgtgatgt acacctccgg ctcgaccggc atcccgaagg gcgtggcggt tccgcatcgc 11100
   agcgtcgcgg agctggccgg gaatcccggc tgggcggtgg agccgggcga cgcggtcctg 11160
   atgcacgcgc cgtacgcctt cgacgcgtcg ctgttcgaga tctgggtgcc gctggtttcc 11220
   gggggccggg tggtgatcgc cgagccgggg ccggtggacg cccggcgcct gcgggaggcg 11280
   atcagctccg gggtgaccag ggcgcatctg accgccggca gcttccgcgc ggtggcggag 11340
   gagtcgccgg agtccttcgc cgggctgcgc gaggtgctga ccggcggtga cgtggtgccg 11400
   gcacacgccg tggcgcgggt ccgctcggcc tgtccccggg tgcggatccg gcacctgtac 11460
   ggcccgacgg agacgacgct gtgcgccaca tggcatcttc tggagccggg ggacgagatc 11520
   ggcccggtgt tgccgatcgg ccgtccgctc ccgggccggc gcgctcaggt gctcgacgcg 11580
   tcgctgcggg ccgtggcgcc gggcgtgatc ggtgacctgt acctgtccgg cgccggtctg 11640
   gctgacggct acctgcgccg ggcagggctg acagcggagc gattcgtggc cgacccgtcc 11700
   gcgcccgggg cgaggatgta ccgcaccggc gacctcgcgc agtggaccgc cgacggtgcg 11760
   ttgctgttcg cgggccgggc cgacgaccag gtgaaggttc gcggcttccg gatcgagccg 11820
   gccgaggtcg aggccgcgtt gaccgcgcag ccgggcgtcc acgaggccgt ggtccgagcg 11880
   gtcgacgggc gcctggtcgg ctatgtggtg gcggaggggg acgcggaacc ggctgtcctg 11940
   cgcgagcgtg tcggtgcggt gctgccggag tacatggtcc cggccgcggt gatcacactg 12000
   gacgcgctgc cgctgaccgg caacggcaag gtggaccggg cggctctgcc ggctccggtc 12060
   ttcgcggcgg acgctccggg gcgcgaaccc ggcaccgagg cggagcgcgt gctgtgcggg 12120
   ctgctgtccg aggtgctcgg cctgaaccgg gtcggagtcg acgagagctt cttcgagctg 12180
   ggcggagact ccatcgcggc gatccggctg gcggcgcgtg cgtcccgggc gggcctgctc 12240
   gtgacgcccg cccagatctt caaggagagg actgtcgcac ggctggcggc cgtggcgggc 12300
   gctgtaccgg ccgggccacc cgtcgaccgc cccctcatcg cgctgaccgc ggaggaggag 12360
   gtcgagctgg cggccgccgt cccgggcgcg gtggaggtct ggccgctggc accgctgcag 12420
   gaggggttgc tcttccagtc gaccatcgac accgagggcc tcgacatcta ccaggcgcag 12480
   tggattctcc agctgaacgc gccgctggac gtgcgccggc tacgggtcgc gtgggaagcg 12540
   gtcttcgcgc ggcacgccga gcttcgagtg agcttcgtcc ggctggcgtc cgggaagacg 12600
   gtgcaggccg ccgccgggca cgtcgtcctg ccgtggcggg aggtggatct gaccgatgcg 12660
   ggcgatgtcg acgcggccct gcaggagctg gccgggcggg agcaggaaca gcgcttcgac 12720
   cttgcccgcg cgccgttgtt ccggttggtg ctggtccggt acggcgagga ccggcaccgg 12780
   ctgctggtcg tccatcacca catcctgacc gacggttggt cggtggcggt catcctcaac 12840
   gaggtggccg aggcctatgc ggccggtggg cggctgcccg accggacggg tccggtgtcc 12900
   tatcggaact acctggcctg gctggaccgg caggacaggg aggcggcgcg cgcggcctgg 12960
   cgggccgagc tgtccggcct cgacgagccc gtgccgatcg ccagggcggc cgccgggacc 13020
   gggtacgaat accgtgtcgc gttcctgccg gcggatgtct gcacgaggct gacggcgtcg 13080
   gtccgtgacc acgggctgac gctgaacacg gtggtgcagg gcgcgtgggc gatggtgctg 13140
   tcgcgacttg tccggcgtac cgatgtggta ttcggcacca cggtggccgt ccgtcccgcg 13200
   gagctcccgg gcgtggagtc gataccgggc ctgatgatga acacggtgcc ggtccgggtg 13260
   acgctggacg gtgggcaatc gctcctcgac ctgctcaccg gcctgcagcg gcggcaggcg 13320
   gccctgatgc cgcaccagca tctggggctg ccggagatcc agaggacggc cgggccgggc 13380
   gcgacgttcg acacgctgct ggtgttcgag aactacccgc gggatttcgc cggccagttc 13440
   acctacctgg gcacgatcga ggggacgcac tatccgctca ccctcggcat catcccggga 13500
   gagcgtctca ggatccagct cgcctaccgg cacgggcaga tcgaggagac cctcgccgcg 13560
   tcggtcctgg acgggttcgt cgcagctctc cgcgcggtgg ccgccgaccc gtccgggttg 13620
   gtgggccgga ccggtgtggg ccgcggcccg gtgcacggct tagccccggc actggtggcg 13680
   gcggagtcgc tgcccgtgct ggtggggcgg gtggtggagc agcggtcgca cgaggtggcc 13740
   gtggtggacg gcgacggcga actgtccttc gggcagttgt gggaccaggc ggtgggcttg 13800
   gcggacctgc tgacggcacg tggagtggga cccgagtgcc gggtgggtgt gctggtgggg 13860
   cggtcggcgt ggtgggtggt cgggatgctg ggcgtctcgc tggcgggtgg cgccttcgtc 13920
   ccggtggatc cggcgtaccc ggccgaacgc atccggctga tcctggccga cgccgatccc 13980
   gcgctcgtgg tgtgtgcggg gaagacccgc gaggcggtgc ccgcggcgtt cgcggatcgg 14040
   ctgctggtgg tcgacgagat ggatctgacc gggggatcgg cggctcgatt gccgcgggta 14100
   cgtccgggtg acgcggcata tgtgatctat acgtcggggt cgacgggcag gccgaagggg 14160
   gtcgtcgtcc cgcacgcggg actggggaac ctggcgctgg cgcagatcga ccggttcggt 14220
   gtgtcgccgt cgtcccgggt cctgcagttc gcggcgctgg gcttcgacgc gatggtgtcg 14280
   gaagtgttga tggcgttgct gtccggggcg cggctggtga tggcgccgga acaccagctg 14340
   ccgccgcgcg tgtcgctggc cgaggcgctg cagcggtggg atgtcacgca cgtgaccgtt 14400
   ccgccgtcgg tgctggccac ggcggaggcg ttgccggccc ggctggagac cgtggtggtg 14460
   gccggggagg catgtccgcc aagcctggcg gaccgctggt cggcgggtct gcggctggtc 14520
   aatgcgtacg ggcccaccga ggccacggtg tgcgcggcga tgagcatgcc gctggtggcg 14580
   agccggcccg tggtcccgat cgggacgccg atcgccgggg gacgctgcta tgtgctggac 14640
   gcgttcctgc ggccgctgcc gccgggcctc accggtgagc tgtacgtggc cgggatcggg 14700
   ctggctcgcg ggtatctggg acgggccgcg ttgacggccg agcggttcgt cgccgacccg 14760
   ttcgtcccgg gtgagcggat gtaccggacc ggagatctgg cgtaccggac cggcgagggt 14820
   gagctggtgt tcgccgggcg tgccgacgac caggtgaagg ttcgtggatt ccggatcgag 14880
   cccggtgagg tcgagtcggc cctgtccggt caccccgggg ttgcccaagc cgcggtgatc 14940
   gtgcgcggtg accgcctact ggcctatgtg tcgccggctg gcgtcgatcc gcaggcggtg 15000
   cgcgaagacc tcgcatcgcg gttgccgccg tacatggtgc ccgcggtggt ggtgccgctg 15060
   gaggcgttgc ccaccacccc gaacgggaag atcgaccggg gcgcgctgcc tgatcccgac 15120
   ttcgcggccg gggcagatgt ccgggagccg cgcaccgagg tcgagcgggc gttgtgcgat 15180
   ctcttcgccg aggtgctcgg tctggaccgg gtcggcgtca ccgacagctt cttcgaggtg 15240
   ggcggcgact ccatcacctc gatgcagttg gtggcacggg cccggcgtgc gggtctgacg 15300
   ttcgccgcgc acgacgtgtt cgaggagcgg accccggagc ggctcgcgca ggtggcggcg 15360
   caggcgccgc ggccggaacg cggccacatg atcggcgacg gtgtgggcga ggtggcgcgg 15420
   acgccggtca tgcggttgct gggcgatgcg gtggtggccg cgggattcgc gcagtgggtc 15480
   gtggtcgccg tgcccgcagg gctcggggag cgcgcgttgg tggcggcgtt gacggcggtg 15540
   gtggacgcgc acgacatgct gcgggtgcgg gtggactcgg ggcggttggt ggtgcgcgcc 15600
   cgtgggtcgg tcgatgtggc cggtgcggtg gagcgggtcg aggccgacac cggcgagatc 15660
   gacgaggtgg cgcgtcgctg tgcggtggcg gtggccgcac ggctggaccc cgctgccggt 15720
   gtggtggtgc gggcggcgtg ggtggacgcc ggcccggatc gctccggccg cttggtgctg 15780
   gccgcgcatc acctggccgt cgacggagtg tcgtggcgtg ttctggtacc ggacctgcag 15840
   gcggcgtgtg aggcggtggc cgccgggcgg cagccggccc tggaaccggt gggagtgtcg 15900
   ttccggcggt gggcggacct gttggggcag tggcggtttt cgccggagcg ggtgggggag 15960
   ttggcgcgat ggcgggcggt ggtgggttcc ggggaccggc ggatcggtga gcccggcccg 16020
   tccgccgggg ccgtgcgttc ccgctcgtgg gtggtgtcgg ggtcacgggc tcaggtgctg 16080
   gtgggacgcg cgccggtggt gttcttctgc ggggtccatg aggtcgtgct ggccggactg 16140
   gcgggcgcgg tggcacggtg gcacggcggg gatgtggtcc tggtcgatgt cgagggtcac 16200
   ggccgtcacc cggtgggcgg actggacctg tcccggacgg tgggttggtt caccagcgtc 16260
   cacccggtgc ggctggatgt cgccggcatc gactgggcgg gcgtgctggc cggtggaccg 16320
   gcggccgggg agctgttgaa aagggtcaag gagcagtcgc gggcggtgcc cggcgacggg 16380
   ctcggttacg ggttgctgcg ttttctcaac gacgagacgg gcccggtact ggcggcgttg 16440
   ccatcgccgc agatcggatt caacttcatg ggacggcatg tggccggtgt gcgggagtgg 16500
   cagccggtcg gggatgtggg cggcgcgctg ggcccgggcg tcgggttgcc gcatgtcctc 16560
   gacgccgacg cggtggtcga ggacggcccc cggggcccgc tgctgaggtt ggtgctgagc 16620
   tggccggacg gtgtgctgcg ggaggacgag gtcgagcggc tgggccgcgg ttggtcggac 16680
   atgttgtccg gactggccgg ccaggcagat gatccgtcgg cggggggaca caccgcgtcc 16740
   gatttcgacc tcctcgacct ggaccaggac gagatcgaga acttcgaagc aatagcggcc 16800
   gaattcggtg gaggtcagga atcgtgacaa ctccgactac gtccccagga tcggcgctcg 16860
   cggaggtctg gccgctgtca ccgatgcagg aggggatgct ctaccatgcc tcgttcgacg 16920
   acgcggcccc cgacatctac gtcatccagc aatcgcagat catcgacggc cctctggaca 16980
   ccgagcgatt ccggaggtcc tgggaggtcc ttctccaccg gcacgcggcg ctgcgagcga 17040
   gtttccaccg ccggaagtcc ggtgagaccg tccagctgat cccccgtgag gtccggcttc 17100
   cctgggccga acgtgacctg tccggcctgc ccgagaaggc ggcgctggcc gaggtgggcg 17160
   agatcgcggc gaaggagcgc gccgaaaggt tcgacctcac gaagcctccc ctgctgcggt 17220
   tgatgctgat ccgcctcggc ccgcagcggc actgtctggt gaccacgagc catcacctgc 17280
   tcatggacgg gtggtcgcgg gccatcctgg aatcggagct gctccatgtc tacgcgtccg 17340
   gtggcaccgt ctcggggctg ccgcccgccg gctcgtaccg tcactatctg tcatggttgg 17400
   gtcggcagga ccgggaggcc gcccgatcgg catggcgcgc ggagctgtcc ggcgccgacg 17460
   gatccgcgct cggaatcgcg gagcggccag ggaaggctcc ggagctgccg gcacgcgagg 17520
   tgctccgcca ttcaccggag ttcacgtccg ctctcgtgga cttcgcccgc cgccacgggt 17580
   tgacgctcaa cacgctggtg cagggcgcct gggcggtggt gctggcccga ttggcgcgcc 17640
   gcacggacgt ggtgttcggc gcgacggtgt cgggacgccc cgcggagctg cccggcgtgg 17700
   aacagcttgt gggcctgttc atcaacaccg tgccggtgcg agtccggctg gacgccgggc 17760
   agccggtcct ggagatgttg acggagctgc agaaacggca gtccgcgctg atctcgcatc 17820
   agcatctcgg attgccggag atccagaagc tcggcggggc gagtttcgac acgatcgtcg 17880
   tcttcgaaaa ctatgtcgat ccggccgcgg cgccggattc tccgcgcggc ctgcgcctga 17940
   ccctgcgaga gttccaccag gcgtcgccgt atgcgatcac gctgggagtg atgccgggcg 18000
   agagcctcga gaccgacgtg cagtatcccc cggagacgct cgatgcccgg atcgccgagg 18060
   aagccctgta cgggctggcc cgcgtcctgg aacagatcat cgccgagccg ggagcggcgg 18120
   tgggccgcct cgacgtggtg agtgcggccg gccggcagct gaccgtcgag cggtggaacg 18180
   agaccggcga cgcggtggac gcgccgtccc cggtcgatct cttccgccgg caggtgcggc 18240
   gggcacccga cgcgacggcc gtgacggccg gggaccgggc ctggtcgttc aaggagctcg 18300
   acgaatggtc cggccggctg gcgcgggccc tgacggatcg gggtgtccgg cgcggcgacc 18360
   gggtcggggt gatgctggag cggtcggccg aggtgctggc ggcatggctc ggggtgtgga 18420
   aggcgggagc cgcgttcgtg ccggtcgatc cgggctaccc ggcggaccgg gtggagttca 18480
   tgctggccga ctccgcggtc gcggcggtgc tgtgccggga gtcgaccgcg ggtgcggtgc 18540
   cggcgggtca cgagctgatc gacatggacc gggtcggcga gggcgtggcc gcgccggtcg 18600
   cgatcggagc cgacgatctg gcgtacgtca tgtacacgtc cggatccacc ggcaagccca 18660
   agggcgtggc cgtcgcgcac gggggtgtgg cggcattggc cggtgatccg gtctgggagg 18720
   tgggggccgg cgacgcggtg ctgatgcacg ctccgcacac cttcgacgcg tcgctctacg 18780
   acgtgtgggt gccgctggtc tccggcgcgc gggtgatgct cgccgagccg ggcgtggtcg 18840
   acgcgcagcg gctcgccgca cacgtggcgg atggtctcac cgcggtgaac ttcaccgccg 18900
   gtcagttccg ggcgctggca caggagtcgc cggaatcctt cgccggactg cgcgacgtgc 18960
   tcgcgggtgg agacgtggtg ccgctcggcg cggtggagcg ggtccggcag gcgtgccccg 19020
   ggctacgggt ctggcacacg tacggcccga cggagaccac gctgtgcgcg acgtggaaga 19080
   agatcgagcc gggtgaccgg ctgggctcga cgctgccgat cggccggccg ctgccgggcc 19140
   gtcggctgta cgtgctggac gtcttcctgc gtccgctgcc gccgggcgtc gcgggtgatc 19200
   tctacatcgc gggcgccggc gtggcccagg gctacctcgg gcgtccggcg ctgaccgccg 19260
   agcggttcgt cgccgacccg ttcgccgcgg gcgagcggat gtaccggacc ggggacgtgg 19320
   cgtactggac cggtgacggc gagctggtgt tcgccgggcg cgccgacaac caggtcaaga 19380
   tccgtggcta tcgggtggaa ccgggcgaga tcgagacggt gctggccgag cagccgggcg 19440
   tcgaccaggc ggtggtgctg gcccgcgacg gccgtttgat cggctacgtg gtgcccggcg 19500
   gcgaggtgga tccggtacgg ctgcgcgagc gggtcgcccg ggtgttgccg gagtacatgg 19560
   tcccggcggc ggtgatggtc ctggacgccc tgccggtgac tccgaacggg aagctggacc 19620
   gggaggctct gccggatccg gacttcagcc gacgcgtctc cggccgggag ccgcgtaccg 19680
   ggtcgagcgg ggcgctgtgc ggtctcttcg ccgaggtgct gggtctggaa cgggtcgggg 19740
   tcgacgacag cttcttcgaa ctcggcggag attcgatcac ctcgatgcag ctcgccgcac 19800
   gcgcccgccg cgacgggatg gccttcggcg cgcgagaggt gttccagcac cggacgccgg 19860
   ccgggatcgc ggcgatcgtc gattccggga acgagccggc ggccggtggc gacggcgagg 19920
   cgacgtggac gccggtgcgg cggttcggcc tcgtcgacct ggaccagcag gaggtcgaag 19980
   aactcgagtc cgagttcggc gaccgttgag cccctctctc ttaagccttt ctctgaaaaa 20040
   gaggagatat ccgtgaccca ggcgcgcatc gaggacatct ggccgctgtc gccgctgcag 20100
   gagggactgc tcttccacac cgtctacgac gacgaggggc cggacgtcta cgtgggccac 20160
   tggatcctcg atctcgacgg cccggtggac gcggcccggc tgcgcgcggc gtgggaggcg 20220
   ctgctggccc ggcacgccgc gctgcgggcc tgcttccggc agcgccggac cggcgagacc 20280
   gtgcaggtcg tcgccggccg ggtggagctg ccgtggcggg tggtcgacct ggcccacctc 20340
   gacgatcccg agcacgccgt gcgggaactg gccgacgagg accggctgag gcccttcgac 20400
   gtcgcgaagc cgccgctgct gcggctgacc ctgatccggc tcgccgacga ccggcaccgc 20460
   ctggtggtga cctgccatca cgcggtcatg gacggctggt cgttgccgat cgtcttcgac 20520
   gagctgaccg cgttgtacgc ggccggcgac ggcccggcac agctgccggc ggtgacgccc 20580
   taccgggagt acctcgcctg gctgagccgg caggacaagc cgagcgcgct ggcggcgtgg 20640
   gccgcggagc tgcgtggcgc gcaggagccg acgttggtgg cccccgccga tccgggacgg 20700
   gcgccgggca tgccggagag cgtcgaggtg cagctgtcgc cggagctcac ccgctcgctg 20760
   gcggagctgg cccgtggccg ggggctgacg ctgaacacgg tgatccaggg cgcctgggcg 20820
   ctggtgctgg cgcggctgac cggccggacg gacgtggtgt tcggggcgac ggtgtcggcg 20880
   cgtccggcgc acctgcccgg cgtggaggca atggtcggcc tgttcctcaa caccgtgccg 20940
   gtgcgggtac ggctgcgggg ttcggcgccg gtcgtcgaga tgctggcgga gctgcagaag 21000
   cggcagtccg ctctcatccc cgaacagttc gtcggactgg cggacatcca gaaggcggcc 21060
   gggcccggcg cggtcttcga caccctgctg gtcttcgaga acttcccccg cgagctcgac 21120
   gattcgcgct cggccgacgc gttcggcatc cgggtgcacc agggccgcga agccgcgcac 21180
   tatccactga cgctggtcgc ggtccccggc gagtcgatgc tgttcaagct cgactacctg 21240
   acggagctgt tcgacgcggc caccgcggcg tccatcctgg aacggttcac ccgggtcctg 21300
   cgccggctga cggacgcggg cgagctcacg gtggccgcga tcgacgtgac cagcgcggcg 21360
   gagcgcgacc gggtggcgcg atggggcgcg gccgtcggtg cgcggccgga ccggctggcg 21420
   ctggacctgt tcgcacgcca ggtcgcgcag cgccccgacg aggtggcggt cgccgatggg 21480
   gaccgggtga tgtcgttcgg ggagctggcc gagcgcgcgg aacggctcgc cggtcacctc 21540
   agcgcgcgcg gggtccggcg cggggaccgg gtggccgtgg tgatggagcg gtccggcgag 21600
   ctgatcgcga cgttgctcgc ggtgtggcgg gccggggcgg cgttcgtacc ggtggatccg 21660
   gcctatcccg cggagcgggt gaagttcctg ctgaccgacg cggagccggt ggcggcggtg 21720
   tgcacggccg cgttccgggc cgccgtgctg gacggcgggc tcgaggcgat cgtggtggac 21780
   gatccgggga cgtggccggc ggtggcgccg tgcccgccgg tccccaccgg acccgacgac 21840
   ctcgcctacg tgatgtacac ctcgggctcg accgggacgc ccaagggcgt cgcggtgtcg 21900
   cacggcgacg tcgcggcgct ggtcggcgac ccgggatggc ggaccggtcc cggcgacacc 21960
   gtgctgatgc acgcctcgca cgcgttcgac atctcgctgt tcgagatctg ggtgccgctg 22020
   ctgtccggcg cccgggtgat gatcgccgga ccgggcgcgg tggacggcgc ggcgctggcc 22080
   gctcaggtgg ccgccggggt gacggccgcg cacctgaccg cgggcgcctt ccgggtgctg 22140
   gccgaggagt cgccggagtc ggtcgccggg ctgcgcgagg tgctgaccgg cggtgacgcg 22200
   gtgccgctgg cggcggtcga acgggtgcgg cgggcgtgtc cggacgtccg ggtgcggcac 22260
   ctgtacggcc cgaccgagac caccctgtgc gccacctggt ggctgctcga accgggcgac 22320
   gagaccggcc cggtgctgcc gatcggtcgc ccgctcgcgg gccggcgcgt ctacgtcctc 22380
   gacgcgttcc tgcggcccct gccgcccggg acgaccggcg agctgtacgt cgccggggcc 22440
   ggtgtggcgc agggctacct gggccgcccg gcgctgaccg ccgagcggtt cgtcgccgac 22500
   cccttcgctc ccggtggccg gatgtaccgc accggcgacc tggcgtactg gaccgagcag 22560
   ggcacgctgg cgttcgccgg acgggccgac gaccaggtca agatccgtgg ctaccgggtg 22620
   gagcccggcg aggtcgaagc ggtcctgggc ggcctgccgg gtgtcgccca ggccgtggtg 22680
   tgcgtccgcg gcgagcacct gatcggttat gtggtggccg aggccggccg ggacctcgac 22740
   ccggaacgcc tgcgcgccag gctcgccgcg accctgcccg agttcatggt cccggccgcg 22800
   gtgctggtgc tggcggacct gccgctgacg gtcaacggca aggtggaccg gccggccctg 22860
   cccgagccgg acttcgccgc caagtcgacc ggccgggccc cggccaccgc ggccgagcgg 22920
   atcctctgcg acgtgttcgc cggcgtcctg ggcctggacc gggtcggcgt cgaggacagc 22980
   ttcttcgcgc tgggcggcga ctcgatctcg tcgatgcagg tggccgcccg cgcccggcgc 23040
   gagggcatcg ccctgacccc gcggcaggtg ttcgagcacc ggaccccgga acggctggcc 23100
   gcgctggcgc cggcggccgg atcggcacga ccggaccggt ccgcggcgga tgccggcctc 23160
   ggcgagatcc cgtggacgcc ggtgatgcga gccctcggcg acgacgcggt gcgccccggc 23220
   ttcgcgcagg cccgagtcgt cgtcgcgccg gccggcctcg acccggacgc gctcaccggc 23280
   gccctgcggg cggtgctgga cacccatgac gtgctgcgcg cgcgggtgga gccggaccgg 23340
   cggctgatcg tgcccgagcg cggcgcggtg gccgcggcgg acctgctgac gcgggtggcg 23400
   gtcgacagtg acggcatcga cgcccgcgcc gagcgcgagg cggcgacggc ggcgggcacg 23460
   ctggatccgt cggccgggat catgctgcgg gcggtgtggc tcgacgccgg cgacgccgag 23520
   ccgggccggc tggcgctcgt ggcgcatcac ctggtggtcg acgcggtgtc gtggagcatc 23580
   ctgctgccgg acctgcaagc ggcgtaccag gaggtgctcg ccggcgcgac cccggctctg 23640
   gaacccgcgg cgacgtcgta ccggcagtgg gcgcggcggc tgaccgagca ggcgtccagc 23700
   ccgagcaccc tggccgagct cgaccactgg gtcaccgtgc tggacgcggc ggaaccaccg 23760
   ctggcggagc accacgggca ggcgcactcg tggtcggcga ccctgtccgg cgccgtggcg 23820
   ggccacctgg tcagccggat gccgggcgcg ttccactgcg ggatccagga ggtgctgctc 23880
   gccggcctgg ccgcggcggt ggcccgctgg cgcggcgacg acgccggggt gctggtggac 23940
   gtggagggcc acggccggca cgccgccgac ggcgaggacc tgttgcgtac ggtggggtgg 24000
   ttcaccagcg tgcacccggt ccgcctggac gtctccggcg tcgacctggc ggccgcggcg 24060
   gccggcgacg cggccgcggg tgagctgctg aaatcggtga aggaccaggt ccgcgcggcg 24120
   cccggcgacg ggttcgggtt cgggctgctg cgccacctca acccggacac cgccgagcgg 24180
   ctcgccgcgt tgcccgcccc gcagatcggg ttcaactacc tcggccggtc cggggtggcc 24240
   gccgaggccg tcccgtggca ggtgcgcggc ggatcgctcg gcgccgggga agccggaccg 24300
   gacctggtcc tggcacaccc gctggaggcc ggcgcggacg tgcgggacac cccggacggc 24360
   ccgctgctgc ggctcaccct ggacggccgg gatctcgccc cggtcacggt ggagctgctc 24420
   ggcgaggcct ggctggagtt gctgaccggt ctggccaccc acgccggcga cccgcgggcc 24480
   ggcgggcaca ccccggccga cttcgacctg gtcgaggtga cgcagcggga cgtgacggcg 24540
   ctggaggccg cggcgccggg cctgaccgac atctggccgc tgtcgccgct gcagcggggc 24600
   atgctcttcg agcgggcgct ggacgaggac ggcgtcgacg tctaccagag ccagcgcatc 24660
   ctggacctcg acgggccgct cgacgcggac cggctgcgcg cggcctggcg gacgctggtc 24720
   gcccggcacg agtcgctgcg caccagcttc catcagctgg agtccggcga gacggtgcag 24780
   gtcgtcgtgg accaggcgga catcggctgg cgggtggccg acgtgtcaca ccgggctgag 24840
   gcggacgcgg ccgcggaggt cggacgactg ctcgccgagg accaggcgca gcggttcgac 24900
   gtcacccggg cgccgctgct gcggctgttg ctggtccggc tcggcgcgga ccggcaccgg 24960
   ctcgtggtga cgtcgcacca catcgtcctg gacggctggt cgacgccgat catcctgggc 25020
   gagatgtcgg cggcgtacgc gggtgcgccg agcacggcga cggcgccctc gccgcgggac 25080
   tacctggcgt ggctgaaccg ccaggacgac gcggcgaccc gcgcggcgtg gcgctccgag 25140
   ctcgccggct gcgacgaacc gaccctggtg gacgccgacg cggacaagac catggtgatg 25200
   ccgggggagc actgcgaagg actgtcggag caggccaccc gccgtctcac cgagttcgca 25260
   cggggtcatg cggtgacgct gagcacgatc ctgcagggcg cctgggcgct ggtgctggcg 25320
   cggctggcgg gccggaccga cgtggtgttc gggacggtgg tgtccggacg ccccgccgag 25380
   gtgccggacg tcgaccggat ggtggggatg ttcatcaaca ccgtcccggt ccgggtgcgc 25440
   ctcgacggcg gccggccggt gctggacctg ctgcacgacc tgcaggcacg ccagtccgcg 25500
   ctgaccgagc accagcacct ggggctgccg gacatccagc gggcggcggg tcccggcgcg 25560
   ctgttcgaca ccatcatgat ggtcgagaac tacccgatgg acgccgccgg cctgggcggc 25620
   gagggtggtg tcaggatcgg ctcggtcctc acccggaccg gcaccagcta tccgctgacg 25680
   atgagcgcga gccccggcga gcgcctgcag atccgggtgt cctaccggcc cgaccgcatc 25740
   gagcgggaga ccgccgccga ggcggccggg caggtcgtac gggtcatcga acgcatcgtg 25800
   gcggagccgt cgctcgcggt gggccgcctg ggtgtgaccg gcgaatcggt gcgcgagcgc 25860
   gtggtggagc gctggaacgc gaccggcgtg gcggccggcg ggtcgtcggt gctgaccggg 25920
   ttccggcggc gtgcgcgcag ctcaccggac gcggtggcgg tcagcggcgg cgggcggacc 25980
   ctgtcctacg ccgccctgga ccgggaatcc gaccggctcg cggggcatct ggccggccgg 26040
   ggtgtgcggc gtggcgaccg cgtcggcgtg gtgatggaac gcggcaccga tctgttcgtc 26100
   gccctcctcg cgatcggcaa ggcgggcgcg gcgtacgtcc cggtgaacct ggactacccc 26160
   cgggaccgga tcgagcggat gctgaccgac gccggcgtat cggtggcggt ctgcgtgcag 26220
   gcgaccagcg gcgcggtgcc ggacggcctc gcgccggtgg tcatggactc accggcgatc 26280
   gccgcggcgc cgtccgaggc cccgccgatc acggtgggcg cgcacgacct ggcgtacgtg 26340
   atgtacacct ccgggtccac cggggtgccg aaaggcgtcg cggtgccgca cggcagcgtg 26400
   gcggcgctgg ccggtgaccc gggctggtcg gtcggccccg gcgacggcgt gctgatgcac 26460
   gccccgcacg cgttcgacgc gtcgttgctg gagatctggg tgccgctgct gagcggggcc 26520
   cacgtggtgg tcgcggaccc gggcgcggtg gacgcgcagc ggctgcgcga ggcgatcgac 26580
   cgcggggtga ccaccgtcca tctgacggcc gggtcgttcc gggtgctggc ggaggagtcg 26640
   ccggacgcct tccgcggact gcgggaggtt ctgaccggcg gcgacgcggt gccgctcgcc 26700
   tccgtcgtac ggttgcgcga gacctgcccc gagatccggg tccggcacct ctacggcccc 26760
   accgagacca ccctgtgcgc cacctggcac ctgatcgagc cgggggtcgc caccggcgac 26820
   accctgccga tcggccggcc gctggccggg cggcgagcgt acgtgctcga cgcgttcctg 26880
   caaccggtgg ccccgaacgt gaccggcgag ctctatctgg ccggcgccgg gctggcgcgc 26940
   ggctatctgg gcgccgccgc cgcgaccgcg gagcggttcg tcgccgaccc gttcgccgcc 27000
   ggtgagcgga tgtaccgcac cggggacctg gcgcgctgga cggaacaggg cgagctgctg 27060
   ttcgccggcc gcgccgacgc ccaggtgaag atccgcggct accgggtcga gccggcggag 27120
   atcgaggcgg cgctgaccgc gatccccgag gtcgcgcagg cggtcgtggt ggcccgcgag 27180
   gacggcccgg gcgagaaacg gctgatcgcc tacgtcaccg cggccgggca gccgggaccg 27240
   gaccccgccg cggtacgcga acacctgggc gagcggctgc cggagttcat ggtgccggcc 27300
   gcggtggtgg tgctggacgg cttcccgctg acgatcaacg gcaagatcga ccgagcggcc 27360
   ctgcccgccc ccgaacgcag cgggaagccg gccggccgcg agccgcgtac cgaggtcgag 27420
   cgggtgttgt gcgacctgtt cgccgacctc ctcgggctgg accgggtcgg ggccgacgac 27480
   agcttcttcg aactgggcgg cgactcgatc gtgtcgatgc agctggccgc ccgcgcacgt 27540
   cgcgagggcg tcgtcttcgg cgcgaaggac gtcttcgagc agaagacgcc ggcggccatc 27600
   gccgcggtcg cggtgcgcgg cggtcagcgg ccggccggtc ccgacgacgg ggtcggcgag 27660
   gtcgcctgga cgccggtgat gcggggcctg ctcgaccgtg accccggcac gatgaaccgc 27720
   agcgccatgg cgcagtgggt gaccgtgggc gcgcccgaca ccctgtcgcg cgacgtgctg 27780
   gcggccgggc tgggagccgt gctcgacgcc catgacatgc tgcgcagccg cgtcgtcgcg 27840
   gccgggacgg aagagccgcg gctggtggtg gcgccgcggg gcgcggtgca cgcggccggg 27900
   ctggtggacc gggtcgaggc gggcgccggc gaggtcgacg acctcaccga gcgcagcgcc 27960
   gccgaggcgg ccacgcggtt ggatccgggc gccggcgtga tggtccgggc ggtgtgggtc 28020
   gacgcgggcc ccggccgcgt cggacggctc gtggtggtgg cgcaccacct ggtggtcgac 28080
   gcggtgtcgt ggcggatcct gctgccggac ctgcgcgcgg cgtgcgaggc ggtggccgcg 28140
   ggccgggagc cggcgctcga tccggtggac gtctcgttcc ggcgctgggc gcggacgctg 28200
   accgatcagg cggccacccg gaccgcggag ctggccacgt ggacgcggat cctgggcggg 28260
   gcgcagccgc ggttcggcgc gctcgacccc ggacgcgaca ccatctcgac cgccggccgc 28320
   cggacgtgga cggtgccgca ggaccgggcc ggcgtgctgg tggagagggt cacctcggcc 28380
   ttccactgtg gtgtgcacga ggtgctgctg gccaccctgg cgggcgcggt ggcccgatgg 28440
   cgcggcggcg ccgccgtcgt ggtggacgtc gaaggtcacg gccgccggcc gctcggcgag 28500
   atggacctgt cgcgcacggt cggctggttc accgacgtgc acccgctccg gctggacgtg 28560
   accgggatcg acacggccga ggtgatcgcc ggtggcggcg cggccggccg cctgctgaag 28620
   accgtcaagg agaacgtgcg tgccgtgccc gacggcgggc tcggctacgg gatgctgcgc 28680
   cacctcaacg ccgagaccgg gcgggtgctg gcggcacagc cggcagccga gatcgggttc 28740
   aactacctgg gccgcttctc cgaggggccc ggcggtgacg tgcagccctg gcagcagctg 28800
   gggacgatcg gcggtacggc ggagccggac atgccgttgc ggcacgccgt ggagatcgac 28860
   gcggccgtgc tcgacgcggc cgacgggccc cggctggagc tgaccgtcac ctgggccggg 28920
   cggcttgtcg gcgcggccga ggccgagtcg ctcggcaggg cttggcaaga cgtgctggcc 28980
   ggtctggcgg tacacgccgg cgacgtccgc gccggcgggc acaccccgtc cgacttcccg 29040
   ctgaccgcgc tgagcgggca ggaggtggcg cagatcgagg ccgccgtgcc ggacctgctc 29100
   gacatctggc cgctctcccc gctgcaggag ggcctgctgt tccatgccgt cgacgagcgc 29160
   ggcccggacg tctacgccag catgcggacc ctggccgtcg acggcccgct ggacgtggcc 29220
   cggttccgcg cgtcctggca ggccgtgctg gaccggcacg ccgccctgcg ggcgagtttc 29280
   catcagctcg agtccggcgc ggccgtgcag gcgatcgccc gcgaggtgac gctgccctgg 29340
   caggagaccg acctgtcgga tctgcccgag gacgtggcgc tggcggagtt cgaccggctc 29400
   gcggcgcagt tgcgcgacga gcggttcgac ctgacccggg cgccgctgct gcgcctgcac 29460
   ctggtgcgtc tcggcgagcg ccggcaccgg ctggccttcg cgtcgcacca catctcgtgc 29520
   gacgggtggt cactcccgat cgtctccacc gaggtgatgg cggcgtacga gggtcgcggc 29580
   ctgccggcgc cgacgtcgta ccgtgactac ctggtctggc tggcccgcca ggacaaggag 29640
   gccgcccggc aggcgtggcg cggcgaactg gccgggctcg acgaggccac ccacgtcgtg 29700
   ccgccggaca cgatcaccgc gccgatcgcg ccgcagcggg tacggttcgg gctcgacgac 29760
   gagctgagcc ggcgcctggt gcggttcacc cgccgccacg gcgtcacgat gaacaccctc 29820
   ttccagggga tctggtcgtt gctgctggcc cggctgaccg gccgcgacga cgtggtcttc 29880
   ggttccgccg tggccgggcg gcccgcggag atccccggcg tcgggtccgt ggtcgggctg 29940
   ttcatgaaca cgctgccggt ccgggtgcgc ctcgacggcg ccgaaccgtt cctggacatg 30000
   ctgaccgacc tgcagcggcg gcaggtcgcg atgatggccc accagcacct cgggctgagc 30060
   gagatcaaac aggtcgccgg tccgggggcg gcgttcgaca cgctggtggt gttcgagaac 30120
   tacccgcgcc cgccgcgccc gtccgacgac ccggacgcgg tggtcatgcg cccggcggag 30180
   atcccggagg acagcgggca ctacccggtg tcgatgcgcg cctccgagga cggcggcgtc 30240
   cacggcgagt tcatcttccg tccggacgcg ttcgaccggg ccgaggccga ggggatgctc 30300
   gccgcgatca tcgaggccct gcggcaggtg gtggccgaac cggggattgc ggtggggcgc 30360
   atcggtctgg tcggcgcggc cgagcgccgg caggtggtgc gggagtggaa cgagaccgat 30420
   gcgccgctga ccgcgcagcc gctgccgatg ctgttccgcc ggcaggcgca gcgctccccg 30480
   gacgcggtgg cggtgcgcga cgcggcgcgc agcctgtcgt tcggcgcgct gctgggcgag 30540
   gccgaggggc tcgcccggcg gctggtggcc tccggcgtac ggcgcgagac ccgggtggcc 30600
   gtgctcgtgg aacgctcggc ggagctggtg gtggccctgc tcggggtgtc gctggccggc 30660
   ggggtgttcg tgcccgtcga cccggactac cccggcgacc ggatcgcgct gatgctggcg 30720
   gatgcggcgc cgcaggtgct ggtgtgcacg gcgcggaccc gctcggtcgt gccgggcgac 30780
   ttcgccggcg cggtgctggc gctggacgag ccgtccgcgg ccggcccgca ggtgtcgctg 30840
   ccgcgggtgg cggcgcggga cggggcgtac gtgatctaca cgtccgggtc gacgggtgtg 30900
   cccaagggcg tgctggtcac ccacgccggg ctcggcaacc tggcgagcgc gcagatcgag 30960
   cggttcggcg tgacgtcgtc ctcgcgggtc ctgcagttcg ccgcgctcgg gttcgacgcc 31020
   gccatctccg aggtgtgcat ggccctgctc tcgggcggga gcatcgtgct ggccgacgcc 31080
   gagcacatgc cgccgcgggt gtcgctgggc gacgcggtac gccggtggga catcacccac 31140
   gtcaccgtgc cgccgagcgt gctggccgtc gaggacgacc tgccggacga cctcaccctg 31200
   atcgtggccg gcgaggcctg cccgcccgcg ctggtggacc gctggtcgcc gggccgccgg 31260
   atgatcaacg gctacggccc caccgagacg accgtgtgcg cgacgatgag ctccccgctg 31320
   tccccgggac gcggcgcggt gcccatcggc ggcccgatcg ccaacatgcg gacctacgtg 31380
   ctcgacgcct tcctgcagcc ggtgccgccg ggcatgaccg gcgagctcta cgtcaccggg 31440
   gtcgggctcg cccgcggata tctcgggcgt ccggggctga ccgcggaacg gttcgtggcc 31500
   gacccgttct cgccgggtgg ccggatgtac cgcaccggcg accgggcccg ctggaccggc 31560
   gacggcgagc tggtcttcgc cggccgggtc gacgcgcagg tcaaggtgcg cggataccgg 31620
   atcgagccgg gggagatcga ggccgtgctg gccgagcacc cgggggtcgc ccaggtggtc 31680
   gtcgtggccc gccaggacgg gccgggcgag aagcagctgg tggcctatgt cgtcccggcg 31740
   gccggcccga ccgccgaggc gagcaccctg atctccgcgc tgcgcgaggc ggcggccgcc 31800
   cgcctgccgg agcacatggt gccggcggcg ttcgtgccgc tggacgcgat gccgctgacg 31860
   cccaacggca aggtcgacca ccgggcgctg caggctcccg acttcgccgg gatgtcggcc 31920
   gggcgggacc cgcgtacggc cttggaggcc aagctgtgcg agctgttcgc cgaggtgctc 31980
   ggcctggagc aggtgggcgc cgacgacagc ttcttcgagc tcggcggcga ctcgatcacc 32040
   tcgatgcagc tgtccacccg ggcccgccgg gaggggctgg agctgacccc gtggcaggtg 32100
   ttcgacgaga agacgccgga acggctggcg gcgctcgtca aagagatccc ggccgacggc 32160
   ggggccaccg gggcgccgga gcccgcggcg ggcacgctcg tcgcgctctc gcctgaacag 32220
   atggaccaac tcgaggccgg accggccggc gaatgacctc cataaggagc agatcgtgac 32280
   cgttgacgac actcgtgcaa agccccgctc cagcgtggaa gacgtctggc cgctgtcgcc 32340
   gctgcaggag ggcatgcttt accacaccgc cctggacggg gacgggccgg acacctacac 32400
   cgtgcagacc gtctacggca tcgacggccc gctggacgcc gggcggctga gagcgtcgtg 32460
   gcaggccctg gtggaccggc atgccgccct gcgggcctgc ttccgctacg tcagcggcgc 32520
   gcagatggtg caggtcattc agcgggacgt cgagctcccg tggcgggaga ccgacctgtc 32580
   cgggctgccg gaggacctcg ccgacggtga ggtggcccgc ctggcggagc aggagatggc 32640
   cgagcgcctg cgcctggagg ccgcgccgct gatgaaactg cacctgatcc ggctcggccc 32700
   gcagcgccac cggctcgtgc acaccctgca ccacgtgctg gtggacggct ggtcgatgcc 32760
   gatcctgcac cgtgagctcg cggcgatcta cgcggcgggc ggggacgcgt ccgggctgcc 32820
   gcccgcggtg tcgtaccgcg actatctggc ctggctgggc cggcaggaca aggagtcggc 32880
   gcgggcggcg tggcgggacg cgctcgccgg gctggacacc ccgaccacgg tcgcgccggc 32940
   cgatccggcc cgcgtgccgg acatcgacac ggcgatggtg gagctgtccc ccgagctgac 33000
   cgacgacctg ctccggctgg cgcatggcca cgacctcacg ctcaacaccg tcgtgcaggg 33060
   cgtgtgggcg gtcgtgctgg cgcagctggc cggccgtacc gacgtggtgt tcggcgcgac 33120
   cgcctcgggg cggccggccg agctggccgg ggtggaggcg atggtcgggc tgctgctggc 33180
   caccctgccg gtgcgggtcc ggctcgacgg gcgccagcgg gtcgtcgaca tgctcgccga 33240
   cctgcagcgc gaccagtcgg cgctgcgcgc gcaccagcat ctcgggctgc aggaggtgca 33300
   ggccgtcgtc gggcccggag cggtcttcga cacgctggtc atctacgaga acttcccgcg 33360
   tacgggcctc ggcggatcgg ccgacgacgg cctggtcatg cggccggtgc agaaggggcg 33420
   cgactcctcg cactacccgt tcacactggt caccggtccg ggcgagcgga tgccggtgat 33480
   cctctactac gaccgggggc tgttcgacca ggcggccgcc acatcggtgc tcggcgcgat 33540
   cgtccgcgtg ctggagcggc tggtcgccga gcccggtgtg ctcgtcggcc ggctgacgct 33600
   gctgggcgag gccgagcgcg ccgtggtggt cggcgactgg aacgcgaccg cgggcccggt 33660
   ccccggcgac tccatggtcg agctgttcga ccggcgggtg gccgccgcgc cggacacggt 33720
   ggcgatcacc gacgccgacg gcgccgacct cacctatgcc gaggtcgatc aggcgtcgaa 33780
   ccggttggcg gcccacctcg ccgatcgggg cgtccgccgc ggcgaccgcg tcggggtggc 33840
   cgtggagcgg tccgcggacc tgccgatcgt gttcctggcg atctggaagg ccggcgccgc 33900
   gtacgtcccg gtggacgtcg ggtacccggc cgagcggatc gcgctgatcc tcgccggctc 33960
   cgcggtctcg acggtgatct gtacgcaggc caccgaggcg gcggtgccgc agaacgtgcc 34020
   gcagcgcgcc acgatcgtcc tcgatgcgcc gccgacgcgg gcggccgtgg acgcctgtgc 34080
   cggcaccgcg ccgacgttcc ggccgagcgc cgccgacctg gcgtacgtca tgtacacctc 34140
   gggctccacc ggcgtcccga agggcgtggc cgtcccgcac ggcgcggtcg ccggcctggc 34200
   cggcgacgcg ggctggcgga tcggccccgg cgacggcgtg ctgatgcacg cgacgcacgt 34260
   gttcgacccc tcgctctacg agatgtgggt gccgctggcc acgggtggcc gggtcctggt 34320
   ggccgctccc ggagtggtcg acgcgggcgg gatccggcag gccgtggcac gcggcgcgac 34380
   cgccgtccac ctcaccgccg gcaccttccg cgcgctggcg gaggcgtccc cggactgttt 34440
   cgccggcctg cgcgaggtcg gcaccggcgg cgacgtggtg cccgcgcaca ccgtggccca 34500
   cctgcggcgg gcccagccac agctgcgggt gcgcaacacg tacgggccga ccgagaccac 34560
   cctgtgcgcc acctggaagc cgatcgagcc gggcgcgcag ctcgggccgg agctgccgat 34620
   cggccgtccg atgaccaacc gcaggatcta catcctggac gccttcctgc gcccggtcgc 34680
   cccgggggtg gccggcgagc tgtacatcgc cggcaccgga ctggcccgcg gatatctctc 34740
   ccggccggac ctgaccgccg agcggttcgt ggcgtgtccg ttcctcgccg gcgagcgcat 34800
   gtaccggacc ggggacctgg cgcgctggaa ccgcgacggc gaggtggtct tcctcgggcg 34860
   cgccgacgac caggtgaaga tccgcggcta ccgggtggag ctggccgagg tggaggcggt 34920
   gctggcggcc cagccgggag tccgcgaggc ggtcgtcgtg gcccgcgagg accggccggg 34980
   cgaacggcgt ctggtcggct acgtcgtctc cgacgccggt gagctggaca ccgaacagat 35040
   ccgccagcag atggcccggg tgctgcccgg ctacatggtt ccggtggcgg tcatcgggct 35100
   ggtcagcctg ccgatcaccg ccaacagcaa ggtggaccgc cgggccctgc ccgcccccga 35160
   cctcgccggc cacgcgtcgg cgaaggcgcc ggagagcgcg accgagaagg tgctgtgcgc 35220
   gctctacgcc gagatcctcg gcgtcgagcg ggtgggcgtc gacgacgcct tccacgacct 35280
   gggcggcagt tcggcgctgg ccatgcggct gatcgcgcgg atccgcgagg agctgggcgt 35340
   ggacctgccg atccgccagc tgttctcctc gcccacaccc gcgggggtgg cccgggcgct 35400
   ggccgcgaag tcgcgccccg cgctggaggc ggtcacccgg ccggagcggg tgccgctcac 35460
   cgcccggcag ctgcgcgcct ggctgctggc ccgcccgtcg gaggagaccc ggggccggca 35520
   cctctcggtc gcgctgcgcc tgcgcggccg gctggacgtg gccgccctgg aggcggcgct 35580
   gcgcgacgtc gcggcccggc acgagatcct gcggaccacc ttcccgggcg acgcgcagac 35640
   cgtccaccag cacatccacg acgccgcgcc ggtccggctg acgccggtcc cggccaccga 35700
   ggaggacctc cccgcacggc tggccgagcg gggcgagcag ctgttcgacc tcacccggga 35760
   catgccgtgg cggtgcgagc tcttcgcgct gtcggagaag gagcacgtgc tgtcggtgac 35820
   ggtgcaccgc atcgccgccg acgacgactc gatggacgtg ttcttccggg acctcgcggc 35880
   cgcgtacggc gcgcggcggg ccggccgggc cccggagcgc gcgccgctgg ccctgcagtt 35940
   cgccgactac gcgatctggg agcagcggct gctcgacggc gagcgcgagc aggacagcct 36000
   gatcaacgac cagatcacct tctggcgcaa ccacctggcc ggcatcgacc aggagaccgt 36060
   gctgccgttc gaccgagcgc ggccggccat cccgtcgcgc cgggccggca cggtcgcgct 36120
   gcggctggac gccggtccgc acgcccggct ggccgaggcg gtggaatcgg ccggcgcgga 36180
   catgccgcag ctcgtgcagg ccgcgctggc catgctgctg acccggtacg gcgcgggcac 36240
   cgacctggtg atcggcacga cgctgccgcg cgacgaggac ctgatcgacc tggagccgat 36300
   gatcgggccg ttcgcccggc cgttcccggt gcgtaccgac ctgtcggcgg atccgacctt 36360
   cctggaggtc gtcgcccggg tgcaggaggc ggtccgcgag gcgcgccagc acctggacgt 36420
   gcccttcgag aagattcccg agctgctggc cctgcccggc tcgctgtcgc gccacccggt 36480
   gtaccaggtc gggctgcagg tgcgcgagga ggacaacggc gcgtgggacg ccgcggaact 36540
   gcccgccctg cgcaccagcg tcgaacccac cggggtcgag gccatcgagc tggacctggc 36600
   gttcgcgctc accgagcgcc gcaacgacga tgacgacgag gacggcatcg agggcgccct 36660
   gcactatgcc gccgacctgt tcgaccacga cacggccgcg tcgctggccc ggcggctggt 36720
   ccgcgtcctg gaacaggtgg ccgaggatcc cgggcggcgg atcagcgacc tggacatcct 36780
   cctcgacgac gccgagcgcg gcgctccggc gatcgcgccg gcgcgctggg ccggggcggt 36840
   gccggcggcg gtcgccgacc tggtcgcggc cggcccgctc ggtgcgctgg tgctcgacga 36900
   ggcacagcgc ccggtggcgc ccgggaccgt cggcgacctg tgggtgaccg gtcccgcggt 36960
   ggacgcggac accggcggcc ggccgagcgt gccgtgtccg ttcgggaccc cggggcaccg 37020
   gatgctgcac accggtctgc tcgcccgtaa gtcgccggcc aggacgctgg tcgtcgtggg 37080
   ggagcggcgc ggggcgagcg cctcggcgaa gaccggtgac tacgaggtgc tgctgccgct 37140
   gcgcgccgcg ggtgaccggg cgccgctgtt ctgcatccac gccagcggcg gcctgagctg 37200
   gaactacggt ccgctgctgc ggcacctgcc gccgaaccag ccggtctacg gggtgcaggc 37260
   gcgtggcctg gcccgtaccg agccgctgcc cggcagcgtg gaggagatgg ccgccgatta 37320
   cgtcgagcag atccgcaccg tgcagccgac cgggccgtac cacctgctcg gctggtctct 37380
   gggcgggcgg atcgcgcagg cgatggccgt gctgctggag gcggacgggg agaaggtcgg 37440
   cctgctcgcc ctgctcgcgg cctatcccgt ctacatggga aggaaggcca ccggcacggt 37500
   caccgaggag gccgcccgcg acgaagacgc cctggaacgg cgcaagcagc aggaactgga 37560
   gctcgccggt cagctggtcc agggggccgg cgcccggtcc cgcttggagg cggtcatgcg 37620
   caacctctgg gaggtcgggc cggcccacac cgcctcgccg ttcgccagtg atgtcctcct 37680
   cttcgtcgcc tccgtggacc gtcccgcgca tctgccggtg tccgaggcga tagccagctg 37740
   gaaagacttc accgggggga cgatcgagcc ccacgagatc gcgaccaacc actacgagat 37800
   ggtgcagccc gcggcgttgg cccagatcgg ggccatcgtc gccgagaaac tccggtcccg 37860
   gccggatgcg tgagaggaca cgatgatgac caatccgttc gacaacgagg acggttcctt 37920
   cctcgtgctc gtcaacggcg agggccagca ttcgctgtgg ccggctttcg ccgaggtccc 37980
   ggacggctgg acgggggtcc acggtccggc ctcccggcag gattgtctcg gctacgtcga 38040
   gcagaactgg acggacctgc ggcccaagag tctgatctcg cagatcagcg actgagcgtc 38100
   cagatgctca atgcggccgg ttggacgagc gttgacgctc gtccgaccgg ccggcaacgt 38160
   cttccggctc gcgcgcctgc acgacgacgg gtcggcgggc gggaccgggc gccgctgccg 38220
   atgcccgcgt ctcggcgacg cgccaccgcg cacggacccg gtcgtctgcg gcgcgcaccg 38280
   gcgccgccga agcgggcgcg ttcccggtgc cggctgccgg accgggcggc gaggatgtgg 38340
   cgcgacctgt ccaaccgtca ccggttgtag gccggcaatg gtgtcaccaa actgatcccc 38400
   atgcctcacg accccggtgc aacccggctc ctggcaattt cccctcatct ggacgacgcg 38460
   gttctgtctt tcggggccgg tctggcccag gcggcacagg acggcgcgaa tgtgctcgtc 38520
   tatacggtat ttgccggcgc cgcacagcct ccttattccc cggcggcgca gcgaatgcac 38580
   acgatctggg gcctcgcgcc ggacgacgac gcggtgctct accgccggaa agaagacatc 38640
   gccgctcttg atcacctgag ggtggctcat cggcacggcc ggttcctcga ctccatctac 38700
   cgtaagttgc cggacggtcg ttggctgacc gcccatgtgg agggcaggca gaagctggcc 38760
   gtcaacgatc actcgccgga cagcgaccat gacctggttg gcgaggtggc ggacgacatc 38820
   aggtcgatca tcgacgagtt cgacccgacg cttgtcgtca cctgtgcggc catcggcgaa 38880
   caccccgacc atgaggcgac gcgagatgcc gcgctgttcg ccacgcacga gaagaacgtt 38940
   ccggtacggc tgtgggagga cctgccgtat gcggtcttca aatcaggtgc ggtcgaattg 39000
   ccgcagggtt ttcgtctcgg ctctgcggat gtcagctcgg tgaagccgga gatgcggtcg 39060
   cagaaattcc aagctgtgga gcggtattcg tctcaaatgg tgctgctcaa cggaagtgaa 39120
   aacaatctgt tcgaccggct ggacgagcat gctcggcaga atgcaccaca cggcggatac 39180
   ggcgagacga cctggccggt cgtccgcagc gacgacagct gacttctgac ctcccgccat 39240
   cgacgccgta tcacgcctgg aggttacgtg gtgtccgaaa cactcactca tccgccgtcc 39300
   caggattccg cgcccgagag ccggcctcac cgctgggcgg tatggcgctc accggccggt 39360
   cagccggcgt gggcccggcc cgcgctgctg ggcatcgcgc tcgtcgcggc ggtgctgtat 39420
   gcctggaacc tgcggctggt cgactacgcg ccgcggtacg cggacgccgt caagagcatg 39480
   tccgggagct ggcgcgcgtt cctgtacggc gccgtcgacc cggaggcgac gtacacgctc 39540
   gacaagctgg ccggctcctt cgtgccccag gtcgtctcgg ccaagatctt cggttatcac 39600
   gcgtggtccc tggcgttgcc gcaggtgatc gagggcgtga tcgcggtgct ggtgatgtac 39660
   cgggtcgtac ggcggtgggt gggcgtggtg cccggcctgc tcgccgccgg catcttcacc 39720
   ctgaccccgg tcgccgcgtc catgttcggg cacagcatgg aggacggcgc actggtcatg 39780
   tgcctggtgc tcgccgtcga cgcgtatcag cgcgcggtcc tggaagggcg gctccggtca 39840
   ctgatcgggg ccggtgtctg ggtcgggctg ggcttccagg ccaagatggt gcaggcgtgg 39900
   atgattctgc ccgccctggc ggtcggctac ctgctgaccg cgccgatcga gctgcgccgc 39960
   cgggtgcggc atctcggggc cgccggggcg gtgatgctgg cggtgtcgct gtcgtgggtc 40020
   atgctctaca ccctcacgcc ggccagcgat cggccctaca tcagtggcac cacgaacaac 40080
   aacgccttcg ccatggtgtt cggatacaac ggtctcggcc gtgtgggcat caacctgccc 40140
   ggcgccgagg aggtcaacgg tcgtaacggc gtgccggcgc ccggtccgct gggcggcccg 40200
   gagggcgagg gccagagggg aacggtcggc acgaaggccc aggaccagag gggaacggtc 40260
   ggcacgaagg cccaggacca gaggggaacg gtcggcacga aggcccagga ccagagggga 40320
   acggtcggca cgaaggccca ggaccagaag ggaatggtcg ggccgaaggc ccatggagga 40380
   ccgccgatgg gaccgggcgc gctccccgag ggatacgagt ccaagatgga gagcagggcc 40440
   gatccgatgg gttggaccaa gctgttcgac ggtcacctcg gcgtcgcgat cggctggctc 40500
   tttccgctga ctctgctcgc cctgctgtgc gggttgtggt ggcgccgccg ggccgagcgc 40560
   accgatccgg tgcgcggcgg gctggtgatg tggggggtgt ggctggtgac cttcggcttt 40620
   gtcttcagtg cgagcgctgt cgcgcacacc gcctacgtgg cctcgctggc gccggcgatc 40680
   gcggccgtct ccgccctcgg catcgtgatg ttcgggcggg cgtatcggcg cggcggcagg 40740
   cagggctgga tactgccgct cgcggtcgcg gtccaactgg cctgggccgc ctggctgtgg 40800
   tcgcactacc cgaacttcct cccctgggcg agatggggca cgctcctgct cggcgtggcc 40860
   gcccttgtcg ggctggtcct ggcgcgcgtg accaggaccg cctcctccgc gctggtcgcg 40920
   gccgcgctcg ccgtcggcgt cggcgtcatg ctggccgcgc ccgccacgta tgccgtctcg 40980
   gtgctcgatc ccgattactc cggcaactcc ttcgatgcca acgcgggacc ggcctcgggc 41040
   accgcgtaag gcgagccggt ggatccgtca ccgccccgcg ggctgcgttt ccgcgcccgg 41100
   ctcggccggc ctttgacatc ggcgcgggtc ttcggctgcc acccgtggtc gctgaccctt 41160
   ccgcttcgga cggcgcccgc cggcgccggc atcgcccgct cggtgccttc cccgccgcct 41220
   cgtcggcggt gccggcgctc gtcgcgggtc aggaatgttc gcccggtcct actcgtggac 41280
   atcgggtccg atccccagga tgggatcgat gacttagcga gtcgtgtgag agaggtgctt 41340
   ctgtggacgt ggtgttgcgc ttcggggggg tgtcgaagaa cgatgacgac cccgatccct 41400
   ggcagaccct gatcgggccg gggacgctgc gcagagtgct cgcctacttc cgcccgcaca 41460
   tcggcaaggt ggcgctcttc gttctcgtcg ccgcgctgga gtcgcttgtc gtcgtcgtag 41520
   ttccgttgtt gctgatggaa ctggtcaacg gcggcatcct caaggacgac gccggggtcg 41580
   tggcatcgat ggccggcctg gccgccggcc tcgccctggt gggcgcgctg ttgcagctgg 41640
   tgtcagccta tatttccggg cagatcgggc agggggtcag ctacgacctc cgggttcagg 41700
   cgctgcgtca cgttcagcag ctgccgatcg cgttcttcac ccgtacccag accggggtgc 41760
   tggtgggccg gctgcacacg gaactgatca tggcgcagca ggcgttcagc cagctcctga 41820
   tggcagccac cagcgccgtc acggtgatcc tggtgatggc cgagctgttc tacctgtcat 41880
   ggcttgtcgc cgtggtcacc ctggtgctga ttccgctgtt cctcgtgccc tggatctacg 41940
   tgggccgggt gatccggcgg cgcaccggac ggctgatgga cgcgaatacc ggccttggtg 42000
   gactcctcca ggagcggttc aacgttcagg gggcgatgct ctccaaactc ttcggccgtc 42060
   cccgcgagga gatggccgag tacgccgatc gtgccgagcg catccgccgc atcggcgtga 42120
   gtctctcggt gtacgggcgg ctcgccttcg tcatgatggc gctgatggcc tcgctcgcca 42180
   cggcgctcgt ctacggcatc ggtggcgggc tcgtgctcgc cggggcgttc cagctcggca 42240
   cgctcgtcgc cctcgccact ctgctcgggc ggttgttcgg gccgatcacg cagctgtccg 42300
   ggatgcagga aaccgcgcag accgtcgtgg tgagcttctc ccggatcttc gaggtgctcg 42360
   atctgaaggg gctgatcgag gagcgtcccg gtgcggtcgc gctcgccaag gacacggcgc 42420
   cggagatcga gttcgacgac gtggcgttcc gctatccgac cgccgacgag gtctcgctgc 42480
   gctcgctgga gtacgtacgc gccgaacggg agcgcgggga gacgacgccg gaggtgctgc 42540
   gcggtgtcag cctgcacgtg ccggccggca ccctcaccgc gctcgtcggc ccgtccggcg 42600
   ccggcaagag cacgctcacc cacctggtgt cacggctgta cgacccgacc ggcggggtca 42660
   ttcgcgtcgg tgggcacgat ctgcgcgacc tcaccttcga ctcgctgcgc gaagcggtgg 42720
   gcgtggtcag ccaggacgcc tacttcttcc acgacacgat ccggcagaac ctgctctacg 42780
   cccgccccac cgccaccgag gacgaactga tcgaggcatg tcgaggggcc cagatctggg 42840
   acctgatcgc ctcgctcccg ggcgggttcg acaccgtcac cggcgatcgt ggctaccgcc 42900
   tgtcgggcgg cgagaagcaa cgcctggcca tcgcccggct gctgctgaag gcgccgacga 42960
   tcgtcgtgct cgacgaagcc accgctcacc tggactccga gtccgaggcc gccgtgcagc 43020
   gggcactcaa gaccgccctg cacaaccgca cctcgttggt gatcgcccac cggttgtcca 43080
   cgattcgcga ggccgaccag atcctcgtga tcgacgacgg acgggtccgg gagcgcggaa 43140
   cgcacgacga gctgctggcc gaggggggtc tgtacgcgga gctttaccac acgcagttcg 43200
   ccaaggccgg aagcgacgca gccgaaccgg cgagcgacgc agccgagctg caccacaggg 43260
   agcccgagcc ggtgcacggc ggaggatgac gatggatcag gcgacgccgg cccggatcgc 43320
   cggccgtcgt cgcccagaac gccggctccg gccggatgcg tgagaggaca cgatgatgac 43380
   caatccgttc gacaacgagg acggttcctt cctcgtgctc gtcaacggcg agggccagca 43440
   ttcgctgtgg ccggctttcg ccgaggtccc ggacggctgg acgggggtcc acggtccggc 43500
   ctcccggcag gattgtctcg gctacgtcga gcagaactgg acggacctgc ggcccaggag 43560
   cctggtcgag caggccgacg cgtgagtgag ccgtccaccc gcccgatccg gacggttgga 43620
   cgagctcgac ggtcgtacga gcggccggaa gcatccatgt cgagcgccgc ttggtcgacc 43680
   gaaaggatct gtggcatgtt cgaggagatc aacgttgtcc gggccgcgga gctgcaccgg 43740
   agggaccgat tcgatccggt gccacagctg cgctcgctga tggccgaggg cccgttgacc 43800
   acgctgggca ccgaggagtc tcccggaggc cggacggcgt ggctcgccac cgggtacgac 43860
   gagatccggc aggttctcag ctccgacgac ttcagcgcac ggctgctcta cgggggaacc 43920
   gctgccggca tcacctggcc cggtttcctc acccagtacg acccgcccga gcacacccga 43980
   ctgcgccgca tggtggcgcc cgccttcgcc gtccgacgga tgcagaagtt ccagccgcag 44040
   gtcgagcggg tcgtccagga cagtttggac gccatcgagg cgctcggcgg accggtcgac 44100
   ttcgtccccc gtttcgggtg gtcggtggcg acgacggcga cctgcgactt cctcggcatt 44160
   ccgcgcgacg atcaggcgga tctggcgcgc agcctccatg ccagccgaac cgaacggtca 44220
   ggcaagcggc gtaccgcggc aggcaacaag ttcatgacgt acatgaacaa gatgacggct 44280
   cgcacccgcc gcgatcccgg tgacgacatg ttcggtgtcg tggtacgcga gtacggcgac 44340
   gagatcaccg acgcggagct gaccggcgtc gccgcgttcg tcatggcgcc gggcgccgac 44400
   caggtggccc gtttcctcgc ggccggcgcg tggctgatgg ccgacgatcc cgagcagttc 44460
   gcactgctgc gggagaagcc ggacaccgtc ccggattggc tggacgaggt gattcggtat 44520
   ctgaccaccg atgagaagac ccatccgcgc gtcgcgacgg acgacgtgcg catcggcgat 44580
   cacctcatca aggccggcga taccgtcacg tgctccctgc tggctgccaa ccgccgcaac 44640
   ttcccgcgcc cggaggatcg attcgacatc acccgggtcc ggccggagca cctggcgttc 44700
   gggcacggca tccatcactg cctgggcagg tctctggccg agctggtgtt ccggacggcg 44760
   attccggccc tggcacaccg cttccccacg ctgaggctgg ccgagccgca ccgagagatc 44820
   agattggggc cgccgccgtt cgacgtggaa gccctgctgc tcgactggta gcgccggaag 44880
   cataggagat ccgatggcac ttcctctgcc gcatcagcgg ctccggctcg atccggtgcc 44940
   ggagttcgaa gagctgcaaa aagctgggcc cctgcacgaa tacgacaccg agccgggcat 45000
   ggacggccgt aagcagtggc tggtcaccgg acatgacgag gtccgcgcga tcctcgccga 45060
   ccacgagcgg ttcagttcca tgcggccggt cgacgacgag gcggatcgcg ccctgttgcc 45120
   gggaatcctg caggcgtacg acccgccgga tcacacccgg ctgcgcagga cggtggcccc 45180
   ggcgtattcc gcccgacgga tggagcggct gcgaccccgc atcgaggaga tcgtcgaaga 45240
   gtgcttggac gacttcgaga gcgtgggcgc gccggtcgac ttcgtccgac atgccgcctg 45300
   gcccatcccc gcgtacatcg cctgcgaatt cctcggcgtc ccgcgtgacg accaggcgga 45360
   gttgtcgcgg atgatcaggg agagccggga gagtcggctt ccccggcagc ggaccctgtc 45420
   gggtctgggc atcgtcaatt acaccaagcg gttgacctcc ggcaaacgcc gtgatcctgg 45480
   cgacgggatg atcggcgtga tcgtgcgcga acacggcgct gagatcagcg acgaggaatt 45540
   ggcggggctc gccgagggga acctgatcat ggcggcggag cagatggccg cgcagctggc 45600
   ggtcgcggtg ctcctgctgg tgacccaccc cgaccagatg gcgctgctgc gcgagaaacc 45660
   ggagctcatc gacagcgcca ccgaggaagt gctgcgccac gcgtccatcg tcgaggcccc 45720
   ggctccccgg gtggcgctcg ctgacgtgcg catggccgga cgcgacatcc atgccggcga 45780
   tgtcctgacg tgttccatgc tggcgaccaa tcgcgccccg ggagatcggt tcgacatcac 45840
   ccgggagaaa gccacccaca tggcattcgg acacggcatc caccactgta tcggggcgcc 45900
   gctggcccgg ctccagctgc gggtggcgct gccggcggtg gtcggccggt tcccgtcttt 45960
   gcggctggcg gtcccggagg aggacctgcg gttcaaaccg ggcaggccgg cacccttcgc 46020
   ggtagaggag cttccccttg agtggtgacg gctcgcctcc gatccacacc cgccggcagg 46080
   gtttcgatcc ggccgacgaa ctgcgcgccg ccggaacgct gacgaagatt tccatcggct 46140
   ccggagcgga cgccgagacc acctggctgg cggccgggca cgccgtcgtg cgccaggttc 46200
   tgggcgatca caagcgattc agcacccggc gccgcttcga ccgacgtgac gagatcggcg 46260
   ggacgggcgt cttccgcccg cgtgagctgg tcggcaacct gatggactac gacccaccgg 46320
   agcacacccg gctccggcac ctgctgaccc cgggattcac ccagcgccgg atgcgtcgtc 46380
   tggcgccgcg catcgaagag atcgtgaccg accgcctcga cgccatggag caggcggggc 46440
   cgccggcgga cctgatcgag ctgttcgccg acgaggtgcc cggagccgtg ctgtgcgagc 46500
   tgatcggagt accccgcgac gatcaggcca tgttcctgca gctgtgtcac cggcatctcg 46560
   acgcctcgct gagtgccagg aaacgggcgg ccgccgggga ggcgttcgcc cgctacctgg 46620
   tggccatgat ggccagggaa cgcaaggacc ccggcgacgg gttcatcggc tcgatcgtcg 46680
   ccgaacacgg cgacacgatc acggatgagg agctgagagg cgtctgtgtg cagctgatgc 46740
   tggccggtga cgacaacgtc tccggcatga tcgggctggg ggtgctggcg ttgctgcgac 46800
   atcccgagca gatcgccgcg ttgcgcgggg acgatcagtc ggcggatcga gctgtcgacg 46860
   agctgatccg gtacctgacg gtcccatacg ccccgacacc gcggaccgcc gtggaggacg 46920
   tcatggttgc cgaccaggtg atcaaggagg gcgagacggt tctctgctct ctcccgatgg 46980
   ccaaccgtga ccgcgccctg ctgccggatg ccgaccgcct cgacgtgacc cgtacgcccg 47040
   ttccgcatgt cgcgttcgga cacggtatcc atcactgtct gggcgccgct ctgacgcgcc 47100
   tgcaactgcg aatcgcctat accgccctgt ggcggcgctt tcccgctttg caactggcgg 47160
   accccgccca ggaaataatg ttccggacga gtaccccggc gtatggatta accagtctgc 47220
   tggttgcctg gtagtgctct gccctctcca ttggacacct tgagccgcgg aagaacttgc 47280
   cggacgatgg catgaccggc gtgcggctca cacggaacct gcgccggcga gctgctacga 47340
   accatcggct cggtgcagac gctgtgcccg gaatcggcca ttgatttccg acgtccgacc 47400
   cggcgaaatc gccgccacgc atctgctggc atcgagatgc cgaccggatg gacgggacaa 47460
   cagcggatat cgcgtagcga ttcatgacct cactcagttc cgacgattcg atcgatggat 47520
   ggtggaaaat gccggtggaa gagttcgacg tggtggtggc cggtggcggg ccggccggtt 47580
   cgaccgtggc cacgctggtg gccatgcagg gccatcgggt gctgctgctg gagaaggaga 47640
   ccttcccccg gtaccagatc ggcgagtcgt tgctgccctc caccgtccac ggggtgtgcc 47700
   ggatgctcgg cgtctcggac gagctggcgg cagccggctt ccccgtcaag cgcggcggca 47760
   ccttccgctg gggcgcccgc ccggagccgt ggacgttctc cttcgccgtc tcgcccctga 47820
   tgaacggccc gacgtcgttc gcgtaccagg tcgaacgggc gcgcttcgac gagatcctgc 47880
   tgcgtaacgc gcagcgcaag ggcgtcgacg tgcgcgaggg atgctcggtc accggggtga 47940
   tcgaaggcga cgagcggatc accggggtgc gttacaccga ccccgacggc ggcgagcacg 48000
   aggtgtcggc gcggttcgtg gtcgacgcgt cgggcaacaa gagccggctg tactcccagg 48060
   tcggcggcgt ccgcaactat tcggagttct tccgcagcct ggcgctgttc ggttacttcg 48120
   agaacggcaa gcggctgccg gagccgtact cgggcaacat cctgagcgtc gcgttcgaca 48180
   gcggctggtt ctggtacatc ccgctgagcg acacgatgac cagcgtcggc gcggtggtgc 48240
   gccgggagat ggcggagaag atccagggcg accgggagaa ggcgctccag gccctgatcg 48300
   ccgaatgccc gctgatctcc gactacctcg ccgacgcgcg ccggatcacc accggccggt 48360
   acggagaact gcgggtccgc aaggactact cgtatcacca gaccagctac tggcggcccg 48420
   ggatgatcct ggtcggggac gccgcgtgct tcgtggaccc ggtgttctcc tcgggcgtgc 48480
   acctggcgac ctacagcgcg ctgctggcgg cgcggtcgat caacagcgtg ctcgccgagg 48540
   acctggacga gaagaccgcg ctgagtgagt tcgaggcccg ctaccgccgc gagtacgggg 48600
   tgttctacga gttcctggtg tccttctacc agatgaacgt caacgaggag tcgtacttct 48660
   ggcaggccaa gaaggtcacc aacaacaagc acaccgagat cgagtcgttc gtggagctga 48720
   tcggcggggt gtcgtcgggt gaggcggcgc tggccacggc cggccggatc gccgagcgca 48780
   gcgccgagtt cgccgcggcc gtggaccaga tggccgccag cgccgacggc aacatggtgc 48840
   cgatgtacaa gtcggaagtg gtcaagcagg tgatgcagga gggcggccag gtgcagatga 48900
   aggcggtgct cggcgcggac gccgagcccg agctgccgct gttccccggc gggctggtgg 48960
   tctcgcccga cgggatgaag tggctgccct accgtcccgc ctgaaccttt ccggaagcgc 49020
   cggggcgccg gacgagcgcc tcgccggcgc gtggcgttgc ggcgggctcg gcgatggcga 49080
   acggcacagg cgagaaggag tgttcaccgg tatggatcac gacatcgatg aggtcgcgcc 49140
   ggtcgtggag ccgacggcga actacatgag gagaacggtc tgcgacccgc acgaggacat 49200
   gttcgctctg cgggcgcacg gtccgctgat ccggatcggc ggggatgccg cggtccagat 49260
   gggcgcggat tatctctggc aggcgatggg ctacgacgtc gtgcggcaga tcctcggtga 49320
   tcatgagaac ttcacgacgc ggctgcggct gaccgcgtcc gaaccgatca gcggggaagg 49380
   cgcgaccaaa ccgcaggagc tggtcggaca gatctcgacc tacgatccgc ccgagcacac 49440
   ccgcctgcgc cggatgctca cgccggagtt cacggtccgg cgcgtccgcc ggctggagcc 49500
   cgcgatccag gagctcatcg acgaccgcat cgatgcgatg gaggccgcgg gaccgcccgt 49560
   ggacgtgcag acggtcttcg ccgatccggt cggcggcggc gcgctgtgcg aactgctcgg 49620
   catcccacgc gatgatcgga accagttcat gcgccgggtc cggcagaacg tcgatctcag 49680
   ccgcggatac aaggcccggg cggccgacag cgcggcgttc aaccggtacc tgcagagcct 49740
   catcacccgg cagcgcaagg accccgacga ggggttcctc ggcatgctgg tgcgcgacca 49800
   cggcgacgac atcacggacg aggagctcaa gggcgtgtgc acggcgctgc tcctcggcgg 49860
   tgtcgagacc gtcgcgggga tgctgggctt cggggtcctc gcgctgctgg accatcccga 49920
   ccagaggcgg ctgctcttcg ccggccgcga ggaggccgac cgcctggtca acgaactgtt 49980
   gcggtatctg tcggcggtgc agcagccgac tccgcggatg gccgtcaagg acgtggtcgt 50040
   cgatgggcaa cagatcaggg cgggggagta cgtgctgtgc tcgatcctca tggccaaccg 50100
   ggacgaagcg ctgacacccc atcccaacgt tctggacgcg gagcgtgagc cggcgccaca 50160
   catcggattc gggcacggca tccatcactg catcggggcg gcggtggcga gaaccgtgct 50220
   gcggatggcc taccagaccc tgtggcgacg acttcccggg ctgcgcctcg cgatacccgc 50280
   cgggcaggtg aagctcagga acgcgttcat cgacagtccg gatcagttgc cggtcacctg 50340
   gtagccgtca accgcaccgg gaagcgggat ggcacctgcc agaggcgccg ccgccaccat 50400
   acccgcgtca tgccgcacga tcgccggccg acgccggagc ctaccgaatg gggatgcgag 50460
   atgcgtgtgt tgttgtcgac gaccggaggg cgcggcgacg tcgaaccgct ggtcgcgctg 50520
   gcggtgcggt tccgcgaact gggtgtggac gcgcgggtct gcgcaccgcc ggactgcgcg 50580
   gaccggctgg ccggcgtcgg tgtgccactg gtggaggtcg gcgagtcggt gcgcgcgatg 50640
   atgcacggag ggaagcggcc gtcgcccgag gacgcgcccc ggctcgacgc cgaggccatc 50700
   gctacgcagt tcgacaaggt cccggcggcc gccgagggct gcgccgcggt ggtgacgacc 50760
   ggcctgttgt ccgccgcggt cgcggtgcgg tccgtggccg agaagctgcg catcccctac 50820
   gtctacgccg cctactgccc gatctatctg ccgtcgccgt actacccgcc gccgccggcc 50880
   ctgggcggcc cgcccgtacc ggaactgacc gacaaccggg cgcagtggaa caggcacagc 50940
   cagggtgcct accggcggtt cggcgccgcg ctcaacagcc accgggcggc gatcggcctg 51000
   ccgccggtgg agaacatcta cgactacgcc ttcagcgatc acccgtggct ggcggccgat 51060
   ccggtcctgg ccccgctgca gccgaccgat ctcgacgtgg tgcagaccgg cgcgtggatc 51120
   ctgcccgacc agcggccgtt gtcggcggag ttggaggggt tcttgagggc tggctcgcct 51180
   ccggtgtacg tgggtttcgg cagtgggccg gcgcctgccg aggccgcgcg ggtggccatc 51240
   gaggcggttc gggctcaggg ccgccgggtg gtgctgtcca gtggttgggc cgggttgggc 51300
   cggatcgacg agggggatga ctgtctggtg gtgggcgagg tgaaccatca ggtgctgttc 51360
   ggccgggtgg ccgccgtcgt ccacgccggc agtgcgggca cgacgaccgc ggtgacccgg 51420
   gcgggcgctc cgcaggtcgt ggtgccccag atgacggatc agccctacta tgccggtcgg 51480
   gtggctgacc tgggtgtcgg cgtggcgcat gacggtccga ccccgaccgt cgagtccctg 51540
   tccgcggcgc tggccacggc attgaccccg ggatccgggg cgcgagcggc ggcggtggcc 51600
   ggcacgatcc gcaccgacgg gacgacggtg gccgcgaaac tgctcctcga ggcgatcagc 51660
   cggcagagat cgtcggtctc cgcgtgaacc gcaccggctg tcggcccgca ccggtgggcg 51720
   gcgaggccgg agccggcccg ggcacctgcc gcggccatga cgttcgtcga agcgatggcg 51780
   gggcgtcccc gcgggtttcg ctggaggtgc aggagagcgt gaaggagtga tgatggatcc 51840
   cgagaccgta cgcatcgcgc tggggttgga ggaacggacc gcggcgtggc tcaccgagct 51900
   cgatgagctc ggtccgcccg ccgagccggt acggctgccg cgcggcgagg aggcacgtga 51960
   tctgctacgg cgtctggagg tgccggagct cgacgccgag gagatcgtgg cggccgctcc 52020
   cgatccggat cgtgacccgg ccctgtggtg gctgctcgaa cgcacccacc acgcgatcgt 52080
   ccggcacatg ggcgaccacc gggcgaagcc acgcggcggt ccgcccctgc cgtacgaggg 52140
   cggcgccgcc gcacgctact tccacgtgta cgtcttcctc gcgacggtgc cggcggtgcg 52200
   ccgcttccac gcggaacggg gtatccccga cgaggtcggc tgggagaccc tgacgcagtt 52260
   gggggagctg gtggccattc atcgccgcaa gtacggccag ggcggcatga acatgcagtg 52320
   gtggaccacc taccacctgc gcggcatcct ctaccggctc ggccgcctgc aattcagtct 52380
   ggccaccgga aaggacggca cgccgcacct cggcctgcac gttcccgagt ggggcggacc 52440
   gctgttgccc aaggcctatg acgaatcgtt gcatcgtgcc cgcccgttct tcgaccggca 52500
   ttttcccgag cacggcgccc gggtggcctg gggaagctcg tggatgctcg atccgcaact 52560
   ggaggagtat ctcaccgagg actccaacat cattcagctt gcgcggttct ggacgctcac 52620
   cgactccgcg ccggagcccg gtaacgcgga cggggactcc tcgatcctgg agttcgtgtt 52680
   ccgctacaac ggccagccgc tggatgaact tccgcagcgt tcctcgctgg agcgcgcggt 52740
   gatcgcccat ctgaaggccg gacgtcactg gcacatgcgg accggcttcg tgaagctgcc 52800
   gtaagcggcg gcggagccgc tgccggcgag cccggccacg accctccgga ggcgctggcc 52860
   gtccggctgc ttgtcgcgga cgctggacac atttgtggaa tcactgcgaa ggtaattcct 52920
   tcagtcattc gatcgtgagg agcggaagtg aatgaggagc gcctctttct ccggcccaac 52980
   accattatcg agcctctggt agacagattt tatgcctcga tgtacgcgac ggctccggtc 53040
   acggccacca tgagtctcgc gttccggtgg attccgatgc tggagtccta cctgcaggcc 53100
   ccggaatggc attacaaggg cagccgggac ccgaagttcc gcggcgggtt cttcgtcaac 53160
   atcgaggact cccgggcgga cgaggtgcgg gcgctgctcg ccgcgatccg gcgcgaccgc 53220
   gccgacatga tccgcttcgc cgaggcgatc gcggaggccg agaagatcgt ccgggaggag 53280
   gcgaccgggt acgacctccg gccgctctac ccgaagctgc ccgccgagct ggcgggcctg 53340
   gtggagatcg cctacgacac cagcaacgcc gcctcgctgc acttccagga gccgctggtc 53400
   taccacagca aggcctacac cgagcagcgg cagtcggtgc agctgtccgt ggagaccggc 53460
   atcgagcggc cgttcgtgat gagcacgccg cggctgccct cgccggacgt gctggagctg 53520
   cccatcccgt tccggcaccc gggcctggag cagctgttcc tgtcccggat ccggcccacc 53580
   accctgaccg cgctgcggga ggcgctggaa ctcggcgatt cgcaagcggc ccagctcgcc 53640
   ggcctgctgg tcccggagcc cgcgctcgcc gcggaccggc acatcgcggc cggcgcccgg 53700
   atccgctact gggggcacgc ctgcctgctc atgcagaccc cggacgtggc catcatgacc 53760
   gacccgttca tcagcgcgga caccgacgcg accggccggt acacctacaa cgacctgccc 53820
   gaccgcatcg actacgtgct gatcacccac gggcactccg accacctcgt cccggagacg 53880
   ctgctgcagc tgcgcggccg ggtgggcacg ttcgtggtgc cgaggacctc ccgcggcaat 53940
   ctgtgcgacc cgtcgctggc gctctacctc aagagcttcg ggttctccgc cgtcgaggtg 54000
   gacgacttcg acgagatctc cttcccgggt ggcaagatcg cggccacgcc gttcttcggc 54060
   gagcacgccg acctggacat ccgcgccaag gcgacgtact ggatcaacct cggcgggaag 54120
   tcgatctatg tgggggcgga ctcctccggc ctcgacccgg cgctctaccg ctacgtccgc 54180
   cggcacctcg ggcgggtcga catcgccttc ctcggcatgg agtgcgacgg cgccccgctg 54240
   aactggcagt accaggcgtt cctcaccaag ccgatcccga acaagatgag cgacagccgc 54300
   aagatgtccg gctccaacgc ggaacaggcc agcgcgatcg tcaccgagct cggcgccgac 54360
   gaggtgtaca tctacgcgat gggcgaggag agctggctcg gccacgtcat ggccaccagc 54420
   tacaacgagg actccttcca gctgcggcag atcgccgagt tcgagacctg gtgcgcgaac 54480
   aacggggtga agtcgggcca cctgctcgac cggcacgaat ggcactggtc gcacccctga 54540
   gcccggtccg gtggcgcgac cagggcgcga cgggttccgg cagtcgttga ccgacgaggt 54600
   ctccggtgga tcgtgcaacg gtgcagctgc cgcacggaga cctcgcggcc ttcgtcgctg 54660
   tgcgacatac cgatggcgcg cgacgcggcc ggcaaccgga cgccccggca tcgcggcggc 54720
   cggctctgca accgatgagc agggagggaa tccccgatgg gctacgacat ggccaccgca 54780
   cccgatctcg taactctgat ccgtcagcat gtcgccgggc ggccggacgc cgacgccgtc 54840
   ggctttctca ccgacccgga cgacatccgc ggcggggtcg tgtcgtggac ctacgggcag 54900
   ctcgaccggg aggctcgcgg gtacgcggcc tggttgcagc agcgcctgcc cgccggctcg 54960
   cgggtgctgc tgctgtatcc caacgggctc gacttcgtcg cggcgttctt cggctgcctg 55020
   tatgccggca tgatcgcggt accggcgccg ctgcccggcc gttatcgcca tcaccaacat 55080
   cgggtcgcga ccatcagcgc cgatgcccgg gtcagcgcgg tcctgaccac ggcggcgcat 55140
   ctgggggagg tccgcgactg ggctcgggcg tgcggactgg accacctgct gatcgcggtc 55200
   ggcgacgaac cggagttcgg cgatccggcg ggctggacgc cggcgtcacc ggaacgtgcg 55260
   accatcgcgc tgttgcagta cacgtccggg tcgaccggcg acccgaaggg agtcgtggtc 55320
   acccacgaca acatcctgta caacttggat gcctgcgtcc ggggcctgcg ctggccggac 55380
   gactggcggg tgggcgggtg gcttccgctc taccacgacc tggcgatgca ggggctgctc 55440
   aacatggccg tcgtgcgcgg cggctatgcc ttgctgatgg agccggtctc gttcgtgcgc 55500
   gatccggtgc gctggctgcg gacgatagcc gaacacgaca tccaggtgac gttcgcgccc 55560
   accttcgcgt accaactgtg cctcgaccgg gtcaccgacg agcagctggc cgctctcgat 55620
   ctgtccggct ggaagatcgc cgggaacgcc gcggagccgg tgaacccggc gatcctggcc 55680
   gcgttcgctg agaagttcgc gccggccggg ttccgccccg agtccttcgc gcccatctac 55740
   gggatggcgg aggccaccgg ctacatcagc ggggaggtgg ggcgtgcgcc gctggtgcgt 55800
   accgtcggcc tcgacgcgct cgcccatggc cggctcgccg acccggcgcc ggacgagccg 55860
   gtacgcgaaa tcgtcagctg cggcactccg aacgaggcgt gcgacatccg tgtggtggac 55920
   ccggagacgt cgcgcgtgcg gcccgacggg tggttgggcg agatctggat acgcgggcgc 55980
   agcgtgtccc cgggctactg gagcgatgcc gggccggcct tcgccgcggt gacggccgag 56040
   ggggaggacg gcttcctgcg taccggcgac ctcggcgtgc tgcaggacgg tgagctgtac 56100
   gtgcacggcc ggctcaagga gacgttcacc gtgcacggtc gccacctcta cccgcacgac 56160
   gtcgagcagg aactgcgcgc acggcacccg gaactcggca agtgcggcgc ggtcttcccg 56220
   ggacgggctc ccggcgccgg cggcgcgcgg ggcgtggtcg tgacccatga ggtgaccaac 56280
   gcggcgcggg atcggctgcc ggagctggcc gccgggttac ggcataccgt ggggcgggca 56340
   ttcggcgtcg aggtctccgc ggtgctgctg ctacgtccgg gtgccgtgct gcgtaccacc 56400
   agcgggaaga tccgccgctc ggccatgcgt gagctgttcc acgagggaaa gctgaccgct 56460
   ctctaccagc atcctccggc gccgcgggag ccgctgccct acgtggggga tccgtcgccg 56520
   gcgaccaggg tgaccagcct ttcgcgtccg tgacgggtgt cgccgggatc cggaccgacg 56580
   cggttgccgc tgtggcggcg gcgcgcgggt gactcccacg cgatggcgcg gggcgtccgg 56640
   ccgggcatga cacgcaccct tcggtctcgg cggcgtggcc cgacggtgcg ccggcccgtg 56700
   gcgcggagat cgtcgaggaa cccggggcag cgggggtacg cacccgtctg accggcacgc 56760
   tgggaccgac tcgccgcggc agcgacgccg cggcgagtcg ctgtccgggc accggtaccg 56820
   cggccgatct ccgtacccgg gtctgtcctg ggcacgtcca tcgcggccgg ctcggccggt 56880
   tccgggcccg ccgcgtcatc tgatgcgaga gaaggagact gtcatggtcg ttgtgatggc 56940
   gccggaggcc acttcggcgg atgtggacgc ggtcgtccgg ctggtcgagt cggtgggcgg 57000
   gcaggcgttc gtcagccgcg ggctgtcccg gaccatcgtc ggcctggtcg gcgacgtggc 57060
   cgacttcgag gcgctcaacc tgggcagcct ggcgggcgtg ctgcgggtga tccgggtgtc 57120
   ggtgccgtac aagctggtca gccgggagca ccacccggcg cgctcggtga tccgggccgg 57180
   cggcgtgccg atcggtcccg gcacgctgac cgtgatcgcc gggccgtgcg cggtggagtc 57240
   cccggagcag acgctggagg cggcccggat ggcgcgctcg gccggggcct cgatgctgcg 57300
   cggcggggcg ttcaagccgc ggacctcacc gtactcctac caggggttgg gggagcgggg 57360
   cctgcgcatc ctggccgacg tgcgggccga gacggggctc gccgtggtca ccgaggtgat 57420
   cgatcccggc agtgtggaca tggtcgcgtc gtacgccgac atgctccaga tcggcgcccg 57480
   gaacatgcag aatttcgcgc tgctgcaggc ggtcgggtcg gccggcaaac cggtgctgct 57540
   caagcgcggg ttcagcgcca cgatcgagga atggctcagc gccgccgagt acatcgcgca 57600
   gcgcggcaat ctgaacatcg tgttgtgtga gcgcgggatc cggacgttcg agaccgctac 57660
   ccgtaacacg ctggacatca gcgcggtgcc ggtcgcccag cggctctccc acctgccggt 57720
   gatcgtggat ccgtcgcact ccggcggccg ccgcgatctg gtggtgccgc tgacccgcgc 57780
   ggcgatcgcg gtgggcgcgg acgggttgct catcgacgtc cacccggagc cgaagaccgc 57840
   tctgtgcgac ggtgaccagg cgctgccgga agccgacatc cgggagatcg ccgacatcgt 57900
   ggcgagcctg cccgcgttcc tggggcggga gccggccgtg ccctcgggtg aattccgaca 57960
   cgttcccgcc gcggtgtgag ctgtggcggc cggcccgtcc accgcatgat cgcccggcgt 58020
   gtcagcgggc cgttcggccc cgcgcggacc ggcctgtatg ccctgctcag aggagtggat 58080
   gaaggacttc cggcgatgac tgcatgatcg cacccggtgg gttcattggg cacaaagggt 58140
   agtgagattg gcattccggg ctaggggtta ggggccggtg tcgctgcgtg agacaggggt 58200
   tacagagcgc tgaaaagcga tgttaatttc gtgccgctgg aagacgatct acccaccatg 58260
   ccgcgctctc gaaccgcgca gcatgttcgc gtcgagcacg gggagaagag tcatggtcac 58320
   tggctgattc cggatgggct gtccgcctga agacgacgcg acccgcatca ccgccgtggt 58380
   cgctgtggac cggctaccgg ttcgcgctcg catacggaac gccataacag ccgagcgttc 58440
   aatttcctgt tcgcccacca ggctcatgtg cgccgctgcc tgcggtgccg gcaggccgga 58500
   ccgacggtct cggccatgcc gcgtaccgcg ctgcgcggac ccgcccaagc cggtcgccgg 58560
   tcccgggcct cgaatcgcac gcaccgtcgt tccccggagc ggcggtagcc cggcgtcgag 58620
   gcccgcaagg cgtaccgcat gagccggctg ggctccctgt cgtgcccgcc ctcatcgggg 58680
   ccgcccgcac gcacgtgcgt gtcggcggtg cctccagcgc gcgcgtcacc agcttaggag 58740
   ctgcattgac acctgacgaa gaggccctca accggcagcc catcatggag atggagatca 58800
   gttcactcag cctcggcgga tcgccccggc tcgccggggg cgatccggtg cacctggagg 58860
   ccatggtcgc ggcgcagggg gaactgccgc cgatcgtggt gcaccgcccg accatgcggg 58920
   tcatcgacgg ctcgcaccgg atccaggcgg ccctccggcg tggggagacg acgatcgccg 58980
   gtcgtttctt cgacggctcc gacgacgagg cgttcgtgat gtcggtgtgg ctcaacgtct 59040
   cgcacggcct gccgctggcc ctggccgacc gcaaacgcgc ggccgaacgc atcgccgtct 59100
   cgcatccgca gtggtcggac cgccgcgtcg ccgcggtgac cggtatctcg ccgagcacgg 59160
   tggccgacat ccgccggcgg gtggccggca cctcggcgcc cgaggccagc cggatcggtc 59220
   aggacggccg ggtccgcccg ctggactgca gcgcgggccg gctgcttgcc gggcggctga 59280
   tggccgagaa tcccgccctg tcgctgcggc aggtggccaa agccgcggcg atctctccgg 59340
   agacggcccg cgacgtccgc aatcggctgc tcagcggcgc tgagctggtg cccaaccggc 59400
   gcccgcgaga cgccgcgccg gtgggggtga agggcggccg ggaccggcgc ccgctgaacc 59460
   tgatccgctc cggagaccgg ccggaacccg tccccgacca cgccgtcgtc atcaaccggc 59520
   tgatgagcga cccggcgctt cggtacaccg ataccggccg taacctgctc cgccttctga 59580
   gcctgcacac ccgctgggcg aaggagtggg aggccatcgt cgacaacctg ccgccgcact 59640
   gcgccgacgc ggtggccgac ctggctcgcc agttcgcgga cctgtgggcc gacttcgcca 59700
   gccgggtcgg cccggaggag cggatggcga gctgaccgcg cgccgcgccc ggacagtacg 59760
   ggcgccggcc ccggctgcgg catccccagc gtgccgcccg gtgccggggc cgcggcgtcc 59820
   accgtctcgc ttctgccacc gtgccgcgcc gggtcgcttc tgcggccgtg ccggaccgcg 59880
   ccgcttctgc cgttccggcg aaccggcccg cttctgcggc cgtgccggat cgggccgctt 59940
   ctgccgcctg tccgaccgcg tggcttctgt cgccccgccc ggccgcctgg gttctgccgt 60000
   cctgtccggc cgcctgggtt ctgccgcctg tccgaccgcg tggcttctgt cgccccgccc 60060
   ggccgcctgg gttctgccgt cctgtccgac cgcctgcttc cgccgccgtg tccaaccggc 60120
   cggaaccggc ggttggacac ggcggcaagc ctcggccggc gccgggtgtc ccaccaatag 60180
   gccatcgaca aggggctata tcacagtcgt aaagtttttg aagtcgtggg ctgaactatg 60240
   tcacaggcaa ctttgagctc gctccgtccc ccgggggagg ggttctgagc tgcggaaaat 60300
   cgtcggttgt ccgatagtga tcggcctttc cttcgttcgg ggcgcttcgg tagggttcga 60360
   tctcgtgtgg tagcgggatt gctcggccgg gggggcccgt gacactgcgt aaacatcagt 60420
   tggaattgct cctgttcgac gaggttcacc gggcatcagc gcgtggcgaa ggtgcggtgc 60480
   tgctcgtcag cggcgcggtg ggcaccggga agaccgcgct gctgcaggcg atggccgcgc 60540
   gtgccggccg ccggggcggc tggtcgttcg tggtcaccgg ttcggcccgc gagcgcgggc 60600
   acccgttcgg cgtgctggac cgcctgatcc ggtcgatgtg cgcggccggg atggccgagc 60660
   cgttcccggg cggcatggac tgcggcgaga acttcttcgt gatgatggac cgggtgggcg 60720
   ccgcgatccg ggagttcgcc ggcgaccggc cggtgctcgt cggtatcgac gacgttcact 60780
   tcgtcgacga gcagtcgctg cgggccgtca gctacctcat ccggcggatc gagtcgagcg 60840
   gtgtggtgct ggtgctcaac gagagcacct cgtacgagcg tgacatggcc ggtctgcgcg 60900
   ccgagatgct gcacctgccg ttctgctacc ggatccggct caccccgctg acctccaccg 60960
   aggtcgccga gcggctgagc gagcggttcg gcggcgtgcc cgaccgggcg tccgtgcaat 61020
   tcggcgtgca ggtcagcggg gggatcccgc tcatcctgca ggccctgatc gacgatctcg 61080
   ccgactccgg cccgggcgtg tgcgagccgg gcgccaactt ccgccaggcc gtgttgcgca 61140
   gcctgcaccg gtgcgcgccg tcgaccaccg cggtggccca ggccatcgcg gtcctcggcg 61200
   actacgcggc gccggagctg gtcgccgagc tcggcggtgt ggacgccgcc ctgatcgagg 61260
   agagcatccg cgacctgcgg gagatggccc tgctggagca cgactggttc cggcacccgc 61320
   acacccgggc cgcggtgctg gccggcatcc cggtgccgca gctgcccgag atgcaccggc 61380
   gcgccgcgga gctgctgcat cagagcgggg cgccggccgg cgccgtggcg gagcacctga 61440
   tcgccgcgca ggacggcggc cgggcgccgt ggcgggtggt catcctgtcg gaggcggccc 61500
   gcgaggcgct ggccgccggc gacgtggaca gcgcggtgct gagcctgcga catgccgtcg 61560
   cggccagctc cgacgagacg cagcgggcgc acgcgggcgt gctgctcgcc gaggcgcagt 61620
   ggcacaccga ccccagccgg gcggcgcgcc gcctgggcga gctgagccag gacgcgcggg 61680
   ccggccggtt caccggcccc gacaccctga tcgcggtcaa ccagctgctc tggtggggcg 61740
   agttcgccga ggccgacgag ctgctgcggc tggccgggga cgaggagtgc ggcgactcca 61800
   gcctcgctca cctgtgggcg atcttcagcc gggccgccgg ggggacggag atctgcgacg 61860
   agcacacccc gtccggcggc tcgccgctgg cgcgatccgg tccgatggcc gtggtcacct 61920
   acctgagctc ggcggcgagc ctggcctccg agagcgtggc ggccgatcgc accgagatgc 61980
   tgctcggcat ccgggccggc gccccgctca ctcccgccct gtacgccctg gtcgtcctgg 62040
   tgcagtccgg ccggctggag gaggcgatca cctggtgcgg tcgcctgctg aaggaggagt 62100
   ggatccaccg gacgccgatg cggcgggtga tgatccagac gatcgagtcg gtggccatgc 62160
   tccgggccgg cgacagcgcg ggcgccctgc ggggcatccg ggaggtgttc gacgccgttc 62220
   cgccaccggc gtggggcgtg gtggcgggcc tgccgctgtc ggtcgcggtg cgggccagca 62280
   ccgaactggg cgacacccat gccgcccggt cgtacctggc ggtcccggtg ccaccggcga 62340
   tgttcgacac gccgttcgcg ctgccgtacc tgctggcgct cggctggtac cacctggcca 62400
   tgggccaccc ggagagctcg ctgaggcaca cccggtcgtg tctggacctg accgcgcgct 62460
   ggggcgtcga cgcggggcgg atcaccgccg ggccggccgg gctggacgtg tcccggccgt 62520
   cgccgccacc ggccgcggac cgggtggacg cgggccaccg gaccccggcc gcggtccggg 62580
   aaccggcgcc ggatcagcgg gaggccacgc cggtgccgcc gcacgccggc ccggacccgg 62640
   cggagcccgc ggcggcgcac cgcggagccg aggacggcgc caggctcacc gacgcggaac 62700
   agcgggtggc cgcgctggcc gccgccggca acaccaaccg gcagatcgcc gagcgcctgt 62760
   tcatcaccgt gagcaccgtc gagcagcatc tcacgaaaat ctatcggaag ctgaatctgc 62820
   gcagccgttc cgggttgcgg cgatacagcc actgatcatc ccgcggcggg cgtgtcgccg 62880
   attccgcagc cgatcccggg acgggcggtt ggacgcggtt tgacgtccat gaattggcgt 62940
   ccataccgtc gcggtgtcgg ttcttcgaaa gattctcgga ggtcagcgat gtccgcggaa 63000
   aaggtcaagg aactggtacg cagtaagcgc cggatgtggg ggtggatgta ccgatgacgg 63060
   ccaccctcga acgcgcttcc gcctcgcccg tcggcatccc gccggccatc tcccggatgg 63120
   tcggggtggg caccgccgtc gccgggacga cggtctccca gccggcattg ctggatcttc 63180
   tcggcatcga ggacccgaag atccgctcga tctacctgaa cagcgccatc ggcacccgcc 63240
   gcctcaccct gccggagccc gacgcgtccg gcgtgcgccg ccccgagccg cagggcgcgc 63300
   tgctggccaa gcacaagcgc ctcgccgtcg agatggcggg gcaggcgctg caggcctgcc 63360
   tcaagaacat cggcgcctcc ctctccgacc tgcggcacct gtgctgcgtg acgtcgaccg 63420
   gcttcctcac cccgggcctg agcgccctga tgatcaagga actcggcatc gacccgcact 63480
   gcagccgctc cgacatcgtg gggatgggct gcaacgccgg cctcaacgcg ctcaacgtcg 63540
   tcgccggctg gtccgccgcc caccccggcg agctcggcgt ggtcctgtgc accgaggcgt 63600
   gctcggccgc gtacgccctg gacggcacca tgcgcaccgc ggtggtcaac agcctcttcg 63660
   gcgacggcgc cgccgccctg gcgctgatca gtgacgctcc cgggcagtcg aggcccggac 63720
   cgcggatcct caagttcgcc agcttcctga tcaccgacgc gatcgacgcc atgcgctacg 63780
   actgggacag cgacctgggc cggttcagct tcttcctgga cccgcagatc ccgtacgtcg 63840
   tcggcgccaa cgcggagacc gtggtggacc gcctgctcgc cggcaccggc ctgcgccgcc 63900
   gtgacatcgc gcagtggctg gtgcactccg gcggcaagaa ggtgatcgac gcggtcgtgg 63960
   tcaatctcgg actgacccgc cacgacgtcc ggcacaccac ccggatcctg cgcgactacg 64020
   gcaacgtctc cagcggctcg ttcctcttct cctacgagcg gctggtcgag gagggcgtca 64080
   cccgccccgg agactacgga gtgctgatga ccatgggccc cggctcgacc atcgaaacgg 64140
   cgctggtcca gtggtgaccg cggccggcgt cgacgacctg gtgctgcgga tcgacgggag 64200
   cgagccggtc acgccggcca ccgtcgaggc ggtgctgggc atctgtgatc gggccgagga 64260
   cgggtacgcc gccgggctgc tgaccgtcca cgtctcgggc gtcccggctc ccggctggac 64320
   gagcgacctc agcgtcggcc tgacgaccaa gtgggagcgc gccgtccgcc ggctggagcg 64380
   cctcggcctg ctcaccgccg gggtggccac cggcgactgc ggcggcgcgg cgctggacct 64440
   gctgctggcc accgacgtgc ggatcgccgt cgccggcgcc cggctggtcg tcccggtgag 64500
   cggcgacgcg acctggccgg gcatggcgct gtaccggctc gcccggcagg gcgccaccgc 64560
   cgggctgcgc cgggccgccc tgctcggcac cccgctcgac gccgctcggg cggtggcggc 64620
   cggggtgatc gacgtggtgg tggaccggcc cgagccggcg ctcgccgaga tcgccgccgc 64680
   ggcggccggc atcaccggca ccgagctggc catccggcgg cagctgatgc tcgacgccgc 64740
   ggagcacagc ttcgaggagg cggtcggctc gcacctggcc gcgtgcgacc ggaccctgcg 64800
   ccgggcggcg gcgtcatgac ggtgaccgcc gtgccgcgcg acctcggcgc cgaccgcaag 64860
   cgcctggccg aggccgcggc ccgcgccgac cggctgatcg cggcgctgcc cccgcccagc 64920
   cggcggacgg cgcagcagcg ggccgaggcg gcggccgccc acgacgcggc gcgccgcctg 64980
   cgggccgggt tcctgaacgc gtacgccgac cgcgtctacg acgagctcac cgaggaccgc 65040
   acccggttcc tgcgcatcga cgagctggcc gaggccgcgg cgtacgcgtt cccgggcctg 65100
   gcgcccaccg cgcagcagct ggcccgggaa cgctcccgcc cccaggccga ccaggagggg 65160
   ctggagatcg accagggcat cctgctgcgg gcggtgctgc ggtcgccgcg ctcgggccgg 65220
   cacctgatgg aggcgatgct gcgccccacc ccgcgggcgt tgcggctgct cgcggagttc 65280
   cggcgcaccg gcgtgctgca gaccgaggcg gtccggctgg agcggcacga gcaggtggcc 65340
   gagctcaccc tgtgccgcga cgactgtctc aacgccgagg acgagcagca ggtcgacgac 65400
   atggagaccg ccgtggacct ggccctgctc gacccggccg tacggatcgg cctggtccgc 65460
   ggcggcgtga tgagccaccc gcggtaccgg ggccgccggg tgttcagcgc cgggatcaac 65520
   ctcaaggcga tcagcgccgg ccggatctcg ctgaccggct tcctgctccg ccgcgaactg 65580
   ggctacctgc acaaactggt gcgcgggttg tccgacgacg acccgcagcg gtggacgccg 65640
   ccggtggaga agccgtgggt ggccgctgtc gacacgttcg ccatcggtgg cggctgccag 65700
   ctgctgctgg tcttcgacca cgtgctggcc gcctcggacg cgtacctgag cctgccggcg 65760
   gcccgggagg ggatcgtccc gggcgccggc aacctgcgtc tgggccggat cgccggggcc 65820
   cggctgtcgc gccaggtcat cctggccggc cgccggatcg ccgccgccga gccggatgcg 65880
   cgcctgctgg tggacgaggt ggtgccgccg gaggagatgg acgaggcggt gcggcgctgc 65940
   ctggcccggc tgtccggcga cgccgtggtg gccaaccggc ggatgatcaa cgtggccgag 66000
   gagccgctcg acgcgctgcg cgtgtacctg gccgagttcg cgctcgaaca ggcgctgcgc 66060
   atccacgccg gcgacgtgat cgacaaggtg ggccggttcg cggagcggtc ggcgtgaccg 66120
   ccgggcggca tccggccgcg accgggtgtc cagcgtgacc gcggcccggg tgcggtacca 66180
   caggaccggc cacgtcgccc gggtcacgct cgaccgtccc gacgtgctca acgccatgga 66240
   tctgcggatg cacgaggaac tggcgcggat ctgggacgac atcgaggccg acgaccggat 66300
   ccgggtcgtg gtgctgaccg gcgccgggga ccgcgcgttc tcggtgggcc aggacctcaa 66360
   ggagcgtgcc cgccgcgacg cggcgggcac cccggggtcc agtttcggca gccgcggcca 66420
   gcccggctgg ccgcggctca ccgagcggtt cgggttcagc aagccggtgg tcgcgcgcgt 66480
   cgacgggtac gccctcggcg gcggcttcga gctcgcgctg gcctgcgatc tgatcatcgc 66540
   atcggaccgc tcggtgttcg ggctgccgga ggtgcggctc gggctcgtgc cgggggccgg 66600
   cggcgtgttc cggctggtcc ggcagctgcc gcagaagacg gcgatgggct acctgctgac 66660
   cggccgtcgc ttcgacgcgg cgaccgcgct ccggttcggg ctggtcaacg aggtggtgcc 66720
   ggcgcagcgg ttggacgctt gcgtggacgg ctgggtcgat gatctcgtca caagcgcgcc 66780
   actgtcggtc cgggcgatca aggaagccgc gctgcgctcg gtcgacctgc cgctgccgga 66840
   cgccttcacc gccacgtacc gctgggagca gcggcgccgc gccagcgcgg acgccgtcga 66900
   ggggccccgg gcgttcgccg agcgccgggc gccgcgctgg tccggcacct gacgtgcacc 66960
   ggcccgttcg atcgtgagga gcgccgtccg atgtccttcg aagcgccgcc gtcgtcactg 67020
   ggcggccatg ccctgctcgc tttcctgatc gcggtggcga cgctgctgct ggtcgcccac 67080
   ctgctggccc gcctggcgga acgggtgcgg atgccggccg tcgtgggtga gctggccacc 67140
   ggcgtcatcc tgggcccgtc cctgctgggc cacctgctgc ccggactgac cgactgggtc 67200
   ttcccgcccg gcaccgacca gatgcacctg ctcgacgcgg taggtcagat cggcatcctg 67260
   ctcctggtcg gactcaccgg cacccacctg gacgtcgcca tggtgcggcg gcgcaaggcc 67320
   accgccgcgc ggatcagcct gggcggcctg ctgctgccgc tggctctcgg ggtcggcgtc 67380
   ggctaccagc tggccggctg gctcagcggc gccgtggtcg gtgaccggct ggtcttcgcc 67440
   ggattcatgg gcgtcgcgat gtgcgtgaca gccatcccgg tgatcgccaa aaccctctcc 67500
   gacatgcggc tgctgcaccg cgacgtgggc cagatgacgc tggcggcggg caccatcgac 67560
   gacgcggtcg gctggttcct gctgtccgtg gtgtcgatgg ccgcgaccgc cgggctcacc 67620
   accggacacg tcgtccaggc ggtcgcctac ctgctcggct tcgtcgtcct cgcggttctg 67680
   ctcggccggc cgctggtccg ccgcgtcatg cggctggccg accgcggcga gggatccggc 67740
   tccagcatcg tcaccgcggt cgtcgtcgtc ctgaccggcg cggcgatcac ccaggcgctg 67800
   ggcatggagc cggtcttcgg cgccttcgtg gcgggcatcc tggtcgggct gcccgcggcg 67860
   gcgaaccagg cgaagctggc ggcgctgcgc acggtcgtgc tcagcgtgct ggcgccgctc 67920
   ttcctcgcca cggccggctt ccggatggac ctgaccgcgc tggccgaccc gcaggtcgcg 67980
   atcgccgcgg tgctcgtgct ggccatcgcc atcgtcggca agttcgccgg ggcgtacgcc 68040
   ggggcccgcc tcagccggct gtcccgctgg gagggcctgg cgatcggcgc cgggatgaac 68100
   tcgcgcggcg tggtcgaggt ggtggtggcg ctcaccggcc tgcgactggg cgtgctgaac 68160
   accgccacct acacgatcgt cgtcctggtc gccatcgtca cctcggtgat ggccccgccg 68220
   ctgctgcgct acgcctgtgc ccgcatcgcc cagaacgagg acgagcggct gcgcaagctg 68280
   caccacgaca cctggaacgg cgtcccggcc ggcgccggcc agtccccctc acccgcagga 68340
   gagcaggcat gaccgtcacg gcgaagcacg tcgggaacac ccggccggcg cccgacctgg 68400
   cgaccgcgac cgcggccgcg gcgaccttcc tcgacgcgct cggcgtcgac ctgagcccgg 68460
   agagcctgca ggagaccccg gcccggatgg cccgggcgta cgccgacctg ctcaccgccc 68520
   gcgagttcaa gctcaccacg ttcgacaacg acgagggcta cgacgagctg gtgatggccc 68580
   gcaacatccc gatccgctcg gtgtgcgagc accacctgct gcccttcatc ggcgtcgcgc 68640
   acgtcggcta cctgcccgcc gcccgcatcc tcggactgtc caagctggcc cgggtcgtcg 68700
   agatgttcgc gcacggcacc caggtgcagg aacggctgac caagcagatc gccgactggc 68760
   tgatcaagga gctggagccg aacggggtgg gcgtggtgat cgaggccgag cacctgtgca 68820
   tgacgttgcg cggggtgcag gcggtcggcg cgaacaccat cacctcgtcc atgctcggcg 68880
   tgctgcgcga cgacgcgcgg tcgcgcggcg agttcctggc cgccgccggg ctgggcgggc 68940
   gctgagccgg ccggtcgccg gtgtgctttc cgggcgcgcc gccggccggg cgcagtcgac 69000
   agtcagttgg acagattgtt tcgccggctc cgcgcgtgtg atcgtggccg caccgggagc 69060
   tccgttggca ggaggaaacg ccgtggaatc gtctgcgctg cgcaagtcgg aattgcatcc 69120
   gagcatttcg gacccggttc tggacacgat gaatttcctc aacgaggtga ccctgcgcta 69180
   tcccgaggcg atctcgttcg cgccgggccg cccgtacgac ggcttcttcc agaccgagga 69240
   ggtcctcgcc cacctgcgcc gctttctcga ccacctggcc gaccagggcg cctcgcccgc 69300
   gcaggtgcgc acggcggtct accagtacgg cccgaccgcc ggccagatcc gcgagatcat 69360
   cgccgactcg ctgcgccggg acgaggggat cgacgtgccg gccgagtcca tcgtggtcac 69420
   cgtcggcgcg caggaggcga tgatgctggt cgtccgcgcg ctgatccgtg acccgcggga 69480
   cgtgctgctg gtgcccagcc cctgctacgt gggcatcacc ggggtggccc ggctgctcga 69540
   cgtgacgctc ggcccggtgc ccgagtcgcc ggacggcttc tccctggaga acgtggcggc 69600
   ggcgatccgg gcgcagcggg ccgccgggcg ccggccgcga gccttctacc tggtgcccga 69660
   ccacgccaac ccgtccggca ccacgctgtc cgagtcggac cgggccgggt tgctgcacct 69720
   ggccgcgaag gaggatctgc tgatcctgga ggacaccccg taccggctgg tcagccccgg 69780
   cgagccggtg cgcacgctga aggcgctgga ccgccgccgc tcggtggtgc acctggggtc 69840
   gtactcaaaa acgatctttc cgggcgcgcg ggtgggcttc gccgtggccg accagacggt 69900
   ggtcggcgac gacggccgga ccggcctgct cgccgacgag ctctcgaaga tcaagagcat 69960
   ggtgacggtc aacacctcgt cgctgagcca ggcggtggtg gccggcgcgc tgctcgcggc 70020
   cggcggccgg gtctccgagc tgaacgccaa ggcggccggg cactacggcg acgccatgcg 70080
   ccgggtgctc gaccggctcg ccgaccggtt gcccgcggcg cggcgcgcgg agctgggcgc 70140
   gcggatccgt accgacgagg cggggacgac gggcgcgtaa tcgaaaagaa agggccgcgc 70200
   tggccgcggg tctcgtcgag ttccaggagg agaagaccga tgagtaatcg agcgggggag 70260
   tgacgatgac cggccacttc caggacctga ccgtcgacca cgtacggatc tactgcgcgg 70320
   acctcgaccc gttgatcgcc caattcggca gctacggcct cgacgtgcgg gccgagggcg 70380
   tcgggccggg cgcggagcac tcggtcgttc tcgggcacgg cgacatccgg ctggtgctga 70440
   cccggcccgg caccggcgac caccccggcg ggatgtacac cgcgcagcac ggctacggcg 70500
   tgtccgacat cgcgctcggt acggccgacg cggccggcgc cttccacgag gcggtccggc 70560
   gcggggcacg gccgatcgcc gcccccgagc ggaccgccgg cgtggtcacc gcctcggtcg 70620
   ccggcttcgg cgacgtcatc cacaccttcg tccagcgcga gccgggagga ccgtggtcgc 70680
   tgccggggct gaacccggtg caccggcccg gcacgcccgg catcggcctg cggctggtcg 70740
   accacttcgc ggtgtgcgtc gaggccggcc ggctcaccga ggtcgtcgag cactacgagc 70800
   gggtgttcgg cttctcggcc atcttcaccg agcggatcgt ggtcggcgag caggcgatgg 70860
   actcccaggt cgtgcagagc gccggcgggg ccgtgacgct caccgtgatc gcgccggaca 70920
   ccacccgccg gccggggcag atcgacacct tcctcaagga ccacggcggc ccgggggtcc 70980
   agcacatcgc gttcgagacc gacgacgtga cccgctcggt cggcgccatg agcgacgccg 71040
   gcatcgagtt cctcaccaca ccggccgcct actacgagag gctgcgggac cggctgcagc 71100
   tcacccggca ctcggtcacc gagctgagcc ggctcaacgt gctcgccgac gaggaccacg 71160
   acggccagct ctaccagatc ttcaccaagt ccacgcaccc gcggggaacg ctgttcttcg 71220
   agatcatcga gcgggtcggg gcccggacct tcggctccgg caacatcaag gccctgtacg 71280
   aggccgtgga gctggaccag gccgcggcag acggccgatg accaccgctg tgctgcggct 71340
   ggacacggtc gacgatttcc ggctgcgcgc cgaggcgacg ctgaccgccg cggtccgtga 71400
   cttcgtggcc ggcggcagcg actcggagac caccctggcg gccaaccggg cggcgctgga 71460
   ccgggtgacg atcgtcccgc gggtgctgac cggcgggccc gacccggaac cgggcgccac 71520
   gctggccggg gcccgcagcg cgctgccgct ggccgtggcg ccgatggcgt accagcggct 71580
   gttgcacccg gagggggagc tggcgacggc gcgcgccgcg gcggccgccg gtgtcccctt 71640
   cgtggtcagc acgctcagca gcgtcccggt cggcgagctg gccgcggccg gcggcgaaca 71700
   gtggttccag ttgtactggc tcaacgacga ccgcgacacg atgaacctgg tccaccgggc 71760
   cgaggacgcc ggctgccggg tgctgatggt gaccgtggac gttccggtga tgggccggcg 71820
   cctgcgggac atccgcaacg agttcgtgct gcccgccgag gtgcgggcgg cgaacgtcgc 71880
   gtccggggcg atgagcgcgg cgcacgatcg cgccgacgcc ggctccgcct tgatcgcgca 71940
   caccaaccgc gcgttccatc cggcgctcac ctgggcgcac ctggaggcgc tgcgctcgcg 72000
   gaccaccctg ccgatcgtgg tgaagggcat cctggacccc gcggacgccc gccgcgcggc 72060
   cgagatcggc gcgaccggcg tggtgatctc caaccacggc ggccgccagc tcgacggcgc 72120
   gccggccagc gtcaccatgc tgcccgccgc cgtcgcggcc gtcccggaca cctgccaggt 72180
   gttcgtggac agcggcatcc gcagcggcac cgacatcctg cgggcgctgg cgctcggcgc 72240
   gcacggtgtg ctgatcgggc ggccgatgct gtgggggctg gcggcgggtg gcgagaccgg 72300
   cgccgccggg gtgctggccg tgctggacgc cgagttgcgc gcggcgatgc ggctggccgg 72360
   ttgcgccgac gtcgcggcgg cccggcggct gaccgccacc accgggtgct gatcggccgc 72420
   gacgagacgc cgaccggccg cgttggacgc tgaccggccg cgacgagacg ctgaccgcct 72480
   ggtcgcggcc gggtgcccgg ctgccggcgg gtccgtcggc cgtccggagc ggcggagggg 72540
   gagaggaatg cccgtacctt ggttcagcgc gccgcgcccg ctcgcggccc cacggttgcg 72600
   gctggtctgt ttcccgtacg ccgggggcaa cgccgccacc taccgctcct gggccggact 72660
   cctgccgccc ggggtggagc tggtcgccgc gctgctgccc ggccgcgccg agcgcctcga 72720
   cgagccgccc ctggtggatc tggacgtgct gctgtccgag ctggtggcgg cggccggccc 72780
   gctgctcggc ccggtcccgc tgatcctgtt cggacacagc ctcggcgcga ccgtcgcgta 72840
   cgagttcggc cgcgccctgg ccaccgagca cggttgcgtg cccgcggccc tgctggtctc 72900
   cggcgccccg gcgcccccgg tgcgccggcg ccgcaccggc cgcggcctct ccgacgacga 72960
   cctcgaacgg atcctgggca aacgcaccga tctcccggcc gggtggctga ccgccgagct 73020
   caagccgttc gtgttcccgg ccctgcgggc cgacctgcag ctgctcgacg gctaccgctg 73080
   gcggcccggc ccgcttcccg gcgtgccggt ggtcgccttc ggcggggatg ccgatccgga 73140
   cgtgtccgcc gccgatctgc gggcgtggca gcggtgcacg accggcccgg cccgcaccca 73200
   cgtgctggcc ggcgaccatt tcttcatcgc gcaccaaccg ttccgcgagc ttctcgccgc 73260
   cacggtgggc gagttcgccg ccagcccgat cgcccggtga gccggcagcg ggccggctgg 73320
   cgcgtccccg gtcacctccg ccgccggccc cgtgcgacga ggcggcagcc gtcccgcgga 73380
   tcccgggacg gctgccggcc ggcggctgct agaggcagtt cgtcacggtg tagcggttcg 73440
   tcggcgtcga cgggaaccag tccggagtga tcgcccaggc gccggcggtg ccgggggtga 73500
   tggtcgcgca ccagccgaca taggtgaagg tgccgctgtt gttgacggcg gacgaggccg 73560
   gcgtcacctt cgcgttcgtc gtgccgcagt tgttgtaggt cacgtagatg gtcgtgcccg 73620
   tgccgttggg ctgctcgacg gaggtccagc agggcaccgc cgcggtcgcg gcgcggagct 73680
   cgacgtgcga accggcatcc ggcttctccg tcgacggact ggcggtcgcc gctccgccgg 73740
   tgacgagtgt ggcggcgagt cccagcgtgg ccactgcggc gccgacaacc ttgaggttcc 73800
   tcatcttgga catgacaatc accatcgtcg atgcttcgat cgcacggaag gcactccagc 73860
   ggtccttggg aggcacattc gg 73882

<210> 2

<211> 447

<212> PRT

<213> Actinoplanes teichomyceticus

<400> 2

Met Ile Pro Leu Ser Leu Glu Ala Ile Ala Ala Val Val Gly Gly Thr
1               5                   10                  15

Val Glu Gly Asp His Thr Val Thr Val Thr Ala Pro Ala Val Leu Asp
            20                  25                  30

Gly Arg Gln Ala Glu Pro Gly Gly Leu Phe Val Ala Phe Ala Gly Glu
        35                  40                  45

His Val Asp Gly His Asp Tyr Ala Gly Gln Ala Gly Gln Ala Gly Ala
    50                  55                  60

Val Ala Val Leu Gly Ser Arg Pro Thr Ala Leu Pro Thr Val Val Val
65                  70                  75                  80

Glu Asp Ala Arg Thr Ala Leu Gln Ala Leu Ala Ala Asp Val Val Ala
                85                  90                  95

Arg Leu Arg Glu Arg Leu Thr Leu Val Gly Val Thr Gly Ser Gln Gly
            100                 105                 110

Lys Thr Ser Thr Lys Asp Leu Leu Ala Ala Val Leu Ser Ser Ala Ala
        115                 120                 125

Pro Thr Ile Ala Thr Lys Gly Asn Leu Asn Asn Glu Leu Gly Val Pro
    130                 135                 140

Leu Thr Met Leu Arg Ala Gly Ala Ala Thr Arg Phe Leu Val Val Glu
145                 150                 155                 160

Met Gly Ala Arg Arg Ile Gly Asp Leu Ala Lys Leu Thr Asp Leu Val
                165                 170                 175

Pro Leu Asp Val Ala Val Val Leu Asn Val Gly Glu Ala His Leu Gly
            180                 185                 190

Val Phe Gly Ser Arg Ala Ala Ile Ala Glu Gly Lys Gly Glu Leu Val
        195                 200                 205

Arg Gly Leu Val Pro Gly Gly Thr Ala Ile Leu Asn Ala Asp Asp Pro
    210                 215                 220

Arg Val Val Ala Met Arg Ser Leu Thr Asp Gly Pro Val Leu Thr Phe
225                 230                 235                 240

Gly His Ala Glu Asp Ala Asp Val Arg Val Arg Asp Leu Thr Leu Asp
                245                 250                 255

Arg Leu Gly Arg Pro Ser Phe Thr Leu Arg Thr Ala Asp Ala Thr Ala
            260                 265                 270

Arg Val Lys Leu Pro Ile Val Gly Ala His Gln Ala Leu Asn Ala Ala
        275                 280                 285

Ala Ala Val Ala Ala Ala Leu Ser Ala Gly Leu Thr Leu Asp Ala Ala

290                 295                 300

Ala Ala Pro Leu Ala Thr Ala Ser Leu Thr Arg Trp Arg Leu Glu Leu
305                 310                 315                 320

Arg Asp Ile Ala Gly Gly Val Thr Leu Leu Asn Asp Ser Tyr Asn Ala
                325                 330                 335

Ser Pro Gly Ser Thr Arg Ala Ala Leu Asp Ala Leu Ala Ala Ile Glu
            340                 345                 350

Gly Gly Arg Arg Val Ala Val Leu Gly Gln Met Leu Glu Leu Gly Asp
        355                 360                 365

Glu Ser Glu Ala Glu His Arg Ala Val Gly Glu Tyr Ala Ala Ala Arg
    370                 375                 380

Ala Asp Val Val Val Thr Val Gly Glu Ala Val Arg Pro Leu Ala Val
385                 390                 395                 400

Gly Ala Gly Glu Arg Ala Val Ala Val Ala Asp Asn Gly Ala Ala Val
                405                 410                 415

Asp Trp Leu Arg Gly His Leu Ala Pro Gly Asp Val Val Leu Val Lys
            420                 425                 430

Ala Ser Arg Gly Ala Arg Leu Asp Glu Val Ala Ala Ala Leu Ser
        435                 440                 445

<210>  3

<211>  405

<212>  PRT

<213>  Actinoplanes teichomyceticus

<400>  3

Met Trp Arg Arg Gly Ser Ala Asp Ala Ile Arg Pro Val Thr Trp Ile
1               5                   10                  15

Ser Thr Thr Arg Gly Ala Gly Phe Leu Asp Asn Gln Leu Thr Tyr Arg
            20                  25                  30

Ala Ala Gly Pro Ser Asp Val Ser Ser Ala Tyr Pro Lys Pro His Thr
        35                  40                  45

Cys Ser Gln His His Leu Pro Leu Thr Gly Arg Met Gly Tyr Ser Glu
    50                  55                  60

Pro Val Arg Ala Ala Ala Pro Arg Thr Arg Ser Leu Ala Ser Ser Ser
65                  70                  75                  80

Ser Pro Val Val Pro Ala Thr Gly Ile Thr Ile Tyr Gly Cys Gly Pro
                85                  90                  95

Asp Glu Ala Ala Leu Phe Arg Glu Leu Ala Pro Arg Phe Gly Val Leu
            100                 105                 110

Pro Ile Ile Thr Glu Ala Ala Val Ser Glu Ala Asn Val Asn Leu Ala
        115                 120                 125

Ser Gly Asn Arg Cys Ile Ser Val Gly His Lys Thr Gln Ile Thr Asn
    130                 135                 140

Gly Thr Leu Leu Ala Leu Ser Arg Ser Gly Val Lys Tyr Ile Ser Thr
145                 150                 155                 160

Arg Ser Ile Gly Tyr Asn His Ile Asp Val Ala Tyr Ala Gln Ser Val
                165                 170                 175

Gly Ile Ser Val Gly Asn Val Ala Tyr Ser Pro Asp Ser Val Ala Asp
            180                 185                 190

Tyr Thr Leu Met Leu Met Leu Met Ala Val Arg His Met Lys Ala Met
        195                 200                 205

Ile Arg Arg Ala Asp Val His Asp Tyr Arg Leu Asn Asp Val Arg Gly
    210                 215                 220

Lys Glu Leu Arg Asp Leu Thr Val Gly Val Val Gly Thr Gly Arg Ile
225                 230                 235                 240

Gly Ser Ala Val Met Asp Arg Leu Trp Gly Phe Gly Cys Arg Val Leu
                245                 250                 255

Ala Tyr Asp Asn Arg Arg Ser Asp Ser Ala His Tyr Val Pro Leu Asp
            260                 265                 270

Asp Leu Val Arg Gln Ser Asp Ile Val Thr Leu His Thr Pro Leu Thr
        275                 280                 285

Ala Ala Thr His His Leu Leu Asn Gln Asp Arg Ile Ala Gln Met Arg

290                 295                 300

Asn Gly Ala Phe Val Ile Asn Thr Gly Arg Gly Ser Leu Ile Asp Thr
305                 310                 315                 320

Glu Ala Leu Val Ala Ala Leu Glu Ser Asp Lys Leu Gly Gly Ala Ala
                325                 330                 335

Leu Asp Val Leu Glu Gly Glu Glu Gly Val Phe Tyr Ala Asp Cys Arg
            340                 345                 350

Asn Lys Leu Ile Glu Ser Lys Ala Leu Leu Arg Leu Gln Glu Met Pro
        355                 360                 365

Asn Val Ile Ile Ser Pro His Met Ala Tyr Tyr Thr Asp His Ala Leu
    370                 375                 380

Ser Asp Thr Val Glu Asn Ser Leu Val Asn Cys Met Asn Phe Glu Arg
385                 390                 395                 400

Glu Gln Gln His Gly
                405

<210>  4

<211>  345

<212>  PRT

<213>  Actinoplanes teichomyceticus

<400>  4

Met Gly Lys Leu Lys Ile Gly Ile Ile Phe Gly Gly Val Ser Glu Glu
1               5                   10                  15

His Pro Val Ser Ile Lys Ser Ala Gln Glu Val Ala Lys Ser Leu Asp
            20                  25                  30

Ala Glu Lys Tyr Glu Pro Tyr Trp Ile Gly Ile Thr Lys Asp Gly Ala
        35                  40                  45

Trp Arg Leu Cys Asp Gly Pro Gly Ala Asp Trp Glu Ile Ser Ser Ala
    50                  55                  60

Arg Pro Ala Met Leu Ser Pro Asp Arg Asn Val His Gly Leu Leu Val
65                  70                  75                  80

Gln Glu Asp Gly Arg Tyr Arg Glu Ile Arg Leu Asp Val Val Leu Pro
                85                  90                  95

Val Leu His Gly Lys Leu Gly Glu Asp Gly Ala Met Gln Gly Leu Phe
            100                 105                 110

Glu Leu Ser Gly Ile Pro Tyr Val Gly Cys Asp Val Gln Ser Ser Ala
        115                 120                 125

Val Cys Met Asp Lys Ser Leu Thr Tyr Ala Val Val Ser Ser Ala Gly
    130                 135                 140

Ile Ala Thr Pro Arg Phe Trp Val Val Thr Pro Asp Glu Ala Pro Asp
145                 150                 155                 160

Pro Ala Gln Leu Thr Tyr Pro Val Phe Val Lys Pro Ala Arg Ser Gly
                165                 170                 175

Ser Ser Phe Gly Val Thr Lys Val Ser Thr Glu Gln Glu Leu Lys Ala
            180                 185                 190

Ala Leu Glu Thr Ala Arg Glu Phe Asp Ser Lys Val Leu Ile Glu Glu
        195                 200                 205

Ala Val Val Gly Ser Glu Ile Gly Cys Ala Ile Met Gly Asn Asp Pro
    210                 215                 220

Asp Leu Val Ala Gly Glu Val Asp Arg Val Ala Leu Ser His Gly Phe
225                 230                 235                 240

Phe Arg Ile His Gln Glu Ser Glu Pro Glu Arg Gly Ser Glu Asn Ser
                245                 250                 255

Thr Phe Ile Cys Pro Ala Asp Ile Pro Ala Glu Ala Arg Ser Leu Val
            260                 265                 270

Gln Glu Thr Ala Lys Ala Ile Tyr Arg Ala Leu Gly Cys Arg Gly Ile
        275                 280                 285

Ala Arg Val Asp Met Phe Leu Lys Glu Asp Gly Thr Val Val Leu Asn
    290                 295                 300

Glu Val Asn Thr Leu Pro Gly Leu Thr Ser Tyr Ser Arg Tyr Pro Arg
305                 310                 315                 320

Met Met Ala Ala Ala Gly Ile Pro Leu Ser Ala Val Ile Asp Gln Ile

325                 330                 335

Val Ser Leu Ala Leu Pro Gly Asp Ile
            340                 345

<210>  5

<211>  202

<212>  PRT

<213>  Actinoplanes teichomyceticus

<400>  5

Met Lys Asp Asp Phe Ala Phe Val Asp Glu Leu Val Pro Gly Ile Arg
1               5                   10                  15

Trp Asp Ala Lys Tyr Ala Thr Trp Asp Asn Phe Thr Gly Lys Pro Val
            20                  25                  30

Asp Gly Tyr Val Ala Asn Arg Ile Val Gly Thr Lys Ala Leu Cys Ala
        35                  40                  45

Ala Leu Ala Arg Ala Gln Glu Glu Ala Glu Thr Leu Gly Phe Gly Leu
    50                  55                  60

Leu Leu Trp Asp Gly Tyr Arg Pro Gln Arg Ala Val Asp Cys Phe Met
65                  70                  75                  80

Arg Trp Ala Glu Gln Pro Glu Asp Gly Arg Lys Lys Leu Arg His Tyr
                85                  90                  95

Pro Asn Ile Gly Arg Pro Glu Met Phe Glu Lys Gly Tyr Val Ala Thr
            100                 105                 110

Lys Ser Gly His Ser Arg Gly Ser Thr Val Asp Leu Thr Leu Tyr Asn
        115                 120                 125

Leu Ala Thr Gly Glu Leu Ala Ser Met Gly Gly Asp His Asp Leu Met
    130                 135                 140

Asp Pro Ile Ser His His Gly Ala Ser Gly Ile Thr Pro Ile Glu Ala
145                 150                 155                 160

Lys Asn Arg Glu Tyr Leu Cys Ser Ile Met Glu Gly Cys Gly Phe Ala
                165                 170                 175

Arg Tyr Asp Tyr Glu Trp Trp His Tyr Thr Leu Lys Asn Glu Pro Tyr
            180                 185                 190

Pro Asp Thr Tyr Phe Asp Phe Pro Ile Val
        195                 200

<210>  6

<211>  179

<212>  PRT

<213>  Actinoplanes teichomyceticus

<400>  6

Met Ser Asp Val Ser Gly Ala Gly Lys Val Val Val Asn Arg Ala Met
1               5                   10                  15

Ser Leu Asp Gly Phe Ile Ala Gly Pro Gly His Ala Met Asp Trp Ile
            20                  25                  30

Phe Glu Tyr Leu Thr Gly Asp Met Phe Pro Glu Val Met Ala Ser Thr
        35                  40                  45

Gly Ala Met Leu Ile Gly Arg Gly Thr Tyr Glu Val Ala Lys Arg Met
    50                  55                  60

Ala Gly Gln Gly Thr Pro Tyr Asp Gly Gly Ala Gln Phe Val Leu Thr
65                  70                  75                  80

His Glu Pro Pro His Glu Pro Asp Pro Ala Val Thr Phe Leu Ser Cys
                85                  90                  95

Asp Ile Glu Glu Ala Val Ala Thr Ala Arg Ser Ala Ala Gly Gly Lys
            100                 105                 110

Asn Leu Glu Ile Leu Gly Ala Asp Val Ala Ala Gln Cys Leu Arg Arg
        115                 120                 125

Gly Leu Val Asp Glu Ile Leu Val Tyr Val Val Pro Val Leu Leu Gly
    130                 135                 140

Asp Gly Val Arg Phe Ser Pro Pro Ser Leu Asp Arg Ile Asp Leu Glu
145                 150                 155                 160

Pro Leu Ser Asn Thr Arg Ser Gly Ala Val Thr Met Leu Arg Phe Arg

165                 170                 175

Val Arg Lys

<210>  7

<211>  363

<212>  PRT

<213>  Actinoplanes teichomyceticus

<400>  7

Val Asp Arg Ala Pro Gly Leu Ser Val Arg Leu Lys Leu Thr Leu Ser
1               5                   10                  15

Tyr Ala Gly Phe Leu Met Leu Ala Gly Ala Ser Leu Leu Ala Thr Val
            20                  25                  30

Trp Val Tyr Leu Leu Arg Gly Leu Pro Asp Thr Ala Met Ser Ser Arg
        35                  40                  45

Gly Leu Val Pro Ile Lys Thr Ile Val Leu His Ala Phe Ala Pro Ala
    50                  55                  60

Thr Ala Gly Val Leu Leu Phe Leu Leu Val Phe Gly Leu Leu Gly Gly
65                  70                  75                  80

Trp Leu Leu Ala Gly Arg Met Leu Ala Pro Leu Thr Arg Ile Thr Glu
                85                  90                  95

Ala Thr Arg Met Ala Ala Asn Gly Ser Leu Ser His Arg Ile Glu Leu
            100                 105                 110

Asp Gly Arg Lys Asp Glu Phe Arg Glu Leu Ala Asp Ala Phe Asp Ile
        115                 120                 125

Met Leu Thr Arg Leu Glu Ala His Ile Ala Glu His Gln Arg Phe Ala
    130                 135                 140

Ala Asn Ala Ser His Glu Leu Arg Thr Pro Leu Ala Ile Thr Lys Thr
145                 150                 155                 160

Leu Leu Asp Val Ala Arg Thr Asp Pro Ala Arg Ser Thr His Glu Leu
                165                 170                 175

Ile Gly Arg Leu His Thr Val Asn Thr Arg Ala Ile Asp Leu Thr Glu
            180                 185                 190

Ala Leu Leu Leu Leu Ser Arg Ala Asp Arg Arg Ser Phe Lys Arg Glu
        195                 200                 205

Tyr Val Asp Leu Ser Leu Asn Val Glu Glu Ala Ile Glu Thr Leu Leu
    210                 215                 220

Pro Leu Ala Glu Lys His Gly Val Thr Ile Glu Ala Ser Gly Asp Ile
225                 230                 235                 240

Thr Pro Thr Val Gly Ser Arg Ala Leu Leu Leu Gln Met Thr Ile Asn
                245                 250                 255

Leu Leu His Asn Ala Ile Val His Asn Val Ala Glu Glu Arg Ile Val
            260                 265                 270

Trp Val Thr Thr Ser Val His Pro Lys Ser Val Thr Leu Thr Val Glu
        275                 280                 285

Asn Thr Gly Glu Gln Leu Ser Ala Gln Leu Val Ser Thr Leu Thr Glu
    290                 295                 300

Pro Phe Gln Arg Gly Thr Glu Arg Ile His His Asp His Ala Gly Val
305                 310                 315                 320

Gly Leu Gly Leu Ala Ile Val Lys Ser Ile Ala His Ala His Asp Gly
                325                 330                 335

Thr Leu Thr Thr Ala Pro Arg Ala Gly Gly Gly Leu His Val Thr Val
            340                 345                 350

Gln Leu Pro Ala Ala Thr Thr Pro Pro Arg Asn
        355                 360

<210>  8

<211>  226

<212>  PRT

<213>  Actinoplanes teichomyceticus


<400>  8

Met Arg Val Leu Ile Val Glu Asp Glu Pro Tyr Met Ala Glu Ala Ile

1               5                   10                  15

Arg Asp Gly Leu Arg Leu Glu Ala Ile Ala Ala Asp Ile Ala Gly Asp
            20                  25                  30

Gly Asp Thr Ala Leu Glu Met Leu Ser Val Asn Ala Tyr Asp Ile Val
        35                  40                  45

Ile Leu Asp Arg Asp Ile Pro Gly Thr Ser Gly Asp Gln Ile Ala Glu
    50                  55                  60

Arg Ile Val Ala Ser Arg Ser Gly Met Pro Ile Leu Met Leu Thr Ala
65                  70                  75                  80

Ala Asp Arg Leu Asp Asp Lys Ala Ser Gly Phe Glu Leu Gly Ala Asp
                85                  90                  95

Asp Tyr Leu Thr Lys Pro Phe Glu Leu Arg Glu Leu Val Leu Arg Leu
            100                 105                 110

Arg Ala Leu Asp Arg Arg Arg Ala Tyr Ser Arg Pro Pro Val Arg Glu
        115                 120                 125

Ile Ala Gly Leu Arg Leu Asp Pro Phe Arg Arg Glu Val Tyr Arg Asp
    130                 135                 140

Asp Arg Tyr Val Ala Leu Thr Arg Lys Gln Phe Ala Val Leu Ala Val
145                 150                 155                 160

Leu Val Ala Ala Glu Gly Gly Val Val Ser Ala Glu Glu Leu Leu Glu
                165                 170                 175

Arg Ala Trp Asp Glu Asn Ala Asp Pro Phe Thr Asn Ala Val Arg Ile
            180                 185                 190

Thr Val Ser Ala Leu Arg Lys Arg Leu Gly Glu Pro Gln Ile Ile Ala
        195                 200                 205

Thr Val Pro Gly Val Gly Tyr Arg Ile Thr Thr Gln Pro Gly Ser Gly
    210                 215                 220

Arg Ala
225

<210>  9

<211>  393

<212>  PRT

<213>  Actinoplanes teichomyceticus

<400>  9

Met Arg Val Leu Phe Ser Ser Tyr Gly Thr Arg Gly Asp Val Glu Pro
1               5                   10                  15

Leu Val Ala Leu Ala Val Arg Leu Arg Glu Leu Gly Ala Glu Val Arg
            20                  25                  30

Met Cys Ala Ser Pro Asp Tyr Val Glu Arg Leu Ala Glu Val Glu Val
        35                  40                  45

Pro Met Val Pro Ile Gly Gln Pro Val Arg Ala Gly Ala Arg Lys Gly
    50                  55                  60

Ala Ala Pro Pro Gly Ala Pro Glu Ala Val Ala Glu Val Ile Ala Glu
65                  70                  75                  80

Gln Phe Asp Arg Val Pro Pro Phe Ala Glu Gly Cys Asp Ala Val Val
                85                  90                  95

Ala Ser Gly Leu Val Ser Thr Ala Val Ala Val Arg Ser Val Ala Glu
            100                 105                 110

Lys Leu Gly Ile His His Tyr Ala Tyr Ala Val Leu Ser Pro Ser Phe
        115                 120                 125

Leu Arg Ala Pro Gly Val Thr Asp Ser Arg Asp Leu Arg Ala Arg Ala
    130                 135                 140

Asn Gln Gly Ala Tyr Arg Gln Phe Gly Gly Pro Leu Asn Ser His Arg
145                 150                 155                 160

Ala Ala Val Gly Leu Pro Pro Val Glu Lys Val Ile Asp Tyr Ala Phe
                165                 170                 175

Thr Glu Arg Pro Trp Leu Ala Ala Asp Pro Val Leu Asp Pro Leu Arg
            180                 185                 190

Pro Thr Asp Pro Asp Val Val Gln Thr Gly Ala Trp Ile Leu Pro Asp
        195                 200                 205

Gln Arg Pro Leu Ser Ala Glu Leu Glu Gly Phe Leu Arg Ala Gly Ser

210                 215                 220

Pro Pro Val Tyr Val Gly Phe Gly Ser Gly Pro Ala Pro Ala Glu Ala
225                 230                 235                 240

Ala Arg Val Ala Ile Glu Ala Val Arg Ala Gln Gly Arg Arg Val Val
                245                 250                 255

Leu Ser Ser Gly Trp Ala Gly Leu Gly Arg Ile Asp Glu Gly Asp Asp
            260                 265                 270

Cys Leu Val Val Gly Glu Val Asn His Gln Val Leu Phe Gly Arg Val
        275                 280                 285

Ala Ala Val Val His His Gly Gly Ala Gly Thr Thr Thr Ala Val Thr
    290                 295                 300

Arg Ala Gly Ala Pro Gln Val Val Val Pro Gln Lys Ala Asp Gln Pro
305                 310                 315                 320

Tyr Tyr Ala Gly Arg Val Ala Asp Leu Gly Val Gly Val Ala His Asp
                325                 330                 335

Gly Pro Thr Pro Thr Val Glu Ser Leu Ser Ala Ala Leu Ala Thr Ala
            340                 345                 350

Leu Thr Pro Gly Ile Arg Ala Arg Ala Ala Ala Val Ala Gly Thr Ile
        355                 360                 365

Arg Thr Asp Gly Thr Thr Val Ala Ala Lys Leu Leu Leu Glu Ala Ile
    370                 375                 380

Ser Arg Gln Arg Ser Ser Val Pro Ala
385                 390

<210>  10

<211>  2076

<212>  PRT

<213>  Actinoplanes teichomyceticus

<400>  10

Met Asn Ser Ala Ala Gln Ala Thr Ser Thr Val Pro Glu Leu Leu Ala
1               5                   10                  15

Arg Gln Val Thr Arg Ala Pro Asp Ala Val Ala Val Val Asp Arg Asp
            20                  25                  30

Arg Val Leu Thr Tyr Arg Glu Leu Asp Glu Leu Ala Gly Arg Leu Ser
        35                  40                  45

Gly Arg Leu Ile Gly Arg Gly Val Arg Arg Gly Asp Arg Val Ala Val
    50                  55                  60

Leu Leu Asp Arg Ser Ala Asp Leu Val Val Thr Leu Leu Ala Ile Trp
65                  70                  75                  80

Lys Ala Gly Ala Ala Tyr Val Pro Val Asp Ala Gly Tyr Pro Ala Pro
                85                  90                  95

Arg Val Ala Phe Met Val Ala Asp Ser Gly Ala Ser Arg Met Val Cys
            100                 105                 110

Ser Ala Ala Thr Arg Asp Gly Val Pro Glu Gly Ile Glu Ala Ile Val
        115                 120                 125

Val Thr Asp Glu Glu Ala Phe Glu Ala Ser Ala Ala Gly Ala Arg Pro
    130                 135                 140

Gly Asp Leu Ala Tyr Val Met Tyr Thr Ser Gly Ser Thr Gly Ile Pro
145                 150                 155                 160

Lys Gly Val Ala Val Pro His Arg Ser Val Ala Glu Leu Ala Gly Asn
                165                 170                 175

Pro Gly Trp Ala Val Glu Pro Gly Asp Ala Val Leu Met His Ala Pro
            180                 185                 190

Tyr Ala Phe Asp Ala Ser Leu Phe Glu Ile Trp Val Pro Leu Val Ser
        195                 200                 205

Gly Gly Arg Val Val Ile Ala Glu Pro Gly Pro Val Asp Ala Arg Arg
    210                 215                 220

Leu Arg Glu Ala Ile Ser Ser Gly Val Thr Arg Ala His Leu Thr Ala
225                 230                 235                 240

Gly Ser Phe Arg Ala Val Ala Glu Glu Ser Pro Glu Ser Phe Ala Gly
                245                 250                 255

Leu Arg Glu Val Leu Thr Gly Gly Asp Val Val Pro Ala His Ala Val

260                 265                 270

Ala Arg Val Arg Ser Ala Cys Pro Arg Val Arg Ile Arg His Leu Tyr
        275                 280                 285

Gly Pro Thr Glu Thr Thr Leu Cys Ala Thr Trp His Leu Leu Glu Pro
    290                 295                 300

Gly Asp Glu Ile Gly Pro Val Leu Pro Ile Gly Arg Pro Leu Pro Gly
305                 310                 315                 320

Arg Arg Ala Gln Val Leu Asp Ala Ser Leu Arg Ala Val Ala Pro Gly
                325                 330                 335

Val Ile Gly Asp Leu Tyr Leu Ser Gly Ala Gly Leu Ala Asp Gly Tyr
            340                 345                 350

Leu Arg Arg Ala Gly Leu Thr Ala Glu Arg Phe Val Ala Asp Pro Ser
        355                 360                 365

Ala Pro Gly Ala Arg Met Tyr Arg Thr Gly Asp Leu Ala Gln Trp Thr
    370                 375                 380

Ala Asp Gly Ala Leu Leu Phe Ala Gly Arg Ala Asp Asp Gln Val Lys
385                 390                 395                 400

Val Arg Gly Phe Arg Ile Glu Pro Ala Glu Val Glu Ala Ala Leu Thr
                405                 410                 415

Ala Gln Pro Gly Val His Glu Ala Val Val Arg Ala Val Asp Gly Arg
            420                 425                 430

Leu Val Gly Tyr Val Val Ala Glu Gly Asp Ala Glu Pro Ala Val Leu
        435                 440                 445

Arg Glu Arg Val Gly Ala Val Leu Pro Glu Tyr Met Val Pro Ala Ala
    450                 455                 460

Val Ile Thr Leu Asp Ala Leu Pro Leu Thr Gly Asn Gly Lys Val Asp
465                 470                 475                 480

Arg Ala Ala Leu Pro Ala Pro Val Phe Ala Ala Asp Ala Pro Gly Arg
                485                 490                 495

Glu Pro Gly Thr Glu Ala Glu Arg Val Leu Cys Gly Leu Leu Ser Glu
            500                 505                 510

Val Leu Gly Leu Asn Arg Val Gly Val Asp Glu Ser Phe Phe Glu Leu
        515                 520                 525

Gly Gly Asp Ser Ile Ala Ala Ile Arg Leu Ala Ala Arg Ala Ser Arg
    530                 535                 540

Ala Gly Leu Leu Val Thr Pro Ala Gln Ile Phe Lys Glu Arg Thr Val
545                 550                 555                 560

Ala Arg Leu Ala Ala Val Ala Gly Ala Val Pro Ala Gly Pro Pro Val
                565                 570                 575

Asp Arg Pro Leu Ile Ala Leu Thr Ala Glu Glu Glu Val Glu Leu Ala
            580                 585                 590

Ala Ala Val Pro Gly Ala Val Glu Val Trp Pro Leu Ala Pro Leu Gln
        595                 600                 605

Glu Gly Leu Leu Phe Gln Ser Thr Ile Asp Thr Glu Gly Leu Asp Ile
    610                 615                 620

Tyr Gln Ala Gln Trp Ile Leu Gln Leu Asn Ala Pro Leu Asp Val Arg
625                 630                 635                 640

Arg Leu Arg Val Ala Trp Glu Ala Val Phe Ala Arg His Ala Glu Leu
                645                 650                 655

Arg Val Ser Phe Val Arg Leu Ala Ser Gly Lys Thr Val Gln Ala Ala
            660                 665                 670

Ala Gly His Val Val Leu Pro Trp Arg Glu Val Asp Leu Thr Asp Ala
        675                 680                 685

Gly Asp Val Asp Ala Ala Leu Gln Glu Leu Ala Gly Arg Glu Gln Glu
    690                 695                 700

Gln Arg Phe Asp Leu Ala Arg Ala Pro Leu Phe Arg Leu Val Leu Val
705                 710                 715                 720

Arg Tyr Gly Glu Asp Arg His Arg Leu Leu Val Val His His His Ile
                725                 730                 735

Leu Thr Asp Gly Trp Ser Val Ala Val Ile Leu Asn Glu Val Ala Glu
            740                 745                 750

Ala Tyr Ala Ala Gly Gly Arg Leu Pro Asp Arg Thr Gly Pro Val Ser

        755                 760                 765

Tyr Arg Asn Tyr Leu Ala Trp Leu Asp Arg Gln Asp Arg Glu Ala Ala
    770                 775                 780

Arg Ala Ala Trp Arg Ala Glu Leu Ser Gly Leu Asp Glu Pro Val Pro
785                 790                 795                 800

Ile Ala Arg Ala Ala Ala Gly Thr Gly Tyr Glu Tyr Arg Val Ala Phe
                805                 810                 815

Leu Pro Ala Asp Val Cys Thr Arg Leu Thr Ala Ser Val Arg Asp His
            820                 825                 830

Gly Leu Thr Leu Asn Thr Val Val Gln Gly Ala Trp Ala Met Val Leu
        835                 840                 845

Ser Arg Leu Val Arg Arg Thr Asp Val Val Phe Gly Thr Thr Val Ala
    850                 855                 860

Val Arg Pro Ala Glu Leu Pro Gly Val Glu Ser Ile Pro Gly Leu Met
865                 870                 875                 880

Met Asn Thr Val Pro Val Arg Val Thr Leu Asp Gly Gly Gln Ser Leu
                885                 890                 895

Leu Asp Leu Leu Thr Gly Leu Gln Arg Arg Gln Ala Ala Leu Met Pro
            900                 905                 910

His Gln His Leu Gly Leu Pro Glu Ile Gln Arg Thr Ala Gly Pro Gly
        915                 920                 925

Ala Thr Phe Asp Thr Leu Leu Val Phe Glu Asn Tyr Pro Arg Asp Phe
    930                 935                 940

Ala Gly Gln Phe Thr Tyr Leu Gly Thr Ile Glu Gly Thr His Tyr Pro
945                 950                 955                 960

Leu Thr Leu Gly Ile Ile Pro Gly Glu Arg Leu Arg Ile Gln Leu Ala
                965                 970                 975

Tyr Arg His Gly Gln Ile Glu Glu Thr Leu Ala Ala Ser Val Leu Asp
            980                 985                 990

Gly Phe Val Ala Ala Leu Arg Ala Val Ala Ala Asp Pro Ser Gly Leu
        995                 1000                1005

Val Gly Arg Thr Gly Val Gly Arg Gly Pro Val His Gly Leu Ala
    1010                1015                1020

Pro Ala Leu Val Ala Ala Glu Ser Leu Pro Val Leu Val Gly Arg
    1025                1030                1035

Val Val Glu Gln Arg Ser His Glu Val Ala Val Val Asp Gly Asp
    1040                1045                1050

Gly Glu Leu Ser Phe Gly Gln Leu Trp Asp Gln Ala Val Gly Leu
    1055                1060                1065

Ala Asp Leu Leu Thr Ala Arg Gly Val Gly Pro Glu Cys Arg Val
    1070                1075                1080

Gly Val Leu Val Gly Arg Ser Ala Trp Trp Val Val Gly Met Leu
    1085                1090                1095

Gly Val Ser Leu Ala Gly Gly Ala Phe Val Pro Val Asp Pro Ala
    1100                1105                1110

Tyr Pro Ala Glu Arg Ile Arg Leu Ile Leu Ala Asp Ala Asp Pro
    1115                1120                1125

Ala Leu Val Val Cys Ala Gly Lys Thr Arg Glu Ala Val Pro Ala
    1130                1135                1140

Ala Phe Ala Asp Arg Leu Leu Val Val Asp Glu Met Asp Leu Thr
    1145                1150                1155

Gly Gly Ser Ala Ala Arg Leu Pro Arg Val Arg Pro Gly Asp Ala
    1160                1165                1170

Ala Tyr Val Ile Tyr Thr Ser Gly Ser Thr Gly Arg Pro Lys Gly
    1175                1180                1185

Val Val Val Pro His Ala Gly Leu Gly Asn Leu Ala Leu Ala Gln
    1190                1195                1200

Ile Asp Arg Phe Gly Val Ser Pro Ser Ser Arg Val Leu Gln Phe
    1205                1210                1215

Ala Ala Leu Gly Phe Asp Ala Met Val Ser Glu Val Leu Met Ala
    1220                1225                1230

Leu Leu Ser Gly Ala Arg Leu Val Met Ala Pro Glu His Gln Leu

1235                1240                1245

Pro Pro Arg Val Ser Leu Ala Glu Ala Leu Gln Arg Trp Asp Val
    1250                1255                1260

Thr His Val Thr Val Pro Pro Ser Val Leu Ala Thr Ala Glu Ala
    1265                1270                1275

Leu Pro Ala Arg Leu Glu Thr Val Val Val Ala Gly Glu Ala Cys
    1280                1285                1290

Pro Pro Ser Leu Ala Asp Arg Trp Ser Ala Gly Leu Arg Leu Val
    1295                1300                1305

Asn Ala Tyr Gly Pro Thr Glu Ala Thr Val Cys Ala Ala Met Ser
    1310                1315                1320

Met Pro Leu Val Ala Ser Arg Pro Val Val Pro Ile Gly Thr Pro
    1325                1330                1335

Ile Ala Gly Gly Arg Cys Tyr Val Leu Asp Ala Phe Leu Arg Pro
    1340                1345                1350

Leu Pro Pro Gly Leu Thr Gly Glu Leu Tyr Val Ala Gly Ile Gly
    1355                1360                1365

Leu Ala Arg Gly Tyr Leu Gly Arg Ala Ala Leu Thr Ala Glu Arg
    1370                1375                1380

Phe Val Ala Asp Pro Phe Val Pro Gly Glu Arg Met Tyr Arg Thr
    1385                1390                1395

Gly Asp Leu Ala Tyr Arg Thr Gly Glu Gly Glu Leu Val Phe Ala
    1400                1405                1410

Gly Arg Ala Asp Asp Gln Val Lys Val Arg Gly Phe Arg Ile Glu
    1415                1420                1425

Pro Gly Glu Val Glu Ser Ala Leu Ser Gly His Pro Gly Val Ala
    1430                1435                1440

Gln Ala Ala Val Ile Val Arg Gly Asp Arg Leu Leu Ala Tyr Val
    1445                1450                1455

Ser Pro Ala Gly Val Asp Pro Gln Ala Val Arg Glu Asp Leu Ala
    1460                1465                1470

Ser Arg Leu Pro Pro Tyr Met Val Pro Ala Val Val Val Pro Leu
    1475                1480                1485

Glu Ala Leu Pro Thr Thr Pro Asn Gly Lys Ile Asp Arg Gly Ala
    1490                1495                1500

Leu Pro Asp Pro Asp Phe Ala Ala Gly Ala Asp Val Arg Glu Pro
    1505                1510                1515

Arg Thr Glu Val Glu Arg Ala Leu Cys Asp Leu Phe Ala Glu Val
    1520                1525                1530

Leu Gly Leu Asp Arg Val Gly Val Thr Asp Ser Phe Phe Glu Val
    1535                1540                1545

Gly Gly Asp Ser Ile Thr Ser Met Gln Leu Val Ala Arg Ala Arg
    1550                1555                1560

Arg Ala Gly Leu Thr Phe Ala Ala His Asp Val Phe Glu Glu Arg
    1565                1570                1575

Thr Pro Glu Arg Leu Ala Gln Val Ala Ala Gln Ala Pro Arg Pro
    1580                1585                1590

Glu Arg Gly His Met Ile Gly Asp Gly Val Gly Glu Val Ala Arg
    1595                1600                1605

Thr Pro Val Met Arg Leu Leu Gly Asp Ala Val Val Ala Ala Gly
    1610                1615                1620

Phe Ala Gln Trp Val Val Val Ala Val Pro Ala Gly Leu Gly Glu
    1625                1630                1635

Arg Ala Leu Val Ala Ala Leu Thr Ala Val Val Asp Ala His Asp
    1640                1645                1650

Met Leu Arg Val Arg Val Asp Ser Gly Arg Leu Val Val Arg Ala
    1655                1660                1665

Arg Gly Ser Val Asp Val Ala Gly Ala Val Glu Arg Val Glu Ala
    1670                1675                1680

Asp Thr Gly Glu Ile Asp Glu Val Ala Arg Arg Cys Ala Val Ala
    1685                1690                1695

Val Ala Ala Arg Leu Asp Pro Ala Ala Gly Val Val Val Arg Ala

1700                1705                1710

Ala Trp Val Asp Ala Gly Pro Asp Arg Ser Gly Arg Leu Val Leu
    1715                1720                1725

Ala Ala His His Leu Ala Val Asp Gly Val Ser Trp Arg Val Leu
    1730                1735                1740

Val Pro Asp Leu Gln Ala Ala Cys Glu Ala Val Ala Ala Gly Arg
    1745                1750                1755

Gln Pro Ala Leu Glu Pro Val Gly Val Ser Phe Arg Arg Trp Ala
    1760                1765                1770

Asp Leu Leu Gly Gln Trp Ala Val Ser Pro Glu Arg Val Gly Glu
    1775                1780                1785

Leu Ala Arg Trp Arg Ala Val Val Gly Ser Gly Asp Arg Arg Ile
    1790                1795                1800

Gly Glu Pro Gly Pro Ser Ala Gly Ala Val Arg Ser Arg Ser Trp
    1805                1810                1815

Val Val Ser Gly Ser Arg Ala Gln Val Leu Val Gly Arg Ala Pro
    1820                1825                1830

Val Val Phe Phe Cys Gly Val His Glu Val Val Leu Ala Gly Leu
    1835                1840                1845

Ala Gly Ala Val Ala Arg Trp His Gly Gly Asp Val Val Leu Val
    1850                1855                1860

Asp Val Glu Gly His Gly Arg His Pro Val Gly Gly Leu Asp Leu
    1865                1870                1875

Ser Arg Thr Val Gly Trp Phe Thr Ser Val His Pro Val Arg Leu
    1880                1885                1890

Asp Val Ala Gly Ile Asp Trp Ala Gly Val Leu Ala Gly Gly Pro
    1895                1900                1905

Ala Ala Gly Glu Leu Leu Lys Arg Val Lys Glu Gln Ser Arg Ala
    1910                1915                1920

Val Pro Gly Asp Gly Leu Gly Tyr Gly Leu Leu Arg Phe Leu Asn
    1925                1930                1935

Asp Glu Thr Gly Pro Val Leu Ala Ala Leu Pro Ser Pro Gln Ile
    1940                1945                1950

Gly Phe Asn Phe Met Gly Arg His Val Ala Gly Val Arg Glu Trp
    1955                1960                1965

Gln Pro Val Gly Asp Val Gly Gly Ala Leu Gly Pro Gly Val Gly
    1970                1975                1980

Leu Pro His Val Leu Asp Ala Asp Ala Val Val Glu Asp Gly Pro
    1985                1990                1995

Arg Gly Pro Leu Leu Arg Leu Val Leu Ser Trp Pro Asp Gly Val
    2000                2005                2010

Leu Arg Glu Asp Glu Val Glu Arg Leu Gly Arg Gly Trp Ser Asp
    2015                2020                2025

Met Leu Ser Gly Leu Ala Gly Gln Ala Asp Asp Pro Ser Ala Gly
    2030                2035                2040

Gly His Thr Ala Ser Asp Phe Asp Leu Leu Asp Leu Asp Gln Asp
    2045                2050                2055

Glu Ile Glu Asn Phe Glu Ala Ile Ala Ala Glu Phe Gly Gly Gly
    2060                2065                2070

Gln Glu Ser
    2075

<210> 11

<211> 1061

<212> PRT

<213> Actinoplanes teichomyceticus

<400> 11

Val Thr Thr Pro Thr Thr Ser Pro Gly Ser Ala Leu Ala Glu Val Trp
1               5                   10                  15

Pro Leu Ser Pro Met Gln Glu Gly Met Leu Tyr His Ala Ser Phe Asp
            20                  25                  30

Asp Ala Ala Pro Asp Ile Tyr Val Ile Gln Gln Ser Gln Ile Ile Asp

35                  40                  45

Gly Pro Leu Asp Thr Glu Arg Phe Arg Arg Ser Trp Glu Val Leu Leu
    50                  55                  60

His Arg His Ala Ala Leu Arg Ala Ser Phe His Arg Arg Lys Ser Gly
65                  70                  75                  80

Glu Thr Val Gln Leu Ile Pro Arg Glu Val Arg Leu Pro Trp Ala Glu
                85                  90                  95

Arg Asp Leu Ser Gly Leu Pro Glu Lys Ala Ala Leu Ala Glu Val Gly
            100                 105                 110

Glu Ile Ala Ala Lys Glu Arg Ala Glu Arg Phe Asp Leu Thr Lys Pro
        115                 120                 125

Pro Leu Leu Arg Leu Met Leu Ile Arg Leu Gly Pro Gln Arg His Cys
    130                 135                 140

Leu Val Thr Thr Ser His His Leu Leu Met Asp Gly Trp Ser Arg Ala
145                 150                 155                 160

Ile Leu Glu Ser Glu Leu Leu His Val Tyr Ala Ser Gly Gly Thr Val
                165                 170                 175

Ser Gly Leu Pro Pro Ala Gly Ser Tyr Arg His Tyr Leu Ser Trp Leu
            180                 185                 190

Gly Arg Gln Asp Arg Glu Ala Ala Arg Ser Ala Trp Arg Ala Glu Leu
        195                 200                 205

Ser Gly Ala Asp Gly Ser Ala Leu Gly Ile Ala Glu Arg Pro Gly Lys
    210                 215                 220

Ala Pro Glu Leu Pro Ala Arg Glu Val Leu Arg His Ser Pro Glu Phe
225                 230                 235                 240

Thr Ser Ala Leu Val Asp Phe Ala Arg Arg His Gly Leu Thr Leu Asn
                245                 250                 255

Thr Leu Val Gln Gly Ala Trp Ala Val Val Leu Ala Arg Leu Ala Arg
            260                 265                 270

Arg Thr Asp Val Val Phe Gly Ala Thr Val Ser Gly Arg Pro Ala Glu
        275                 280                 285

Leu Pro Gly Val Glu Gln Leu Val Gly Leu Phe Ile Asn Thr Val Pro
    290                 295                 300

Val Arg Val Arg Leu Asp Ala Gly Gln Pro Val Leu Glu Met Leu Thr
305                 310                 315                 320

Glu Leu Gln Lys Arg Gln Ser Ala Leu Ile Ser His Gln His Leu Gly
                325                 330                 335

Leu Pro Glu Ile Gln Lys Leu Gly Gly Ala Ser Phe Asp Thr Ile Val
            340                 345                 350

Val Phe Glu Asn Tyr Val Asp Pro Ala Ala Ala Pro Asp Ser Pro Arg
        355                 360                 365

Gly Leu Arg Leu Thr Leu Arg Glu Phe His Gln Ala Ser Pro Tyr Ala
    370                 375                 380

Ile Thr Leu Gly Val Met Pro Gly Glu Ser Leu Glu Thr Asp Val Gln
385                 390                 395                 400

Tyr Pro Pro Glu Thr Leu Asp Ala Arg Ile Ala Glu Glu Ala Leu Tyr
                405                 410                 415

Gly Leu Ala Arg Val Leu Glu Gln Ile Ile Ala Glu Pro Gly Ala Ala
            420                 425                 430

Val Gly Arg Leu Asp Val Val Ser Ala Ala Gly Arg Gln Leu Thr Val
        435                 440                 445

Glu Arg Trp Asn Glu Thr Gly Asp Ala Val Asp Ala Pro Ser Pro Val
    450                 455                 460

Asp Leu Phe Arg Arg Gln Val Arg Arg Ala Pro Asp Ala Thr Ala Val
465                 470                 475                 480

Thr Ala Gly Asp Arg Ala Trp Ser Phe Lys Glu Leu Asp Glu Trp Ser
                485                 490                 495

Gly Arg Leu Ala Arg Ala Leu Thr Asp Arg Gly Val Arg Arg Gly Asp
            500                 505                 510

Arg Val Gly Val Met Leu Glu Arg Ser Ala Glu Val Leu Ala Ala Trp
        515                 520                 525

Leu Gly Val Trp Lys Ala Gly Ala Ala Phe Val Pro Val Asp Pro Gly

    530                 535                 540

Tyr Pro Ala Asp Arg Val Glu Phe Met Leu Ala Asp Ser Ala Val Ala
545                 550                 555                 560

Ala Val Leu Cys Arg Glu Ser Thr Ala Gly Ala Val Pro Ala Gly His
                565                 570                 575

Glu Leu Ile Asp Met Asp Arg Val Gly Glu Gly Val Ala Ala Pro Val
            580                 585                 590

Ala Ile Gly Ala Asp Asp Leu Ala Tyr Val Met Tyr Thr Ser Gly Ser
        595                 600                 605

Thr Gly Lys Pro Lys Gly Val Ala Val Ala His Gly Gly Val Ala Ala
    610                 615                 620

Leu Ala Gly Asp Pro Val Trp Glu Val Gly Ala Gly Asp Ala Val Leu
625                 630                 635                 640

Met His Ala Pro His Thr Phe Asp Ala Ser Leu Tyr Asp Val Trp Val
                645                 650                 655

Pro Leu Val Ser Gly Ala Arg Val Met Leu Ala Glu Pro Gly Val Val
            660                 665                 670

Asp Ala Gln Arg Leu Ala Ala His Val Ala Asp Gly Leu Thr Ala Val
        675                 680                 685

Asn Phe Thr Ala Gly Gln Phe Arg Ala Leu Ala Gln Glu Ser Pro Glu
    690                 695                 700

Ser Phe Ala Gly Leu Arg Asp Val Leu Ala Gly Gly Asp Val Val Pro
705                 710                 715                 720

Leu Gly Ala Val Glu Arg Val Arg Gln Ala Cys Pro Gly Leu Arg Val
                725                 730                 735

Trp His Thr Tyr Gly Pro Thr Glu Thr Thr Leu Cys Ala Thr Trp Lys
            740                 745                 750

Lys Ile Glu Pro Gly Asp Arg Leu Gly Ser Thr Leu Pro Ile Gly Arg
        755                 760                 765

Pro Leu Pro Gly Arg Arg Leu Tyr Val Leu Asp Val Phe Leu Arg Pro
    770                 775                 780

Leu Pro Pro Gly Val Ala Gly Asp Leu Tyr Ile Ala Gly Ala Gly Val
785                 790                 795                 800

Ala Gln Gly Tyr Leu Gly Arg Pro Ala Leu Thr Ala Glu Arg Phe Val
                805                 810                 815

Ala Asp Pro Phe Ala Ala Gly Glu Arg Met Tyr Arg Thr Gly Asp Val
            820                 825                 830

Ala Tyr Trp Thr Gly Asp Gly Glu Leu Val Phe Ala Gly Arg Ala Asp
        835                 840                 845

Asn Gln Val Lys Ile Arg Gly Tyr Arg Val Glu Pro Gly Glu Ile Glu
    850                 855                 860

Thr Val Leu Ala Glu Gln Pro Gly Val Asp Gln Ala Val Val Leu Ala
865                 870                 875                 880

Arg Asp Gly Arg Leu Ile Gly Tyr Val Val Pro Gly Gly Glu Val Asp
                885                 890                 895

Pro Val Arg Leu Arg Glu Arg Val Ala Arg Val Leu Pro Glu Tyr Met
            900                 905                 910

Val Pro Ala Ala Val Met Val Leu Asp Ala Leu Pro Val Thr Pro Asn
        915                 920                 925

Gly Lys Leu Asp Arg Glu Ala Leu Pro Asp Pro Asp Phe Ser Arg Arg
    930                 935                 940

Val Ser Gly Arg Glu Pro Arg Thr Gly Val Glu Arg Ala Leu Cys Gly
945                 950                 955                 960

Leu Phe Ala Glu Val Leu Gly Leu Glu Arg Val Gly Val Asp Asp Ser
                965                 970                 975

Phe Phe Glu Leu Gly Gly Asp Ser Ile Thr Ser Met Gln Leu Ala Ala
            980                 985                 990

Arg Ala Arg Arg Asp Gly Met Ala  Phe Gly Ala Arg Glu  Val Phe Gln
        995                 1000                 1005

His Arg  Thr Pro Ala Gly Ile  Ala Ala Ile Val Asp  Ser Gly Asn
    1010                 1015                 1020

Glu Pro  Ala Ala Gly Gly Asp  Gly Glu Ala Thr Trp  Thr Pro Val

1025                1030                1035

Arg Arg  Phe Gly Leu Val Asp  Leu Asp Gln Gln Glu  Val Glu Glu
    1040                1045                1050

Leu Glu  Ser Glu Phe Gly Asp  Arg
    1055                1060

<210>  12

<211>  4067

<212>  PRT

<213>  Actinoplanes teichomyceticus

<400>  12

Val Thr Gln Ala Arg Ile Glu Asp Ile Trp Pro Leu Ser Pro Leu Gln
1               5                   10                  15

Glu Gly Leu Leu Phe His Thr Val Tyr Asp Asp Glu Gly Pro Asp Val
            20                  25                  30

Tyr Val Gly His Trp Ile Leu Asp Leu Asp Gly Pro Val Asp Ala Ala
        35                  40                  45

Arg Leu Arg Ala Ala Trp Glu Ala Leu Leu Ala Arg His Ala Ala Leu
    50                  55                  60

Arg Ala Cys Phe Arg Gln Arg Arg Thr Gly Glu Thr Val Gln Val Val
65                  70                  75                  80

Ala Gly Arg Val Glu Leu Pro Trp Arg Val Val Asp Leu Ala His Leu
                85                  90                  95

Asp Asp Pro Glu His Ala Val Arg Glu Leu Ala Asp Glu Asp Arg Leu
            100                 105                 110

Arg Pro Phe Asp Val Ala Lys Pro Pro Leu Leu Arg Leu Thr Leu Ile
        115                 120                 125

Arg Leu Ala Asp Asp Arg His Arg Leu Val Val Thr Cys His His Ala
    130                 135                 140

Val Met Asp Gly Trp Ser Leu Pro Ile Val Phe Asp Glu Leu Thr Ala
145                 150                 155                 160

Leu Tyr Ala Ala Gly Asp Gly Pro Ala Gln Leu Pro Ala Val Thr Pro
                165                 170                 175

Tyr Arg Glu Tyr Leu Ala Trp Leu Ser Arg Gln Asp Lys Pro Ser Ala
            180                 185                 190

Leu Ala Ala Trp Ala Ala Glu Leu Arg Gly Ala Gln Glu Pro Thr Leu
        195                 200                 205

Val Ala Pro Ala Asp Pro Gly Arg Ala Pro Gly Met Pro Glu Ser Val
    210                 215                 220

Glu Val Gln Leu Ser Pro Glu Leu Thr Arg Ser Leu Ala Glu Leu Ala
225                 230                 235                 240

Arg Gly Arg Gly Leu Thr Leu Asn Thr Val Ile Gln Gly Ala Trp Ala
                245                 250                 255

Leu Val Leu Ala Arg Leu Thr Gly Arg Thr Asp Val Val Phe Gly Ala
            260                 265                 270

Thr Val Ser Ala Arg Pro Ala His Leu Pro Gly Val Glu Ala Met Val
        275                 280                 285

Gly Leu Phe Leu Asn Thr Val Pro Val Arg Val Arg Leu Arg Gly Ser
    290                 295                 300

Ala Pro Val Val Glu Met Leu Ala Glu Leu Gln Lys Arg Gln Ser Ala
305                 310                 315                 320

Leu Ile Pro Glu Gln Phe Val Gly Leu Ala Asp Ile Gln Lys Ala Ala
                325                 330                 335

Gly Pro Gly Ala Val Phe Asp Thr Leu Leu Val Phe Glu Asn Phe Pro
            340                 345                 350

Arg Glu Leu Asp Asp Ser Arg Ser Ala Asp Ala Phe Gly Ile Arg Val
        355                 360                 365

His Gln Gly Arg Glu Ala Ala His Tyr Pro Leu Thr Leu Val Ala Val
    370                 375                 380

Pro Gly Glu Ser Met Leu Phe Lys Leu Asp Tyr Leu Thr Glu Leu Phe
385                 390                 395                 400

Asp Ala Ala Thr Ala Ala Ser Ile Leu Glu Arg Phe Thr Arg Val Leu

                405                 410                 415

Arg Arg Leu Thr Asp Ala Gly Glu Leu Thr Val Ala Ala Ile Asp Val
            420                 425                 430

Thr Ser Ala Ala Glu Arg Asp Arg Val Ala Arg Trp Gly Ala Ala Val
        435                 440                 445

Gly Ala Arg Pro Asp Arg Leu Ala Leu Asp Leu Phe Ala Arg Gln Val
    450                 455                 460

Ala Gln Arg Pro Asp Glu Val Ala Val Ala Asp Gly Asp Arg Val Met
465                 470                 475                 480

Ser Phe Gly Glu Leu Ala Glu Arg Ala Glu Arg Leu Ala Gly His Leu
                485                 490                 495

Ser Ala Arg Gly Val Arg Arg Gly Asp Arg Val Ala Val Val Met Glu
            500                 505                 510

Arg Ser Gly Glu Leu Ile Ala Thr Leu Leu Ala Val Trp Arg Ala Gly
        515                 520                 525

Ala Ala Phe Val Pro Val Asp Pro Ala Tyr Pro Ala Glu Arg Val Lys
    530                 535                 540

Phe Leu Leu Thr Asp Ala Glu Pro Val Ala Ala Val Cys Thr Ala Ala
545                 550                 555                 560

Phe Arg Ala Ala Val Leu Asp Gly Gly Leu Glu Ala Ile Val Val Asp
                565                 570                 575

Asp Pro Gly Thr Trp Pro Ala Val Ala Pro Cys Pro Pro Val Pro Thr
            580                 585                 590

Gly Pro Asp Asp Leu Ala Tyr Val Met Tyr Thr Ser Gly Ser Thr Gly
        595                 600                 605

Thr Pro Lys Gly Val Ala Val Ser His Gly Asp Val Ala Ala Leu Val
    610                 615                 620

Gly Asp Pro Gly Trp Arg Thr Gly Pro Gly Asp Thr Val Leu Met His
625                 630                 635                 640

Ala Ser His Ala Phe Asp Ile Ser Leu Phe Glu Ile Trp Val Pro Leu
                645                 650                 655

Leu Ser Gly Ala Arg Val Met Ile Ala Gly Pro Gly Ala Val Asp Gly
            660                 665                 670

Ala Ala Leu Ala Ala Gln Val Ala Ala Gly Val Thr Ala Ala His Leu
        675                 680                 685

Thr Ala Gly Ala Phe Arg Val Leu Ala Glu Glu Ser Pro Glu Ser Val
    690                 695                 700

Ala Gly Leu Arg Glu Val Leu Thr Gly Gly Asp Ala Val Pro Leu Ala
705                 710                 715                 720

Ala Val Glu Arg Val Arg Arg Ala Cys Pro Asp Val Arg Val Arg His
                725                 730                 735

Leu Tyr Gly Pro Thr Glu Thr Thr Leu Cys Ala Thr Trp Trp Leu Leu
            740                 745                 750

Glu Pro Gly Asp Glu Thr Gly Pro Val Leu Pro Ile Gly Arg Pro Leu
        755                 760                 765

Ala Gly Arg Arg Val Tyr Val Leu Asp Ala Phe Leu Arg Pro Leu Pro
    770                 775                 780

Pro Gly Thr Thr Gly Glu Leu Tyr Val Ala Gly Ala Gly Val Ala Gln
785                 790                 795                 800

Gly Tyr Leu Gly Arg Pro Ala Leu Thr Ala Glu Arg Phe Val Ala Asp
                805                 810                 815

Pro Phe Ala Pro Gly Gly Arg Met Tyr Arg Thr Gly Asp Leu Ala Tyr
            820                 825                 830

Trp Thr Glu Gln Gly Thr Leu Ala Phe Ala Gly Arg Ala Asp Asp Gln
        835                 840                 845

Val Lys Ile Arg Gly Tyr Arg Val Glu Pro Gly Glu Val Glu Ala Val
    850                 855                 860

Leu Gly Gly Leu Pro Gly Val Ala Gln Ala Val Val Cys Val Arg Gly
865                 870                 875                 880

Glu His Leu Ile Gly Tyr Val Val Ala Glu Ala Gly Arg Asp Leu Asp
                885                 890                 895

Pro Glu Arg Leu Arg Ala Arg Leu Ala Ala Thr Leu Pro Glu Phe Met

900                 905                 910

Val Pro Ala Ala Val Leu Val Leu Ala Asp Leu Pro Leu Thr Val Asn
        915                 920                 925

Gly Lys Val Asp Arg Pro Ala Leu Pro Glu Pro Asp Phe Ala Ala Lys
    930                 935                 940

Ser Thr Gly Arg Ala Pro Ala Thr Ala Ala Glu Arg Ile Leu Cys Asp
945                 950                 955                 960

Val Phe Ala Gly Val Leu Gly Leu Asp Arg Val Gly Val Glu Asp Ser
                965                 970                 975

Phe Phe Ala Leu Gly Gly Asp Ser Ile Ser Ser Met Gln Val Ala Ala
            980                 985                 990

Arg Ala Arg Arg Glu Gly Ile Ala  Leu Thr Pro Arg Gln  Val Phe Glu
        995                 1000                 1005

His Arg  Thr Pro Glu Arg Leu  Ala Ala Leu Ala Pro  Ala Ala Gly
    1010                 1015                 1020

Ser Ala  Arg Pro Asp Arg Ser  Ala Ala Asp Ala Gly  Leu Gly Glu
    1025                 1030                 1035

Ile Pro  Trp Thr Pro Val Met  Arg Ala Leu Gly Asp  Asp Ala Val
    1040                 1045                 1050

Arg Pro  Gly Phe Ala Gln Ala  Arg Val Val Val Ala  Pro Ala Gly
    1055                 1060                 1065

Leu Asp  Pro Asp Ala Leu Thr  Gly Ala Leu Arg Ala  Val Leu Asp
    1070                 1075                 1080

Thr His  Asp Val Leu Arg Ala  Arg Val Glu Pro Asp  Arg Arg Leu
    1085                 1090                 1095

Ile Val  Pro Glu Arg Gly Ala  Val Ala Ala Ala Asp  Leu Leu Thr
    1100                 1105                 1110

Arg Val  Ala Val Asp Ser Asp  Gly Ile Asp Ala Arg  Ala Glu Arg
    1115                 1120                 1125

Glu Ala  Ala Thr Ala Ala Gly  Thr Leu Asp Pro Ser  Ala Gly Ile
    1130                 1135                 1140

Met Leu Arg Ala Val Trp Leu Asp Ala Gly Asp Ala Glu Pro Gly
    1145                1150                1155

Arg Leu Ala Leu Val Ala His His Leu Val Val Asp Ala Val Ser
    1160                1165                1170

Trp Ser Ile Leu Leu Pro Asp Leu Gln Ala Ala Tyr Gln Glu Val
    1175                1180                1185

Leu Ala Gly Ala Thr Pro Ala Leu Glu Pro Ala Ala Thr Ser Tyr
    1190                1195                1200

Arg Gln Trp Ala Arg Arg Leu Thr Glu Gln Ala Ser Ser Pro Ser
    1205                1210                1215

Thr Leu Ala Glu Leu Asp His Trp Val Thr Val Leu Asp Ala Ala
    1220                1225                1230

Glu Pro Pro Leu Ala Glu His His Gly Gln Ala His Ser Trp Ser
    1235                1240                1245

Ala Thr Leu Ser Gly Ala Val Ala Gly His Leu Val Ser Arg Met
    1250                1255                1260

Pro Gly Ala Phe His Cys Gly Ile Gln Glu Val Leu Leu Ala Gly
    1265                1270                1275

Leu Ala Ala Ala Val Ala Arg Trp Arg Gly Asp Asp Ala Gly Val
    1280                1285                1290

Leu Val Asp Val Glu Gly His Gly Arg His Ala Ala Asp Gly Glu
    1295                1300                1305

Asp Leu Leu Arg Thr Val Gly Trp Phe Thr Ser Val His Pro Val
    1310                1315                1320

Arg Leu Asp Val Ser Gly Val Asp Leu Ala Ala Ala Ala Ala Gly
    1325                1330                1335

Asp Ala Ala Ala Gly Glu Leu Leu Lys Ser Val Lys Asp Gln Val
    1340                1345                1350

Arg Ala Ala Pro Gly Asp Gly Phe Gly Phe Gly Leu Leu Arg His
    1355                1360                1365

Leu Asn Pro Asp Thr Ala Glu Arg Leu Ala Ala Leu Pro Ala Pro

1370                1375                1380

Gln Ile Gly Phe Asn Tyr Leu Gly Arg Ser Gly Val Ala Ala Glu
    1385                1390                1395

Ala Val Pro Trp Gln Val Arg Gly Gly Ser Leu Gly Ala Gly Glu
    1400                1405                1410

Ala Gly Pro Asp Leu Val Leu Ala His Pro Leu Glu Ala Gly Ala
    1415                1420                1425

Asp Val Arg Asp Thr Pro Asp Gly Pro Leu Leu Arg Leu Thr Leu
    1430                1435                1440

Asp Gly Arg Asp Leu Ala Pro Val Thr Val Glu Leu Leu Gly Glu
    1445                1450                1455

Ala Trp Leu Glu Leu Leu Thr Gly Leu Ala Thr His Ala Gly Asp
    1460                1465                1470

Pro Arg Ala Gly Gly His Thr Pro Ala Asp Phe Asp Leu Val Glu
    1475                1480                1485

Val Thr Gln Arg Asp Val Thr Ala Leu Glu Ala Ala Ala Pro Gly
    1490                1495                1500

Leu Thr Asp Ile Trp Pro Leu Ser Pro Leu Gln Arg Gly Met Leu
    1505                1510                1515

Phe Glu Arg Ala Leu Asp Glu Asp Gly Val Asp Val Tyr Gln Ser
    1520                1525                1530

Gln Arg Ile Leu Asp Leu Asp Gly Pro Leu Asp Ala Asp Arg Leu
    1535                1540                1545

Arg Ala Ala Trp Arg Thr Leu Val Ala Arg His Glu Ser Leu Arg
    1550                1555                1560

Thr Ser Phe His Gln Leu Glu Ser Gly Glu Thr Val Gln Val Val
    1565                1570                1575

Val Asp Gln Ala Asp Ile Gly Trp Arg Val Ala Asp Val Ser His
    1580                1585                1590

Arg Ala Glu Ala Asp Ala Ala Ala Glu Val Gly Arg Leu Leu Ala
    1595                1600                1605

Glu Asp Gln Ala Gln Arg Phe Asp Val Thr Arg Ala Pro Leu Leu
    1610                1615                1620

Arg Leu Leu Leu Val Arg Leu Gly Ala Asp Arg His Arg Leu Val
    1625                1630                1635

Val Thr Ser His His Ile Val Leu Asp Gly Trp Ser Thr Pro Ile
    1640                1645                1650

Ile Leu Gly Glu Met Ser Ala Ala Tyr Ala Gly Ala Pro Ser Thr
    1655                1660                1665

Ala Thr Ala Pro Ser Pro Arg Asp Tyr Leu Ala Trp Leu Asn Arg
    1670                1675                1680

Gln Asp Asp Ala Ala Thr Arg Ala Ala Trp Arg Ser Glu Leu Ala
    1685                1690                1695

Gly Cys Asp Glu Pro Thr Leu Val Asp Ala Asp Ala Asp Lys Thr
    1700                1705                1710

Met Val Met Pro Gly Glu His Cys Glu Gly Leu Ser Glu Gln Ala
    1715                1720                1725

Thr Arg Arg Leu Thr Glu Phe Ala Arg Gly His Ala Val Thr Leu
    1730                1735                1740

Ser Thr Ile Leu Gln Gly Ala Trp Ala Leu Val Leu Ala Arg Leu
    1745                1750                1755

Ala Gly Arg Thr Asp Val Val Phe Gly Thr Val Val Ser Gly Arg
    1760                1765                1770

Pro Ala Glu Val Pro Asp Val Asp Arg Met Val Gly Met Phe Ile
    1775                1780                1785

Asn Thr Val Pro Val Arg Val Arg Leu Asp Gly Gly Arg Pro Val
    1790                1795                1800

Leu Asp Leu Leu His Asp Leu Gln Ala Arg Gln Ser Ala Leu Thr
    1805                1810                1815

Glu His Gln His Leu Gly Leu Pro Asp Ile Gln Arg Ala Ala Gly
    1820                1825                1830

Pro Gly Ala Leu Phe Asp Thr Ile Met Met Val Glu Asn Tyr Pro

1835                1840                1845

Met Asp Ala Ala Gly Leu Gly Gly Glu Gly Gly Val Arg Ile Gly
    1850                1855                1860

Ser Val Leu Thr Arg Thr Gly Thr Ser Tyr Pro Leu Thr Met Ser
    1865                1870                1875

Ala Ser Pro Gly Glu Arg Leu Gln Ile Arg Val Ser Tyr Arg Pro
    1880                1885                1890

Asp Arg Ile Glu Arg Glu Thr Ala Ala Glu Ala Ala Gly Gln Val
    1895                1900                1905

Val Arg Val Ile Glu Arg Ile Val Ala Glu Pro Ser Leu Ala Val
    1910                1915                1920

Gly Arg Leu Gly Val Thr Gly Glu Ser Val Arg Glu Arg Val Val
    1925                1930                1935

Glu Arg Trp Asn Ala Thr Gly Val Ala Ala Gly Gly Ser Ser Val
    1940                1945                1950

Leu Thr Gly Phe Arg Arg Arg Ala Arg Ser Ser Pro Asp Ala Val
    1955                1960                1965

Ala Val Ser Gly Gly Gly Arg Thr Leu Ser Tyr Ala Ala Leu Asp
    1970                1975                1980

Arg Glu Ser Asp Arg Leu Ala Gly His Leu Ala Gly Arg Gly Val
    1985                1990                1995

Arg Arg Gly Asp Arg Val Gly Val Val Met Glu Arg Gly Thr Asp
    2000                2005                2010

Leu Phe Val Ala Leu Leu Ala Ile Gly Lys Ala Gly Ala Ala Tyr
    2015                2020                2025

Val Pro Val Asn Leu Asp Tyr Pro Arg Asp Arg Ile Glu Arg Met
    2030                2035                2040

Leu Thr Asp Ala Gly Val Ser Val Ala Val Cys Val Gln Ala Thr
    2045                2050                2055

Ser Gly Ala Val Pro Asp Gly Leu Ala Pro Val Val Met Asp Ser
    2060                2065                2070

Pro Ala Ile Ala Ala Ala Pro Ser Glu Ala Pro Pro Ile Thr Val
    2075                2080                2085

Gly Ala His Asp Leu Ala Tyr Val Met Tyr Thr Ser Gly Ser Thr
    2090                2095                2100

Gly Val Pro Lys Gly Val Ala Val Pro His Gly Ser Val Ala Ala
    2105                2110                2115

Leu Ala Gly Asp Pro Gly Trp Ser Val Gly Pro Gly Asp Gly Val
    2120                2125                2130

Leu Met His Ala Pro His Ala Phe Asp Ala Ser Leu Leu Glu Ile
    2135                2140                2145

Trp Val Pro Leu Leu Ser Gly Ala His Val Val Val Ala Asp Pro
    2150                2155                2160

Gly Ala Val Asp Ala Gln Arg Leu Arg Glu Ala Ile Asp Arg Gly
    2165                2170                2175

Val Thr Thr Val His Leu Thr Ala Gly Ser Phe Arg Val Leu Ala
    2180                2185                2190

Glu Glu Ser Pro Asp Ala Phe Arg Gly Leu Arg Glu Val Leu Thr
    2195                2200                2205

Gly Gly Asp Ala Val Pro Leu Ala Ser Val Val Arg Leu Arg Glu
    2210                2215                2220

Thr Cys Pro Glu Ile Arg Val Arg His Leu Tyr Gly Pro Thr Glu
    2225                2230                2235

Thr Thr Leu Cys Ala Thr Trp His Leu Ile Glu Pro Gly Val Ala
    2240                2245                2250

Thr Gly Asp Thr Leu Pro Ile Gly Arg Pro Leu Ala Gly Arg Arg
    2255                2260                2265

Ala Tyr Val Leu Asp Ala Phe Leu Gln Pro Val Ala Pro Asn Val
    2270                2275                2280

Thr Gly Glu Leu Tyr Leu Ala Gly Ala Gly Leu Ala Arg Gly Tyr
    2285                2290                2295

Leu Gly Ala Ala Ala Ala Thr Ala Glu Arg Phe Val Ala Asp Pro

2300                2305                2310

Phe Ala Ala Gly Glu Arg Met Tyr Arg Thr Gly Asp Leu Ala Arg
    2315                2320                2325

Trp Thr Glu Gln Gly Glu Leu Leu Phe Ala Gly Arg Ala Asp Ala
    2330                2335                2340

Gln Val Lys Ile Arg Gly Tyr Arg Val Glu Pro Ala Glu Ile Glu
    2345                2350                2355

Ala Ala Leu Thr Ala Ile Pro Glu Val Ala Gln Ala Val Val Val
    2360                2365                2370

Ala Arg Glu Asp Gly Pro Gly Glu Lys Arg Leu Ile Ala Tyr Val
    2375                2380                2385

Thr Ala Ala Gly Gln Pro Gly Pro Asp Pro Ala Ala Val Arg Glu
    2390                2395                2400

His Leu Gly Glu Arg Leu Pro Glu Phe Met Val Pro Ala Ala Val
    2405                2410                2415

Val Val Leu Asp Gly Phe Pro Leu Thr Ile Asn Gly Lys Ile Asp
    2420                2425                2430

Arg Ala Ala Leu Pro Ala Pro Glu Arg Ser Gly Lys Pro Ala Gly
    2435                2440                2445

Arg Glu Pro Arg Thr Glu Val Glu Arg Val Leu Cys Asp Leu Phe
    2450                2455                2460

Ala Asp Leu Leu Gly Leu Asp Arg Val Gly Ala Asp Asp Ser Phe
    2465                2470                2475

Phe Glu Leu Gly Gly Asp Ser Ile Val Ser Met Gln Leu Ala Ala
    2480                2485                2490

Arg Ala Arg Arg Glu Gly Val Val Phe Gly Ala Lys Asp Val Phe
    2495                2500                2505

Glu Gln Lys Thr Pro Ala Ala Ile Ala Ala Val Ala Val Arg Gly
    2510                2515                2520

Gly Gln Arg Pro Ala Gly Pro Asp Asp Gly Val Gly Glu Val Ala
    2525                2530                2535

Trp Thr Pro Val Met Arg Gly Leu Leu Asp Arg Asp Pro Gly Thr
    2540                2545                2550

Met Asn Arg Ser Ala Met Ala Gln Trp Val Thr Val Gly Ala Pro
    2555                2560                2565

Asp Thr Leu Ser Arg Asp Val Leu Ala Ala Gly Leu Gly Ala Val
    2570                2575                2580

Leu Asp Ala His Asp Met Leu Arg Ser Arg Val Val Ala Ala Gly
    2585                2590                2595

Thr Glu Glu Pro Arg Leu Val Val Ala Pro Arg Gly Ala Val His
    2600                2605                2610

Ala Ala Gly Leu Val Asp Arg Val Glu Ala Gly Ala Gly Glu Val
    2615                2620                2625

Asp Asp Leu Thr Glu Arg Ser Ala Ala Glu Ala Ala Thr Arg Leu
    2630                2635                2640

Asp Pro Gly Ala Gly Val Met Val Arg Ala Val Trp Val Asp Ala
    2645                2650                2655

Gly Pro Gly Arg Val Gly Arg Leu Val Val Val Ala His His Leu
    2660                2665                2670

Val Val Asp Ala Val Ser Trp Arg Ile Leu Leu Pro Asp Leu Arg
    2675                2680                2685

Ala Ala Cys Glu Ala Val Ala Ala Gly Arg Glu Pro Ala Leu Asp
    2690                2695                2700

Pro Val Asp Val Ser Phe Arg Arg Trp Ala Arg Thr Leu Thr Asp
    2705                2710                2715

Gln Ala Ala Thr Arg Thr Ala Glu Leu Ala Thr Trp Thr Arg Ile
    2720                2725                2730

Leu Gly Gly Ala Gln Pro Arg Phe Gly Ala Leu Asp Pro Gly Arg
    2735                2740                2745

Asp Thr Ile Ser Thr Ala Gly Arg Arg Thr Trp Thr Val Pro Gln
    2750                2755                2760

Asp Arg Ala Gly Val Leu Val Glu Arg Val Thr Ser Ala Phe His

2765                    2770                    2775

Cys Gly Val His Glu Val Leu Leu Ala Thr Leu Ala Gly Ala Val
    2780                    2785                    2790

Ala Arg Trp Arg Gly Gly Ala Ala Val Val Val Asp Val Glu Gly
    2795                    2800                    2805

His Gly Arg Arg Pro Leu Gly Glu Met Asp Leu Ser Arg Thr Val
    2810                    2815                    2820

Gly Trp Phe Thr Asp Val His Pro Leu Arg Leu Asp Val Thr Gly
    2825                    2830                    2835

Ile Asp Thr Ala Glu Val Ile Ala Gly Gly Gly Ala Ala Gly Arg
    2840                    2845                    2850

Leu Leu Lys Thr Val Lys Glu Asn Val Arg Ala Val Pro Asp Gly
    2855                    2860                    2865

Gly Leu Gly Tyr Gly Met Leu Arg His Leu Asn Ala Glu Thr Gly
    2870                    2875                    2880

Arg Val Leu Ala Ala Gln Pro Ala Ala Glu Ile Gly Phe Asn Tyr
    2885                    2890                    2895

Leu Gly Arg Phe Ser Glu Gly Pro Gly Gly Asp Val Gln Pro Trp
    2900                    2905                    2910

Gln Gln Leu Gly Thr Ile Gly Gly Thr Ala Glu Pro Asp Met Pro
    2915                    2920                    2925

Leu Arg His Ala Val Glu Ile Asp Ala Ala Val Leu Asp Ala Ala
    2930                    2935                    2940

Asp Gly Pro Arg Leu Glu Leu Thr Val Thr Trp Ala Gly Arg Leu
    2945                    2950                    2955

Val Gly Ala Ala Glu Ala Glu Ser Leu Gly Arg Ala Trp Gln Asp
    2960                    2965                    2970

Val Leu Ala Gly Leu Ala Val His Ala Gly Asp Val Arg Ala Gly
    2975                    2980                    2985

Gly His Thr Pro Ser Asp Phe Pro Leu Thr Ala Leu Ser Gly Gln
    2990                    2995                    3000

Glu Val Ala Gln Ile Glu Ala Ala Val Pro Asp Leu Leu Asp Ile
    3005                3010                3015

Trp Pro Leu Ser Pro Leu Gln Glu Gly Leu Leu Phe His Ala Val
    3020                3025                3030

Asp Glu Arg Gly Pro Asp Val Tyr Ala Ser Met Arg Thr Leu Ala
    3035                3040                3045

Val Asp Gly Pro Leu Asp Val Ala Arg Phe Arg Ala Ser Trp Gln
    3050                3055                3060

Ala Val Leu Asp Arg His Ala Ala Leu Arg Ala Ser Phe His Gln
    3065                3070                3075

Leu Glu Ser Gly Ala Ala Val Gln Ala Ile Ala Arg Glu Val Thr
    3080                3085                3090

Leu Pro Trp Gln Glu Thr Asp Leu Ser Asp Leu Pro Glu Asp Val
    3095                3100                3105

Ala Leu Ala Glu Phe Asp Arg Leu Ala Ala Gln Leu Arg Asp Glu
    3110                3115                3120

Arg Phe Asp Leu Thr Arg Ala Pro Leu Leu Arg Leu His Leu Val
    3125                3130                3135

Arg Leu Gly Glu Arg Arg His Arg Leu Ala Phe Ala Ser His His
    3140                3145                3150

Ile Ser Cys Asp Gly Trp Ser Leu Pro Ile Val Ser Thr Glu Val
    3155                3160                3165

Met Ala Ala Tyr Glu Gly Arg Gly Leu Pro Ala Pro Thr Ser Tyr
    3170                3175                3180

Arg Asp Tyr Leu Val Trp Leu Ala Arg Gln Asp Lys Glu Ala Ala
    3185                3190                3195

Arg Gln Ala Trp Arg Gly Glu Leu Ala Gly Leu Asp Glu Ala Thr
    3200                3205                3210

His Val Val Pro Pro Asp Thr Ile Thr Ala Pro Ile Ala Pro Gln
    3215                3220                3225

Arg Val Arg Phe Gly Leu Asp Asp Glu Leu Ser Arg Arg Leu Val

3230                3235                3240

Arg Phe Thr Arg Arg His Gly Val Thr Met Asn Thr Leu Phe Gln
    3245                3250                3255

Gly Ile Trp Ser Leu Leu Leu Ala Arg Leu Thr Gly Arg Asp Asp
    3260                3265                3270

Val Val Phe Gly Ser Ala Val Ala Gly Arg Pro Ala Glu Ile Pro
    3275                3280                3285

Gly Val Gly Ser Val Val Gly Leu Phe Met Asn Thr Leu Pro Val
    3290                3295                3300

Arg Val Arg Leu Asp Gly Ala Glu Pro Phe Leu Asp Met Leu Thr
    3305                3310                3315

Asp Leu Gln Arg Arg Gln Val Ala Met Met Ala His Gln His Leu
    3320                3325                3330

Gly Leu Ser Glu Ile Lys Gln Val Ala Gly Pro Gly Ala Ala Phe
    3335                3340                3345

Asp Thr Leu Val Val Phe Glu Asn Tyr Pro Arg Pro Pro Arg Pro
    3350                3355                3360

Ser Asp Asp Pro Asp Ala Val Val Met Arg Pro Ala Glu Ile Pro
    3365                3370                3375

Glu Asp Ser Gly His Tyr Pro Val Ser Met Arg Ala Ser Glu Asp
    3380                3385                3390

Gly Gly Val His Gly Glu Phe Ile Phe Arg Pro Asp Ala Phe Asp
    3395                3400                3405

Arg Ala Glu Ala Glu Gly Met Leu Ala Ala Ile Ile Glu Ala Leu
    3410                3415                3420

Arg Gln Val Val Ala Glu Pro Gly Ile Ala Val Gly Arg Ile Gly
    3425                3430                3435

Leu Val Gly Ala Ala Glu Arg Arg Gln Val Val Arg Glu Trp Asn
    3440                3445                3450

Glu Thr Asp Ala Pro Leu Thr Ala Gln Pro Leu Pro Met Leu Phe
    3455                3460                3465

Arg Arg Gln Ala Gln Arg Ser Pro Asp Ala Val Ala Val Arg Asp
    3470                3475                3480

Ala Ala Arg Ser Leu Ser Phe Gly Ala Leu Leu Gly Glu Ala Glu
    3485                3490                3495

Gly Leu Ala Arg Arg Leu Val Ala Ser Gly Val Arg Arg Glu Thr
    3500                3505                3510

Arg Val Ala Val Leu Val Glu Arg Ser Ala Glu Leu Val Val Ala
    3515                3520                3525

Leu Leu Gly Val Ser Leu Ala Gly Gly Val Phe Val Pro Val Asp
    3530                3535                3540

Pro Asp Tyr Pro Gly Asp Arg Ile Ala Leu Met Leu Ala Asp Ala
    3545                3550                3555

Ala Pro Gln Val Leu Val Cys Thr Ala Arg Thr Arg Ser Val Val
    3560                3565                3570

Pro Gly Asp Phe Ala Gly Ala Val Leu Ala Leu Asp Glu Pro Ser
    3575                3580                3585

Ala Ala Gly Pro Gln Val Ser Leu Pro Arg Val Ala Ala Arg Asp
    3590                3595                3600

Gly Ala Tyr Val Ile Tyr Thr Ser Gly Ser Thr Gly Val Pro Lys
    3605                3610                3615

Gly Val Leu Val Thr His Ala Gly Leu Gly Asn Leu Ala Ser Ala
    3620                3625                3630

Gln Ile Glu Arg Phe Gly Val Thr Ser Ser Ser Arg Val Leu Gln
    3635                3640                3645

Phe Ala Ala Leu Gly Phe Asp Ala Ala Ile Ser Glu Val Cys Met
    3650                3655                3660

Ala Leu Leu Ser Gly Gly Ser Ile Val Leu Ala Asp Ala Glu His
    3665                3670                3675

Met Pro Pro Arg Val Ser Leu Gly Asp Ala Val Arg Arg Trp Asp
    3680                3685                3690

Ile Thr His Val Thr Val Pro Pro Ser Val Leu Ala Val Glu Asp

3695                3700                3705

Asp Leu Pro Asp Asp Leu Thr Leu Ile Val Ala Gly Glu Ala Cys
    3710                3715                3720

Pro Pro Ala Leu Val Asp Arg Trp Ser Pro Gly Arg Arg Met Ile
    3725                3730                3735

Asn Gly Tyr Gly Pro Thr Glu Thr Thr Val Cys Ala Thr Met Ser
    3740                3745                3750

Ser Pro Leu Ser Pro Gly Arg Gly Ala Val Pro Ile Gly Gly Pro
    3755                3760                3765

Ile Ala Asn Met Arg Thr Tyr Val Leu Asp Ala Phe Leu Gln Pro
    3770                3775                3780

Val Pro Pro Gly Met Thr Gly Glu Leu Tyr Val Thr Gly Val Gly
    3785                3790                3795

Leu Ala Arg Gly Tyr Leu Gly Arg Pro Gly Leu Thr Ala Glu Arg
    3800                3805                3810

Phe Val Ala Asp Pro Phe Ser Pro Gly Gly Arg Met Tyr Arg Thr
    3815                3820                3825

Gly Asp Arg Ala Arg Trp Thr Gly Asp Gly Glu Leu Val Phe Ala
    3830                3835                3840

Gly Arg Val Asp Ala Gln Val Lys Val Arg Gly Tyr Arg Ile Glu
    3845                3850                3855

Pro Gly Glu Ile Glu Ala Val Leu Ala Glu His Pro Gly Val Ala
    3860                3865                3870

Gln Val Val Val Val Ala Arg Gln Asp Gly Pro Gly Glu Lys Gln
    3875                3880                3885

Leu Val Ala Tyr Val Val Pro Ala Ala Gly Pro Thr Ala Glu Ala
    3890                3895                3900

Ser Thr Leu Ile Ser Ala Leu Arg Glu Ala Ala Ala Ala Arg Leu
    3905                3910                3915

Pro Glu His Met Val Pro Ala Ala Phe Val Pro Leu Asp Ala Met
    3920                3925                3930

Pro Leu Thr Pro Asn Gly Lys Val Asp His Arg Ala Leu Gln Ala
    3935                3940                3945

Pro Asp Phe Ala Gly Met Ser Ala Gly Arg Asp Pro Arg Thr Ala
    3950                3955                3960

Leu Glu Ala Lys Leu Cys Glu Leu Phe Ala Glu Val Leu Gly Leu
    3965                3970                3975

Glu Gln Val Gly Ala Asp Asp Ser Phe Phe Glu Leu Gly Gly Asp
    3980                3985                3990

Ser Ile Thr Ser Met Gln Leu Ser Thr Arg Ala Arg Arg Glu Gly
    3995                4000                4005

Leu Glu Leu Thr Pro Trp Gln Val Phe Asp Glu Lys Thr Pro Glu
    4010                4015                4020

Arg Leu Ala Ala Leu Val Lys Glu Ile Pro Ala Asp Gly Gly Ala
    4025                4030                4035

Thr Gly Ala Pro Glu Pro Ala Ala Gly Thr Leu Val Ala Leu Ser
    4040                4045                4050

Pro Glu Gln Met Asp Gln Leu Glu Ala Gly Pro Ala Gly Glu
    4055                4060                4065

<210> 13

<211> 1865

<212> PRT

<213> Actinoplanes teichomyceticus

<400> 13

Val Thr Val Asp Asp Thr Arg Ala Lys Pro Arg Ser Ser Val Glu Asp
1               5                   10                  15

Val Trp Pro Leu Ser Pro Leu Gln Glu Gly Met Leu Tyr His Thr Ala
            20                  25                  30

Leu Asp Gly Asp Gly Pro Asp Thr Tyr Thr Val Gln Thr Val Tyr Gly
        35                  40                  45

Ile Asp Gly Pro Leu Asp Ala Gly Arg Leu Arg Ala Ser Trp Gln Ala

50                  55                  60

Leu Val Asp Arg His Ala Ala Leu Arg Ala Cys Phe Arg Tyr Val Ser
65                  70                  75                  80

Gly Ala Gln Met Val Gln Val Ile Gln Arg Asp Val Glu Leu Pro Trp
                85                  90                  95

Arg Glu Thr Asp Leu Ser Gly Leu Pro Glu Asp Leu Ala Asp Gly Glu
            100                 105                 110

Val Ala Arg Leu Ala Glu Gln Glu Met Ala Glu Arg Leu Arg Leu Glu
        115                 120                 125

Ala Ala Pro Leu Met Lys Leu His Leu Ile Arg Leu Gly Pro Gln Arg
    130                 135                 140

His Arg Leu Val His Thr Leu His His Val Leu Val Asp Gly Trp Ser
145                 150                 155                 160

Met Pro Ile Leu His Arg Glu Leu Ala Ala Ile Tyr Ala Ala Gly Gly
                165                 170                 175

Asp Ala Ser Gly Leu Pro Pro Ala Val Ser Tyr Arg Asp Tyr Leu Ala
            180                 185                 190

Trp Leu Gly Arg Gln Asp Lys Glu Ser Ala Arg Ala Ala Trp Arg Asp
        195                 200                 205

Ala Leu Ala Gly Leu Asp Thr Pro Thr Thr Val Ala Pro Ala Asp Pro
    210                 215                 220

Ala Arg Val Pro Asp Ile Asp Thr Ala Met Val Glu Leu Ser Pro Glu
225                 230                 235                 240

Leu Thr Asp Asp Leu Leu Arg Leu Ala His Gly His Asp Leu Thr Leu
                245                 250                 255

Asn Thr Val Val Gln Gly Val Trp Ala Val Val Leu Ala Gln Leu Ala
            260                 265                 270

Gly Arg Thr Asp Val Val Phe Gly Ala Thr Ala Ser Gly Arg Pro Ala
        275                 280                 285

Glu Leu Ala Gly Val Glu Ala Met Val Gly Leu Leu Leu Ala Thr Leu
    290                 295                 300

Pro Val Arg Val Arg Leu Asp Gly Arg Gln Arg Val Val Asp Met Leu
305                 310                 315                 320

Ala Asp Leu Gln Arg Asp Gln Ser Ala Leu Arg Ala His Gln His Leu
                325                 330                 335

Gly Leu Gln Glu Val Gln Ala Val Val Gly Pro Gly Ala Val Phe Asp
            340                 345                 350

Thr Leu Val Ile Tyr Glu Asn Phe Pro Arg Thr Gly Leu Gly Gly Ser
        355                 360                 365

Ala Asp Asp Gly Leu Val Met Arg Pro Val Gln Lys Gly Arg Asp Ser
    370                 375                 380

Ser His Tyr Pro Phe Thr Leu Val Thr Gly Pro Gly Glu Arg Met Pro
385                 390                 395                 400

Val Ile Leu Tyr Tyr Asp Arg Gly Leu Phe Asp Gln Ala Ala Ala Thr
                405                 410                 415

Ser Val Leu Gly Ala Ile Val Arg Val Leu Glu Arg Leu Val Ala Glu
            420                 425                 430

Pro Gly Val Leu Val Gly Arg Leu Thr Leu Leu Gly Glu Ala Glu Arg
        435                 440                 445

Ala Val Val Val Gly Asp Trp Asn Ala Thr Ala Gly Pro Val Pro Gly
    450                 455                 460

Asp Ser Met Val Glu Leu Phe Asp Arg Arg Val Ala Ala Ala Pro Asp
465                 470                 475                 480

Thr Val Ala Ile Thr Asp Ala Asp Gly Ala Asp Leu Thr Tyr Ala Glu
                485                 490                 495

Val Asp Gln Ala Ser Asn Arg Leu Ala Ala His Leu Ala Asp Arg Gly
            500                 505                 510

Val Arg Arg Gly Asp Arg Val Gly Val Ala Val Glu Arg Ser Ala Asp
        515                 520                 525

Leu Pro Ile Val Phe Leu Ala Ile Trp Lys Ala Gly Ala Ala Tyr Val
    530                 535                 540

Pro Val Asp Val Gly Tyr Pro Ala Glu Arg Ile Ala Leu Ile Leu Ala

545                 550                 555                 560

Gly Ser Ala Val Ser Thr Val Ile Cys Thr Gln Ala Thr Glu Ala Ala
                565                 570                 575

Val Pro Gln Asn Val Pro Gln Arg Ala Thr Ile Val Leu Asp Ala Pro
            580                 585                 590

Pro Thr Arg Ala Ala Val Asp Ala Cys Ala Gly Thr Ala Pro Thr Phe
        595                 600                 605

Arg Pro Ser Ala Ala Asp Leu Ala Tyr Val Met Tyr Thr Ser Gly Ser
    610                 615                 620

Thr Gly Val Pro Lys Gly Val Ala Val Pro His Gly Ala Val Ala Gly
625                 630                 635                 640

Leu Ala Gly Asp Ala Gly Trp Arg Ile Gly Pro Gly Asp Gly Val Leu
                645                 650                 655

Met His Ala Thr His Val Phe Asp Pro Ser Leu Tyr Glu Met Trp Val
            660                 665                 670

Pro Leu Ala Thr Gly Gly Arg Val Leu Val Ala Ala Pro Gly Val Val
        675                 680                 685

Asp Ala Gly Gly Ile Arg Gln Ala Val Ala Arg Gly Ala Thr Ala Val
    690                 695                 700

His Leu Thr Ala Gly Thr Phe Arg Ala Leu Ala Glu Ala Ser Pro Asp
705                 710                 715                 720

Cys Phe Ala Gly Leu Arg Glu Val Gly Thr Gly Gly Asp Val Val Pro
                725                 730                 735

Ala His Thr Val Ala His Leu Arg Arg Ala Gln Pro Gln Leu Arg Val
            740                 745                 750

Arg Asn Thr Tyr Gly Pro Thr Glu Thr Thr Leu Cys Ala Thr Trp Lys
        755                 760                 765

Pro Ile Glu Pro Gly Ala Gln Leu Gly Pro Glu Leu Pro Ile Gly Arg
    770                 775                 780

Pro Met Thr Asn Arg Arg Ile Tyr Ile Leu Asp Ala Phe Leu Arg Pro
785                 790                 795                 800

Val Ala Pro Gly Val Ala Gly Glu Leu Tyr Ile Ala Gly Thr Gly Leu
                805                 810                 815

Ala Arg Gly Tyr Leu Ser Arg Pro Asp Leu Thr Ala Glu Arg Phe Val
            820                 825                 830

Ala Cys Pro Phe Leu Ala Gly Glu Arg Met Tyr Arg Thr Gly Asp Leu
        835                 840                 845

Ala Arg Trp Asn Arg Asp Gly Glu Val Val Phe Leu Gly Arg Ala Asp
    850                 855                 860

Asp Gln Val Lys Ile Arg Gly Tyr Arg Val Glu Leu Ala Glu Val Glu
865                 870                 875                 880

Ala Val Leu Ala Ala Gln Pro Gly Val Arg Glu Ala Val Val Val Ala
                885                 890                 895

Arg Glu Asp Arg Pro Gly Glu Arg Arg Leu Val Gly Tyr Val Val Ser
            900                 905                 910

Asp Ala Gly Glu Leu Asp Thr Glu Gln Ile Arg Gln Gln Met Ala Arg
        915                 920                 925

Val Leu Pro Gly Tyr Met Val Pro Val Ala Val Ile Gly Leu Val Ser
    930                 935                 940

Leu Pro Ile Thr Ala Asn Ser Lys Val Asp Arg Arg Ala Leu Pro Ala
945                 950                 955                 960

Pro Asp Leu Ala Gly His Ala Ser Ala Lys Ala Pro Glu Ser Ala Thr
                965                 970                 975

Glu Lys Val Leu Cys Ala Leu Tyr Ala Glu Ile Leu Gly Val Glu Arg
            980                 985                 990

Val Gly Val Asp Asp Ala Phe His Asp Leu Gly Gly Ser Ser Ala Leu
        995                 1000                1005

Ala Met Arg Leu Ile Ala Arg Ile Arg Glu Glu Leu Gly Val Asp
    1010                1015                1020

Leu Pro Ile Arg Gln Leu Phe Ser Ser Pro Thr Pro Ala Gly Val
    1025                1030                1035

Ala Arg Ala Leu Ala Ala Lys Ser Arg Pro Ala Leu Glu Ala Val

1040                    1045                    1050

Thr Arg Pro Glu Arg Val Pro Leu Thr Ala Arg Gln Leu Arg Ala
    1055                    1060                    1065

Trp Leu Leu Ala Arg Pro Ser Glu Glu Thr Arg Gly Arg His Leu
    1070                    1075                    1080

Ser Val Ala Leu Arg Leu Arg Gly Arg Leu Asp Val Ala Ala Leu
    1085                    1090                    1095

Glu Ala Ala Leu Arg Asp Val Ala Ala Arg His Glu Ile Leu Arg
    1100                    1105                    1110

Thr Thr Phe Pro Gly Asp Ala Gln Thr Val His Gln His Ile His
    1115                    1120                    1125

Asp Ala Ala Pro Val Arg Leu Thr Pro Val Pro Ala Thr Glu Glu
    1130                    1135                    1140

Asp Leu Pro Ala Arg Leu Ala Glu Arg Gly Glu Gln Leu Phe Asp
    1145                    1150                    1155

Leu Thr Arg Asp Met Pro Trp Arg Cys Glu Leu Phe Ala Leu Ser
    1160                    1165                    1170

Glu Lys Glu His Val Leu Ser Val Thr Val His Arg Ile Ala Ala
    1175                    1180                    1185

Asp Asp Asp Ser Met Asp Val Phe Phe Arg Asp Leu Ala Ala Ala
    1190                    1195                    1200

Tyr Gly Ala Arg Arg Ala Gly Arg Ala Pro Glu Arg Ala Pro Leu
    1205                    1210                    1215

Ala Leu Gln Phe Ala Asp Tyr Ala Ile Trp Glu Gln Arg Leu Leu
    1220                    1225                    1230

Asp Gly Glu Arg Glu Gln Asp Ser Leu Ile Asn Asp Gln Ile Thr
    1235                    1240                    1245

Phe Trp Arg Asn His Leu Ala Gly Ile Asp Gln Glu Thr Val Leu
    1250                    1255                    1260

Pro Phe Asp Arg Ala Arg Pro Ala Ile Pro Ser Arg Arg Ala Gly
    1265                    1270                    1275

Thr Val Ala Leu Arg Leu Asp Ala Gly Pro His Ala Arg Leu Ala
    1280                1285                1290

Glu Ala Val Glu Ser Ala Gly Ala Asp Met Pro Gln Leu Val Gln
    1295                1300                1305

Ala Ala Leu Ala Met Leu Leu Thr Arg Tyr Gly Ala Gly Thr Asp
    1310                1315                1320

Leu Val Ile Gly Thr Thr Leu Pro Arg Asp Glu Asp Leu Ile Asp
    1325                1330                1335

Leu Glu Pro Met Ile Gly Pro Phe Ala Arg Pro Phe Pro Val Arg
    1340                1345                1350

Thr Asp Leu Ser Ala Asp Pro Thr Phe Leu Glu Val Val Ala Arg
    1355                1360                1365

Val Gln Glu Ala Val Arg Glu Ala Arg Gln His Leu Asp Val Pro
    1370                1375                1380

Phe Glu Lys Ile Pro Glu Leu Leu Ala Leu Pro Gly Ser Leu Ser
    1385                1390                1395

Arg His Pro Val Tyr Gln Val Gly Leu Gln Val Arg Glu Glu Asp
    1400                1405                1410

Asn Gly Ala Trp Asp Ala Ala Glu Leu Pro Ala Leu Arg Thr Ser
    1415                1420                1425

Val Glu Pro Thr Gly Val Glu Ala Ile Glu Leu Asp Leu Ala Phe
    1430                1435                1440

Ala Leu Thr Glu Arg Arg Asn Asp Asp Asp Asp Glu Asp Gly Ile
    1445                1450                1455

Glu Gly Ala Leu His Tyr Ala Ala Asp Leu Phe Asp His Asp Thr
    1460                1465                1470

Ala Ala Ser Leu Ala Arg Arg Leu Val Arg Val Leu Glu Gln Val
    1475                1480                1485

Ala Glu Asp Pro Gly Arg Arg Ile Ser Asp Leu Asp Ile Leu Leu
    1490                1495                1500

Asp Asp Ala Glu Arg Gly Ala Pro Ala Ile Ala Pro Ala Arg Trp

1505                1510                1515

Ala Gly Ala Val Pro Ala Ala Val Ala Asp Leu Val Ala Ala Gly
    1520                1525                1530

Pro Leu Gly Ala Leu Val Leu Asp Glu Ala Gln Arg Pro Val Ala
    1535                1540                1545

Pro Gly Thr Val Gly Asp Leu Trp Val Thr Gly Pro Ala Val Asp
    1550                1555                1560

Ala Asp Thr Gly Gly Arg Pro Ser Val Pro Cys Pro Phe Gly Thr
    1565                1570                1575

Pro Gly His Arg Met Leu His Thr Gly Leu Leu Ala Arg Lys Ser
    1580                1585                1590

Pro Ala Arg Thr Leu Val Val Val Gly Glu Arg Arg Gly Ala Ser
    1595                1600                1605

Ala Ser Ala Lys Thr Gly Asp Tyr Glu Val Leu Leu Pro Leu Arg
    1610                1615                1620

Ala Ala Gly Asp Arg Ala Pro Leu Phe Cys Ile His Ala Ser Gly
    1625                1630                1635

Gly Leu Ser Trp Asn Tyr Gly Pro Leu Leu Arg His Leu Pro Pro
    1640                1645                1650

Asn Gln Pro Val Tyr Gly Val Gln Ala Arg Gly Leu Ala Arg Thr
    1655                1660                1665

Glu Pro Leu Pro Gly Ser Val Glu Glu Met Ala Ala Asp Tyr Val
    1670                1675                1680

Glu Gln Ile Arg Thr Val Gln Pro Thr Gly Pro Tyr His Leu Leu
    1685                1690                1695

Gly Trp Ser Leu Gly Gly Arg Ile Ala Gln Ala Met Ala Val Leu
    1700                1705                1710

Leu Glu Ala Asp Gly Glu Lys Val Gly Leu Leu Ala Leu Leu Ala
    1715                1720                1725

Ala Tyr Pro Val Tyr Met Gly Arg Lys Ala Thr Gly Thr Val Thr
    1730                1735                1740

Glu Glu Ala Ala Arg Asp Glu Asp Ala Leu Glu Arg Arg Lys Gln
    1745                    1750                    1755

Gln Glu Leu Glu Leu Ala Gly Gln Leu Val Gln Gly Ala Gly Ala
    1760                    1765                    1770

Arg Ser Arg Leu Glu Ala Val Met Arg Asn Leu Trp Glu Val Gly
    1775                    1780                    1785

Pro Ala His Thr Ala Ser Pro Phe Ala Ser Asp Val Leu Leu Phe
    1790                    1795                    1800

Val Ala Ser Val Asp Arg Pro Ala His Leu Pro Val Ser Glu Ala
    1805                    1810                    1815

Ile Ala Ser Trp Lys Asp Phe Thr Gly Gly Thr Ile Glu Pro His
    1820                    1825                    1830

Glu Ile Ala Thr Asn His Tyr Glu Met Val Gln Pro Ala Ala Leu
    1835                    1840                    1845

Ala Gln Ile Gly Ala Ile Val Ala Glu Lys Leu Arg Ser Arg Pro
    1850                    1855                    1860

Asp Ala
    1865

<210>  14

<211>  69

<212>  PRT

<213>  Actinoplanes teichomyceticus

<400>  14

Met Thr Asn Pro Phe Asp Asn Glu Asp Gly Ser Phe Leu Val Leu Val
1               5                   10                  15

Asn Gly Glu Gly Gln His Ser Leu Trp Pro Ala Phe Ala Glu Val Pro
            20                  25                  30

Asp Gly Trp Thr Gly Val His Gly Pro Ala Ser Arg Gln Asp Cys Leu
        35                  40                  45

Gly Tyr Val Glu Gln Asn Trp Thr Asp Leu Arg Pro Lys Ser Leu Ile

50                  55                  60

Ser Gln Ile Ser Asp
65

<210>  15

<211>  273

<212>  PRT

<213>  Actinoplanes teichomyceticus

<400>  15

Met Pro His Asp Pro Gly Ala Thr Arg Leu Leu Ala Ile Ser Pro His
1               5                   10                  15

Leu Asp Asp Ala Val Leu Ser Phe Gly Ala Gly Leu Ala Gln Ala Ala
            20                  25                  30

Gln Asp Gly Ala Asn Val Leu Val Tyr Thr Val Phe Ala Gly Ala Ala
        35                  40                  45

Gln Pro Pro Tyr Ser Pro Ala Ala Gln Arg Met His Thr Ile Trp Gly
    50                  55                  60

Leu Ala Pro Asp Asp Asp Ala Val Leu Tyr Arg Arg Lys Glu Asp Ile
65                  70                  75                  80

Ala Ala Leu Asp His Leu Arg Val Ala His Arg His Gly Arg Phe Leu
                85                  90                  95

Asp Ser Ile Tyr Arg Lys Leu Pro Asp Gly Arg Trp Leu Thr Ala His
            100                 105                 110

Val Glu Gly Arg Gln Lys Leu Ala Val Asn Asp His Ser Pro Asp Ser
        115                 120                 125

Asp His Asp Leu Val Gly Glu Val Ala Asp Asp Ile Arg Ser Ile Ile
    130                 135                 140

Asp Glu Phe Asp Pro Thr Leu Val Val Thr Cys Ala Ala Ile Gly Glu
145                 150                 155                 160

His Pro Asp His Glu Ala Thr Arg Asp Ala Ala Leu Phe Ala Thr His
                165                 170                 175

Glu Lys Asn Val Pro Val Arg Leu Trp Glu Asp Leu Pro Tyr Ala Val
            180                 185                 190

Phe Lys Ser Gly Ala Val Glu Leu Pro Gln Gly Phe Arg Leu Gly Ser
        195                 200                 205

Ala Asp Val Ser Ser Val Lys Pro Glu Met Arg Ser Gln Lys Phe Gln
    210                 215                 220

Ala Val Glu Arg Tyr Ser Ser Gln Met Val Leu Leu Asn Gly Ser Glu
225                 230                 235                 240

Asn Asn Leu Phe Asp Arg Leu Asp Glu His Ala Arg Gln Asn Ala Pro
                245                 250                 255

His Gly Gly Tyr Gly Glu Thr Thr Trp Pro Val Val Arg Ser Asp Asp
            260                 265                 270

Ser

<210>  16

<211>  593

<212>  PRT

<213>  Actinoplanes teichomyceticus

<400>  16

Val Val Ser Glu Thr Leu Thr His Pro Pro Ser Gln Asp Ser Ala Pro
1               5                   10                  15

Glu Ser Arg Pro His Arg Trp Ala Val Trp Arg Ser Pro Ala Gly Gln
            20                  25                  30

Pro Ala Trp Ala Arg Pro Ala Leu Leu Gly Ile Ala Leu Val Ala Ala
        35                  40                  45

Val Leu Tyr Ala Trp Asn Leu Arg Leu Val Asp Tyr Ala Pro Arg Tyr
    50                  55                  60

Ala Asp Ala Val Lys Ser Met Ser Gly Ser Trp Arg Ala Phe Leu Tyr
65                  70                  75                  80

Gly Ala Val Asp Pro Glu Ala Thr Tyr Thr Leu Asp Lys Leu Ala Gly

                85                  90                  95

Ser Phe Val Pro Gln Val Val Ser Ala Lys Ile Phe Gly Tyr His Ala
            100                 105                 110

Trp Ser Leu Ala Leu Pro Gln Val Ile Glu Gly Val Ile Ala Val Leu
        115                 120                 125

Val Met Tyr Arg Val Val Arg Arg Trp Val Gly Val Val Pro Gly Leu
    130                 135                 140

Leu Ala Ala Gly Ile Phe Thr Leu Thr Pro Val Ala Ala Ser Met Phe
145                 150                 155                 160

Gly His Ser Met Glu Asp Gly Ala Leu Val Met Cys Leu Val Leu Ala
                165                 170                 175

Val Asp Ala Tyr Gln Arg Ala Val Leu Glu Gly Arg Leu Arg Ser Leu
            180                 185                 190

Ile Gly Ala Gly Val Trp Val Gly Leu Gly Phe Gln Ala Lys Met Val
        195                 200                 205

Gln Ala Trp Met Ile Leu Pro Ala Leu Ala Val Gly Tyr Leu Leu Thr
    210                 215                 220

Ala Pro Ile Glu Leu Arg Arg Arg Val Arg His Leu Gly Ala Ala Gly
225                 230                 235                 240

Ala Val Met Leu Ala Val Ser Leu Ser Trp Val Met Leu Tyr Thr Leu
                245                 250                 255

Thr Pro Ala Ser Asp Arg Pro Tyr Ile Ser Gly Thr Thr Asn Asn Asn
            260                 265                 270

Ala Phe Ala Met Val Phe Gly Tyr Asn Gly Leu Gly Arg Val Gly Ile
        275                 280                 285

Asn Leu Pro Gly Ala Glu Glu Val Asn Gly Arg Asn Gly Val Pro Ala
    290                 295                 300

Pro Gly Pro Leu Gly Gly Pro Glu Gly Glu Gly Gln Arg Gly Thr Val
305                 310                 315                 320

Gly Thr Lys Ala Gln Asp Gln Arg Gly Thr Val Gly Thr Lys Ala Gln
                325                 330                 335

Asp Gln Arg Gly Thr Val Gly Thr Lys Ala Gln Asp Gln Arg Gly Thr
            340                 345                 350

Val Gly Thr Lys Ala Gln Asp Gln Lys Gly Met Val Gly Pro Lys Ala
        355                 360                 365

His Gly Gly Pro Pro Met Gly Pro Gly Ala Leu Pro Glu Gly Tyr Glu
    370                 375                 380

Ser Lys Met Glu Ser Arg Ala Asp Pro Met Gly Trp Thr Lys Leu Phe
385                 390                 395                 400

Asp Gly His Leu Gly Val Ala Ile Gly Trp Leu Phe Pro Leu Thr Leu
                405                 410                 415

Leu Ala Leu Leu Cys Gly Leu Trp Trp Arg Arg Arg Ala Glu Arg Thr
            420                 425                 430

Asp Pro Val Arg Gly Gly Leu Val Met Trp Gly Val Trp Leu Val Thr
        435                 440                 445

Phe Gly Phe Val Phe Ser Ala Ser Ala Val Ala His Thr Ala Tyr Val
    450                 455                 460

Ala Ser Leu Ala Pro Ala Ile Ala Ala Val Ser Ala Leu Gly Ile Val
465                 470                 475                 480

Met Phe Gly Arg Ala Tyr Arg Arg Gly Gly Arg Gln Gly Trp Ile Leu
                485                 490                 495

Pro Leu Ala Val Ala Val Gln Leu Ala Trp Ala Ala Trp Leu Trp Ser
            500                 505                 510

His Tyr Pro Asn Phe Leu Pro Trp Ala Arg Trp Gly Thr Leu Leu Leu
        515                 520                 525

Gly Val Ala Ala Leu Val Gly Leu Val Leu Ala Arg Val Thr Arg Thr
    530                 535                 540

Ala Ser Ser Ala Leu Val Ala Ala Ala Leu Ala Val Gly Val Gly Val
545                 550                 555                 560

Met Leu Ala Ala Pro Ala Thr Tyr Ala Val Ser Val Leu Asp Pro Asp
                565                 570                 575

Tyr Ser Gly Asn Ser Phe Asp Ala Asn Ala Gly Pro Ala Ser Gly Thr

580                 585                 590

Ala

<210>  17

<211>  648

<212>  PRT

<213>  Actinoplanes teichomyceticus

<400>  17
   Val Asp Val Val Leu Arg Phe Gly Gly Val Ser Lys Asn Asp Asp Asp
   1               5                   10                  15
   Pro Asp Pro Trp Gln Thr Leu Ile Gly Pro Gly Thr Leu Arg Arg Val
               20                  25                  30
   Leu Ala Tyr Phe Arg Pro His Ile Gly Lys Val Ala Leu Phe Val Leu
           35                  40                  45
   Val Ala Ala Leu Glu Ser Leu Val Val Val Val Val Pro Leu Leu Leu
       50                  55                  60
   Met Glu Leu Val Asn Gly Gly Ile Leu Lys Asp Asp Ala Gly Val Val
   65                  70                  75                  80
   Ala Ser Met Ala Gly Leu Ala Ala Gly Leu Ala Leu Val Gly Ala Leu
                   85                  90                  95
   Leu Gln Leu Val Ser Ala Tyr Ile Ser Gly Gln Ile Gly Gln Gly Val
               100                 105                 110
   Ser Tyr Asp Leu Arg Val Gln Ala Leu Arg His Val Gln Gln Leu Pro
           115                 120                 125
   Ile Ala Phe Phe Thr Arg Thr Gln Thr Gly Val Leu Val Gly Arg Leu
       130                 135                 140
   His Thr Glu Leu Ile Met Ala Gln Gln Ala Phe Ser Gln Leu Leu Met
   145                 150                 155                 160
   Ala Ala Thr Ser Ala Val Thr Val Ile Leu Val Met Ala Glu Leu Phe
                   165                 170                 175
   Tyr Leu Ser Trp Leu Val Ala Val Val Thr Leu Val Leu Ile Pro Leu
   180 185 190
   Phe Leu Val Pro Trp Ile Tyr Val Gly Arg Val Ile Arg Arg Arg Thr
   195 200 205
   Gly Arg Leu Met Asp Ala Asn Thr Gly Leu Gly Gly Leu Leu Gln Glu
   210 215 220
   Arg Phe Asn Val Gln Gly Ala Met Leu Ser Lys Leu Phe Gly Arg Pro
   225 230 235 240
   Arg Glu Glu Met Ala Glu Tyr Ala Asp Arg Ala Glu Arg Ile Arg Arg
   245 250 255
   Ile Gly Val Ser Leu Ser Val Tyr Gly Arg Leu Ala Phe Val Met Met
   260 265 270
   Ala Leu Met Ala Ser Leu Ala Thr Ala Leu Val Tyr Gly Ile Gly Gly
   275 280 285
   Gly Leu Val Leu Ala Gly Ala Phe Gln Leu Gly Thr Leu Val Ala Leu
   290 295 300
   Ala Thr Leu Leu Gly Arg Leu Phe Gly Pro Ile Thr Gln Leu Ser Gly
   305 310 315 320
   Met Gln Glu Thr Ala Gln Thr Val Val Val Ser Phe Ser Arg Ile Phe
   325 330 335
   Glu Val Leu Asp Leu Lys Gly Leu Ile Glu Glu Arg Pro Gly Ala Val
   340 345 350
   Ala Leu Ala Lys Asp Thr Ala Pro Glu Ile Glu Phe Asp Asp Val Ala
   355 360 365
   Phe Arg Tyr Pro Thr Ala Asp Glu Val Ser Leu Arg Ser Leu Glu Tyr
   370 375 380
   Val Arg Ala Glu Arg Glu Arg Gly Glu Thr Thr Pro Glu Val Leu Arg
   385 390 395 400
   Gly Val Ser Leu His Val Pro Ala Gly Thr Leu Thr Ala Leu Val Gly
   405 410 415
   Pro Ser Gly Ala Gly Lys Ser Thr Leu Thr His Leu Val Ser Arg Leu
   420 425 430
   Tyr Asp Pro Thr Gly Gly Val Ile Arg Val Gly Gly His Asp Leu Arg
   435 440 445
   Asp Leu Thr Phe Asp Ser Leu Arg Glu Ala Val Gly Val Val Ser Gln
   450 455 460
   Asp Ala Tyr Phe Phe His Asp Thr Ile Arg Gln Asn Leu Leu Tyr Ala
   465 470 475 480
   Arg Pro Thr Ala Thr Glu Asp Glu Leu Ile Glu Ala Cys Arg Gly Ala
   485 490 495
   Gln Ile Trp Asp Leu Ile Ala Ser Leu Pro Gly Gly Phe Asp Thr Val
   500 505 510
   Thr Gly Asp Arg Gly Tyr Arg Leu Ser Gly Gly Glu Lys Gln Arg Leu
   515 520 525
   Ala Ile Ala Arg Leu Leu Leu Lys Ala Pro Thr Ile Val Val Leu Asp
   530 535 540
   Glu Ala Thr Ala His Leu Asp Ser Glu Ser Glu Ala Ala Val Gln Arg
   545 550 555 560
   Ala Leu Lys Thr Ala Leu His Asn Arg Thr Ser Leu Val Ile Ala His
   565 570 575
   Arg Leu Ser Thr Ile Arg Glu Ala Asp Gln Ile Leu Val Ile Asp Asp
   580 585 590
   Gly Arg Val Arg Glu Arg Gly Thr His Asp Glu Leu Leu Ala Glu Gly
   595 600 605
   Gly Leu Tyr Ala Glu Leu Tyr His Thr Gln Phe Ala Lys Ala Gly Ser
   610 615 620
   Asp Ala Ala Glu Pro Ala Ser Asp Ala Ala Glu Leu His His Arg Glu
   625 630 635 640
   Pro Glu Pro Val His Gly Gly Gly
   645

<210>  18

<211>  70

<212>  PRT

<213>  Actinoplanes teichomyceticus

<400>  18

Met Met Thr Asn Pro Phe Asp Asn Glu Asp Gly Ser Phe Leu Val Leu
1               5                   10                  15

Val Asn Gly Glu Gly Gln His Ser Leu Trp Pro Ala Phe Ala Glu Val
            20                  25                  30

Pro Asp Gly Trp Thr Gly Val His Gly Pro Ala Ser Arg Gln Asp Cys
        35                  40                  45

Leu Gly Tyr Val Glu Gln Asn Trp Thr Asp Leu Arg Pro Arg Ser Leu
    50                  55                  60

Val Glu Gln Ala Asp Ala
65                  70

<210>  19

<211>  391

<212>  PRT

<213>  Actinoplanes teichomyceticus

<400>  19

Met Phe Glu Glu Ile Asn Val Val Arg Ala Ala Glu Leu His Arg Arg
1               5                   10                  15

Asp Arg Phe Asp Pro Val Pro Gln Leu Arg Ser Leu Met Ala Glu Gly
            20                  25                  30

Pro Leu Thr Thr Leu Gly Thr Glu Glu Ser Pro Gly Gly Arg Thr Ala
        35                  40                  45

Trp Leu Ala Thr Gly Tyr Asp Glu Ile Arg Gln Val Leu Ser Ser Asp
    50                  55                  60

Asp Phe Ser Ala Arg Leu Leu Tyr Gly Gly Thr Ala Ala Gly Ile Thr
65                  70                  75                  80

Trp Pro Gly Phe Leu Thr Gln Tyr Asp Pro Pro Glu His Thr Arg Leu

85                  90                  95

Arg Arg Met Val Ala Pro Ala Phe Ala Val Arg Arg Met Gln Lys Phe
            100                 105                 110

Gln Pro Gln Val Glu Arg Val Val Gln Asp Ser Leu Asp Ala Ile Glu
        115                 120                 125

Ala Leu Gly Gly Pro Val Asp Phe Val Pro Arg Phe Gly Trp Ser Val
    130                 135                 140

Ala Thr Thr Ala Thr Cys Asp Phe Leu Gly Ile Pro Arg Asp Asp Gln
145                 150                 155                 160

Ala Asp Leu Ala Arg Ser Leu His Ala Ser Arg Thr Glu Arg Ser Gly
                165                 170                 175

Lys Arg Arg Thr Ala Ala Gly Asn Lys Phe Met Thr Tyr Met Asn Lys
            180                 185                 190

Met Thr Ala Arg Thr Arg Arg Asp Pro Gly Asp Asp Met Phe Gly Val
        195                 200                 205

Val Val Arg Glu Tyr Gly Asp Glu Ile Thr Asp Ala Glu Leu Thr Gly
    210                 215                 220

Val Ala Ala Phe Val Met Gly Ala Gly Ala Asp Gln Val Ala Arg Phe
225                 230                 235                 240

Leu Ala Ala Gly Ala Trp Leu Met Ala Asp Asp Pro Glu Gln Phe Ala
                245                 250                 255

Leu Leu Arg Glu Lys Pro Asp Thr Val Pro Asp Trp Leu Asp Glu Val
            260                 265                 270

Ile Arg Tyr Leu Thr Thr Asp Glu Lys Thr His Pro Arg Val Ala Thr
        275                 280                 285

Asp Asp Val Arg Ile Gly Asp His Leu Ile Lys Ala Gly Asp Thr Val
    290                 295                 300

Thr Cys Ser Leu Leu Ala Ala Asn Arg Arg Asn Phe Pro Arg Pro Glu
305                 310                 315                 320

Asp Arg Phe Asp Ile Thr Arg Val Arg Pro Glu His Leu Ala Phe Gly
                325                 330                 335

His Gly Ile His His Cys Leu Gly Arg Ser Leu Ala Glu Leu Val Phe
            340                 345                 350

Arg Thr Ala Ile Pro Ala Leu Ala His Arg Phe Pro Thr Leu Arg Leu
        355                 360                 365

Ala Glu Pro His Arg Glu Ile Arg Leu Gly Pro Pro Pro Phe Asp Val
    370                 375                 380

Glu Ala Leu Leu Leu Asp Trp
385                 390

<210> 20

<211> 384

<212> PRT

<213> Actinoplanes teichomyceticus

<400> 20

Met Ala Leu Pro Leu Pro His Gln Arg Leu Arg Leu Asp Pro Val Pro
1               5                   10                  15

Glu Phe Glu Glu Leu Gln Lys Ala Gly Pro Leu His Glu Tyr Asp Thr
            20                  25                  30

Glu Pro Gly Met Asp Gly Arg Lys Gln Trp Leu Val Thr Gly His Asp
        35                  40                  45

Glu Val Arg Ala Ile Leu Ala Asp His Glu Arg Phe Ser Ser Met Arg
    50                  55                  60

Pro Val Asp Asp Glu Ala Asp Arg Ala Leu Leu Pro Gly Ile Leu Gln
65                  70                  75                  80

Ala Tyr Asp Pro Pro Asp His Thr Arg Leu Arg Arg Thr Val Ala Pro
                85                  90                  95

Ala Tyr Ser Ala Arg Arg Met Glu Arg Leu Arg Pro Arg Ile Glu Glu
            100                 105                 110

Ile Val Glu Glu Cys Leu Asp Asp Phe Glu Ser Val Gly Ala Pro Val
        115                 120                 125

Asp Phe Val Arg His Ala Ala Trp Pro Ile Pro Ala Tyr Ile Ala Cys

130                 135                 140

Glu Phe Leu Gly Val Pro Arg Asp Asp Gln Ala Glu Leu Ser Arg Met
145                 150                 155                 160

Ile Arg Glu Ser Arg Glu Ser Arg Leu Pro Arg Gln Arg Thr Leu Ser
                165                 170                 175

Gly Leu Gly Ile Val Asn Tyr Thr Lys Arg Leu Thr Ser Gly Lys Arg
            180                 185                 190

Arg Asp Pro Gly Asp Gly Met Ile Gly Val Ile Val Arg Glu His Gly
        195                 200                 205

Ala Glu Ile Ser Asp Glu Glu Leu Ala Gly Leu Ala Glu Gly Asn Leu
    210                 215                 220

Ile Met Ala Ala Glu Gln Met Ala Ala Gln Leu Ala Val Ala Val Leu
225                 230                 235                 240

Leu Leu Val Thr His Pro Asp Gln Met Ala Leu Leu Arg Glu Lys Pro
                245                 250                 255

Glu Leu Ile Asp Ser Ala Thr Glu Glu Val Leu Arg His Ala Ser Ile
            260                 265                 270

Val Glu Ala Pro Ala Pro Arg Val Ala Leu Ala Asp Val Arg Met Ala
        275                 280                 285

Gly Arg Asp Ile His Ala Gly Asp Val Leu Thr Cys Ser Met Leu Ala
    290                 295                 300

Thr Asn Arg Ala Pro Gly Asp Arg Phe Asp Ile Thr Arg Glu Lys Ala
305                 310                 315                 320

Thr His Met Ala Phe Gly His Gly Ile His His Cys Ile Gly Ala Pro
                325                 330                 335

Leu Ala Arg Leu Gln Leu Arg Val Ala Leu Pro Ala Val Val Gly Arg
            340                 345                 350

Phe Pro Ser Leu Arg Leu Ala Val Pro Glu Glu Asp Leu Arg Phe Lys
        355                 360                 365

Pro Gly Arg Pro Ala Pro Phe Ala Val Glu Glu Leu Pro Leu Glu Trp
    370                 375                 380

<210> 21

<211> 398

<212> PRT

<213> Actinoplanes teichomyceticus

<400> 21

Leu Ser Gly Asp Gly Ser Pro Pro Ile His Thr Arg Arg Gln Gly Phe
1               5                   10                  15

Asp Pro Ala Asp Glu Leu Arg Ala Ala Gly Thr Leu Thr Lys Ile Ser
            20                  25                  30

Ile Gly Ser Gly Ala Asp Ala Glu Thr Thr Trp Leu Ala Ala Gly His
        35                  40                  45

Ala Val Val Arg Gln Val Leu Gly Asp His Lys Arg Phe Ser Thr Arg
    50                  55                  60

Arg Arg Phe Asp Arg Arg Asp Glu Ile Gly Gly Thr Gly Val Phe Arg
65                  70                  75                  80

Pro Arg Glu Leu Val Gly Asn Leu Met Asp Tyr Asp Pro Pro Glu His
                85                  90                  95

Thr Arg Leu Arg His Leu Leu Thr Pro Gly Phe Thr Gln Arg Arg Met
            100                 105                 110

Arg Arg Leu Ala Pro Arg Ile Glu Glu Ile Val Thr Asp Arg Leu Asp
        115                 120                 125

Ala Met Glu Gln Ala Gly Pro Pro Ala Asp Leu Ile Glu Leu Phe Ala
    130                 135                 140

Asp Glu Val Pro Gly Ala Val Leu Cys Glu Leu Ile Gly Val Pro Arg
145                 150                 155                 160

Asp Asp Gln Ala Met Phe Leu Gln Leu Cys His Arg His Leu Asp Ala
                165                 170                 175

Ser Leu Ser Ala Arg Lys Arg Ala Ala Ala Gly Glu Ala Phe Ala Arg
            180                 185                 190

Tyr Leu Val Ala Met Met Ala Arg Glu Arg Lys Asp Pro Gly Asp Gly

195                 200                 205

Phe Ile Gly Ser Ile Val Ala Glu His Gly Asp Thr Ile Thr Asp Glu
    210                 215                 220

Glu Leu Arg Gly Val Cys Val Gln Leu Met Leu Ala Gly Asp Asp Asn
225                 230                 235                 240

Val Ser Gly Met Ile Gly Leu Gly Val Leu Ala Leu Leu Arg His Pro
                245                 250                 255

Glu Gln Ile Ala Ala Leu Arg Gly Asp Asp Gln Ser Ala Asp Arg Ala
            260                 265                 270

Val Asp Glu Leu Ile Arg Tyr Leu Thr Val Pro Tyr Ala Pro Thr Pro
        275                 280                 285

Arg Thr Ala Val Glu Asp Val Met Val Ala Asp Gln Val Ile Lys Glu
    290                 295                 300

Gly Glu Thr Val Leu Cys Ser Leu Pro Met Ala Asn Arg Asp Arg Ala
305                 310                 315                 320

Leu Leu Pro Asp Ala Asp Arg Leu Asp Val Thr Arg Thr Pro Val Pro
                325                 330                 335

His Val Ala Phe Gly His Gly Ile His His Cys Leu Gly Ala Ala Leu
            340                 345                 350

Thr Arg Leu Gln Leu Arg Ile Ala Tyr Thr Ala Leu Trp Arg Arg Phe
        355                 360                 365

Pro Ala Leu Gln Leu Ala Asp Pro Ala Gln Glu Ile Met Phe Arg Thr
    370                 375                 380

Ser Thr Pro Ala Tyr Gly Leu Thr Ser Leu Leu Val Ala Trp
385                 390                 395

<210>  22

<211>  506

<212>  PRT

<213>  Actinoplanes teichomyceticus

<400>  22

Met Thr Ser Leu Ser Ser Asp Asp Ser Ile Asp Gly Trp Trp Lys Met
1               5                   10                  15

Pro Val Glu Glu Phe Asp Val Val Val Ala Gly Gly Gly Pro Ala Gly
            20                  25                  30

Ser Thr Val Ala Thr Leu Val Ala Met Gln Gly His Arg Val Leu Leu
        35                  40                  45

Leu Glu Lys Glu Thr Phe Pro Arg Tyr Gln Ile Gly Glu Ser Leu Leu
    50                  55                  60

Pro Ser Thr Val His Gly Val Cys Arg Met Leu Gly Val Ser Asp Glu
65                  70                  75                  80

Leu Ala Ala Ala Gly Phe Pro Val Lys Arg Gly Gly Thr Phe Arg Trp
                85                  90                  95

Gly Ala Arg Pro Glu Pro Trp Thr Phe Ser Phe Ala Val Ser Pro Leu
            100                 105                 110

Met Asn Gly Pro Thr Ser Phe Ala Tyr Gln Val Glu Arg Ala Arg Phe
        115                 120                 125

Asp Glu Ile Leu Leu Arg Asn Ala Gln Arg Lys Gly Val Asp Val Arg
    130                 135                 140

Glu Gly Cys Ser Val Thr Gly Val Ile Glu Gly Asp Glu Arg Ile Thr
145                 150                 155                 160

Gly Val Arg Tyr Thr Asp Pro Asp Gly Gly Glu His Glu Val Ser Ala
                165                 170                 175

Arg Phe Val Val Asp Ala Ser Gly Asn Lys Ser Arg Leu Tyr Ser Gln
            180                 185                 190

Val Gly Gly Val Arg Asn Tyr Ser Glu Phe Phe Arg Ser Leu Ala Leu
        195                 200                 205

Phe Gly Tyr Phe Glu Asn Gly Lys Arg Leu Pro Glu Pro Tyr Ser Gly
    210                 215                 220

Asn Ile Leu Ser Val Ala Phe Asp Ser Gly Trp Phe Trp Tyr Ile Pro
225                 230                 235                 240

Leu Ser Asp Thr Met Thr Ser Val Gly Ala Val Val Arg Arg Glu Met

                245                 250                 255

Ala Glu Lys Ile Gln Gly Asp Arg Glu Lys Ala Leu Gln Ala Leu Ile
            260                 265                 270

Ala Glu Cys Pro Leu Ile Ser Asp Tyr Leu Ala Asp Ala Arg Arg Ile
        275                 280                 285

Thr Thr Gly Arg Tyr Gly Glu Leu Arg Val Arg Lys Asp Tyr Ser Tyr
    290                 295                 300

His Gln Thr Ser Tyr Trp Arg Pro Gly Met Ile Leu Val Gly Asp Ala
305                 310                 315                 320

Ala Cys Phe Val Asp Pro Val Phe Ser Ser Gly Val His Leu Ala Thr
                325                 330                 335

Tyr Ser Ala Leu Leu Ala Ala Arg Ser Ile Asn Ser Val Leu Ala Glu
            340                 345                 350

Asp Leu Asp Glu Lys Thr Ala Leu Ser Glu Phe Glu Ala Arg Tyr Arg
        355                 360                 365

Arg Glu Tyr Gly Val Phe Tyr Glu Phe Leu Val Ser Phe Tyr Gln Met
    370                 375                 380

Asn Val Asn Glu Glu Ser Tyr Phe Trp Gln Ala Lys Lys Val Thr Asn
385                 390                 395                 400

Asn Lys His Thr Glu Ile Glu Ser Phe Val Glu Leu Ile Gly Gly Val
                405                 410                 415

Ser Ser Gly Glu Ala Ala Leu Ala Thr Ala Gly Arg Ile Ala Glu Arg
            420                 425                 430

Ser Ala Glu Phe Ala Ala Ala Val Asp Gln Met Ala Ala Ser Ala Asp
        435                 440                 445

Gly Asn Met Val Pro Met Tyr Lys Ser Glu Val Val Lys Gln Val Met
    450                 455                 460

Gln Glu Gly Gly Gln Val Gln Met Lys Ala Val Leu Gly Ala Asp Ala
465                 470                 475                 480

Glu Pro Glu Leu Pro Leu Phe Pro Gly Gly Leu Val Val Ser Pro Asp
                485                 490                 495

Gly Met Lys Trp Leu Pro Tyr Arg Pro Ala
            500                 505

<210>  23

<211>  392

<212>  PRT

<213>  Actinoplanes teichomyceticus

<400>  23

Met Arg Arg Thr Val Cys Asp Pro His Glu Asp Met Phe Ala Leu Arg
1               5                   10                  15

Ala His Gly Pro Leu Ile Arg Ile Gly Gly Asp Ala Ala Val Gln Met
            20                  25                  30

Gly Ala Asp Tyr Leu Trp Gln Ala Met Gly Tyr Asp Val Val Arg Gln
        35                  40                  45

Ile Leu Gly Asp His Glu Asn Phe Thr Thr Arg Leu Arg Leu Thr Ala
    50                  55                  60

Ser Glu Pro Ile Ser Gly Glu Gly Ala Thr Lys Pro Gln Glu Leu Val
65                  70                  75                  80

Gly Gln Ile Ser Thr Tyr Asp Pro Pro Glu His Thr Arg Leu Arg Arg
                85                  90                  95

Met Leu Thr Pro Glu Phe Thr Val Arg Arg Val Arg Arg Leu Glu Pro
            100                 105                 110

Ala Ile Gln Glu Leu Ile Asp Asp Arg Ile Asp Ala Met Glu Ala Ala
        115                 120                 125

Gly Pro Pro Val Asp Val Gln Thr Val Phe Ala Asp Pro Val Gly Gly
    130                 135                 140

Gly Ala Leu Cys Glu Leu Leu Gly Ile Pro Arg Asp Asp Arg Asn Gln
145                 150                 155                 160

Phe Met Arg Arg Val Arg Gln Asn Val Asp Leu Ser Arg Gly Tyr Lys
                165                 170                 175

Ala Arg Ala Ala Asp Ser Ala Ala Phe Asn Arg Tyr Leu Gln Ser Leu

180                 185                 190

Ile Thr Arg Gln Arg Lys Asp Pro Asp Glu Gly Phe Leu Gly Met Leu
        195                 200                 205

Val Arg Asp His Gly Asp Asp Ile Thr Asp Glu Glu Leu Lys Gly Val
    210                 215                 220

Cys Thr Ala Leu Leu Leu Gly Gly Val Glu Thr Val Ala Gly Met Leu
225                 230                 235                 240

Gly Phe Gly Val Leu Ala Leu Leu Asp His Pro Asp Gln Arg Arg Leu
                245                 250                 255

Leu Phe Ala Gly Arg Glu Glu Ala Asp Arg Leu Val Asn Glu Leu Leu
            260                 265                 270

Arg Tyr Leu Ser Ala Val Gln Gln Pro Thr Pro Arg Met Ala Val Lys
        275                 280                 285

Asp Val Val Val Asp Gly Gln Gln Ile Arg Ala Gly Glu Tyr Val Leu
    290                 295                 300

Cys Ser Ile Leu Met Ala Asn Arg Asp Glu Ala Leu Thr Pro His Pro
305                 310                 315                 320

Asn Val Leu Asp Ala Glu Arg Glu Pro Ala Pro His Ile Gly Phe Gly
                325                 330                 335

His Gly Ile His His Cys Ile Gly Ala Ala Val Ala Arg Thr Val Leu
            340                 345                 350

Arg Met Ala Tyr Gln Thr Leu Trp Arg Arg Leu Pro Gly Leu Arg Leu
        355                 360                 365

Ala Ile Pro Ala Gly Gln Val Lys Leu Arg Asn Ala Phe Ile Asp Ser
    370                 375                 380

Pro Asp Gln Leu Pro Val Thr Trp
385                 390

<210>  24

<211>  408

<212>  PRT

<213>  Actinoplanes teichomyceticus

<400> 24

Met Arg Val Leu Leu Ser Thr Thr Gly Gly Arg Gly Asp Val Glu Pro
1               5                   10                  15

Leu Val Ala Leu Ala Val Arg Phe Arg Glu Leu Gly Val Asp Ala Arg
            20                  25                  30

Val Cys Ala Pro Pro Asp Cys Ala Asp Arg Leu Ala Gly Val Gly Val
        35                  40                  45

Pro Leu Val Glu Val Gly Glu Ser Val Arg Ala Met Met His Gly Gly
    50                  55                  60

Lys Arg Pro Ser Pro Glu Asp Ala Pro Arg Leu Asp Ala Glu Ala Ile
65                  70                  75                  80

Ala Thr Gln Phe Asp Lys Val Pro Ala Ala Ala Glu Gly Cys Ala Ala
                85                  90                  95

Val Val Thr Thr Gly Leu Leu Ser Ala Ala Val Ala Val Arg Ser Val
            100                 105                 110

Ala Glu Lys Leu Arg Ile Pro Tyr Val Tyr Ala Ala Tyr Cys Pro Ile
        115                 120                 125

Tyr Leu Pro Ser Pro Tyr Tyr Pro Pro Pro Pro Ala Leu Gly Gly Pro
    130                 135                 140

Pro Val Pro Glu Leu Thr Asp Asn Arg Ala Gln Trp Asn Arg His Ser
145                 150                 155                 160

Gln Gly Ala Tyr Arg Arg Phe Gly Ala Ala Leu Asn Ser His Arg Ala
                165                 170                 175

Ala Ile Gly Leu Pro Pro Val Glu Asn Ile Tyr Asp Tyr Ala Phe Ser
            180                 185                 190

Asp His Pro Trp Leu Ala Ala Asp Pro Val Leu Ala Pro Leu Gln Pro
        195                 200                 205

Thr Asp Leu Asp Val Val Gln Thr Gly Ala Trp Ile Leu Pro Asp Gln
    210                 215                 220

Arg Pro Leu Ser Ala Glu Leu Glu Gly Phe Leu Arg Ala Gly Ser Pro

225                 230                 235                 240

Pro Val Tyr Val Gly Phe Gly Ser Gly Pro Ala Pro Ala Glu Ala Ala
                245                 250                 255

Arg Val Ala Ile Glu Ala Val Arg Ala Gln Gly Arg Arg Val Val Leu
            260                 265                 270

Ser Ser Gly Trp Ala Gly Leu Gly Arg Ile Asp Glu Gly Asp Asp Cys
        275                 280                 285

Leu Val Val Gly Glu Val Asn His Gln Val Leu Phe Gly Arg Val Ala
    290                 295                 300

Ala Val Val His Ala Gly Ser Ala Gly Thr Thr Thr Ala Val Thr Arg
305                 310                 315                 320

Ala Gly Ala Pro Gln Val Val Val Pro Gln Met Thr Asp Gln Pro Tyr
                325                 330                 335

Tyr Ala Gly Arg Val Ala Asp Leu Gly Val Gly Val Ala His Asp Gly
            340                 345                 350

Pro Thr Pro Thr Val Glu Ser Leu Ser Ala Ala Leu Ala Thr Ala Leu
        355                 360                 365

Thr Pro Gly Ile Arg Ala Arg Ala Ala Ala Val Ala Gly Thr Ile Arg
    370                 375                 380

Thr Asp Gly Thr Thr Val Ala Ala Lys Leu Leu Leu Glu Ala Ile Ser
385                 390                 395                 400

Arg Gln Arg Ser Ser Val Ser Ala
                405

<210>  25

<211>  323

<212>  PRT

<213>  Actinoplanes teichomyceticus

<400>  25

Met Asp Pro Glu Thr Val Arg Ile Ala Leu Gly Leu Glu Glu Arg Thr
1               5                   10                  15

Ala Ala Trp Leu Thr Glu Leu Asp Glu Leu Gly Pro Pro Ala Glu Pro
            20                  25                  30

Val Arg Leu Pro Arg Gly Glu Glu Ala Arg Asp Leu Leu Arg Arg Leu
        35                  40                  45

Glu Val Pro Glu Leu Asp Ala Glu Glu Ile Val Ala Ala Ala Pro Asp
    50                  55                  60

Pro Asp Arg Asp Pro Ala Leu Trp Trp Leu Leu Glu Arg Thr His His
65                  70                  75                  80

Ala Ile Val Arg His Met Gly Asp His Arg Ala Lys Pro Arg Gly Gly
                85                  90                  95

Pro Pro Leu Pro Tyr Glu Gly Gly Ala Ala Ala Arg Tyr Phe His Val
            100                 105                 110

Tyr Val Phe Leu Ala Thr Val Pro Ala Val Arg Arg Phe His Ala Glu
        115                 120                 125

Arg Gly Ile Pro Asp Glu Val Gly Trp Glu Thr Leu Thr Gln Leu Gly
    130                 135                 140

Glu Leu Val Ala Ile His Arg Arg Lys Tyr Gly Gln Gly Gly Met Asn
145                 150                 155                 160

Met Gln Trp Trp Thr Thr Tyr His Leu Arg Gly Ile Leu Tyr Arg Leu
                165                 170                 175

Gly Arg Leu Gln Phe Ser Leu Ala Thr Gly Lys Asp Gly Thr Pro His
            180                 185                 190

Leu Gly Leu His Val Pro Glu Trp Gly Gly Pro Leu Leu Pro Lys Ala
        195                 200                 205

Tyr Asp Glu Ser Leu His Arg Ala Arg Pro Phe Phe Asp Arg His Phe
    210                 215                 220

Pro Glu His Gly Ala Arg Val Ala Trp Gly Ser Ser Trp Met Leu Asp
225                 230                 235                 240

Pro Gln Leu Glu Glu Tyr Leu Thr Glu Asp Ser Asn Ile Ile Gln Leu
                245                 250                 255

Ala Arg Phe Trp Thr Leu Thr Asp Ser Ala Pro Glu Pro Gly Asn Ala

            260                 265                 270

Asp Gly Asp Ser Ser Ile Leu Glu Phe Val Phe Arg Tyr Asn Gly Gln
        275                 280                 285

Pro Leu Asp Glu Leu Pro Gln Arg Ser Ser Leu Glu Arg Ala Val Ile
    290                 295                 300

Ala His Leu Lys Ala Gly Arg His Trp His Met Arg Thr Gly Phe Val
305                 310                 315                 320

Lys Leu Pro

<210> 26

<211> 530

<212> PRT

<213> Actinoplanes teichomyceticus

<400> 26

Val Asn Glu Glu Arg Leu Phe Leu Arg Pro Asn Thr Ile Ile Glu Pro
1               5                   10                  15

Leu Val Asp Arg Phe Tyr Ala Ser Met Tyr Ala Thr Ala Pro Val Thr
            20                  25                  30

Ala Thr Met Ser Leu Ala Phe Arg Trp Ile Pro Met Leu Glu Ser Tyr
        35                  40                  45

Leu Gln Ala Pro Glu Trp His Tyr Lys Gly Ser Arg Asp Pro Lys Phe
    50                  55                  60

Arg Gly Gly Phe Phe Val Asn Ile Glu Asp Ser Arg Ala Asp Glu Val
65                  70                  75                  80

Arg Ala Leu Leu Ala Ala Ile Arg Arg Asp Arg Ala Asp Met Ile Arg
                85                  90                  95

Phe Ala Glu Ala Ile Ala Glu Ala Glu Lys Ile Val Arg Glu Glu Ala
            100                 105                 110

Thr Gly Tyr Asp Leu Arg Pro Leu Tyr Pro Lys Leu Pro Ala Glu Leu
        115                 120                 125

Ala Gly Leu Val Glu Ile Ala Tyr Asp Thr Ser Asn Ala Ala Ser Leu
    130                 135                 140

His Phe Gln Glu Pro Leu Val Tyr His Ser Lys Ala Tyr Thr Glu Gln
145                 150                 155                 160

Arg Gln Ser Val Gln Leu Ser Val Glu Thr Gly Ile Glu Arg Pro Phe
                165                 170                 175

Val Met Ser Thr Pro Arg Leu Pro Ser Pro Asp Val Leu Glu Leu Pro
            180                 185                 190

Ile Pro Phe Arg His Pro Gly Leu Glu Gln Leu Phe Leu Ser Arg Ile
        195                 200                 205

Arg Pro Thr Thr Leu Thr Ala Leu Arg Glu Ala Leu Glu Leu Gly Asp
    210                 215                 220

Ser Gln Ala Ala Gln Leu Ala Gly Leu Leu Val Pro Glu Pro Ala Leu
225                 230                 235                 240

Ala Ala Asp Arg His Ile Ala Ala Gly Ala Arg Ile Arg Tyr Trp Gly
                245                 250                 255

His Ala Cys Leu Leu Met Gln Thr Pro Asp Val Ala Ile Met Thr Asp
            260                 265                 270

Pro Phe Ile Ser Ala Asp Thr Asp Ala Thr Gly Arg Tyr Thr Tyr Asn
        275                 280                 285

Asp Leu Pro Asp Arg Ile Asp Tyr Val Leu Ile Thr His Gly His Ser
    290                 295                 300

Asp His Leu Val Pro Glu Thr Leu Leu Gln Leu Arg Gly Arg Val Gly
305                 310                 315                 320

Thr Phe Val Val Pro Arg Thr Ser Arg Gly Asn Leu Cys Asp Pro Ser
                325                 330                 335

Leu Ala Leu Tyr Leu Lys Ser Phe Gly Phe Ser Ala Val Glu Val Asp
            340                 345                 350

Asp Phe Asp Glu Ile Ser Phe Pro Gly Gly Lys Ile Ala Ala Thr Pro
        355                 360                 365

Phe Phe Gly Glu His Ala Asp Leu Asp Ile Arg Ala Lys Ala Thr Tyr

370                 375                 380

Trp Ile Asn Leu Gly Gly Lys Ser Ile Tyr Val Gly Ala Asp Ser Ser
385                 390                 395                 400

Gly Leu Asp Pro Ala Leu Tyr Arg Tyr Val Arg Arg His Leu Gly Arg
                405                 410                 415

Val Asp Ile Ala Phe Leu Gly Met Glu Cys Asp Gly Ala Pro Leu Asn
            420                 425                 430

Trp Gln Tyr Gln Ala Phe Leu Thr Lys Pro Ile Pro Asn Lys Met Ser
        435                 440                 445

Asp Ser Arg Lys Met Ser Gly Ser Asn Ala Glu Gln Ala Ser Ala Ile
    450                 455                 460

Val Thr Glu Leu Gly Ala Asp Glu Val Tyr Ile Tyr Ala Met Gly Glu
465                 470                 475                 480

Glu Ser Trp Leu Gly His Val Met Ala Thr Ser Tyr Asn Glu Asp Ser
                485                 490                 495

Phe Gln Leu Arg Gln Ile Ala Glu Phe Glu Thr Trp Cys Ala Asn Asn
            500                 505                 510

Gly Val Lys Ser Gly His Leu Leu Asp Arg His Glu Trp His Trp Ser
        515                 520                 525

His Pro
    530

<210>  27

<211>  598

<212>  PRT

<213>  Actinoplanes teichomyceticus

<400>  27

Met Gly Tyr Asp Met Ala Thr Ala Pro Asp Leu Val Thr Leu Ile Arg
1               5                   10                  15

Gln His Val Ala Gly Arg Pro Asp Ala Asp Ala Val Gly Phe Leu Thr
            20                  25                  30

Asp Pro Asp Asp Ile Arg Gly Gly Val Val Ser Trp Thr Tyr Gly Gln
        35                  40                  45

Leu Asp Arg Glu Ala Arg Gly Tyr Ala Ala Trp Leu Gln Gln Arg Leu
    50                  55                  60

Pro Ala Gly Ser Arg Val Leu Leu Leu Tyr Pro Asn Gly Leu Asp Phe
65                  70                  75                  80

Val Ala Ala Phe Phe Gly Cys Leu Tyr Ala Gly Met Ile Ala Val Pro
                85                  90                  95

Ala Pro Leu Pro Gly Arg Tyr Arg His His Gln His Arg Val Ala Thr
            100                 105                 110

Ile Ser Ala Asp Ala Arg Val Ser Ala Val Leu Thr Thr Ala Ala His
        115                 120                 125

Leu Gly Glu Val Arg Asp Trp Ala Arg Ala Cys Gly Leu Asp His Leu
    130                 135                 140

Leu Ile Ala Val Gly Asp Glu Pro Glu Phe Gly Asp Pro Ala Gly Trp
145                 150                 155                 160

Thr Pro Ala Ser Pro Glu Arg Ala Thr Ile Ala Leu Leu Gln Tyr Thr
                165                 170                 175

Ser Gly Ser Thr Gly Asp Pro Lys Gly Val Val Val Thr His Asp Asn
            180                 185                 190

Ile Leu Tyr Asn Leu Asp Ala Cys Val Arg Gly Leu Arg Trp Pro Asp
        195                 200                 205

Asp Trp Arg Val Gly Gly Trp Leu Pro Leu Tyr His Asp Leu Ala Met
    210                 215                 220

Gln Gly Leu Leu Asn Met Ala Val Val Arg Gly Gly Tyr Ala Leu Leu
225                 230                 235                 240

Met Glu Pro Val Ser Phe Val Arg Asp Pro Val Arg Trp Leu Arg Thr
                245                 250                 255

Ile Ala Glu His Asp Ile Gln Val Thr Phe Ala Pro Thr Phe Ala Tyr
            260                 265                 270

Gln Leu Cys Leu Asp Arg Val Thr Asp Glu Gln Leu Ala Ala Leu Asp

        275                 280                 285

Leu Ser Gly Trp Lys Ile Ala Gly Asn Ala Ala Glu Pro Val Asn Pro
    290                 295                 300

Ala Ile Leu Ala Ala Phe Ala Glu Lys Phe Ala Pro Ala Gly Phe Arg
305                 310                 315                 320

Pro Glu Ser Phe Ala Pro Ile Tyr Gly Met Ala Glu Ala Thr Gly Tyr
                325                 330                 335

Ile Ser Gly Glu Val Gly Arg Ala Pro Leu Val Arg Thr Val Gly Leu
            340                 345                 350

Asp Ala Leu Ala His Gly Arg Leu Ala Asp Pro Ala Pro Asp Glu Pro
        355                 360                 365

Val Arg Glu Ile Val Ser Cys Gly Thr Pro Asn Glu Ala Cys Asp Ile
    370                 375                 380

Arg Val Val Asp Pro Glu Thr Ser Arg Val Arg Pro Asp Gly Trp Leu
385                 390                 395                 400

Gly Glu Ile Trp Ile Arg Gly Arg Ser Val Ser Pro Gly Tyr Trp Ser
                405                 410                 415

Asp Ala Gly Pro Ala Phe Ala Ala Val Thr Ala Glu Gly Glu Asp Gly
            420                 425                 430

Phe Leu Arg Thr Gly Asp Leu Gly Val Leu Gln Asp Gly Glu Leu Tyr
        435                 440                 445

Val His Gly Arg Leu Lys Glu Thr Phe Thr Val His Gly Arg His Leu
    450                 455                 460

Tyr Pro His Asp Val Glu Gln Glu Leu Arg Ala Arg His Pro Glu Leu
465                 470                 475                 480

Gly Lys Cys Gly Ala Val Phe Pro Gly Arg Ala Pro Gly Ala Gly Gly
                485                 490                 495

Ala Arg Gly Val Val Val Thr His Glu Val Thr Asn Ala Ala Arg Asp
            500                 505                 510

Arg Leu Pro Glu Leu Ala Ala Gly Leu Arg His Thr Val Gly Arg Ala
        515                 520                 525

Phe Gly Val Glu Val Ser Ala Val Leu Leu Leu Arg Pro Gly Ala Val
    530                 535                 540

Leu Arg Thr Thr Ser Gly Lys Ile Arg Arg Ser Ala Met Arg Glu Leu
545                 550                 555                 560

Phe His Glu Gly Lys Leu Thr Ala Leu Tyr Gln His Pro Pro Ala Pro
                565                 570                 575

Arg Glu Pro Leu Pro Tyr Val Gly Asp Pro Ser Pro Ala Thr Arg Val
            580                 585                 590

Thr Ser Leu Ser Arg Pro
        595

<210>  28

<211>  351

<212>  PRT

<213>  Actinoplanes teichomyceticus

<400>  28

Met Val Val Val Met Ala Pro Glu Ala Thr Ser Ala Asp Val Asp Ala
1               5                   10                  15

Val Val Arg Leu Val Glu Ser Val Gly Gly Gln Ala Phe Val Ser Arg
            20                  25                  30

Gly Leu Ser Arg Thr Ile Val Gly Leu Val Gly Asp Val Ala Asp Phe
        35                  40                  45

Glu Ala Leu Asn Leu Gly Ser Leu Ala Gly Val Leu Arg Val Ile Arg
    50                  55                  60

Val Ser Val Pro Tyr Lys Leu Val Ser Arg Glu His His Pro Ala Arg
65                  70                  75                  80

Ser Val Ile Arg Ala Gly Gly Val Pro Ile Gly Pro Gly Thr Leu Thr
                85                  90                  95

Val Ile Ala Gly Pro Cys Ala Val Glu Ser Pro Glu Gln Thr Leu Glu
            100                 105                 110

Ala Ala Arg Met Ala Arg Ser Ala Gly Ala Ser Met Leu Arg Gly Gly

115                 120                 125

Ala Phe Lys Pro Arg Thr Ser Pro Tyr Ser Tyr Gln Gly Leu Gly Glu
    130                 135                 140

Arg Gly Leu Arg Ile Leu Ala Asp Val Arg Ala Glu Thr Gly Leu Ala
145                 150                 155                 160

Val Val Thr Glu Val Ile Asp Pro Gly Ser Val Asp Met Val Ala Ser
                165                 170                 175

Tyr Ala Asp Met Leu Gln Ile Gly Ala Arg Asn Met Gln Asn Phe Ala
            180                 185                 190

Leu Leu Gln Ala Val Gly Ser Ala Gly Lys Pro Val Leu Leu Lys Arg
        195                 200                 205

Gly Phe Ser Ala Thr Ile Glu Glu Trp Leu Ser Ala Ala Glu Tyr Ile
    210                 215                 220

Ala Gln Arg Gly Asn Leu Asn Ile Val Leu Cys Glu Arg Gly Ile Arg
225                 230                 235                 240

Thr Phe Glu Thr Ala Thr Arg Asn Thr Leu Asp Ile Ser Ala Val Pro
                245                 250                 255

Val Ala Gln Arg Leu Ser His Leu Pro Val Ile Val Asp Pro Ser His
            260                 265                 270

Ser Gly Gly Arg Arg Asp Leu Val Val Pro Leu Thr Arg Ala Ala Ile
        275                 280                 285

Ala Val Gly Ala Asp Gly Leu Leu Ile Asp Val His Pro Glu Pro Lys
    290                 295                 300

Thr Ala Leu Cys Asp Gly Asp Gln Ala Leu Pro Glu Ala Asp Ile Arg
305                 310                 315                 320

Glu Ile Ala Asp Ile Val Ala Ser Leu Pro Ala Phe Leu Gly Arg Glu
                325                 330                 335

Pro Ala Val Pro Ser Gly Glu Phe Arg His Val Pro Ala Ala Val
            340                 345                 350

<210>  29

<211>  329

<212>  PRT

<213>  Actinoplanes teichomyceticus

<400>  29

Leu Thr Pro Asp Glu Glu Ala Leu Asn Arg Gln Pro Ile Met Glu Met
1               5                   10                  15

Glu Ile Ser Ser Leu Ser Leu Gly Gly Ser Pro Arg Leu Ala Gly Gly
            20                  25                  30

Asp Pro Val His Leu Glu Ala Met Val Ala Ala Gln Gly Glu Leu Pro
        35                  40                  45

Pro Ile Val Val His Arg Pro Thr Met Arg Val Ile Asp Gly Ser His
    50                  55                  60

Arg Ile Gln Ala Ala Leu Arg Arg Gly Glu Thr Thr Ile Ala Gly Arg
65                  70                  75                  80

Phe Phe Asp Gly Ser Asp Asp Glu Ala Phe Val Met Ser Val Trp Leu
                85                  90                  95

Asn Val Ser His Gly Leu Pro Leu Ala Leu Ala Asp Arg Lys Arg Ala
            100                 105                 110

Ala Glu Arg Ile Ala Val Ser His Pro Gln Trp Ser Asp Arg Arg Val
        115                 120                 125

Ala Ala Val Thr Gly Ile Ser Pro Ser Thr Val Ala Asp Ile Arg Arg
    130                 135                 140

Arg Val Ala Gly Thr Ser Ala Pro Glu Ala Ser Arg Ile Gly Gln Asp
145                 150                 155                 160

Gly Arg Val Arg Pro Leu Asp Cys Ser Ala Gly Arg Leu Leu Ala Gly
                165                 170                 175

Arg Leu Met Ala Glu Asn Pro Ala Leu Ser Leu Arg Gln Val Ala Lys
            180                 185                 190

Ala Ala Ala Ile Ser Pro Glu Thr Ala Arg Asp Val Arg Asn Arg Leu
        195                 200                 205

Leu Ser Gly Ala Glu Leu Val Pro Asn Arg Arg Pro Arg Asp Ala Ala

210                 215                 220

Pro Val Gly Val Lys Gly Gly Arg Asp Arg Arg Pro Leu Asn Leu Ile
225                 230                 235                 240

Arg Ser Gly Asp Arg Pro Glu Pro Val Pro Asp His Ala Val Val Ile
                245                 250                 255

Asn Arg Leu Met Ser Asp Pro Ala Leu Arg Tyr Thr Asp Thr Gly Arg
            260                 265                 270

Asn Leu Leu Arg Leu Leu Ser Leu His Thr Arg Trp Ala Lys Glu Trp
        275                 280                 285

Glu Ala Ile Val Asp Asn Leu Pro Pro His Cys Ala Asp Ala Val Ala
    290                 295                 300

Asp Leu Ala Arg Gln Phe Ala Asp Leu Trp Ala Asp Phe Ala Ser Arg
305                 310                 315                 320

Val Gly Pro Glu Glu Arg Met Ala Ser
                325

<210>  30

<211>  792

<212>  PRT

<213>  Actinoplanes teichomyceticus

<400>  30

Val Leu Leu Val Ser Gly Ala Val Gly Thr Gly Lys Thr Ala Leu Leu
1               5                   10                  15

Gln Ala Met Ala Ala Arg Ala Gly Arg Arg Gly Gly Trp Ser Phe Val
            20                  25                  30

Val Thr Gly Ser Ala Arg Glu Arg Gly His Pro Phe Gly Val Leu Asp
        35                  40                  45

Arg Leu Ile Arg Ser Met Cys Ala Ala Gly Met Ala Glu Pro Phe Pro
    50                  55                  60

Gly Gly Met Asp Cys Gly Glu Asn Phe Phe Val Met Met Asp Arg Val
65                  70                  75                  80

Gly Ala Ala Ile Arg Glu Phe Ala Gly Asp Arg Pro Val Leu Val Gly
                85                  90                  95

Ile Asp Asp Val His Phe Val Asp Glu Gln Ser Leu Arg Ala Val Ser
            100                 105                 110

Tyr Leu Ile Arg Arg Ile Glu Ser Ser Gly Val Val Leu Val Leu Asn
        115                 120                 125

Glu Ser Thr Ser Tyr Glu Arg Asp Met Ala Gly Leu Arg Ala Glu Met
    130                 135                 140

Leu His Leu Pro Phe Cys Tyr Arg Ile Arg Leu Thr Pro Leu Thr Ser
145                 150                 155                 160

Thr Glu Val Ala Glu Arg Leu Ser Glu Arg Phe Gly Gly Val Pro Asp
                165                 170                 175

Arg Ala Ser Val Gln Phe Gly Val Gln Val Ser Gly Gly Ile Pro Leu
            180                 185                 190

Ile Leu Gln Ala Leu Ile Asp Asp Leu Ala Asp Ser Gly Pro Gly Val
        195                 200                 205

Cys Glu Pro Gly Ala Asn Phe Arg Gln Ala Val Leu Arg Ser Leu His
    210                 215                 220

Arg Cys Ala Pro Ser Thr Thr Ala Val Ala Gln Ala Ile Ala Val Leu
225                 230                 235                 240

Gly Asp Tyr Ala Ala Pro Glu Leu Val Ala Glu Leu Gly Gly Val Asp
                245                 250                 255

Ala Ala Leu Ile Glu Glu Ser Ile Arg Asp Leu Arg Glu Met Gly Leu
            260                 265                 270

Leu Glu His Asp Trp Phe Arg His Pro His Thr Arg Ala Ala Val Leu
        275                 280                 285

Ala Gly Ile Pro Val Pro Gln Leu Pro Glu Met His Arg Arg Ala Ala
    290                 295                 300

Glu Leu Leu His Gln Ser Gly Ala Pro Ala Gly Ala Val Ala Glu His
305                 310                 315                 320

Leu Ile Ala Ala Gln Asp Gly Gly Arg Ala Pro Trp Arg Val Val Ile

                325                 330                 335

Leu Ser Glu Ala Ala Arg Glu Ala Leu Ala Ala Gly Asp Val Asp Ser
            340                 345                 350

Ala Val Leu Ser Leu Arg His Ala Val Ala Ala Ser Ser Asp Glu Thr
        355                 360                 365

Gln Arg Ala His Ala Gly Val Leu Leu Ala Glu Ala Gln Trp His Thr
    370                 375                 380

Asp Pro Ser Arg Ala Ala Arg Arg Leu Gly Glu Leu Ser Gln Asp Ala
385                 390                 395                 400

Arg Ala Gly Arg Phe Thr Gly Pro Asp Thr Leu Ile Ala Val Asn Gln
                405                 410                 415

Leu Leu Trp Trp Gly Glu Phe Ala Glu Ala Asp Glu Leu Leu Arg Leu
            420                 425                 430

Ala Gly Asp Glu Glu Cys Gly Asp Ser Ser Leu Ala His Leu Trp Ala
        435                 440                 445

Ile Phe Ser Arg Ala Ala Gly Gly Thr Glu Ile Cys Asp Glu His Thr
    450                 455                 460

Pro Ser Gly Gly Ser Pro Leu Ala Arg Ser Gly Pro Met Ala Val Val
465                 470                 475                 480

Thr Tyr Leu Ser Ser Ala Ala Ser Leu Ala Ser Glu Ser Val Ala Ala
                485                 490                 495

Asp Arg Thr Glu Met Leu Leu Gly Ile Arg Ala Gly Ala Pro Leu Thr
            500                 505                 510

Pro Ala Leu Tyr Ala Leu Val Val Leu Val Gln Ser Gly Arg Leu Glu
        515                 520                 525

Glu Ala Ile Thr Trp Cys Gly Arg Leu Leu Lys Glu Glu Trp Ile His
    530                 535                 540

Arg Thr Pro Met Arg Arg Val Met Ile Gln Thr Ile Glu Ser Val Ala
545                 550                 555                 560

Met Leu Arg Ala Gly Asp Ser Ala Gly Ala Leu Arg Gly Ile Arg Glu
                565                 570                 575

Val Phe Asp Ala Val Pro Pro Pro Ala Trp Gly Val Val Ala Gly Leu
            580                 585                 590

Pro Leu Ser Val Ala Val Arg Ala Ser Thr Glu Leu Gly Asp Thr His
        595                 600                 605

Ala Ala Arg Ser Tyr Leu Ala Val Pro Val Pro Pro Ala Met Phe Asp
    610                 615                 620

Thr Pro Phe Ala Leu Pro Tyr Leu Leu Ala Leu Gly Trp Tyr His Leu
625                 630                 635                 640

Ala Met Gly His Pro Glu Ser Ser Leu Arg His Thr Arg Ser Cys Leu
                645                 650                 655

Asp Leu Thr Ala Arg Trp Gly Val Asp Ala Gly Arg Ile Thr Ala Gly
            660                 665                 670

Pro Ala Gly Leu Asp Val Ser Arg Pro Ser Pro Pro Pro Ala Ala Asp
        675                 680                 685

Arg Val Asp Ala Gly His Arg Thr Pro Ala Ala Val Arg Glu Pro Ala
    690                 695                 700

Pro Asp Gln Arg Glu Ala Thr Pro Val Pro Pro His Ala Gly Pro Asp
705                 710                 715                 720

Pro Ala Glu Pro Ala Ala Ala His Arg Gly Ala Glu Asp Gly Ala Arg
                725                 730                 735

Leu Thr Asp Ala Glu Gln Arg Val Ala Ala Leu Ala Ala Ala Gly Asn
            740                 745                 750

Thr Asn Arg Gln Ile Ala Glu Arg Leu Phe Ile Thr Val Ser Thr Val
        755                 760                 765

Glu Gln His Leu Thr Lys Ile Tyr Arg Lys Leu Asn Leu Arg Ser Arg
    770                 775                 780

Ser Gly Leu Arg Arg Tyr Ser His
785                 790

<210> 31

<211> 373

<212> PRT

<213>  Actinoplanes teichomyceticus

<400>  31

Val Gly Val Asp Val Pro Met Thr Ala Thr Leu Glu Arg Ala Ser Ala
1               5                   10                  15

Ser Pro Val Gly Ile Pro Pro Ala Ile Ser Arg Met Val Gly Val Gly
            20                  25                  30

Thr Ala Val Ala Gly Thr Thr Val Ser Gln Pro Ala Leu Leu Asp Leu
        35                  40                  45

Leu Gly Ile Glu Asp Pro Lys Ile Arg Ser Ile Tyr Leu Asn Ser Ala
    50                  55                  60

Ile Gly Thr Arg Arg Leu Thr Leu Pro Glu Pro Asp Ala Ser Gly Val
65                  70                  75                  80

Arg Arg Pro Glu Pro Gln Gly Ala Leu Leu Ala Lys His Lys Arg Leu
                85                  90                  95

Ala Val Glu Met Gly Gly Gln Ala Leu Gln Ala Cys Leu Lys Asn Ile
            100                 105                 110

Gly Ala Ser Leu Ser Asp Leu Arg His Leu Cys Cys Val Thr Ser Thr
        115                 120                 125

Gly Phe Leu Thr Pro Gly Leu Ser Ala Leu Met Ile Lys Glu Leu Gly
    130                 135                 140

Ile Asp Pro His Cys Ser Arg Ser Asp Ile Val Gly Met Gly Cys Asn
145                 150                 155                 160

Ala Gly Leu Asn Ala Leu Asn Val Val Ala Gly Trp Ser Ala Ala His
                165                 170                 175

Pro Gly Glu Leu Gly Val Val Leu Cys Thr Glu Ala Cys Ser Ala Ala
            180                 185                 190

Tyr Ala Leu Asp Gly Thr Met Arg Thr Ala Val Val Asn Ser Leu Phe
        195                 200                 205

Gly Asp Gly Ala Ala Ala Leu Ala Leu Ile Ser Asp Ala Pro Gly Gln
    210                 215                 220

Ser Arg Pro Gly Pro Arg Ile Leu Lys Phe Ala Ser Phe Leu Ile Thr
225                 230                 235                 240

Asp Ala Ile Asp Ala Met Arg Tyr Asp Trp Asp Ser Asp Leu Gly Arg
                245                 250                 255

Phe Ser Phe Phe Leu Asp Pro Gln Ile Pro Tyr Val Val Gly Ala Asn
            260                 265                 270

Ala Glu Thr Val Val Asp Arg Leu Leu Ala Gly Thr Gly Leu Arg Arg
        275                 280                 285

Arg Asp Ile Ala Gln Trp Leu Val His Ser Gly Gly Lys Lys Val Ile
    290                 295                 300

Asp Ala Val Val Val Asn Leu Gly Leu Thr Arg His Asp Val Arg His
305                 310                 315                 320

Thr Thr Arg Ile Leu Arg Asp Tyr Gly Asn Val Ser Ser Gly Ser Phe
                325                 330                 335

Leu Phe Ser Tyr Glu Arg Leu Val Glu Glu Gly Val Thr Arg Pro Gly
            340                 345                 350

Asp Tyr Gly Val Leu Met Thr Met Gly Pro Gly Ser Thr Ile Glu Thr
        355                 360                 365

Ala Leu Val Gln Trp
    370

<210>  32

<211>  221

<212>  PRT

<213>  Actinoplanes teichomyceticus

<400>  32

Val Thr Ala Ala Gly Val Asp Asp Leu Val Leu Arg Ile Asp Gly Ser
1               5                   10                  15

Glu Pro Val Thr Pro Ala Thr Val Glu Ala Val Leu Gly Ile Cys Asp
            20                  25                  30

Arg Ala Glu Asp Gly Tyr Ala Ala Gly Leu Leu Thr Val His Val Ser

35                  40                  45

Gly Val Pro Ala Pro Gly Trp Thr Ser Asp Leu Ser Val Gly Leu Thr
    50                  55                  60

Thr Lys Trp Glu Arg Ala Val Arg Arg Leu Glu Arg Leu Gly Leu Leu
65                  70                  75                  80

Thr Ala Gly Val Ala Thr Gly Asp Cys Gly Gly Ala Ala Leu Asp Leu
                85                  90                  95

Leu Leu Ala Thr Asp Val Arg Ile Ala Val Ala Gly Ala Arg Leu Val
            100                 105                 110

Val Pro Val Ser Gly Asp Ala Thr Trp Pro Gly Met Ala Leu Tyr Arg
        115                 120                 125

Leu Ala Arg Gln Gly Ala Thr Ala Gly Leu Arg Arg Ala Ala Leu Leu
    130                 135                 140

Gly Thr Pro Leu Asp Ala Ala Arg Ala Val Ala Ala Gly Val Ile Asp
145                 150                 155                 160

Val Val Val Asp Arg Pro Glu Pro Ala Leu Ala Glu Ile Ala Ala Ala
                165                 170                 175

Ala Ala Gly Ile Thr Gly Thr Glu Leu Ala Ile Arg Arg Gln Leu Met
            180                 185                 190

Leu Asp Ala Ala Glu His Ser Phe Glu Glu Ala Val Gly Ser His Leu
        195                 200                 205

Ala Ala Cys Asp Arg Thr Leu Arg Arg Ala Ala Ala Ser
    210                 215                 220

<210>  33

<211>  433

<212>  PRT

<213>  Actinoplanes teichomyceticus


<400>  33

Met Thr Val Thr Ala Val Pro Arg Asp Leu Gly Ala Asp Arg Lys Arg
1               5                   10                  15

Leu Ala Glu Ala Ala Ala Arg Ala Asp Arg Leu Ile Ala Ala Leu Pro
            20                  25                  30

Pro Pro Ser Arg Arg Thr Ala Gln Gln Arg Ala Glu Ala Ala Ala Ala
        35                  40                  45

His Asp Ala Ala Arg Arg Leu Arg Ala Gly Phe Leu Asn Ala Tyr Ala
    50                  55                  60

Asp Arg Val Tyr Asp Glu Leu Thr Glu Asp Arg Thr Arg Phe Leu Arg
65                  70                  75                  80

Ile Asp Glu Leu Ala Glu Ala Ala Ala Tyr Ala Phe Pro Gly Leu Ala
                85                  90                  95

Pro Thr Ala Gln Gln Leu Ala Arg Glu Arg Ser Arg Pro Gln Ala Asp
            100                 105                 110

Gln Glu Gly Leu Glu Ile Asp Gln Gly Ile Leu Leu Arg Ala Val Leu
        115                 120                 125

Arg Ser Pro Arg Ser Gly Arg His Leu Met Glu Ala Met Leu Arg Pro
    130                 135                 140

Thr Pro Arg Ala Leu Arg Leu Leu Ala Glu Phe Arg Arg Thr Gly Val
145                 150                 155                 160

Leu Gln Thr Glu Ala Val Arg Leu Glu Arg His Glu Gln Val Ala Glu
                165                 170                 175

Leu Thr Leu Cys Arg Asp Asp Cys Leu Asn Ala Glu Asp Glu Gln Gln
            180                 185                 190

Val Asp Asp Met Glu Thr Ala Val Asp Leu Ala Leu Leu Asp Pro Ala
        195                 200                 205

Val Arg Ile Gly Leu Val Arg Gly Gly Val Met Ser His Pro Arg Tyr
    210                 215                 220

Arg Gly Arg Arg Val Phe Ser Ala Gly Ile Asn Leu Lys Ala Ile Ser
225                 230                 235                 240

Ala Gly Arg Ile Ser Leu Thr Gly Phe Leu Leu Arg Arg Glu Leu Gly
                245                 250                 255

Tyr Leu His Lys Leu Val Arg Gly Leu Ser Asp Asp Asp Pro Gln Arg

            260                 265                 270

Trp Thr Pro Pro Val Glu Lys Pro Trp Val Ala Ala Val Asp Thr Phe
        275                 280                 285

Ala Ile Gly Gly Gly Cys Gln Leu Leu Leu Val Phe Asp His Val Leu
    290                 295                 300

Ala Ala Ser Asp Ala Tyr Leu Ser Leu Pro Ala Ala Arg Glu Gly Ile
305                 310                 315                 320

Val Pro Gly Ala Gly Asn Leu Arg Leu Gly Arg Ile Ala Gly Ala Arg
                325                 330                 335

Leu Ser Arg Gln Val Ile Leu Ala Gly Arg Arg Ile Ala Ala Ala Glu
            340                 345                 350

Pro Asp Ala Arg Leu Leu Val Asp Glu Val Val Pro Pro Glu Glu Met
        355                 360                 365

Asp Glu Ala Val Arg Arg Cys Leu Ala Arg Leu Ser Gly Asp Ala Val
    370                 375                 380

Val Ala Asn Arg Arg Met Ile Asn Val Ala Glu Glu Pro Leu Asp Ala
385                 390                 395                 400

Leu Arg Val Tyr Leu Ala Glu Phe Ala Leu Glu Gln Ala Leu Arg Ile
                405                 410                 415

His Ala Gly Asp Val Ile Asp Lys Val Gly Arg Phe Ala Glu Arg Ser
            420                 425                 430

Ala

<210> 34

<211> 268

<212> PRT

<213> Actinoplanes teichomyceticus

<400> 34

Val Ser Ser Val Thr Ala Ala Arg Val Arg Tyr His Arg Thr Gly His
1               5                   10                  15

Val Ala Arg Val Thr Leu Asp Arg Pro Asp Val Leu Asn Ala Met Asp
            20                  25                  30

Leu Arg Met His Glu Glu Leu Ala Arg Ile Trp Asp Asp Ile Glu Ala
        35                  40                  45

Asp Asp Arg Ile Arg Val Val Val Leu Thr Gly Ala Gly Asp Arg Ala
    50                  55                  60

Phe Ser Val Gly Gln Asp Leu Lys Glu Arg Ala Arg Arg Asp Ala Ala
65                  70                  75                  80

Gly Thr Pro Gly Ser Ser Phe Gly Ser Arg Gly Gln Pro Gly Trp Pro
                85                  90                  95

Arg Leu Thr Glu Arg Phe Gly Phe Ser Lys Pro Val Val Ala Arg Val
            100                 105                 110

Asp Gly Tyr Ala Leu Gly Gly Gly Phe Glu Leu Ala Leu Ala Cys Asp
        115                 120                 125

Leu Ile Ile Ala Ser Asp Arg Ser Val Phe Gly Leu Pro Glu Val Arg
    130                 135                 140

Leu Gly Leu Val Pro Gly Ala Gly Gly Val Phe Arg Leu Val Arg Gln
145                 150                 155                 160

Leu Pro Gln Lys Thr Ala Met Gly Tyr Leu Leu Thr Gly Arg Arg Phe
                165                 170                 175

Asp Ala Ala Thr Ala Leu Arg Phe Gly Leu Val Asn Glu Val Val Pro
            180                 185                 190

Ala Gln Arg Leu Asp Ala Cys Val Asp Gly Trp Val Asp Asp Leu Val
        195                 200                 205

Thr Ser Ala Pro Leu Ser Val Arg Ala Ile Lys Glu Ala Ala Leu Arg
    210                 215                 220

Ser Val Asp Leu Pro Leu Pro Asp Ala Phe Thr Ala Thr Tyr Arg Trp
225                 230                 235                 240

Glu Gln Arg Arg Arg Ala Ser Ala Asp Ala Val Glu Gly Pro Arg Ala
                245                 250                 255

Phe Ala Glu Arg Arg Ala Pro Arg Trp Ser Gly Thr

260                 265

<210>  35

<211>  453

<212>  PRT

<213>  Actinoplanes teichomyceticus

<400>  35

Met Ser Phe Glu Ala Pro Pro Ser Ser Leu Gly Gly His Ala Leu Leu
1               5                   10                  15

Ala Phe Leu Ile Ala Val Ala Thr Leu Leu Leu Val Ala His Leu Leu
            20                  25                  30

Ala Arg Leu Ala Glu Arg Val Arg Met Pro Ala Val Val Gly Glu Leu
        35                  40                  45

Ala Thr Gly Val Ile Leu Gly Pro Ser Leu Leu Gly His Leu Leu Pro
    50                  55                  60

Gly Leu Thr Asp Trp Val Phe Pro Pro Gly Thr Asp Gln Met His Leu
65                  70                  75                  80

Leu Asp Ala Val Gly Gln Ile Gly Ile Leu Leu Leu Val Gly Leu Thr
                85                  90                  95

Gly Thr His Leu Asp Val Ala Met Val Arg Arg Arg Lys Ala Thr Ala
            100                 105                 110

Ala Arg Ile Ser Leu Gly Gly Leu Leu Leu Pro Leu Ala Leu Gly Val
        115                 120                 125

Gly Val Gly Tyr Gln Leu Ala Gly Trp Leu Ser Gly Ala Val Val Gly
    130                 135                 140

Asp Arg Leu Val Phe Ala Gly Phe Met Gly Val Ala Met Cys Val Thr
145                 150                 155                 160

Ala Ile Pro Val Ile Ala Lys Thr Leu Ser Asp Met Arg Leu Leu His
                165                 170                 175

Arg Asp Val Gly Gln Met Thr Leu Ala Ala Gly Thr Ile Asp Asp Ala
            180                 185                 190

Val Gly Trp Phe Leu Leu Ser Val Val Ser Met Ala Ala Thr Ala Gly
        195                 200                 205

Leu Thr Thr Gly His Val Val Gln Ala Val Ala Tyr Leu Leu Gly Phe
    210                 215                 220

Val Val Leu Ala Val Leu Leu Gly Arg Pro Leu Val Arg Arg Val Met
225                 230                 235                 240

Arg Leu Ala Asp Arg Gly Glu Gly Ser Gly Ser Ser Ile Val Thr Ala
                245                 250                 255

Val Val Val Val Leu Thr Gly Ala Ala Ile Thr Gln Ala Leu Gly Met
            260                 265                 270

Glu Pro Val Phe Gly Ala Phe Val Ala Gly Ile Leu Val Gly Leu Pro
        275                 280                 285

Ala Ala Ala Asn Gln Ala Lys Leu Ala Ala Leu Arg Thr Val Val Leu
    290                 295                 300

Ser Val Leu Ala Pro Leu Phe Leu Ala Thr Ala Gly Phe Arg Met Asp
305                 310                 315                 320

Leu Thr Ala Leu Ala Asp Pro Gln Val Ala Ile Ala Ala Val Leu Val
                325                 330                 335

Leu Ala Ile Ala Ile Val Gly Lys Phe Ala Gly Ala Tyr Ala Gly Ala
            340                 345                 350

Arg Leu Ser Arg Leu Ser Arg Trp Glu Gly Leu Ala Ile Gly Ala Gly
        355                 360                 365

Met Asn Ser Arg Gly Val Val Glu Val Val Val Ala Leu Thr Gly Leu
    370                 375                 380

Arg Leu Gly Val Leu Asn Thr Ala Thr Tyr Thr Ile Val Val Leu Val
385                 390                 395                 400

Ala Ile Val Thr Ser Val Met Ala Pro Pro Leu Leu Arg Tyr Ala Cys
                405                 410                 415

Ala Arg Ile Ala Gln Asn Glu Asp Glu Arg Leu Arg Lys Leu His His
            420                 425                 430

Asp Thr Trp Asn Gly Val Pro Ala Gly Ala Gly Gln Ser Pro Ser Pro

435                 440                 445

Ala Gly Glu Gln Ala
    450

<210>  36

<211>  198

<212>  PRT

<213>  Actinoplanes teichomyceticus

<400>  36

Met Thr Val Thr Ala Lys His Val Gly Asn Thr Arg Pro Ala Pro Asp
1               5                   10                  15

Leu Ala Thr Ala Thr Ala Ala Ala Ala Thr Phe Leu Asp Ala Leu Gly
            20                  25                  30

Val Asp Leu Ser Pro Glu Ser Leu Gln Glu Thr Pro Ala Arg Met Ala
        35                  40                  45

Arg Ala Tyr Ala Asp Leu Leu Thr Ala Arg Glu Phe Lys Leu Thr Thr
    50                  55                  60

Phe Asp Asn Asp Glu Gly Tyr Asp Glu Leu Val Met Ala Arg Asn Ile
65                  70                  75                  80

Pro Ile Arg Ser Val Cys Glu His His Leu Leu Pro Phe Ile Gly Val
                85                  90                  95

Ala His Val Gly Tyr Leu Pro Ala Ala Arg Ile Leu Gly Leu Ser Lys
            100                 105                 110

Leu Ala Arg Val Val Glu Met Phe Ala His Gly Thr Gln Val Gln Glu
        115                 120                 125

Arg Leu Thr Lys Gln Ile Ala Asp Trp Leu Ile Lys Glu Leu Glu Pro
    130                 135                 140

Asn Gly Val Gly Val Val Ile Glu Ala Glu His Leu Cys Met Thr Leu
145                 150                 155                 160

Arg Gly Val Gln Ala Val Gly Ala Asn Thr Ile Thr Ser Ser Met Leu
                165                 170                 175

Gly Val Leu Arg Asp Asp Ala Arg Ser Arg Gly Glu Phe Leu Ala Ala
            180                 185                 190

Ala Gly Leu Gly Gly Arg
        195

<210>  37

<211>  365

<212>  PRT

<213>  Actinoplanes teichomyceticus

<400>  37

Val Glu Ser Ser Ala Leu Arg Lys Ser Glu Leu His Pro Ser Ile Ser
1               5                   10                  15

Asp Pro Val Leu Asp Thr Met Asn Phe Leu Asn Glu Val Thr Leu Arg
            20                  25                  30

Tyr Pro Glu Ala Ile Ser Phe Ala Pro Gly Arg Pro Tyr Asp Gly Phe
        35                  40                  45

Phe Gln Thr Glu Glu Val Leu Ala His Leu Arg Arg Phe Leu Asp His
    50                  55                  60

Leu Ala Asp Gln Gly Ala Ser Pro Ala Gln Val Arg Thr Ala Val Tyr
65                  70                  75                  80

Gln Tyr Gly Pro Thr Ala Gly Gln Ile Arg Glu Ile Ile Ala Asp Ser
                85                  90                  95

Leu Arg Arg Asp Glu Gly Ile Asp Val Pro Ala Glu Ser Ile Val Val
            100                 105                 110

Thr Val Gly Ala Gln Glu Ala Met Met Leu Val Val Arg Ala Leu Ile
        115                 120                 125

Arg Asp Pro Arg Asp Val Leu Leu Val Pro Ser Pro Cys Tyr Val Gly
    130                 135                 140

Ile Thr Gly Val Ala Arg Leu Leu Asp Val Thr Leu Gly Pro Val Pro
145                 150                 155                 160

Glu Ser Pro Asp Gly Phe Ser Leu Glu Asn Val Ala Ala Ala Ile Arg

                165                 170                 175

Ala Gln Arg Ala Ala Gly Arg Arg Pro Arg Ala Phe Tyr Leu Val Pro
            180                 185                 190

Asp His Ala Asn Pro Ser Gly Thr Thr Leu Ser Glu Ser Asp Arg Ala
        195                 200                 205

Gly Leu Leu His Leu Ala Ala Lys Glu Asp Leu Leu Ile Leu Glu Asp
    210                 215                 220

Thr Pro Tyr Arg Leu Val Ser Pro Gly Glu Pro Val Arg Thr Leu Lys
225                 230                 235                 240

Ala Leu Asp Arg Arg Arg Ser Val Val His Leu Gly Ser Tyr Ser Lys
                245                 250                 255

Thr Ile Phe Pro Gly Ala Arg Val Gly Phe Ala Val Ala Asp Gln Thr
            260                 265                 270

Val Val Gly Asp Asp Gly Arg Thr Gly Leu Leu Ala Asp Glu Leu Ser
        275                 280                 285

Lys Ile Lys Ser Met Val Thr Val Asn Thr Ser Ser Leu Ser Gln Ala
    290                 295                 300

Val Val Ala Gly Ala Leu Leu Ala Ala Gly Gly Arg Val Ser Glu Leu
305                 310                 315                 320

Asn Ala Lys Ala Ala Gly His Tyr Gly Asp Ala Met Arg Arg Val Leu
                325                 330                 335

Asp Arg Leu Ala Asp Arg Leu Pro Ala Ala Arg Arg Ala Glu Leu Gly
            340                 345                 350

Ala Arg Ile Arg Thr Asp Glu Ala Gly Thr Thr Gly Ala
        355                 360                 365

<210> 38

<211> 351

<212> PRT

<213> Actinoplanes teichomyceticus

<400> 38

Met Thr Gly His Phe Gln Asp Leu Thr Val Asp His Val Arg Ile Tyr
1               5                   10                  15

Cys Ala Asp Leu Asp Pro Leu Ile Ala Gln Phe Gly Ser Tyr Gly Leu
            20                  25                  30

Asp Val Arg Ala Glu Gly Val Gly Pro Gly Ala Glu His Ser Val Val
        35                  40                  45

Leu Gly His Gly Asp Ile Arg Leu Val Leu Thr Arg Pro Gly Thr Gly
    50                  55                  60

Asp His Pro Gly Gly Met Tyr Thr Ala Gln His Gly Tyr Gly Val Ser
65                  70                  75                  80

Asp Ile Ala Leu Gly Thr Ala Asp Ala Ala Gly Ala Phe His Glu Ala
                85                  90                  95

Val Arg Arg Gly Ala Arg Pro Ile Ala Ala Pro Glu Arg Thr Ala Gly
            100                 105                 110

Val Val Thr Ala Ser Val Ala Gly Phe Gly Asp Val Ile His Thr Phe
        115                 120                 125

Val Gln Arg Glu Pro Gly Gly Pro Trp Ser Leu Pro Gly Leu Asn Pro
    130                 135                 140

Val His Arg Pro Gly Thr Pro Gly Ile Gly Leu Arg Leu Val Asp His
145                 150                 155                 160

Phe Ala Val Cys Val Glu Ala Gly Arg Leu Thr Glu Val Val Glu His
                165                 170                 175

Tyr Glu Arg Val Phe Gly Phe Ser Ala Ile Phe Thr Glu Arg Ile Val
            180                 185                 190

Val Gly Glu Gln Ala Met Asp Ser Gln Val Val Gln Ser Ala Gly Gly
        195                 200                 205

Ala Val Thr Leu Thr Val Ile Ala Pro Asp Thr Thr Arg Arg Pro Gly
    210                 215                 220

Gln Ile Asp Thr Phe Leu Lys Asp His Gly Gly Pro Gly Val Gln His
225                 230                 235                 240

Ile Ala Phe Glu Thr Asp Asp Val Thr Arg Ser Val Gly Ala Met Ser

245                 250                 255

Asp Ala Gly Ile Glu Phe Leu Thr Thr Pro Ala Ala Tyr Tyr Glu Arg
            260                 265                 270

Leu Arg Asp Arg Leu Gln Leu Thr Arg His Ser Val Thr Glu Leu Ser
        275                 280                 285

Arg Leu Asn Val Leu Ala Asp Glu Asp His Asp Gly Gln Leu Tyr Gln
    290                 295                 300

Ile Phe Thr Lys Ser Thr His Pro Arg Gly Thr Leu Phe Phe Glu Ile
305                 310                 315                 320

Ile Glu Arg Val Gly Ala Arg Thr Phe Gly Ser Gly Asn Ile Lys Ala
                325                 330                 335

Leu Tyr Glu Ala Val Glu Leu Asp Gln Ala Ala Ala Asp Gly Arg
            340                 345                 350

<210>  39

<211>  364

<212>  PRT

<213>  Actinoplanes teichomyceticus

<400>  39

Met Thr Thr Ala Val Leu Arg Leu Asp Thr Val Asp Asp Phe Arg Leu
1               5                   10                  15

Arg Ala Glu Ala Thr Leu Thr Ala Ala Val Arg Asp Phe Val Ala Gly
            20                  25                  30

Gly Ser Asp Ser Glu Thr Thr Leu Ala Ala Asn Arg Ala Ala Leu Asp
        35                  40                  45

Arg Val Thr Ile Val Pro Arg Val Leu Thr Gly Gly Pro Asp Pro Glu
    50                  55                  60

Pro Gly Ala Thr Leu Ala Gly Ala Arg Ser Ala Leu Pro Leu Ala Val
65                  70                  75                  80

Ala Pro Met Ala Tyr Gln Arg Leu Leu His Pro Glu Gly Glu Leu Ala
                85                  90                  95

Thr Ala Arg Ala Ala Ala Ala Ala Gly Val Pro Phe Val Val Ser Thr
            100                 105                 110

Leu Ser Ser Val Pro Val Gly Glu Leu Ala Ala Ala Gly Gly Glu Gln
        115                 120                 125

Trp Phe Gln Leu Tyr Trp Leu Asn Asp Asp Arg Asp Thr Met Asn Leu
    130                 135                 140

Val His Arg Ala Glu Asp Ala Gly Cys Arg Val Leu Met Val Thr Val
145                 150                 155                 160

Asp Val Pro Val Met Gly Arg Arg Leu Arg Asp Ile Arg Asn Glu Phe
                165                 170                 175

Val Leu Pro Ala Glu Val Arg Ala Ala Asn Val Ala Ser Gly Ala Met
            180                 185                 190

Ser Ala Ala His Asp Arg Ala Asp Ala Gly Ser Ala Leu Ile Ala His
        195                 200                 205

Thr Asn Arg Ala Phe His Pro Ala Leu Thr Trp Ala His Leu Glu Ala
    210                 215                 220

Leu Arg Ser Arg Thr Thr Leu Pro Ile Val Val Lys Gly Ile Leu Asp
225                 230                 235                 240

Pro Ala Asp Ala Arg Arg Ala Ala Glu Ile Gly Ala Thr Gly Val Val
                245                 250                 255

Ile Ser Asn His Gly Gly Arg Gln Leu Asp Gly Ala Pro Ala Ser Val
            260                 265                 270

Thr Met Leu Pro Ala Ala Val Ala Ala Val Pro Asp Thr Cys Gln Val
        275                 280                 285

Phe Val Asp Ser Gly Ile Arg Ser Gly Thr Asp Ile Leu Arg Ala Leu
    290                 295                 300

Ala Leu Gly Ala His Gly Val Leu Ile Gly Arg Pro Met Leu Trp Gly
305                 310                 315                 320

Leu Ala Ala Gly Gly Glu Thr Gly Ala Ala Gly Val Leu Ala Val Leu
                325                 330                 335

Asp Ala Glu Leu Arg Ala Ala Met Arg Leu Ala Gly Cys Ala Asp Val

340                 345                 350

Ala Ala Ala Arg Arg Leu Thr Ala Thr Thr Gly Cys
        355                 360

<210>  40

<211>  250

<212>  PRT

<213>  Actinoplanes teichomyceticus

<400>  40

Met Pro Val Pro Trp Phe Ser Ala Pro Arg Pro Leu Ala Ala Pro Arg
1               5                   10                  15

Leu Arg Leu Val Cys Phe Pro Tyr Ala Gly Gly Asn Ala Ala Thr Tyr
            20                  25                  30

Arg Ser Trp Ala Gly Leu Leu Pro Pro Gly Val Glu Leu Val Ala Ala
        35                  40                  45

Leu Leu Pro Gly Arg Ala Glu Arg Leu Asp Glu Pro Pro Leu Val Asp
    50                  55                  60

Leu Asp Val Leu Leu Ser Glu Leu Val Ala Ala Ala Gly Pro Leu Leu
65                  70                  75                  80

Gly Pro Val Pro Leu Ile Leu Phe Gly His Ser Leu Gly Ala Thr Val
                85                  90                  95

Ala Tyr Glu Phe Gly Arg Ala Leu Ala Thr Glu His Gly Cys Val Pro
            100                 105                 110

Ala Ala Leu Leu Val Ser Gly Ala Pro Ala Pro Pro Val Arg Arg Arg
        115                 120                 125

Arg Thr Gly Arg Gly Leu Ser Asp Asp Asp Leu Glu Arg Ile Leu Gly
    130                 135                 140

Lys Arg Thr Asp Leu Pro Ala Gly Trp Leu Thr Ala Glu Leu Lys Pro
145                 150                 155                 160

Phe Val Phe Pro Ala Leu Arg Ala Asp Leu Gln Leu Leu Asp Gly Tyr
                165                 170                 175

Arg Trp Arg Pro Gly Pro Leu Pro Gly Val Pro Val Val Ala Phe Gly
            180                 185                 190

Gly Asp Ala Asp Pro Asp Val Ser Ala Ala Asp Leu Arg Ala Trp Gln
        195                 200                 205

Arg Cys Thr Thr Gly Pro Ala Arg Thr His Val Leu Ala Gly Asp His
    210                 215                 220

Phe Phe Ile Ala His Gln Pro Phe Arg Glu Leu Leu Ala Ala Thr Val
225                 230                 235                 240

Gly Glu Phe Ala Ala Ser Pro Ile Ala Arg
                245                 250

<210> 41

<211> 18

<212> DNA

<213> synthetic

<400> 41
atgcgcgtgt tgatctcg 18

<210> 42

<211> 18

<212> DNA

<213> synthetic

<400> 42
cggctgaccg cggcgaac 18

<210> 43

<211> 20

<212> DNA

<213> synthetic

<400> 43
cgtgggggtg gatgtatcga 20

<210> 44

<211> 17

<212> DNA

<213> synthetic

<400> 44
tcaccattgg atcagcg 17

<210> 45

<211> 20

<212> DNA

<213> synthetic

<400> 45
ttcctcgacc ggcgcatccg 20

<210> 46

<211> 21

<212> DNA

<213> synthetic

<400> 46
ggtgagcggc aggacactga g 21

<210> 47

<211> 21

<212> DNA

<213> synthetic

<400> 47
aggcagttcg tcacggtgta g 21

<210> 48

<211> 21

<212> DNA

<213>  synthetic

<400>  48
gccagcgtgc tcttcccgat c                                                21

<210>  49

<211>  36

<212>  DNA

<213>  synthetic

<400>  49
tttttcatat gcgtgtgttg ttttcgacga ccggag                                36

<210>  50

<211>  35

<212>  DNA

<213>  synthetic

<400>  50
tttttgtcga ccgcggagac cgacgatctc tgccg                                 35

## Description

### BACKGROUND OF THE INVENTION

Actinomycetes produce structurally diverse and biologically active secondary metabolites, many of which have found commercial application. Important metabolites are industrially produced not only by *Streptomyces* spp. (studied in most detail) but, for example, also by *Amycolatopsis, Actinoplanes* and *Saccharopolyspora* species. The genetic elements governing the biosynthesis of secondary metabolites are organized in gene clusters, which contain all the genes required for biosynthesis, regulation and resistance.

Many different secondary metabolites share a common biosynthetic route, where similar enzymes intervene. This has been thoroughly documented for polyketides (Katz and McDaniel 1999), non-ribosomally synthesized peptides (Marahiel 1997) and deoxysugars (Rodriguez et al. 2000). However, despite this similarity, the organization of the gene cluster involved in the synthesis of a particular secondary metabolite in a given microorganism cannot be defined a priori, and synthesis of very similar secondary metabolites may be governed by differently organized clusters, especially when the corresponding producer strains do not belong to the same genus. Furthermore, the identification of a desired cluster within a producer strain is complicated in actinomycetes by the occurrence of multiple clusters specifying enzymes for the same pathway (Omura et al. 2001; Bentley et al. 2002). Consequently, one cannot know *a priori* the organization, nucleotide sequence, or extent of identity of a new cluster as compared to those already known.

Glycopeptides, also known as dalbaheptides because of their mechanism of action (Parenti and Cavalleri 1989), are an important class of antibiotics, interfering with synthesis of the bacterial cell wall, with vancomycin and teicoplanin currently in clinical use. They are often used for treating life-threatening infections caused by multi-resistant Gram-positive bacteria. The emergence of resistance to glycopeptides among enterococci and the fear that this high-level resistance may eventually become widespread in methicillin-resistant *Staphylococcus aureus* has prompted the search for second-generation drugs of this class. Promising results have been obtained with the development of semi-synthetic derivatives with improved activity, expanded antibacterial spectrum or better pharmacokinetics (Malabarba and Ciabatti 2001).

Therefore, there exists the utility to obtain improved glycopeptides by manipulation of occurring natural compounds. However, glycopeptides are structurally complex molecules and their accessibility to chemistry is limited to a few positions in the molecule. For example, while the sugars can be easily removed chemically from a glycopeptide, generating the corresponding aglycone, the regioselective attachment of a different sugar to a particular position by chemical means is extremely difficult. In addition, glycopeptides of the teicoplanin family contain an acyl chain linked to the glucosamine attached to the arylamino acid at position 4, while compounds of the vancomycin class do not. Acylation and deacylation of glycopeptides has been reported either chemically or by biotransformation (Lancini and Cavalleri 1997), but it usually results in overall low yields. In light of the above, it would be desirable to have genes and enzymes useful for redirecting these steps in glycopeptide formation, in order to obtain derivatives that are hard or impossible to make by chemical means.

An attractive alternative would be to generate improved antibiotics by engineering the biosynthetic processes for naturally occurring glycopeptides. For example, it has been possible to selectively glycosylate glycopeptide aglycons both in vitro and in vivo after the expression of glycosyltransferases from the vancomycin and chloroeremomycin gene clusters (Solenberg et al. 1997; Loosey et al. 2001). However, none of the enzymes described so far is able to attach a glucosamine residue at desired positions. Similarly, inactivation of selected genes in the balhimycin producer *A. mediterranei* has led to the obtainment of balhimycin derivatives (Pelzer et al. 1999). However, no such experiments have been described for strains producing glycopeptides of the teicoplanin family.

The antibiotic teicoplanin (INN; USP Dictionary of USAN and International Drug Names, 1995; C.A. Fleeger, Editor. U.S. Pharmacopeial Convention Inc. Rockville" MD) belongs to the lipoglycopeptide family of dalbaheptides (Parenti and Cavalleri 1989). It consists of a complex of closely related molecules, denoted A₂₋₁ to A₂₋₅, which differ only in the nature of the acyl residue, accompanied by minor amounts of a pseudo-aglycone defined as A₃₋₁. Teicoplanin A₂₋₂ is the major component of the complex. The core structure is a modified linear heptapeptide with a rigid scaffold determined by ether bonds between amino acids 1-3, 2-4 and 4-6, and a C-C bond between amino acids 5-7. Three sugar moieties are attached to the aryl groups: a-D-mannose to amino acid 7, N-acetyl-β-D-glucosamine to amino acid 6 and N-acyl-β-D-glucosamine to amino acid 4. In addition two chlorine atoms are present on each of the tyrosine residues (amino acids 2 and 6). The structure of the components of teicoplanin complex is represented by the formula (I) shown below.

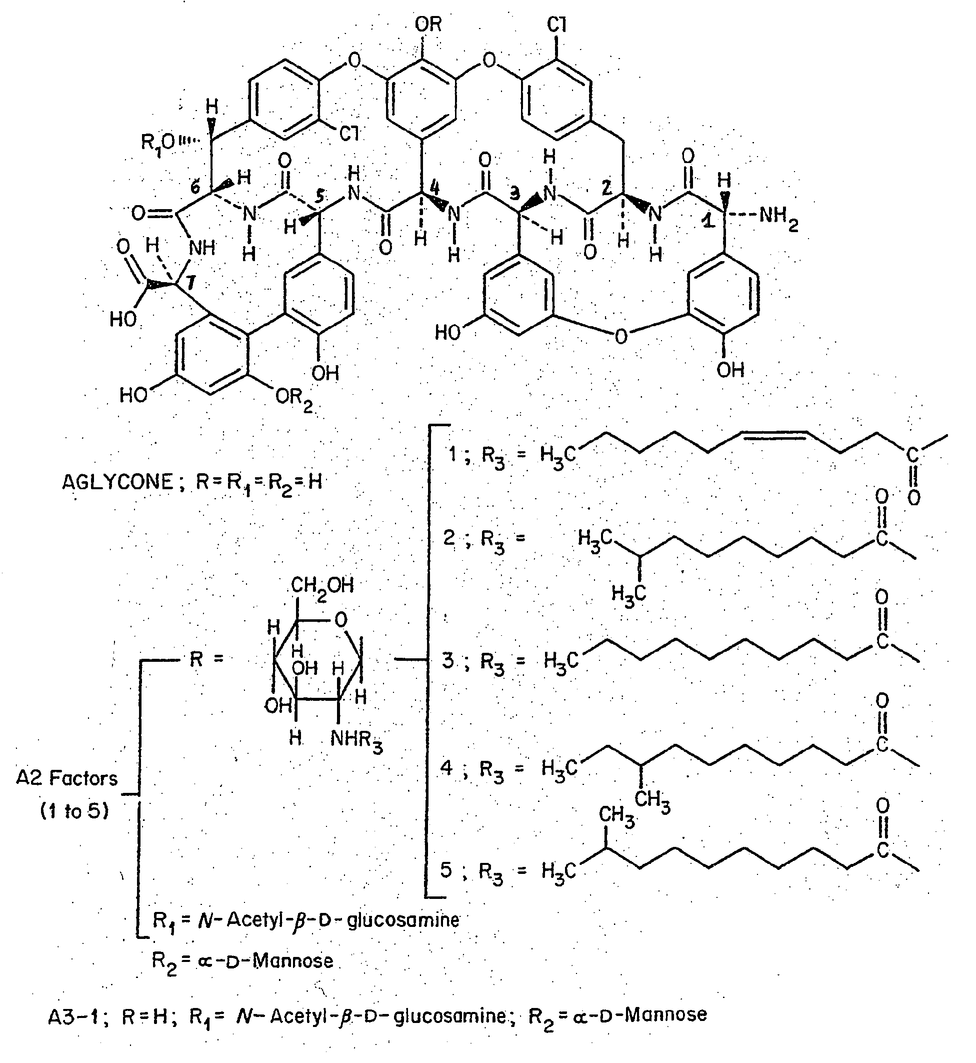

Teicoplanin, produced by *Actinoplanes teichomyceticus* ATCC31121 (Parenti et al. 1978) is currently used in human medicine. Efforts at generating new semi-synthetic derivatives of glycopeptides have been undertaken so as to address the emergence of glycopeptides resistance among enterococci and staphylococci (Malabarba and Ciabatti 2001). Therefore, the genes required for and regulating the formation of teicoplanin can also be useful in optimizing the production process and in manipulating the teicoplanin structure.

Gene clusters involved in the formation of the glycopeptides chloroeremomycin (van Wageningen et al. 1998), balhimycin (Pelzer et al. 1999), complestatin (Chiu et al. 2001) and A47934 (Pootoolal et al. 2002) have been described. These clusters, designated *cep, bal, com* and *sta*, respectively, were obtained from *Amycolatopsis orientalis, Amycolatopsis mediterranei, Streptomyces lavendulae* and *Streptomyces toyocaensis,* respectively. These clusters have provided several genes useful for manipulating glycopeptide pathways. However, certain steps cannot be performed with the genes derived from the described clusters. For example, the available gene clusters do not encode functions capable of attaching glucosamine residues to amino acids 4 and 6, or of attaching a fatty acid chain. All these functions are also described in the present invention.

The improvement of current antibiotic production processes by empirical, trial and error approaches, remains time consuming and may result in bacterial cultures that are unstable, perform inconsistently and accumulate unwanted by-products. In recent years, rational methods, involving the manipulation of key regulatory elements present within the gene cluster of interest or the overexpression of rate-limiting steps in the pathway, have been applied successfully to increase the level of antibiotic produced by *Streptomyces* spp.. Therefore, the existence, nature, number and sequence of cluster-associated regulators cannot be predicted by comparison to other cluster, even those specifying a related antibiotic. As an example, the tylosin gene cluster encodes four distinct regulators, while none has been found in the cluster specifying the related macrolide antibiotic erythromycin (Bate et al. 1999). Similarly, the nature and reason for a rate-limiting step in a biosynthetic pathway cannot be established *a priori.*

### SUMMARY OF THE INVENTION

The present invention provides a set of isolated polynucleotide molecules required for the biosynthesis of the glycopeptide teicoplanin in microorganisms. In one form of the invention, polynucleotide molecules are selected from the contiguous DNA sequence (SEQ ID NO: 1), which represents the *tcp* gene cluster as isolated from *Actinoplanes teichomyceticus* ATCC31121 and consists of 39 ORFs encoding the polypeptides required for teicoplanin formation. The amino acid sequences of the polypeptide encoded by said 39 ORFs are provided in SEQ ID NOS: 2 to 40.

The present invention provides an isolated nucleic acid comprising a nucleotide sequence selected from a group consisting of:
a)the *tcp* gene cluster encoding the polypeptides required for the synthesis of teicoplanin (SEQ ID NO: 1);
b)a nucleotide sequence encoding the same polypeptides encoded by the *tcp* gene cluster (SEQ ID NO: 1), other than the nucleotide sequence of the *tcp* gene cluster itself;
c)any nucleotide sequence of *tcp* ORFs 1 to 39, encoding the polypeptides of SEQ ID NOS: 2 to 40;
d)a nucleotide sequence encoding the same polypeptide encoded by any of *tcp* ORFs 1 to 39 (SEQ ID NOS: 2 to 40), other than the nucleotide sequence of said ORF.

A further object of this invention is to provide an isolated nucleic acid comprising a nucleotide sequence selected from the group consisting of:
e)a nucleotide sequence of any of the *tcp* ORFs 1 to 5, 9 to 14, 16 to 22, 25, 30 to 34 and 36 to 38 encoding the polypeptides specified in SEQ D NOS: 2 to 6, 10 to 15, 17 to 23, 26, 31 to 35 and 37 to 39;
f)a nucleotide sequence encoding the same polypeptide encoded by any of *tcp* of OFRs 1 to 5, 9 to 14, 16 to 22, 25, 30 to 34 and 36 to 38 encoding the polypeptides specified in SEQ ID NOS: 2 to 6, 10 to 15, 17 to 23, 26, 31 to 35 and 37 to 39 other than the nucleotide sequence of said *tcp* ORFs;
g)a nucleotide sequence of any of *tcp* ORFs 6 to 8, 15, 23 to 24, 26 to 29, 35 and 39 encoding the polypeptides specified in SEQ ID NOS: 7 to 9, 16, 24 to 25, 27 to 30, 36 and 40;
h)a nucleotide sequence encoding the same polypeptide encoded by any of *tcp* ORFs 6 to 8, 15, 23 to 24, 26 to 29, 35 and 39 encoding the polypeptides specified in SEQ ID NOS: 7 to 9, 16, 24 to 25, 27 to 30, 36 and 40 other than the nucleotide sequence of said *tcp* ORF;
i)a nucleotide sequence encoding a polypeptide that is at least 80%, preferably 86%, more preferably 90%, most preferably 95% or more, identical in amino acid sequence to a polypeptide encoded by any of *tcp* ORFs 8, 15, 23 to 24, 26 to 27, 29, 35 and 39 (SEQ ID NOS: 9, 16, 24 to 25, 27 to 28, 30, 36 and 40); and
j)a nucleotide sequence encoding a polypeptide that is at least 87%, preferably 90%; more preferably 95% or more, identical in amino acid sequence to the polypeptide encoded by *tcp* ORF 28 (SEQ ID NO: 29).

In one embodiment the isolated nucleic acids of this invention comprise combinations of ORFs selected from ORFs 1 to 39 (SEQ ID NOS: 2 to 40), which encode polypeptides required for the synthesis of the 4-hydroxyphenylglycine (HPG) residues of teicoplanin. In another embodiment, the nucleic acid comprises combinations of ORFs selected from ORFs 1 to 39 (SEQ ID NOS: 2 to 40), which encode the polypeptides required for the synthesis of the 3,5-dihydroxyphenylglycine (DPG) residues of teicoplanin. In yet another embodiment, the nucleic acid comprises combinations of ORFs selected from ORFs 1 to 39 (SEQ ID NOS: 2 to 40), which encode the polypeptides required for the synthesis of the heptapeptide skeleton of teicoplanin. According to another embodiment, in a nucleic acid of this invention, combinations of ORFs selected from ORFs 1 to 39 (SEQ ID NOS: 2 to 40) are provided which encode a polypeptide required for the chlorination of the aromatic residues of amino acids 3 and 6 of teicoplanin. In yet another embodiment, nucleic acid comprising combinations of ORFs selected from ORFs 1 to 39 (SEQ ID NOS: 2 to 40) are provided, which encode a polypeptide required for the β-hydroxylation of the tyrosine residue of aminoacid 6 of teicoplanin. In yet another embodiment, nucleic acid comprising combinations of ORFs selected from ORFs 1 to 39 (SEQ ID NOS: 2 to 40) are provided, which encode polypeptides required for the cross-linking of the aromatic residues of amino acids at positions 2 and 4, 4 and 6, 1 and 3, and 5 and 7 of teicoplanin. According to another embodiment, in the nucleic acid of this invention, combinations of ORFs selected from ORFs 1 to 39 (SEQ ID NOS: 2 to 40) are provided which encode the polypeptides required for the addition and formation of the N-acetyl-β-D-glucosamine and N-acyl-β-D-glucosamine residues. In yet another embodiment, nucleic acids are provided which comprise combinations of ORFs selected from ORFs 1 to 39 (SEQ ID NOS: 2 to 40), encoding a polypeptide required for the attachment of the mannosyl residue. In yet another embodiment, nucleic acids are provided which comprise combinations of ORFs selected from ORFs 1 to 39 (SEQ ID NOS: 2 to 40), encoding a polypeptide required for teicoplanin resistance. According to yet another embodiment, nucleic acids are provided which comprise combinations of ORFs selected from ORFs 1 to 39 (SEQ ID NOS: 2 to 40), encoding polypeptides required for the export of teicoplanin. In yet another embodiment, nucleic acids are provided which comprise combinations of ORFs selected from ORFs 1 to 39 (SEQ ID NOS: 2 to 40), encoding polypeptides required for regulating the expression of the *tcp* cluster. In yet another embodiment, nucleic acids are provided which comprise one or more DNA segments selected from SEQ ID NO: 1, enhancing the expression level of an ORF selected from ORFs 1 through 39 (SEQ ID NOS: 2 to 40).

Those skilled in the art understand that the present invention, having provided the nucleotide sequences encoding polypeptides of the teicoplanin biosynthetic pathway, also provides nucleotides encoding fragments derived from such polypeptides. In addition, those skilled in the art understand that, since the genetic code is degenerate, the same polypeptides specified in SEQ ID NOS: 2 to 40 can be encoded by natural or artificial variants of ORFs 1 to 39, i.e. by nucleotide sequences other than the genomic nucleotide sequences specified by ORFs 1 to 39 but which encode the same polypeptides. Furthermore, it is also understood that naturally occurring or artificially manufactured variants can occur of the polypeptides specified in SEQ ID NOS: 2 to 40, said variants having the same function(s) as the above mentioned original polypeptides but containing addition, deletion or substitution of amino acid not essential for folding or catalytic function, or conservative substitution of essential amino acids.

Those skilled in the art understand also that, having provided the nucleotide sequence of the entire cluster required for teicoplanin biosynthesis, the present invention also provides nucleotide sequences required for the expression of the genes present in said cluster. Such regulatory sequences include but are not limited to promoter and enhancer sequences, antisense sequences, transcription terminator and antiterminator sequences. These sequences are useful for regulating the expression of the genes present in the *tcp* gene cluster. Cells carrying said nucleotide sequences, alone or fused to other nucleotide sequences, fall also within the scope of the present invention.

In one aspect, the present invention provides isolated nucleic acids comprising nucleotide sequences encoding the ORF8 polypeptide (SEQ ID NO: 9), or naturally occurring variants or derivatives of said polypeptide, useful for the attachment of N-acyl-β-D-glucosamine residue to the core structure of a glycopeptide antibiotic precursor. In one aspect, the present invention provides isolated nucleic acids comprising nucleotide sequences encoding the ORF23 polypeptide (SEQ ID NO: 24), or naturally occurring variants or derivatives of said polypeptide, useful for the attachment of N-acetyl-β-D-glucosamine to the core structure of a glycopeptide antibiotic precursor. In another aspect, the present invention provides nucleic acids comprising nucleotide sequences encoding the ORF24 and 26 polypeptide (SEQ ID NO: 25 and 27), or naturally occurring variants or derivatives of said polypeptide, useful for the attachment of fatty acid residues to the core structure of a glycopeptide antibiotic precursor. In another aspect, the present invention provides nucleic acids comprising nucleotide sequences encoding the ORF21 polypeptide (SEQ ID NO: 22), or naturally occurring variants or derivatives of said polypeptide, useful for the chlorination of β-hydroxytyrosine and tyrosine residues in a core glycopeptide antibiotic precursor. In another aspect, the present invention provides nucleic acids comprising nucleotide sequences encoding the ORF 15 polypeptide (SEQ ID NO: 16), or naturally occurring variants or derivatives of said polypeptide, useful for the attachment of mannosyl residues to the core structure of a glycopeptide antibiotic precursor.

In another aspect, the present invention provides nucleic acids comprising nucleotide sequences encoding the polypeptides encoded by ORFs 16 and 34 (SEQ ID NOS: 17 and 35), or naturally or artificially occurring variants or derivatives of said polypeptides, useful for export out of the cells of a glycopeptide antibiotic or a glycopeptide antibiotic precursor. In another aspect, the present invention provides nucleic acids comprising nucleotide sequences encoding the ORF1 to 4 polypeptide (SEQ ID NO: 2 to 5), or naturally or artificially occurring variants or derivatives of said polypeptide, useful for conferring resistance to the producing strain to a glycopeptide antibiotic or a glycopeptide antibiotic precursor. In another aspect, the present invention provides nucleic acids comprising nucleotide sequences encoding the ORFs 27, 35 and 39 polypeptides (SEQ ID NOS: 28, 36 and 40), or naturally or artificially occurring variants or derivatives of said polypeptide, useful for increasing the yield of a glycopeptide antibiotic precursor. In another aspect, the present invention provides nucleic acids comprising nucleotide sequences encoding the ORFs 6, 7, 28 and 29 polypeptides (SEQ ID NOS: 7, 8 29 and 30), or naturally or artificially occurring variants or derivatives of said polypeptide, useful for altering the level of expression of one or more genes of a glycopeptide gene cluster.

In one embodiment, the present invention provides a glycopeptide producing strain carrying extra copies of the nucleotide sequences specifying at least one ORF selected from any of ORFs 1 through 39 (SEQ ID NOS: 2 to 40). In one preferred embodiment, such glycopeptide producing strain is any strain belonging to the order *Actinomycetales.* In yet another preferred embodiment, such glycopeptide producing strain is a member of the genus *Actinoplanes.* In one further aspect, the present invention provides a *Actinoplanes* strain containing one or more variations in the nucleotide sequence specified in SEQ ID NO: 1, such variation resulting in an increased or decreased expression of one or more of ORFs 1 through 39 (SEQ ID NOS: 2 to 40).

In one preferred embodiment, the present invention provides nucleic acids comprising a nucleotide sequence specified by SEQ ID NO: 1, or a portion thereof, carried on one or more vectors, useful for the production of teicoplanin, one or more of its precursors, or a derivative thereof, by another cell. In one preferred embodiment, said nucleotide sequence or portion thereof is carried on a single vector. In yet another preferred embodiment, such vector is a bacterial artificial chromosome. In yet another aspect, said bacterial artificial chromosome is an ESAC vector (as described in WO99/63674). In another preferred embodiment, the present invention provides a recombinant actinomycete strain other than *Actinoplanes* sp. ATCC 31121 containing the gene cluster specified by SEQ ID NO: 1, said gene cluster being carried in an ESAC vector which is integrated into the chromosome of said recombinant actinomycete strain.

In one aspect, the present invention provides a method for increasing the production of teicoplanin, said method comprising the following steps: (a) transforming with a recombinant DNA vector a microorganism that produces teicoplanin or a teicoplanin precursor by means of a biosynthetic pathway, said vector comprising a DNA sequence, chosen from any of ORFs 1 through 39 (SEQ ID NO: 2 through 40), that codes for an activity that is rate limiting in said pathway; and (b) culturing said microorganism transformed with said vector under conditions suitable for cell growth, expression of said gene and production of said antibiotic or antibiotic precursor.

In another aspect, the present invention provides a method for producing a derivative of teicoplanin or a precursor thereof, said method comprising the following steps: (a) cloning in a suitable vector a segment chosen from the nucleotide sequence defined by SEQ ID NO: 1, said segment containing at least a portion of one of ORFs 1 through 39 (SEQ ID NO: 2 through 40), said ORF encoding a polypeptide that catalyzes a biosynthetic step that one wishes to bypass; (b) inactivating said ORF or portion therof by removing or replacing one or more codons that specify for amino acids that are essential for the activity of said polypeptide; (c) transforming with said recombinant DNA vector a microorganism that produces teicoplanin or a teicoplanin precursor by means of a biosynthetic pathway; (d) screening the resulting transformants for those where said DNA sequence has been replaced by the mutated copy; and (e) culturing said mutant cells under conditions suitable for cell growth, expression of said pathway and production of said pathway analogue.

In yet another aspect, the present invention provides a method for producing novel glycopeptides, said method comprising the following steps: (a) transforming with a recombinant DNA vector a microorganism that produces a glycopeptide or a glycopeptide precursor different from teicoplanin or a precursor thereof by means of a biosynthetic pathway, said vector comprising one or more ORFs, chosen among ORFs 1 through 39 (SEQ ID NOS: 2 through 40), that codes for the expression of an enzymatic activity that modifies said glycopeptide or glycopeptide precursor; and (b) culturing said microorganism transformed with said vector under conditions suitable for cell growth, expression of said gene and production of said antibiotic or antibiotic precursor.

Examples of microorganisms that produce a glycopeptide or a glycopeptide precursor suitable for carrying out this method are *Nonomuraea* sp. ATCC 39727, *Streptomyces toyocaensis and Amycolatopsis mediterranei*

In yet another aspect, the present invention provides a further method for producing novel glycopeptides, said method comprising the following steps: (a) transforming with a recombinant DNA vector a microorganism, said vector comprising one or more ORFs, chosen among ORFs 1 through 39 (SEQ ID NOS: 2 through 40), that codes for one or more polypeptides that modify a glycopeptide or glycopeptide precursor, and said microorganism being selected among those that do not produce glycopeptides or glycopeptide precursors and that can efficiently express the introduced OFR(s); (b) preparing a cell extract or cell fraction of said microorganism under conditions suitable for the presence of active polypeptide(s), said cell extract or cell fraction containing at least said polypeptide(s); and (c) adding a glycopeptide or glycopeptide precursor to said cell extract or cell fraction, and incubating said mixture under conditions where said polypeptide(s) can modify said glycopeptide or glycopeptide precursor.

Examples of microorganisms which are suitable for performing the above method are *E. coli, B. subtilis and S. lividans.*

A further aspect of this invention includes an isolated polypeptide involved in the biosynthetic pathway of teicoplanin selected from
a) an ORF polypeptide encoded by any one of *tcp* ORFs 1 to 39 (SEQ ID NOS: 2 through 40);
b)a polypeptide which is at least 90%, preferably 95% or more, identical in amino acid sequence to a polypeptide encoded by any one of *tcp* ORFs 1 to 5, 9 to 14, 16 to 22, 25, 30 to 34 and 36 to 38 (SEQ ID NOS: 2 to 6, 10 to 15, 17 to 23, 26, 31 to 35 and 37 to 39);
c)a polypeptide which is at least 80% preferably 86%, more preferably 90%, most preferably 95% or more, identical in amino acid sequence to a polypeptide encoded by any of *tcp* ORFs 6 to 8, 15, 23 to 24, 26 to 27, 29, 35 and 39 (SEQ ID NOS: 7 to 9, 16, 24 to 25, 27 to 28, 30, 36 and 40); and
d)a polypeptide which is at least 87%, preferably 90%, more preferably 95% or more, identical in amino acid sequence to a polypeptide encoded by ORF 28 (SEQ ID NO: 29).

### DEFINITIONS

The term "isolated nucleic acid" refers to a DNA molecule, either as genomic DNA or a complementary DNA (cDNA), which can be single or double stranded, of natural and synthetic origin. This term refers also to an RNA molecule, of natural or synthetic origin.

The term "nucleotide sequence" refers to full length or partial length sequences of ORFs and intergenic regions as disclosed herein. Any one of the nucleotide sequences of the invention as shown in the sequence listing is (a) a coding sequence, (b) an RNA molecule derived from transcription of (a), (c) a coding sequence which uses the degeneracy of the genetic code to encode an identical polypeptide, or (d) an intergenic region, containing promoters, enhancers, terminator and antiterminator sequences.

The terms "gene cluster", "cluster" and "biosynthesis cluster" all designate a contiguous segment of a microorganism's genome that contains all the genes required for the synthesis of a secondary metabolite.

The term *"tcp"* refers to a genetic element responsible for teicoplanin biosynthesis in *Actinoplanes teichomyceticus* ATCC31121.

The term "ORF" refers to a genomic nucleotide sequence that encodes one polypeptide. In the context of the present invention, the term ORF is synonymous with "gene".

The term "ORF polypeptide" refers to a polypeptide encoded by an ORF.

The term "*tcp* ORF" refers to an ORF comprised within the *tcp* gene cluster.

The term "NRPS" refers to a non-ribosomal peptide synthetase which is a complex of enzymatic activities responsible for the incorporation of amino acids into an oligopeptide skeleton of a secondary metabolite. A functional NRPS is one that catalyzes the incorporation of one or more amino acid into an oligopeptide.

The term "NRPS module", or "module", refers to a segment of a NRPS that directs the activation, incorporation and possible modification of one amino acid into an oligopeptide.

The term "NRPS gene" refers to a gene that encodes a NRPS.

The term "secondary metabolite" refers to a bioactive substance produced by a microorganism through the expression of a set of genes specified by a gene cluster.

The term "production host" is a microorganism where the formation of a secondary metabolite is directed by a gene cluster derived from a donor organism.

The term "ESAC" identifies an *"Escherichia coli-Streptomyces* Artificial Chromosome", i.e. a recombinant vector that carries and maintains large DNA inserts in an *Escherichia coli* host and that can be introduced and maintained in an actinomycete production host. Examples of ESACs are given in WO99/67374.

### BRIEF DESCRIPTION OF THE DRAWINGS

**Figure 1**. Isolated DNA segments derived from the chromosome of *Actinoplanes teichomyceticus* ATCC31121. The thick line denotes the segment described in SEQ ID NO: 1. The cosmids carrying said isolated DNA segments are designated pAT9, pAT17, pAT32, pAT42, pAT52 and pAT53.
**Figure 2**. Genetic organization of the *tcp* cluster. Each ORF is represented by an arrow, and numbered as in Table 1. The orientation is the same as in Fig. 1. Numbers on the scale bars indicate sequence coordinates (in kb).

### DETAILED DESCRIPTION OF THE INVENTION

### A. THE tcp GENES FROM ACTINOPLANES TEICHOMYCETICUS

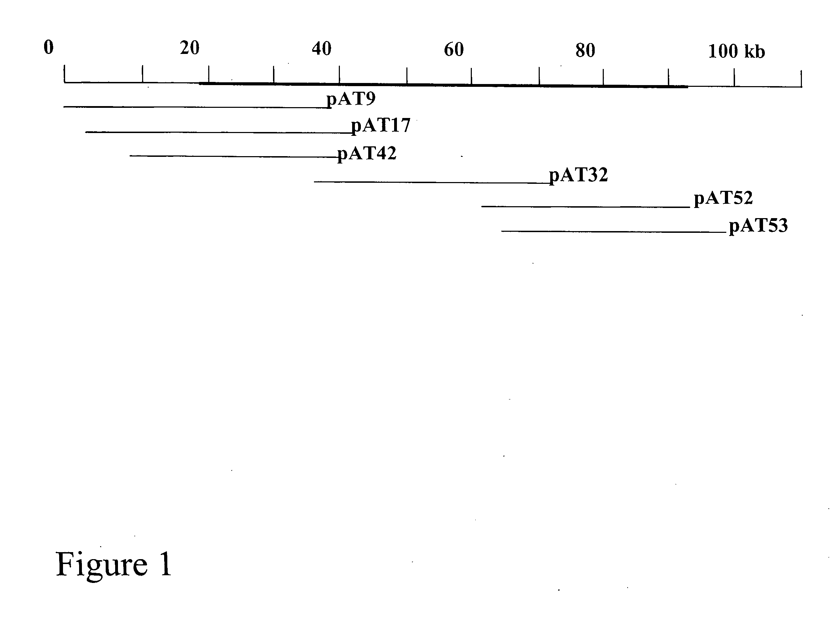

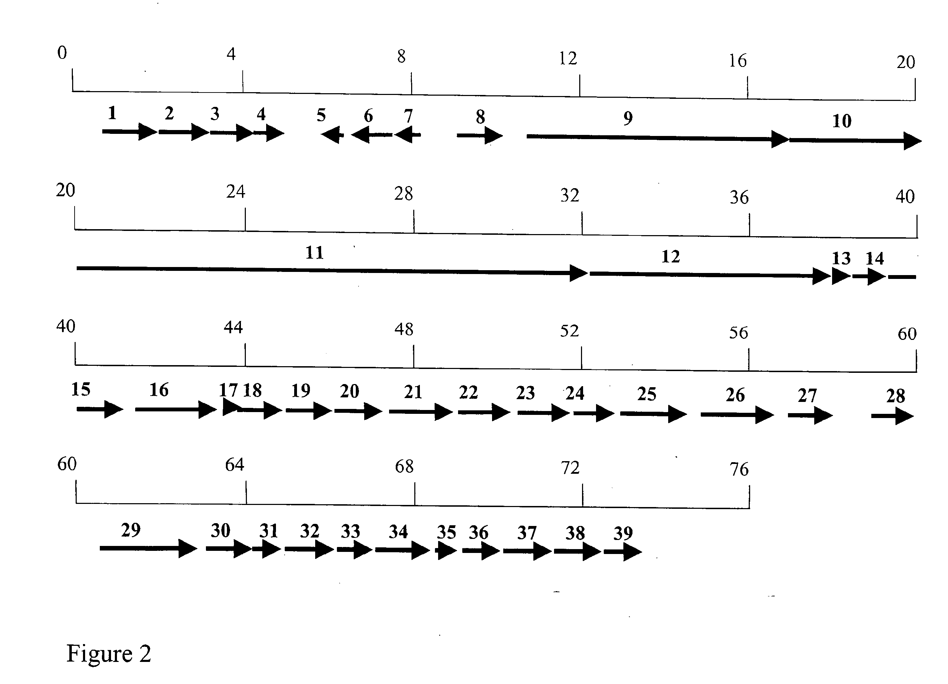

Teicoplanin is a complex of closely related glycopeptide antibiotics produced by *Actinoplanes teichomyceticus* ATCC31121. The present invention provides nucleic acid sequences and characterization of the gene cluster for the biosynthesis of teicoplanin. The physical organization of the teicoplanin gene cluster, together with flanking DNA sequences, is reported in Fig. 1, which illustrates the physical map of a 110-kb genomic segment from the genome of *Actinoplanes teichomyceticus* ATCC31121, together with a set of cosmids defining such segment. The genetic organization of the DNA segment governing teicoplanin biosynthesis, designated as the *tcp* cluster, is shown in Fig. 2 and its nucleotide sequence is reported as SEQ ID NO: 1.

The precise boundary of the cluster can be established by comparison with other glycopeptide clusters and from the functions of its gene products. Therefore, on the left end (Fig. 1), the *tcp* cluster is delimited by *tcp* ORF1, encoding the enzyme MurF (SEQ ID NO: 2) similar to D-alanyl-D-alanine-adding enzyme, involved in the mechanism of resistance to teicoplanin. On the right side, the *tcp* cluster is delimited by *tcp* ORF39 (SEQ ID NO: 40), encoding a type II thioesterase. The *tcp* cluster spans 73,882 base pairs and contains 39 ORFs, designated *tcp* ORF1 through ORF39. The contiguous nucleotide sequence of SEQ ID NO: 1 encodes the 39 deduced proteins listed in SEQ ID NOS: 2 to 40. ORF1 (SEQ ID NO: 2) represents 447 amino acids deduced from translating SEQ ID NO: 1 from nucleotides 652 to 1995. ORF2 (SEQ ID NO: 3) represents 405 amino acids deduced from translating SEQ ID NO: 1 from nucleotides 2017 to 3234. ORF3 (SEQ ID NO: 4) represents 345 amino acids deduced from translating SEQ ID NO: 1 from nucleotides 3227 to 4264. ORF4 (SEQ ID NO: 5) represents 202 amino acids deduced from translating SEQ ID NO: 1 from nucleotides 4264 to 4872. ORF5 (SEQ ID NO: 6) represents 179 amino acids deduced from translating SEQ ID NO: 1 from nucleotides 6173 to 5634 on the complementary strand. ORF6 (SEQ ID NO: 7) represents 363 amino acids deduced from translating SEQ ID NO: 1 from nucleotides 7377 to 6286 on the complementary strand. ORF7 (SEQ ID NO: 8) represents 226 amino acids deduced from translating SEQ ID NO: 1 from nucleotides 8065 to 7385 on the complementary strand. ORF8 (SEQ ID NO: 9) represents 393 amino acids deduced from translating SEQ ID NO: 1 from nucleotides 8924 to 10105. ORF9 (SEQ ID NO: 10) represents 2076 amino acids deduced from translating SEQ ID NO: 1 from nucleotides 10597 to 16827. ORF10 (SEQ ID NO: 11) represents 1061 amino acids deduced from translating SEQ ID NO: 1 from nucleotides 16824 to 20009. ORF11 (SEQ ID NO: 12) represents 4067 amino acids deduced from translating SEQ ID NO: 1 from nucleotides 20053 to 32256. ORF12 (SEQ ID NO: 13) represents 1865 amino acids deduced from translating SEQ ID NO: 1 from nucleotides 32276 to 37873. ORF13 (SEQ ID NO: 14) represents 69 amino acids deduced from translating SEQ ID NO: 1 from nucleotides 37886 to 38095. ORF14 (SEQ ID NO: 15) represents 273 amino acids deduced from translating SEQ ID NO: 1 from nucleotides 38401 to 39222. ORF15 (SEQ ID NO: 16) represents 593 amino acids deduced from translating SEQ ID NO: 1 from nucleotides 39268 to 41049. ORF16 (SEQ ID NO: 17) represents 648 amino acids deduced from translating SEQ ID NO: 1 from nucleotides 41343 to 43289. ORF17 (SEQ ID NO: 18) represents 70 amino acids deduced from translating SEQ ID NO: 1 from nucleotides 43373 to 43585. ORF18 (SEQ ID NO: 19) represents 391 amino acids deduced from translating SEQ ID NO: 1 from nucleotides 43696 to 44871. ORF19 (SEQ ID NO: 20) represents 384 amino acids deduced from translating SEQ ID NO: 1 from nucleotides 44894 to 46048. ORF20 (SEQ ID NO: 21) represents 398 amino acids deduced from translating SEQ ID NO: 1 from nucleotides 46038 to 47234. ORF21 (SEQ ID NO: 22) represents 506 amino acids deduced from translating SEQ ID NO: 1 from nucleotides 47484 to 49004. ORF22 (SEQ ID NO: 23) represents 392 amino acids deduced from translating SEQ ID NO: 1 from nucleotides 49166 to 50344. ORF23 (SEQ ID NO: 24) represents 408 amino acids deduced from translating SEQ ID NO: 1 from nucleotides 50461 to 51687. ORF24 (SEQ ID NO: 25) represents 323 amino acids deduced from translating SEQ ID NO: 1 from nucleotides 51833 to 52804. ORF25 (SEQ ID NO: 26) represents 530 amino acids deduced from translating SEQ ID NO: 1 from nucleotides 52948 to 54540. ORF26 (SEQ ID NO: 27) represents 598 amino acids deduced from translating SEQ ID NO: 1 from nucleotides 54757 to 56553. ORF27 (SEQ ID NO: 28) represents 351 amino acids deduced from translating SEQ ID NO: 1 from nucleotides 56924 to 57979. ORF28 (SEQ ID NO: 29) represents 329 amino acids deduced from translating SEQ ID NO: 1 from nucleotides 58746 to 59735. ORF29 (SEQ ID NO: 30) represents 792 amino acids deduced from translating SEQ ID NO: 1 from nucleotides 60477 to 62855. ORF30 (SEQ ID NO: 31) represents 373 amino acids deduced from translating SEQ ID NO: 1 from nucleotides 63036 to 64157. ORF31 (SEQ ID NO: 32) represents 221 amino acids deduced from translating SEQ ID NO: 1 from nucleotides 64154 to 64819. ORF32 (SEQ ID NO: 33) represents 433 amino acids deduced from translating SEQ ID NO: 1 from nucleotides 64816 to 66117. ORF33 (SEQ ID NO: 34) represents 268 amino acids deduced from translating SEQ ID NO: 1 from nucleotides 66146 to 66952. ORF34 (SEQ ID NO: 35) represents 453 amino acids deduced from translating SEQ ID NO: 1 from nucleotides 66991 to 68352. ORF35 (SEQ ID NO: 36) represents 198 amino acids deduced from translating SEQ ID NO: 1 from nucleotides 68349 to 68945. ORF36 (SEQ ID NO: 37) represents 365 amino acids deduced from translating SEQ ID NO: 1 from nucleotides 69083 to 70180. ORF37 (SEQ ID NO: 38) represents 351 amino acids deduced from translating SEQ ID NO: 1 from nucleotides 70266 to 71321. ORF38 (SEQ ID NO: 39) represents 364 amino acids deduced from translating SEQ ID NO: 1 from nucleotides 71318 to 72412. ORF39 (SEQ ID NO: 40) represents 250 amino acids deduced from translating SEQ ID NO: 1 from nucleotides 72548 to 73300.

The *tcp* cluster is characterized by the presence of several ORFs that do not find homologs in the *bal, cep, com* and *sta* clusters. These include *tcp* ORFs 5, 15, 24, 26 to 27, 29, 35 and 39 (SEQ ID NOS: 6, 16, 25, 27 to 28, 30, 36 and 40). A comparison among the five *bal, cep, com, sta* and *tcp* clusters is summarized in Table 1. In conclusion, the genetic organization of the *tcp* cluster as described herein is different from those of other clusters involved in the synthesis of other glycopeptides. It therefore represents the first example of a cluster with such a genetic organization.

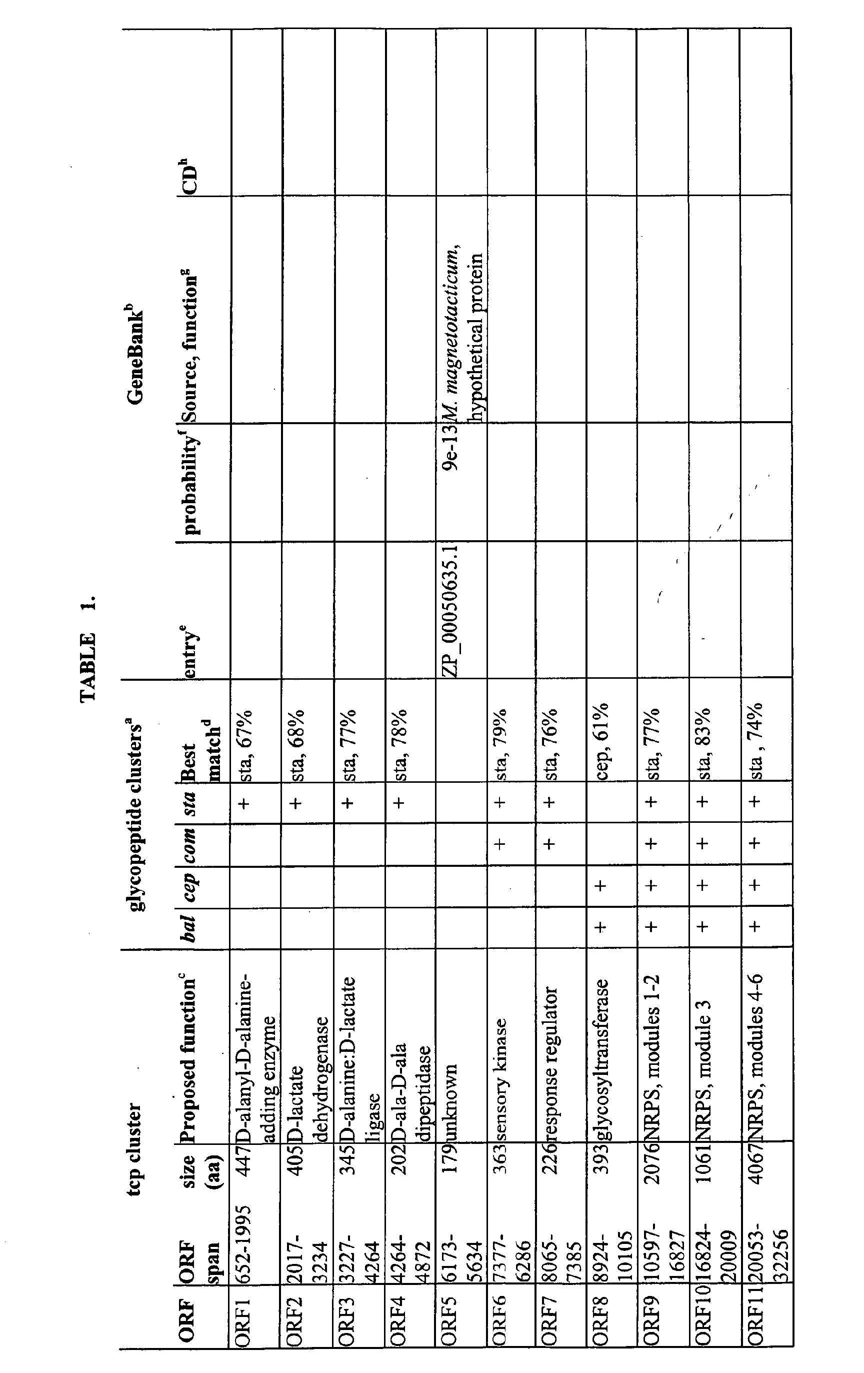

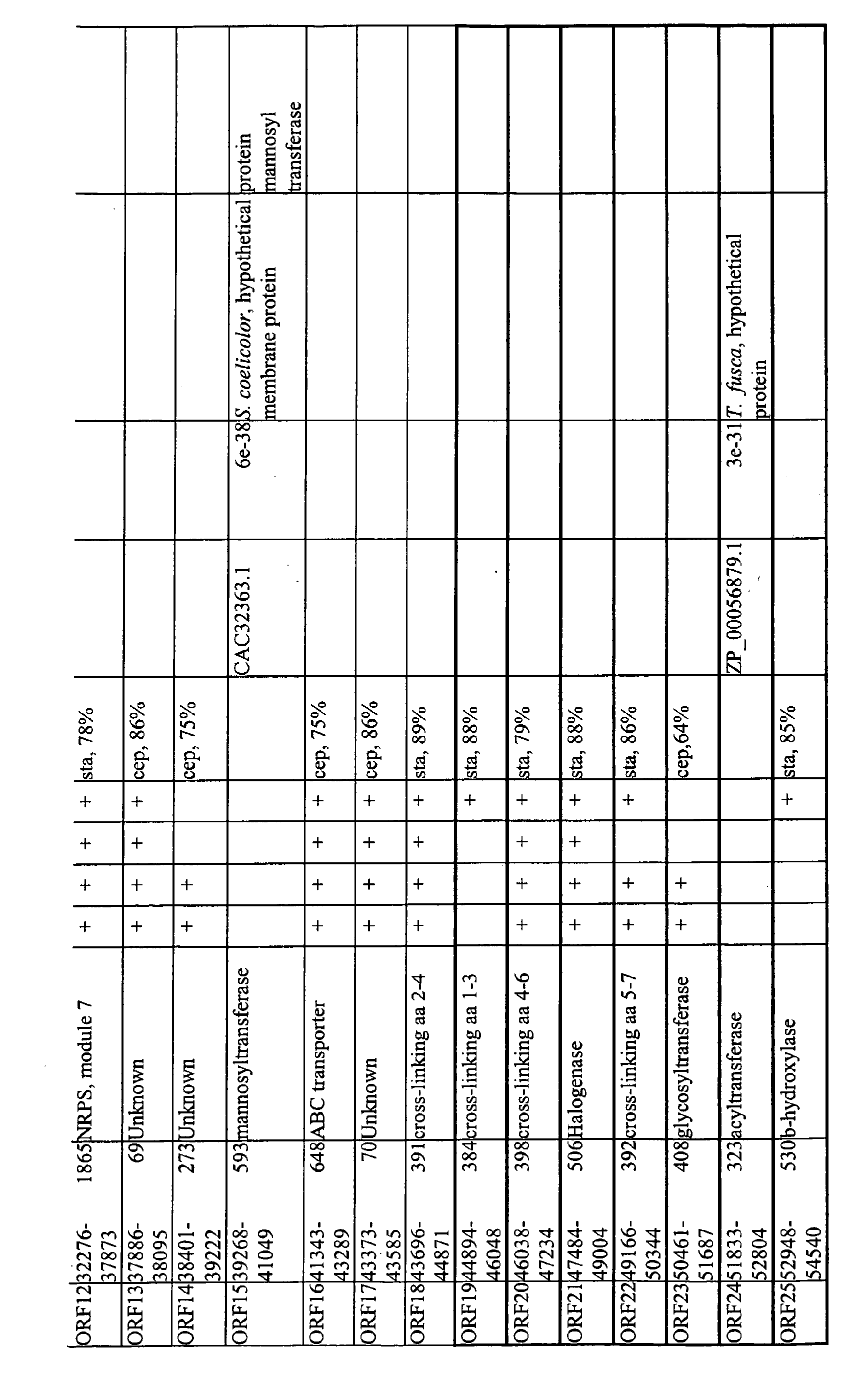

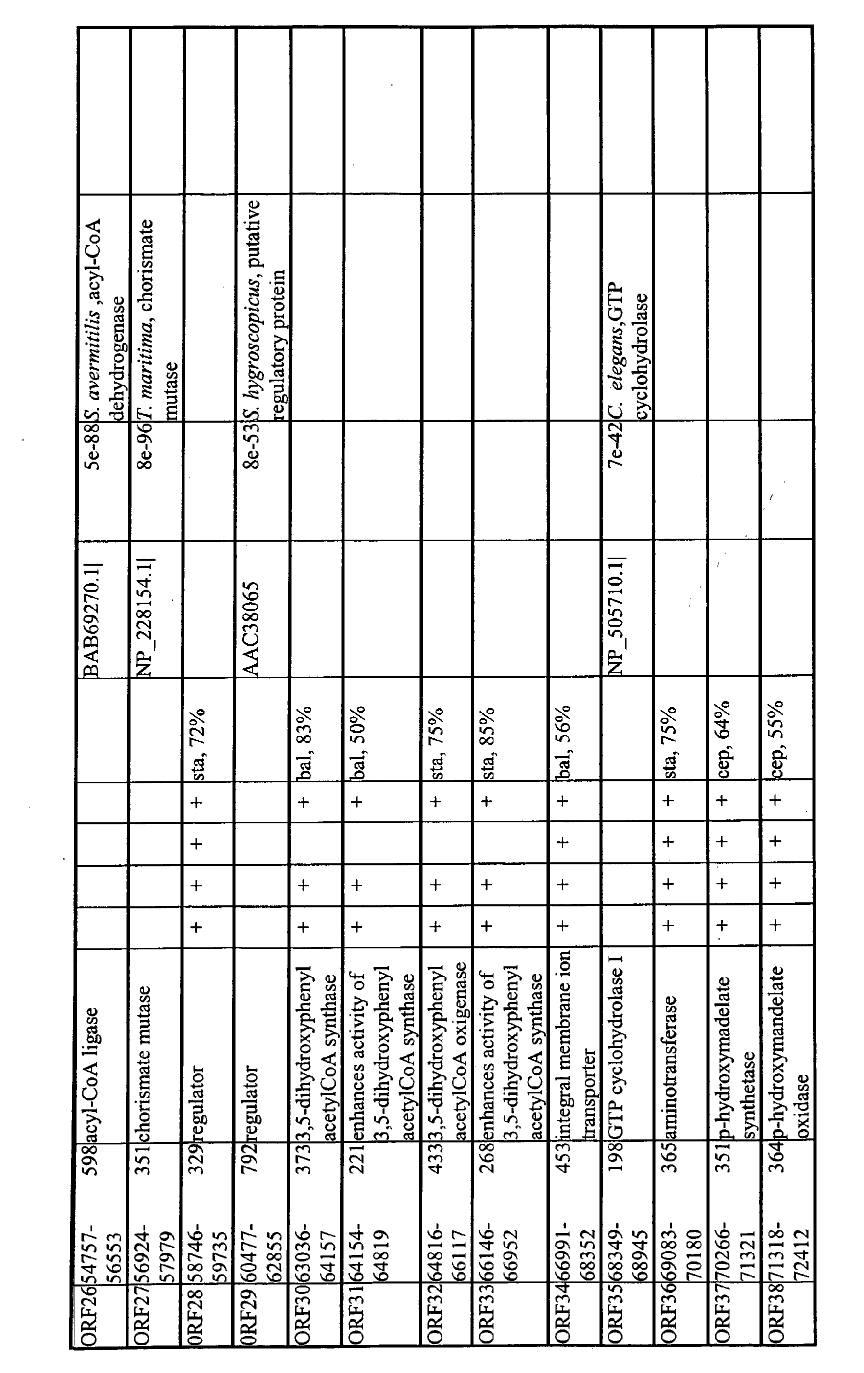

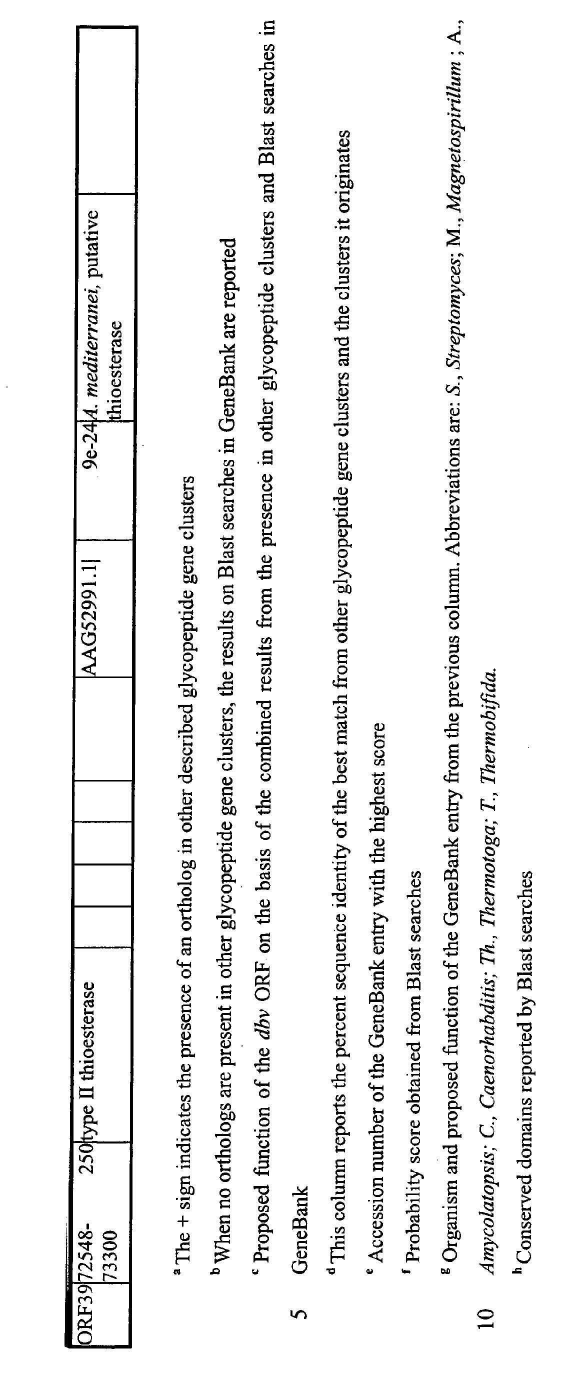

### B. ROLES OF THE tcp GENES

The present invention discloses, in particular, the DNA sequence encoding the NRPS responsible for the synthesis of the heptapeptide precursor of teicoplanin. This NRPS consists of four polypeptides, each containing between 1 and 3 modules. These are designated *tcp* ORF9 to ORF12 (SEQ ID NOS: 10 to 13). Peptide synthesis by NRPSs is carried out by modular systems, where a loading module is followed by a series of elongating modules. In NRPSs, each elongating module is characterized by the presence of at least three domains: an adenylation (A) domain, responsible for substrate recognition and activation; a thiolation (T) domain, which covalently binds as thioesters amino acids and elongating peptides; and a condensation (C) domain, which catalyzes peptide bond formation. In addition to these core domains, the last module contains a thioesterase (Te) domain, which hydrolyzes the ester bond linking the completed peptide to the NRPS. Some modules convert an L-amino acid into the D-form through the action of an epimerization (E) domain. The *dbv* NRPS consists of seven modules, for a total of seven A domains, seven T domains, six C domains, three E domains and one Te domain. Specifically, *tcp* ORF9 (SEQ ID NO: 10) encodes NRPS modules 1 and 2, specifies the sequence of domains A-T-C-A-E-T and is required for the incorporation of a HPG and a Tyr residue (first two amino acids) in the heptapeptide core of teicoplanin; *tcp* ORF10 (SEQ ID NO: 11) encodes NRPS module 3, specifies the sequence of domains C-A-T and is responsible for incorporating a DPG residue; *tcp* ORF11 (SEQ ID NO: 12) encodes NRPS modules 4 through 6, specifies the sequence of domains C-A-E-T-C-A-E-T-C-A-T and is responsible for incorporating two HPG and a Tyr residue in the teicoplanin heptapeptide core; and *tcp* ORF12 (SEQ ID NO: 13) encodes NRPS module 7, specifies the sequence of domains C-A-T-C*-T-Te (C* denotes an atypical condensation domain of unknown function) and is required for incorporation of the last DPG residue and in the release of the heptapeptide precursor of teicoplanin.

Other genes present in the *tcp* cluster represent novel genetic elements useful for increasing production of teicoplanin or for synthesizing novel metabolites. Among these, *tcp* ORF8 (SEQ ID NO: 9) encodes the glycosyltransferase that attaches an N-acetyl-β-D-glucosamine residue to the hydroxyl group of the tyrosine residue at position 6 (Formula (I)). This gene can be cloned and expressed in a heterologous host to yield an active enzyme capable of attaching a glucosamine residue to other glycopeptide aglycones. Alternatively, *tcp* ORF8 can be inactivated in the producing strain, resulting in the formation of teicoplanin derivatives devoid of the sugar attached to amino acid 6.

Yet other preferred nucleic acid molecules of the present invention include *tcp* ORF23 (SEQ ID NO: 24), which encodes the glycosyltransferase that attaches a b-D-glucosamine residue to the phenolic hydroxyl of the HPG residue at position 4 (Formula (I)). This gene can be cloned and expressed in a heterologous host to yield an active enzyme capable of attaching a glucosamine residue to other glycopeptide aglycones. Alternatively, *tcp* ORF23 can be inactivated in the producing strain, resulting in the formation of teicoplanin derivatives devoid of the sugar attached to amino acid 4.

Yet other preferred nucleic acid molecules of the present invention include *tcp* ORFs 24 and 26 (SEQ ID NO: 25 and 27), which encode the polypeptides participating in N-acylation of the glucosamine residue at amino acid 4. *tcp* ORF24 and ORF26 represent novel genetic elements, absent from the *cep, com, sta* and *bal* clusters. These genes can be cloned and expressed in a heterologous host to yield active enzymes capable of N-acylating sugar moieties of different glycopeptides. Alternatively, *tcp* ORF24 and ORF26 can be inactivated in the producing strain, resulting in the formation of deacyl derivatives of teicoplanin.

Yet other preferred nucleic acid molecules of the present invention include *tcp* ORF15 (SEQ ID NO: 16), which encodes a mannosyltransferase, responsible for attaching a mannosyl residue to amino acid 7. It thus represents a novel genetic element, absent from the *cep, sta, com* and *bal* clusters. This gene can be cloned and expressed in another glycopeptide producer strain to yield glycopeptides carrying a mannosyl residue attached to amino acid 7. Alternatively, *tcp* ORF15 can be inactivated in the producing strain, resulting in the formation of desmannosyl -teicoplanin derivatives. While this compound can be obtained by other means (Lancini and Cavalleri 1997), it may be desirable to produce it through a single fermentation process.

Yet other preferred nucleic acid molecules of the present invention include *tcp* ORF39 (SEQ ID NO: 40), which encodes a thioesterase, responsible for hydrolyzing aberrant intermediate peptides from the NRPS. Similarly to other type II thioesterases (Kotowska et al. 2002), the product of *tcp* ORF39 is responsible for maintaining an efficient NRPS during teicoplanin biosynthesis, by hydrolyzing all those thioesters that are not processed further into heptapeptides. It thus represents a novel genetic element, absent from the *cep,* sta, *com* and *bal* clusters. This gene can be cloned and expressed in another glycopeptide producer strain to increase the yield of product formed. Host strains include but are not limited to strains belonging to the order *Actinomycetales,* to the families *Micromonosporaceae, Streptosporangiaceae, Pseudonocardiaceae* and *Streptomycetaceae,* to the genera *Actinoplanes,* *Nonomureae, Amycolatopsis, Streptomyces* and the like.

The *tcp* cluster also includes a number of genes responsible for the synthesis of the non-proteinogenic amino acids HPG and DPG. For the synthesis of the former, the products of ORFs 27, 36, 37 and 38 (SEQ ID NOS: 28, 37, 38 and 39) are required. Synthesis of DPG requires the participation of *tcp* ORFs 30 to 33 (SEQ ID NOS: 31 to 34), in addition to ORF36 (SEQ ID NO: 37). Their roles are summarized in Table 1. Since HPG and DPG are non-proteinogenic amino acids, synthesis of the heptapeptide by the NRPS depends on their availability. Consequently, the activity of these enzymes is a limiting step in glycopeptide biosynthesis. Increased yield of glycopeptides can thus be obtained by increasing the expression of these ORFs. These genes can be overexpressed, individually or in any combination of them, in the teicoplanin producing strain to increase the yield of teicoplanin.

The *tcp* cluster also includes a number of genes responsible for exporting glycopeptide intermediates or finished products out of the cytoplasm and for conferring resistance to the producer cell. These genes include *tcp* ORFs 1 to 4, 16 and 34 (SEQ ID NOS: 2 to 5, 17 and 35). *tcp* ORF1 encodes a D-alanyl-D-alanyl adding enzyme, responsible for adding a dipeptide or a depsipeptide during peptidoglycan synthesis. *tcp* ORFs 2 to 4 encode a D-lactate dehydrogenase, a D-alanine:D-lactate ligase, and a D-alanyl-D-alanine dipeptidase, respectively. *tcp* ORFs 1 to 4 (SEQ ID NOS: 2 to 5) participate in the synthesis of a modified peptidoglycan, resulting in reduced binding of glycopeptides to the bacterial cell wall. These ORFs contribute to conferring teicoplanin resistance to the producer strain. *tcp* ORF16 encodes an ATP-dependent transporter, responsible for exporting teicoplanin or its intermediates utilizing ATP-derived energy. ORF34 encodes an Na/K ion-antiporter, responsible for exporting teicoplanin or its intermediates against a proton gradient. These genes can be cloned and expressed, either individually or in any combination of them, in another glycopeptide producer strain to increase the yield of product formed. Host strains include but are not limited to strains belonging to the order *Actinomycetales,* to the families *Micromonosporaceae, Streptosporangiaceae, Pseudonocardiaceae* and *Streptomycetaceae,* to the genera *Actinoplanes, Nonomureae, Amycolatopsis, Streptomyces* and the like. Alternatively, these genes can be overexpressed, individually or in any combination of them, in the teicoplanin producing strain to increase the yield of teicoplanin.

The *tcp* cluster also includes a number of regulatory genes, responsible for activating, directly or indirectly, the expression of biosynthetic and resistance genes during teicoplanin production. These genes include *tcp* ORFs 6 to 7 and 28 to 29 (SEQ ID NOS: 7 to 8 and 29 to 30). *tcp* ORFs 6 and 7 together encode the sensor kinase and the response regulator, respectively, of a two-component signal transduction system. *tcp* ORF29 is highly related to HygR, a positive regulator present in a gene cluster from *Streptomyces hygroscopicus* (Ruan et al. 1997). It represents a novel genetic element, absent from the *cep, com, bal* and *sta* clusters. *tcp* ORF28 is highly related to similar regulators present in other glycopeptide clusters. These four genes can be cloned and expressed, either individually or in any combination of them, in another glycopeptide producer strain to increase the yield of product formed. Host strains include but are not limited to strains belonging to the order *Actinomycetales,* to the families *Streptosporangiaceae, Micromonosporaceae, Pseudonocardiaceae* and *Streptomycetaceae,* to the genera *Nonomureae,* *Actinoplanes, Amycolatopsis, Streptomyces* and the like. Alternatively, these genes can be overexpressed, individually or in any combination of them, in the teicoplanin producing strain to increase the yield of teicoplanin. Examples of the expression of such regulatory genes for cluster of related compound have been described(Hutchinson CR.,1998; Bruheim et al., 2002)

### C. USES OF THE tcp CLUSTER

The present invention provides also nucleic acids for the expression of the entire teicoplanin molecule, any of its precursors or a derivative thereof. Such nucleic acids include isolated gene cluster(s) comprising ORFs encoding polypeptides sufficient to direct the assembly of teicoplanin. In one example, the entire *tcp* cluster (SEQ ID NO: 1) can be introduced into a suitable vector and used to transform a desired production host. In one aspect, this DNA segment is introduced into a suitable vector capable of carrying large DNA segments. Examples of such vectors include but are not limited to Bacterial Artificial Chromosome (BAC) vectors or specialized derivatives such as ESAC vectors (Shizuya et al. 1992; Ioannou et al. 1994; Sosio et al. 2000b). In another aspect, the *tcp* cluster is cloned as two separate segments into two distinct vectors, which can be compatible in the desired production host. In yet another aspect, the *tcp* cluster can be subdivided into three segments, each cloned into a separate, compatible vector. Examples of the use of one-, two- or three-vector systems have been described in the literature (e.g. Xue et al. 1999).

Once the *tcp* cluster has been suitably cloned into one or more vectors, it can be introduced into a number of suitable production hosts, where production of glycopeptide antibiotics might occur with greater efficiency than in the native host. Preferred host cells are those of species or strains that can efficiently express actinomycetes genes. Such host include but are not limited to *Actinomycetales, Micromonosporaceae, Streptosporangiaceae, Pseudonocardiaceae* and *Streptomycetaceae, Actinoplanes, Nonomuraea, Amycolatopsis* and *Streptomyces* and the like. Alternatively, a second copy of the *tcp* cluster, cloned into one or more suitable vectors, can be introduced in the teicoplanin producing strain, where the second copy of *tcp* genes will increase the yield of teicoplanin.

The transfer of the producing capability to a well characterized host can substantially improve several portions of the process of lead optimization and development: the titer of the natural product in the producing strain can be more effectively increased; the purification of the natural product can be carried out in a known background of possible interfering activities; the composition of the complex can be more effectively controlled; altered derivatives of the natural product can be more effectively produced through manipulation of the fermentation conditions or by pathway engineering.

Alternatively, the biosynthetic gene cluster can be modified, inserted into a host cell and used to synthesize or chemically modify a wide variety of metabolites: for example the open reading frames can be re-ordered, modified and combined with other glycopeptide biosynthesis gene cluster. Using the information provided herein, cloning and expression of teicoplanin nucleic acids can be accomplished using routine and well known methods.

In another possible use, selected ORFs from the *tcp* gene cluster are isolated and inactivated by the use of routine molecular biology techniques. The mutated ORF(s), cloned in a suitable vector containing DNA segments that flank said ORF in the *Actinoplanes* ATCC31131 chromosome, are introduced into said Actinoplanes strain, where two double cross-over events of homologous recombination result in the inactivation of said ORF(s) in the producer strain. This procedure is useful for the production of precursors or derivatives of teicoplanin in an efficient manner.

In another possible use, selected ORFs from the *tcp* gene cluster are isolated and placed under the control of a desirable promoter. The engineered ORF(s), cloned in a suitable vector, are then introduced into *Actinoplanes* ATCC 31131 or a teicoplanin producing variant or mutant thereof containing said ORF(s), either by replacing the original ORF as described above, or as an additional copy of said ORF(s). This procedure is useful for increasing or decreasing the expression level of ORF(s) that are critical for production of the teicoplanin molecule, precursors or derivatives thereof.

### EXAMPLES

The following examples serve to illustrate the principles and methodologies through which the teicoplanin gene cluster is identified and the *tcp* genes are analyzed and manipulated. These examples serve to illustrate the principles and methodologies of the present invention, but are not meant to limit its scope.

### General methods

Unless otherwise indicated, bacterial strains and cloning vectors can all be obtained from public collections or commercial sources. Standard procedures are used for molecular biology (e.g. Sambrook and Russell 2001; Kieser et al. 2000). *Actinoplanes teichomyceticus* ATCC31131 was grown in HT agar (Kieser et al. 2000) and in Rare3 medium (10 g/l glucose, 4 g/l yeast extract, 10 g/l malt extract, 2 g/l peptone, 2 g/l MgCl₂, 0.5% glycerol). Glycopeptides are isolated following published procedures (Lancini and Cavalleri, 1997). Sequence analyses are performed using the programs GCG from the Wisconsin package, version 9.1 (Accelrys).
Database searches are performed at with Blast or Fasta programs at public sites (http://www.ncbi.nlm.nih.gov/blast/index.html and http://www.ebi.ac.uk/fasta33).

### Example 1 - Isolation of teicoplanin biosynthesis genes

The teicoplanin biosynthesis gene cluster was isolated as described (Sosio et al. 2000c). Briefly, total DNA from *Actinoplanes teichomyceticus* ATCC31131 was partially digested with *Sau*3AI in order to optimize fragment sizes in the 40 kb range. The partially digested DNA was treated with alkaline phosphatase and ligated to pWE15 (Stratagene, La Jolla, CA 92037), previously digested with *Bam*HI. The ligation mixture was packaged in vitro and used to transfect *E. coli* XL1Blue cells. The resulting cosmid library was screened by hybridization with two probes obtained from PCR amplification of segments from the *bal* cluster using *A. mediterranei* DSM 5908 genomic DNA as template. These probes were: *bgtfA,* obtained by amplification with oligos 5'-ATGCGCGTGTTGATCTCG-3' (SEQ ID NO: 41) and 5'-CGGCTGACCGCGGCGAAC-3' (SEQ ID NO: 42); and *dpgA,* obtained from amplification with oligos 5'-CGTGGGGGTGGATGTATCGA-3' (SEQ ID NO: 43) and 5'-TCACCATTGGATCAGCG-3' (SEQ ID NO: 44). All oligos were designed from the sequence deposited in GenBank with accession No. Y16952. Further hybridization was performed with the oligonucleotide Pep8 (Sosio et al. 2000a). The cosmids positive to one or more of these probes were isolated and physically mapped with restriction enzymes. From such experiments, the cosmids reported in Fig. 1 were identified. The segment thus identified from the genome of *Actinoplanes teichomyceticus* ATCC31131 contains the *tcp* gene cluster responsible for the synthesis of the antibiotic teicoplanin. The precise boundary of the cluster can be defined by comparison with the reported cluster of other glycopeptides as described above under the chapter A "The *tcp* Genes from *Actinoplanes Teichomyceticus".*

The above example serves to illustrate the principle and methodologies through which the *tcp* cluster can be isolated. It will occur to those skilled in the art that the *tcp* cluster can be cloned in a variety of vectors. However, those skilled in the art understand that, given the large size of the *tcp* cluster, preferred vectors are those capable of carrying large inserts, such as lambda, cosmid and BAC vectors. Those skilled in the art understand that other probes can be used to identify the *tcp* cluster from such a library. From the sequence reported in SEQ ID NO: 1, any fragment can be PCR-amplified from *Actinoplanes teichomyceticus* ATCC31131 DNA and used to screen a library made with such DNA. One or more clones from said library can be identified that include any segment covered by SEQ ID NO: 1. Furthermore, it is also possible to identify the *tcp* cluster through the use of heterologous probes, such as those derived from the *cep, bal, com* and *sta* cluster, using the information provided in Table 1. Alternatively, other gene clusters directing the synthesis of secondary metabolites contain genes sufficiently related to the *tcp* genes as to allow heterologous hybridizations. All these variations fall within the scope of the present invention.

### Example 2 - Sequence analysis of teicoplanin gene cluster

The *tcp* cluster, identified as described under Example 1, was sequenced by the shotgun approach. The sequence of the *tcp* cluster is provided herein as SEQ ID NO: 1. The resulting DNA sequence was analyzed with Codonpreference [GCG, (Genetic Computer group, Madison, WI 53711) version 9.1] to identify likely coding sequences. Next, each coding sequence identified in this way was analyzed by comparison against the *bal, cep, com* and *sta* clusters using the program Tfasta. Coding sequences not identifying matches in any of these clusters were then searched against GenBank, employing the programs Blast, or against SwissProt, using Fasta. Finally, the exact start codon for each ORF was established by multiple alignment of related sequences with the program Pileup or by searching for an upstream ribosomal binding site. In total, 39 ORFs, denominated *tcp* ORF1 through *tcp* ORF39, were identified. The results of these analyses are summarized in Table 1, and provided herein in the sequence listing as SEQ ID NO: 2 through SEQ ID NO: 40. Details are given below.

### 2A. Synthesis of specialized amino acids HPG and DPG

Seven proteins encoded by the *tcp* cluster participate in the synthesis of the specialized amino acids HPG and DPG. Namely, OR37 and ORF38 (SEQ ID NOS: 38 and 39) are involved in the synthesis of the HPG residues required for teicoplanin formation and they encode the p-hydroxymandelate synthetase and the p-hydroxymandelate oxidase , respectively. Homologs of these ORFs are found in other glycopeptide clusters (Table 1) and their roles have been established experimentally (Li et al. 2001; Hubbard et al. 2000). ORFs 30 to 33 (SEQ ID NOS: 31 to 34) are involved in the synthesis of the DPG residues required for teicoplanin formation. Homologs of these ORFs are found in other glycopeptide clusters that direct the synthesis of heptapeptide containing DPG residues (Table 1) and the involvement of the corresponsing gene products has been determined experimentally (Pfeifer et al. 2001; Chen et al. 2001). ORF36 (SEQ ID NO: 37) encodes the amino transferase required for the transamination of both p-hydroxyphenylglyoxylate and 3,5-dihydroxyphenylglyoxylate, to yield HPG and DPG, respectively. Its role has been experimentally established (Pfeifer et al. 2001; Hubbard et al. 2000), and it utilizes preferentially tyrosine as an amino donor (Hubbard et al. 2000). This reaction results in the formation of p-hydroxyphenylpyruvate, which can then be converted into p-hydroxymandelate by the action of the gene product of ORF38 (SEQ ID NO: 39).

Another ORF participating indirectly in the synthesis of HPG is also found in the *tcp* cluster, namely ORF27 (SEQ ID NO: 28). ORF27 (SEQ ID NO: 28) encodes a chorismate mutase that participates in the synthesis of p-hydroxyphenylpyruvate, the substrate for the product of ORF37 (SEQ ID NO: 38). This ORF therefore encodes the enzyme that primes the cycle for converting tyrosine into HPG. The expression level of this ORF is therefore important in supplying adequate levels of HPG for teicoplanin formation.

### 2B. Synthesis of the heptapeptide precursor of teicoplanin

Four proteins, encoded by ORFs 9 to 12 (SEQ ID NOS: 10 to 13) are involved in the synthesis of the heptapeptide core of teicoplanin. All of these show significant similarity to other NRPSs. Based on alignments with other NRPS systems, the proposed domain composition and specificities of the proteins encoded by these four ORFs are reported in Table 2.

**Table 2 :**

| Domain composition and roles of *tcp* NRPS | | | | |
|---|---|---|---|---|
| dbv ORF | modules | domains | Amino acids | peptide bonds |
| ORF9 | 1-2 | AT-CATE | HPG, Tyr | 1-2 |
| ORF10 | 3 | CAT | DPG | 2-3 |
| ORF11 | 4-6 | CATE-CATE-CAT | HPG, HPG, Tyr | 3-4, 4-5, 5-6 |
| ORF12 | 7 | CATC*Te | DPG | 6-7 |

The assignment of the specific roles of the *tcp* NRPS genes could be predicted by their genetic localization within the *tcp* cluster. In fact, for all the glycopeptide clusters reported thus far, there is colinearity between the genetic order of the modules and the order in which the corresponding amino acids are incorporated into the polypeptide.

Other ORFs participating indirectly in the synthesis of the heptapeptide precursor of teicoplanin are also found in the *tcp* cluster, namely ORF13, ORF17 and 39 (SEQ ID NOS: 14, 18 and 40. ORFs 13 and 17 (SEQ ID NO: 14 and 18) are very related, and each encodes a short polypeptide of unknown function. Homologs of this gene product are found in many clusters encoding NRPS systems. ORF39 (SEQ ID NO: 40) encodes a type II thioesterase, a protein often encoded by other clusters containing NRPS or polyketide synthase genes. The proposed role for these thioesterases is to enhance the efficiency by which NRPS and PKS systems operate, by removing aberrant intermediates covalently attached to the enzymes (Kotowska et al. 2002). No orthologs of this protein are encoded by the other known glycopeptide clusters (Table 1) .

### 2C. Cross-linking of the aromatic residues in the heptapeptide

Four proteins, encoded by ORFs 18, 19, 20 and 22 (SEQ ID NOS: 19, 20, 21 and 23) are involved in the cross-linking reactions that join together the aromatic residues of the teicoplanin heptapeptide precursors (Table 1). On the basis of the level of identities with the P450 monooxygenases found in other glycopeptide clusters, and on the basis of the roles predicted for the P450 monooxygenases encoded by the genes present in the *bal* cluster (Bischoff et al. 2001), the following predictions can be made. Namely, the product of ORF 22 (SEQ ID NO: 23) is likely to be involved in the cross-linking of the aromatic residues of amino acids 2 and 4; the product of ORF 19 (SEQ ID NO: 20) is likely to be involved in the cross-linking of the aromatic residues of amino acids 4 and 6; and the product of ORF 18 (SEQ ID NO: 19) is likely to be involved in the cross-linking of the aromatic residues of amino acids 5 and 7. An ortholog of ORF 20 (SEQ ID NO: 21) is not present in the *bal, cep* and *com* clusters, but it is found in the *sta* cluster (Table 1). Since the structure of A47934, like that of teicoplanin, contains an extra cross-link between the aromatic residues of amino acids 1 and 3, the product of ORF20 (SEQ ID NO: 21) is likely to be involved in this cross-linking reactions.

### 2D. Formation of b-hydroxytyrosine and chlorination of aromatic residues

Two proteins, encoded by ORF21 and ORF25 (SEQ ID NOS: 22 and 26), are involved in the formation of a β-hydroxyltyrosine residue and in the chlorination of the aromatic residues of amino acids 2 and 6 in the heptapeptide. On the basis of the level of identities with the genes encoding halogenases found in other glycopeptide clusters, and on the basis of the roles predicted for the halogenase gene present in the bal cluster (Puk et al. 2002), the product of ORF 21 (SEQ ID NO: 22) is likely to be involved in the introduction of a chlorine atom into the aromatic residues of both amino acids 2 and 6. The product of ORF25 (SEQ ID NO: 26) is highly related to a family of proteins that contain motifs typical of non-heme iron dioxygenases. One such protein is predicted from the sta cluster (Pootoolal et al. 2002) and is suggested to be involved in the β-hydroxylation of tyrosine. The exact timing of this hydroxylation reaction is not currently known. It could occur before incorporation of amino acid 6 into the heptapeptide, as it happens in the synthesis of balhimycin (Bischoff et al. 2001); it could occur during heptapeptide synthesis, or after completion of the heptapeptide skeleton.

### 2E. Addition and modification of sugars

Five proteins, encoded by *tcp* ORFs 8, 15, 23, 24 and 26 (SEQ ID NOS: 9, 16, 24, 25 and 27) are involved in some of the late steps in teicoplanin biosynthesis. Their predicted roles are as follows.

*tcp* ORF8 (SEQ ID NO: 9) is highly related to proteins encoded by other glycopeptide clusters (Table 1), which have been demonstrated to be involved in the attachment of sugars to the hydroxyl group of the amino acid residue present at position 6 (Solenberg et al. 1997). Specifically, ORF8 (SEQ ID NO: 9) encodes a glycosyltransferase involved in the attachment of the N-acetyl-glucosamine residue to the teicoplanin aglycone. No other glycosyltransferase with such a specificity is encoded by the other described glycopeptide clusters.

Homologs of *tcp* ORF15 (SEQ ID NO: 16) are not found in the other described glycopeptide clusters. This protein contains motifs typical of the family of protein mannosyltransferases (Table 1). Furthermore, homologs of this ORF have been identified in the *S. coelicolor* genome (Table 1), as well as in the *Actinoplanes* spp. cluster specifying the synthesis of the antibiotic ramoplanin (WO 02/31155). Since ramoplanin contains a mannosyl residue attached to the peptide core, all these data point to a role for ORF15 (SEQ ID NO: 16) in attaching the mannosyl residue to the hydroxyl group of amino acid 7. This putative role is also demonstrated in Example 4 below.

*tcp* ORF23 (SEQ ID NO: 24) is highly related to proteins encoded by other glycopeptide clusters (Table 1), which have been demonstrated to be involved in the attachment of sugars to the hydroxyl group of the amino acid residue present at position 4 (Solenberg et al. 1997). Specifically, ORF23 (SEQ ID NO: 24) encodes a glycosyltransferase involved in the attachment of the N-acetyl-glucosamine residue, subsequently transacilated by another protein, to the teicoplanin aglycone. No other glycosyltransferase with such a specificity is encoded by the other described glycopeptide clusters.

Homologs of *tcp* ORFs 24 and 26 (SEQ ID NOS: 25 and 27) are not found in the other described glycopeptide clusters. These proteins are likely to be involved in the attachment of fatty acid chain to teicoplanin precursor (Table 1). Since teicoplanin contains an acyl residue attached to the NH₂ group of the aminosugar residue, the products of these ORFs are likely to be directly or indirectly involved in acylation of the teicoplanin precursor, resulting in the family of compounds that characterize the teicoplanin complex.

### 2F. Export and resistance

Five proteins, encoded by ORFs 1 to 4, 16 and 34 (SEQ ID NOS: 2 to 5, 17 and 35) are involved in exporting teicoplanin or some of its precursor outside the cytoplasm and in conferring resistance to the producing strain. Their predicted roles are as follows.

Homologs of *tcp* ORFs 1 to 4 (SEQ ID NOS: 2 to 5) are found in the *sta* cluster. The roles of these proteins in the mechanism of resistance have been established experimentally (Pootoolal et al., 2002) but are best studied in some glycopeptide-resistant enterococci. It has been established that homologs of ORFs 2 to 4 are involved in the synthesis of the D-alanyl-D-alanine moiety at the termini of the pentapeptide chains in nascent peptidoglycan, thus reducing the extent of glycopeptide binding to its molecular target (Evers et al. 1996). Homologs of *tcp* ORF1 are described as D-alanyl-D-alanine adding enzymes involved in bacterial cell-wall biosynthesis. *tcp* ORFs 1 to 4 (SEQ ID NOS: 2 to 5) are therefore likely to be involved in conferring some level of resistance to teicoplanin in the producing strain *Actinoplanes* ATCC31131.

Homologs of *tcp* ORFs 16 and 34 (SEQ ID NOS: 17 and 35) are present in other glycopeptide clusters (Table 1). They are predicted to encode ABC-type and ion-dependent transmembrane transporters, respectively. They are thus likely to be involved in export or compartimentalization of teicoplanin or some of its precursors.

### 2G. Regulation

Four proteins, encoded by ORFs 6, 7, 28 and 29 (SEQ ID NOS: 7, 8, 29 and 30) are involved in regulating the expression of one or more of the *tcp* genes. ORF6 and 7 (SEQ ID NOS: 7 and 8) encode the two members of a bacterial two-component signal transduction system. The former protein is a likely response regulator (Table 1). The latter protein is a likely transmembrane histidine kinase. ORFs 6 and 7 (SEQ ID NOS: 7 and 8) are therefore likely to be involved in sensing a signal that triggers the expression of one or more genes in the *tcp* cluster Homologs of ORF28 (SEQ ID NO: 29) are present in other glycopeptide clusters (Table 1). Homologs of ORF29 (SEQ ID NO: 30) are not found in the other described glycopeptide clusters. This protein contains motifs typical of positive regulators of the LuxR family, and is mostly related to one positive regulator found in a PKS cluster from *Streptomyces hygroscopicus* (Ruan et al. 1997). ORFs 28 and 29 (SEQ ID NOS: 29 and 30) are therefore likely to be required for the expression of one or more of the *tcp* genes.

### 2H. Additional functions

Two additional ORFs are present in the *tcp* cluster: ORF5 (SEQ ID NO: 6) and ORF35 (SEQ ID NO: 36). The former is related to a protein of unknown function present in A. *orientalis* (Table 1) , which however lies outside the *cep* cluster (van Wageningen et al. 1999). The latter ORF shows considerable sequence identity with type I GTP cyclohydrolases (Table 1). However, its precise role in teicoplanin biosynthesis cannot be predicted yet.

### Example 3 - Isolation of the tcp cluster in an ESAC vector

Using the information provided in Example 2, the *tcp* cluster was isolated in an ESAC vector as follows. DNA from *Actinoplanes teichomyceticus* ATCC31131 was prepared embedded in agarose plugs as described (Sosio et al. 2000b; WO99/67374), and partially digested with *Sau*3AI, in order to optimize fragment sizes in the 100-200 kb range. The resulting DNA fragments were briefly run on a PFGE gel, recovered and released from the agarose gel as described (Sosio et al. 2000b; WO99/67374). The resulting steps, including preparation of the vector pPAC-S1 (Sosio et al. 2000b), ligation and electroporation of *E. coli* DH10B competent cells, were performed as described (Sosio et al. 2000b; WO99/67374). The resulting colonies were arrayed onto nylon filters and screened by hybridization with two probes, PCR-amplified from *Actinoplanes teichomyceticus* ATCC31131 DNA. Probe A was obtained using oligos 5'-TTCCTCGACCGGCGCATCCG-3' (SEQ ID NO: 45) and 5'-GGTGAGCGGCAGGACACTGAG-3' (SEQ ID NO: 46); and probe B with 5'-AGGCAGTTCGTCACGGTGTAG-3' (SEQ ID NO: 47) and 5'-GCCAGCGTGCTCTTCCCGATC-3' (SEQ ID NO: 48). All these sequences were derived from SEQ ID NO: 1. The ESAC clones positive to all these probes were then isolated and physically mapped by digestion with *Eco*RI and *Eco*RV. From one such experiment, the ESAC clone AtES1, containing an insert of about 80 kb, was isolated. AtES1 spans the entire *tcp* cluster (SEQ ID NO: 1) and extends it for about 3 kb 5' to nucleotide 1 of SEQ ID NO: 1, and for about 4 kb 3' to nt 73,883 of SEQ ID NO: 1.

The above example serves to illustrate the principle and methodologies through which the *tcp* cluster can be obtained in an ESAC vector. It will occur to those skilled in the art that the vector pPAC-S1 is just one example of an ESAC vector that can be used for this purpose. Other vectors useful for cloning the entire *tcp* gene cluster and transferring into a suitable actinomycete host have been described (Sosio et al. 2000b; WO99/67374). Furthermore, other methods for preparing a large insert library of *Actinoplanes teichomyceticus* ATCC31131 DNA, including but not limited to partial digestion, fragment separation and recovery, vector preparation, ligation and transformation of *E. coli* cells, also fall within the scope of the present invention. It will also occur to those skilled in the art that, once the boundaries of the *tcp* cluster are established as in SEQ ID NO: 1, any probe or probe combination other than probes A and B as described above, can be used to screen a library made with *Actinoplanes teichomyceticus* ATCC31131 DNA to identify clones whose inserts span the entire *tcp* cluster. Alternatively, with the information provided in SEQ ID NO: 1 and in Table 1, other useful probes can be obtained from other gene clusters that contain genes sufficiently related to the *tcp* genes as to allow heterologous hybridizations. All these variations fall within the scope of the present invention.

### Example 4 - In vitro glycosylation of the teicoplanin aglycone

Using the information provided in Example 2, *tcp* ORF23 was overexpressed in *E. coli* as follows. A 1.2 kb fragment, obtained by amplification with oligos 5'-TTTTTCATATGCGTGTGTTGTTTTCGACGACCGGAG-3' (SEQ ID NO: 49) and 5'-TTTTTGTCGACCGCGGAGACCGACGATCTCTGCCG-3' (SEQ ID NO: 50), was digested with NcoI and SalI and ligated to pET22b, previously digested with *Nco*I and *Xho*I. After transformation of *E. coli* DH5a cells, the resulting plasmid pAtGtfA, recognizable for the presence of fragments of 5.5 kb and 1.2 kb after digestion with *Nde*I and *Xho*I, was introduced into *E. coli* BL21(DE3) cells. Cultures of *E. coli* BL21(DE3) cells harbouring pAtGtfA were grown at 20 °C in LB to an OD₆₀₀ of 0.6. Then, IPTG was added to 1 mM and cells grown for further 6 h. Cells were harvested, ruptured by sonication and the His-tagged ORF23 polypeptide was recovered from a Ni-agarose column as described (Quiagen GmbH, Düsseldorf, Germany). In vitro glycosylation of substrates was carried out as described (Losey et al. 2001), using 6 mM UDP-N-acetylglucosamine, 2 mM teicoplanin aglycone and 100 nM of the ORF23 polypeptide. Formation of the glucosaminyl-derivative of the teicoplanin aglycone was confirmed by comparison with an authentic standard (Malabarba and Ciabatti 2001).

The above example serves to illustrate the principle and methodologies through which an ORF chosen among any of those specified by SEQ ID NOS: 2 to 40 can be overexpressed in a convenient host, the resulting enzyme overproduced and used for transforming a glycopeptide derivative into a different compound. It will occur to those skilled in the art that ORF23 (SEQ ID NO: 24) is just used as an example of the methodologies for overproducing any polypeptide encoded by SEQ ID NO: 1. Those skilled in the art understand also that other methods for overproducing proteins, including but not limited to the use of different affinity tags, the use of different vectors, of different host strains, and of methods of induction, can also be used to overproduce one or more of the polypeptides specified by ORFs 1 to 39 (SEQ ID NOS: 2 to 40).

### REFERENCES

Bate N, Butler AR, Gandecha AR, Cundliffe E (1999) Chem Biol 6: 617-624.
Benning MM, Wesenberg G, Liu R, Taylor KL, Dunaway-Mariano D, Holden HM (1998) J Biol Chem 273: 33572-33579.
Bentley SD, Chater KF, Cerdeno-Tarraga AM, Challis GL, Thomson NR, James KD, Harris DE, Quail MA, Kieser H, Harper D, Bateman A, Brown S, Chandra G, Chen CW, Collins M, Cronin A, Fraser A, Goble A, Hidalgo J, Hornsby T, Howarth S, Huang CH, Kieser T, Larke L, Murphy L, Oliver K, O'Neil S, Rabbinowitsch E, Rajandream MA, Rutherford K, Rutter S, Seeger K, Saunders D, Sharp S, Squares R, Squares S, Taylor K, Warren T, Wietzorrek A, Woodwardm J, Barrell BG, Parkhill J, Hopwood DA (2002) Nature 417: 141-147.
Bierman R, Logan K, O'Brien ET, Seno R, Nagaraja R, Schoner BE (1992) Gene 116: 43-49.
Bischoff D, Pelzer S, Holtzel A, Nicholson GJ, Stockert S, Wohlleben W, Jung G, Sussmuth RD (2001) Angew Chem Int Ed Engl 40: 1693-1696.
Bruheim P, Sletta H, Bibb MJ, White J, Levine DW. (2002) J Ind Microbiol Biotechnol 28:103-11.
Chater KF, Bibb M (1997) in Biotechnology, vol 6, pp. 57-105, VCH, Weinheim, Germany.
(Kleinkauf H, von Dohren H eds), VCH, Weinheim, Germany.
Chen H, Tseng CC, Hubbard BK, Walsh CT (2001) Proc Natl Acad Sci USA 98: 14901-14906.
Chiu HT, Hubbard BK, Shah AN, Eide J, Fredenburg RA, Walsh CT, Khosla C (2001) Proc Natl Acad Sci USA 98: 8548-8553.
Evers S, Quintiliani R Jr, Courvalin P (1996) Microb Drug Resist 2: 219-223.
Heathcote ML, Staunton J, Leadlay PF (2001) Chem Biol 8: 207-220.
Hubbard BK ,Thomas MG, Walsh CT (2000) Chem Biol 7: 931-942.
Hutchinson CR (1998) Curr Opin Microbiol 1(3) :319-29 Katz L, McDaniel R (1999) Med. Res. Rev. 19: 543-58.
Kieser T, Bibb MJ, Buttner MJ, Chater KF, Hopwood DA (2000) Practical *Streptomyces* Genetics, The John Innes Foundation, Norwich, UK.
Kotowska M, Pawlik K, Butler AR, Cundliffe E, Takano E, Kuczek K (2002) Microbiology 148: 1777-1883.
Ioannou PA. Amemiya CT, Garnes J, Kroisel PM, Shizuya H, Chen C, Batzer MA, de Jong PJ (1994) Nat Genet 6: 84-89.
Lancini GC, Cavalleri B (1990) In: Kleinkauf H, von Döhren H (eds), Biochemistry of Peptide Antibiotics pp. 159-178 Walter de Gruyter Berlin, New York.
Lancini GC, Cavalleri B (1997) Glycopeptide antibiotics (Dalbaaheptides) in Biotechnology, vol 7, pp.369-396, VCH, Weinheim, Germany.
Li TL, Choroba OW, Hong H, Williams DH, Spencer JB (2001) Chem Commun 20: 2156-2157.
Losey HC, Peczuh MW, Chen Z, Eggert US, Dong SD, Pelczer I, Kahne D, Walsh CT (2001) Biochemistry 40: 4745-4755.
Malabarba A, Ciabatti R (2001) Curr Med Chem 8: 1759-1773.
Malabarba A. Ciabatti R, Gerli E, Ripamonti F, Ferrari P, Colombo L, Olsufyeva EN, Pavlov AY, Reznikova MI, Lazhko EI, Preobrazhenskaya MN (1997) J Antibiot 50: 70-81.
Marahiel MA (1997) Chem Biol 4: 561-577.
Mendez C, Salas JA. (2001) Trends Biotechnol 19: 449-456.
Omura S, Ikeda H, Ishikawa J, Hanamoto A, Takahashi C, Shinose M, Takahashi Y, Horikawa H, Nakazawa H, Osonoe T, Kikuchi H, Shiba T, Sakaki Y, Hattori M (2001) Proc Natl Acad Sci USA 98: 12215-12220.
Parenti F, Cavalleri B (1989) J Antibiot 42:1882-1883.
Parenti F, Cavalleri B (1990) Drugs of the future 15: 57-72.
Pelzer S, Sussmuth R, Heckmann D, Recktenwald J, Huber P, Jung G, Wohlleben W (1999) Antimicrob Agents Chemother 43: 1565-1573.
Pfeifer V, Nicholson GJ, Ries J, Recktenwald J, Schefer AB, Shawky RM, Schroder J, Wohlleben W, Pelzer S (2001) J Biol Chem 276: 38370-38377.
Pootoolal J, Thomas MG, Marshall CG, Neu JM, Hubbard BK, Walsh CT, Wright GD (2002) Proc Natl Acad Sci USA 99: 8962-8967.
Puk O, Huber P, Bischoff D, Recktenwald J, Jung G, Sussmuth RD, van Pee KH, Wohlleben W, Pelzer S (2002) Chem Biol 9: 225-235.
Rodriguez L, Aguirrezabalaga I, Allende N, Brana AF, Mendez C, Salas JA (2002) Chem Biol. 9:721-729.
Ruan X, Stassi D, Lax SA, Katz L (1997) Gene 203: 1-9.
Sambrook J, Russel D. (2001) Molecular Cloning: A laboratory Manual (Cold Spring Harbor Lab. Press, Cold Spring Harbor NY).
Shizuya H, Birren B, Kim UJ, Mancino V, Slepak T, Tachiri Y, Simon M (1992) Proc Natl Acad Sci USA 89: 8794-8797.
Solenberg PJ, Matsushima P, Stack DR, Wilkie SC, Thompson RC, Baltz RH (1997) Chem Biol 4:195-202.
Sosio M, Bossi E, Bianchi A, Donadio S (2000a) Mol Gen Genet 264: 213-221.
Sosio M, Giusino F, Cappellano C, Bossi E, Puglia AM, Donadio S (2000b) Nat Biotechnol 18: 343-345.
Steiert M, Schmitz FJ (2002) Curr Opin Investig Drugs 3: 229-233.
Sosio M, Bianchi A, Bossi E, Donadio S (2000c) Antonie Van Leeuwenhoek 78: 379-84.
van Wageningen AM, Kirkpatrick PN, Williams DH, Harris BR, Kershaw JK, Lennard NJ, Jones M, Jones SJ, Solenberg PJ (1998) Chem Biol 5: 155-162.
van Veen HW, Konings WN (1998) Biochim Biophys Acta 1365: 31-36.
Xue Q, Ashley G, Hutchinson CR, Santi DV (1999) Proc Natl Acad Sci USA 96: 11740-11745.
Zhang Z, Wang Y. Ruan J (1998) Int J Syst Bacteriol 48:411-422.

## Claims

**1.** An isolated nucleic acid comprising a nucleotide sequence selected from the group consisting of:
a) the *tcp* gene cluster encoding the polypeptides required for the synthesis of teicoplanin (SEQ ID NO: 1);
b)a nucleotide sequence encoding the same polypeptides encoded by the *tcp* gene cluster (SEQ ID NO: 1), other than the nucleotide sequence of the *tcp* gene cluster;
c) any nucleotide sequence of *tcp* ORFs 1 to 39, encoding the polypeptides of SEQ ID NOS: 2 to 40; and
d) a nucleotide sequence encoding the same polypeptides encoded by any of *tcp* ORFs 1 to 39 (SEQ ID NOS: 2 to 40), other than the nucleotide sequence of said ORF.

**2.** An isolated nucleic acid of claim 1 comprising a nucleotide sequence selectd from the group consisting of:
e) a nucleotide sequence of any of the *tcp* ORFs 1 to 5, 9 to 14, 16 to 22, 25, 30 to 34 and 36 to 38 encoding the polypeptides specified in SEQ D NOS: 2 to 6, 10 to 15, 17 to 23, 26, 31 to 35 and 37 to 39;
f)a nucleotide sequence encoding the same polypeptide encoded by any of *tcp* of OFRs 1 to 5, 9 to 14, 16 to 22, 25, 30 to 34 and 36 to 38 encoding the polypeptides specified in SEQ ID NOS: 2 to 6, 10 to 15, 17 to 23, 26, 31 to 35 and 37 to 39 other than the nucleotide sequence of said *tcp* ORFs;
g)a nucleotide sequence of any of *tcp* ORFs 6 to 8, 15, 23 to 24, 26 to 29, 35 and 39 encoding the polypeptides specified in SEQ ID NOS: 7 to 9, 16, 24 to 25, 27 to 30, 36 and 40;
h)a nucleotide sequence encoding the same polypeptide encoded by any of *tcp* ORFs 6 to 8, 15, 23 to 24, 26 to 29, 35 and 39 encoding the polypeptides specified in SEQ ID NOS: 7 to 9, 16, 24 to 25, 27 to 30, 36 and 40 other than the nucleotide sequence of said *tcp* ORF;
i)a nucleotide sequence encoding a polypeptide that is at least 80%, preferably 86%, more preferably 90%, most preferably 95% or more, identical in amino acid sequence to a polypeptide encoded by any of *tcp* ORFs 8, 15, 23 to 24, 26 to 27, 29, 35 and 39 (SEQ ID NOS: 9, 16, 24 to 25, 27 to 28, 30, 36 and 40); and
j)a nucleotide sequence encoding a polypeptide that is at least 87%, preferably 90%; more preferably 95% or more, identical in amino acid sequence to the polypeptide encoded by *tcp* ORF 28 (SEQ ID NO: 29).

**3.** An isolated nucleic acid according to claim 2 comprising a combination of nucleotide sequences which encode polypeptides required for the synthesis of the 4-hydroxy-phenylglycine residues of teicoplanin consisting of *tcp* ORFs 36 to 38 (SEQ ID NOS: 37 to 39), or nucleotide sequences encoding the same polypeptides, other than the nucleotide sequences of said ORFs.

**4.** An isolated nucleic acid according to claim 2 comprising a combination of nucleotide sequences which encode polypeptides required for the synthesis of the 3,5-dihydroxy-phenylglycine residues of teicoplanin consisting of *tcp* ORFs 30 to 33, and 36 (SEQ ID NOS: 31 to 34, and 37), or nucleotide sequences encoding the same polypeptides, other than the nucleotide sequences of said ORFs.

**5.** An isolated nucleic acid according to claim 2 comprising a combination of nucleotide sequences which encode polypeptides required for the synthesis of the heptapeptide skeleton of teicoplanin consisting of *tcp* ORFs 9 to 12 (SEQ ID NOS: 10 to 13), or nucleotide sequences encoding the same polypeptides, other than the nucleotide sequences of said ORFs.

**6.** An isolated nucleic acid according to claim 2 comprising a nucleotide sequence which encodes a polypeptide required for the chlorination of the aromatic residues of amino acids 2 and 6 of teicoplanin consisting of *tcp* ORF 21 (SEQ ID NO: 22), or nucleotide sequences encoding the same polypeptide, other than the nucleotide sequence of said ORF.

**7.** An isolated nucleic acid according to claim 2 comprising a nucleotide sequence which encodes a polypeptide required for the ß-hydroxylation of the tyrosine residue of amino acid 6 of teicoplanin consisting of *tcp* ORF 25 (SEQ ID NO: 26), or nucleotide sequences encoding the same polypeptide, other than the nucleotide sequence of said ORF.

**8.** An isolated nucleic acid according to claim 2 comprising a combination of nucleotide sequences which encode polypeptides required for the cross-linking of the aromatic residues of amino acids at positions 2 and 4, 4 and 6, 1 and 3, and 5 and 7 of teicoplanin consisting of *tcp* ORFs 18 to 20, and 22 (SEQ ID NOS: 19 to 21 and 23), or nucleotide sequences encoding the same polypeptides, other than the nucleotide sequences of said ORFs.

**9.** An isolated nucleic acid according to claim 2 comprising a combination of nucleotide sequences which encode polypeptides required for the addition and formation of the two N-acyl-β-D-glucosamine residues to the core structure of teicoplanin consisting of ORFs 8, 23 to 24 and 26 (SEQ ID NOS: 9, 24 to 25 and 27), or nucleotide sequences encoding the same polypeptides, other than the nucleotide sequences of said ORFs.

**10.** An isolated nucleic acid according to claim 2 comprising a nucleotide sequence which encodes a polypeptide required for the attachment of the mannosyl residue of teicoplanin consisting of *tcp* ORF 15 (SEQ ID NO: 16), or nucleotide sequences encoding the same polypeptide, other than the nucleotide sequence of said ORF.

**11.** An isolated nucleic acid according to claim 2 comprising a combination of nucleotide sequences which encode polypeptides required for export of teicoplanin or some of its precursors outside of the cytoplasm and for conferring resistance to teicoplanin to the producing strain consisting of *tcp* ORFs 1 to 6, 16 and 34 (SEQ ID NOS: 2 to 6, 17 and 35), or nucleotide sequences encoding the same polypeptides, other than the nucleotide sequences of said ORFs.

**12.** An isolated nucleic acid according to claim 2 comprising a combination of nucleotide sequences which encode polypeptides required for regulating the expression of one or more genes of the *tcp* gene cluster consisting of *tcp* ORFs 6 to 7, 28 to 29 (SEQ ID NOS: 7 to 8, 29 to 30), or nucleotide sequences encoding the same polypeptides, other than the nucleotide sequences of said ORFs.

**13.** An isolated nucleic acid according to claim 1 comprising a nucleotide sequence consisting of the *tcp* gene cluster encoding the polypeptides required for the synthesis of a teicoplanin wherein an in frame deletion has been introduced in the nucleotide sequence encoding the polypeptides required for the attachment of the mannosyl residue.

**14.** An isolated nucleic acid sequence according to claim 1 comprising a nucleotide sequence carrying at least one extra-copy of at least one of the *tcp* ORFs 1 to 39 (SEQ ID NOS: 2 to 40) or of a nucleotide sequence encoding the same polypeptides encoded by said *tcp* ORF(s), other than the nucleotide sequence of said *tcp* ORF(s).

**15.** An isolated nucleic acid of any of claims 1 to 14 wherein the nucleotide sequence is a DNA sequence.

**16.** A recombinant DNA vector which comprises a DNA sequence as defined in any of claims 1 to 14.

**17.** A recombinant vector according to claim 16 which is an ESAC vector.

**18.** A host cell transformed with a vector of any of claims 16 or 17.

**19.** A transformed host cell according to claim 18 which belongs to the order *Actinomycetales,* preferably to the family *Micromonosporaceae, Streptosporangiaceae, Pseudonocardiaceae* or *Streptomycetaceae,* more preferably to the genera *Actinoplanes, Nonomureae, Amycolatopsis, Streptomyces* or the like.

**20.** A method for increasing production of teicoplanin by a microorganism capable of producing teicoplanin or a precursor thereof by means of a biosynthetic pathway, said method comprising:
a)transforming with a recombinant DNA vector of claim 16 a microorganism that produces teicoplanin or a teicoplanin precursor by means of a biosynthetic pathway, wherein said DNA vector codes for the expression of an activity that is rate limiting in said pathway; and
b)culturing said microorganism transformed with said vector under conditions suitable for cell growth, expression of said gene and production of said antibiotic or antibiotic presursor.

**21.** A method for producing a derivative of teicoplanin or a precursor thereof which comprises
(a) cloning in a suitable vector a segment chosen from the nucleotide sequence defined by SEQ ID NO: 1, said segment containing at least a portion of one of ORFs 1 through 39 (SEQ ID NO: 2 through 40), said ORF encoding a polypeptide that catalyzes a biosynthetic step that one wishes to bypass;
(b) inactivating said ORF or portion therof by removing or replacing one or more codons that specify for amino acids that are essential for the activity of said polypeptide;
(c) transforming with said recombinant DNA vector a microorganism that produces teicoplanin or a teicoplanin precursor by means of a biosynthetic pathway;
(d) screening the resulting transformants for those where said DNA sequence has been replaced by the mutated copy; and
(e) culturing said mutant cells under conditions suitable for cell growth, expression of said pathway and production of said pathway analogue.

**22.** A transformed microorganism producing teicoplanin or a precursor or a derivative thereof, wherein the teicoplanin biosynthetic genes in its genome have been modified by insertion of a nucleotide sequence according to claim 14.

**23.** A process for producing teicoplanin or a precursor or a derivative thereof which comprises cultivating a transformed teicoplanin-producing microorganism of claim 22.

**24.** A transformed teicoplanin-producing microorganism having teicoplanin biosynthetic genes in its genome wherein at least one of the teicoplanin biosynthetic genes, selected from *tcp* ORFs 1 to 39 (SEQ ID NOS: 2 to 40), is inactivated according to the process of claim 21.

**25.** A transformed microorganism according to claim 24, wherein the biosynthetic gene which is inactivated is the gene involved in the attachment of the mannosyl residue.

**26.** A method for producing novel glycopeptides, said method comprising the following steps:
a)transforming with a recombinant DNA vector a microorganism that produces a glycopeptide or a glycopeptide precursor different from teicoplanin or a precursor thereof by means of a biosynthetic pathway, said vector or portion thereof comprising a nucleotide sequence of any of claim 2 to 12, that codes for the expression of an enzymatic activity that modifies said glycopeptide or glycopeptide precursor; and
b)culturing said microorganism transformed with said vector under conditions suitable for cell growth, expression of said gene and production of said antibiotic or antibiotic precursor; or
a)transforming with a recombinant DNA vector a microorganism, said vector comprising one or more ORFs, chosen among ORFs 1 through 39 (SEQ ID NOS: 2 through 40), that codes for one or more polypeptides that modify a glycopeptides or glycopeptide precursor, and said microorganism being selected among those that do not produce glycopeptide or glycopeptide precursors and that can efficiently express the introduced ORF(s);
b)preparing a cell extract or cell fraction of said microorganism under conditions suitable for the presence of active polypeptide(s), said cell extract or cell fraction containing at least said polypeptide(s); and
c)adding a glycopeptide or glycopeptide precursor to said cell extract or cell fraction, and incubating said mixture under conditions where said polypeptide(s) can modify said glycopeptide or glycopeptide precursor.

**27.** An isolated polypeptide involved in the biosynthetic pathway of teicoplanin selected from:
a) an ORF polypeptide encoded by any one of *tcp* ORFs 1 to 39 (SEQ ID NOS: 2 through 40);
b)a polypeptide which is at least 90%, preferably 95% or more, identical in amino acid sequence to a polypeptide encoded by any one of *tcp* ORFs 1 to 5, 9 to 14, 16 to 22, 25, 30 to 34 and 36 to 38 (SEQ ID NOS: 2 to 6, 10 to 15, 17 to 23, 26, 31 to 35 and 37 to 39);
c)a polypeptide which is at least 80% preferably 86%, more preferably 90%, most preferably 95% or more, identical in amino acid sequence to a polypeptide encoded by any of *tcp* ORFs 6 to 8, 15, 23 to 24, 26 to 27, 29, 35 and 39 (SEQ ID NOS: 7 to 9, 16, 24 to 25, 27 to 28, 30, 36 and 40), and .
d)a polypeptide which is at least 87%, preferably 90%, more preferably 95% or more, identical in amino acid sequence to a polypeptide encoded by ORF 28 (SEQ ID NO: 29) .

**30.** An isolated polypeptide comprising a polypeptide involved in the biosynthetic pathway of teicoplanin selected from the polypeptides encoded by any of the nucleic acids of any of claims 3 to 14.
